# EUROPEAN PATENT APPLICATION

(11) **EP 2 151 495 A1**
(43) Date of publication of application: **10.02.2010**
(21) Application number: 08752225.6
(22) Date of filing: 28.04.2008
(51) Int. Cl.: C12N 15/00, C07K 14/18, C12N 5/10, C12N 7/00, C12Q 1/02, C12Q 1/68

(54) **HCV GENE**

(30) Priority: 27.04.2007 JP 2007119667
(71) Applicant: Advanced Life Science Institute, Inc., Wako-shi, Saitama 351-0112 (JP)
(72) Inventor: MORI, Kenichi, Wako-shi Saitama 351-0112 (JP); MAKI, Noboru, Wako-shi Saitama 351-0112 (JP); FUKAI, Hiromi, Wako-shi Saitama 351-0112 (JP); OHUE, Chiharu, Wako-shi Saitama 351-0112 (JP)
(74) Representative: Sampson, Catherine
(86) International application number: PCT/JP2008/058215
(87) International publication number: WO 2008/136470

(57) **Abstract**

The present invention provides a hepatitis C virus gene, a replicon RNA derived from the gene, a replicon-replicating cell into which the replicon RNA is introduced, and a method for screening a drug using the replicon-replicating cell. By introducing a replicon RNA comprising (A) a polynucleotide having the nucleic acid sequence shown in SEQ ID NO: 5; (B) a polynucleotide having the nucleic acid sequence shown in SEQ ID NO: 7; (C) a polynucleotide coding for a polypeptide having the amino acid sequence shown in SEQ ID NO: 6; (D) a polynucleotide coding for a polypeptide having the amino acid sequence shown in SEQ ID NO: 8; or (E) a polynucleotide having a nucleic acid sequence having a homology of not less than 90% with the nucleic acid sequence shown in SEQ ID NO: 5 or SEQ ID NO: 7; or a replicon RNA of the genotype 1b comprising nucleotides coding for the 1804th amino acid leucine and the 1966th amino acid lysine in the amino acid sequence of a hepatitis C virus polyprotein and a polynucleotide coding for an NS4B protein, into a cell, the replicon-replicating cell can be prepared. By using the replicon-replicating cell, the screening for the drug can be carried out.

SEQUENCE LISTING

<110>  Advanced Life Science Institute, Inc.

<120>  HCV genome

<130>  CPS/FP6666879

<140>  08752225.6
<141>  2008-04-28

<150>  PCT/JP2008/058215
<151>  2008-04-28

<150>  2007-119667
<151>  2007-04-27

<160>  68

<170>  PatentIn version 3.1

<210>  1
<211>  9594
<212>  RNA
<213>  Hepatitis C virus

<400>  1
gccagccccc ugaugggggc gacacuccac cauagaucac uccccuguga ggaacuacug      60

ucuucacgca gaaagcgucu agccauggcg uuaguaugag ugucgugcag ccuccaggac     120

ccccccuccc gggagagcca uaguggucug cggaaccggu gaguacaccg gaauugccag     180

gacgaccggg uccuuucuug gaucaacccg cucaaugccu ggagauuugg gcgugccccc     240

gcgagacugc uagccgagua guguuggguc gcgaaaggcc uugugguacu gccugauagg     300

gugcuugcga gugccccggg aggucucgua gaccgugcau caugagcaca aauccuaaac     360

cucaaagaaa aaccaaacgu aacaccaacc gccgcccaca ggacgucaag uucccgggcg     420

guggccagau cguuggugga guuuaccugu ugccgcgcag gggccccagg uugggugugc     480

gcgcgacuag gaagacuucc gagcggucgc aaccucgugg aaggcgacaa ccuaucccca     540

aggcucgcca gcccgagggc agggccuggg cucagcccgg guauccuugg ccccucuaug     600

gcaacgaggg ucuggggugg gcaggauggc uccugucacc ccguggcucu cggccuaguu     660

ggggccccac ggacccccgg cguaggucgc guaauuuggg uaaggucauc gauacccuca     720

caugcggcuu cgccgaccuc augggguaca uuccgcucgu cggcgccccc cuaggaggcg     780

cugccagggc ccuggcgcau ggcguccggg uucuggagga cggcgugaac uaugcaacag     840

ggaaucugcc cgguugcccu uucucuaucu uccucuuagc uuugcugucc uguuugacca     900

ucccagcuuc cgcucacgaa gugcgcaacg uauccgggcu guaccauguc acgaacgacu     960

gcuccaacuc aagcauugug uaugaggcag cggacaugau caugcacacc cccgggugcg    1020

ugcccugcgu ccgggagggu aacuccuccc gcugcugggu agcgcucacu cccacgcucg 1080

cggccaggaa uagcagcguc cccacugcga caauacgacg ccaugucgau uugcucgucg 1140

gggcggcugc uuucuguucc gcuauguacg ugggggaucu uugcggaucu guuuuccucg 1200

ucucccagcu guucaccuuu ucaccucgcc gguacgagac gguacaggac ugcaauugcu 1260

cacucuaucc cggccacgua ucaggccauc gcauggcuug ggauaugaug augaacuggu 1320

caccuacaac agccuuagug guaucgcagu uacuccggau cccacaagcc gucguggaua 1380

uggugguagg ggcccacugg ggaguccugg cgggccuugc cuacuauucc auggugggga 1440

acugggcuaa ggucuugauu gugaugcuac ucuuugccgg cgucgacggg aagaccuacg 1500

ugacaggggg ggcgcagagc cgagccacuc aaggcuuugc gucccucuuu acacgggggc 1560

cgucucagaa acuccagcuu guaaauucca acggcagcug gcacauuaac aggacugccu 1620

ugaacugcaa ugacuccuuc cagacugggu uccuugccgc gcuguuuuac gcacaccguu 1680

ucaacucguc cggaugccca gagcgcaugg ccagcugccg ccccaucgac acguucgauc 1740

aggggugggg ccccaucacu caugucgcgc gucgcacauc ggaccagagg ccuuauugcu 1800

ggcacuacgc accucaaccg ugugguauug uacccgcguu gcagguaugu gguccagugu 1860

auugcuucac cccaagcccc gucguggugg ggacgaccga ucgcuucggc gcccccacgu 1920

acaacugggg ggagaaugag acggacgugc uacuccucaa caauacgcgg ccgccgcacg 1980

gcaacugguu cggcuguaca uggaugaaua guaccggguu caccaagacg ugugggggcc 2040

cccccugcaa caucgggggg uuuggcaaca acaccuugac cugcccuacg gauugcuucc 2100

ggaagcaccc cgaggccacu uacaccaaau gcggcucggg gcccugguug acgccuaggu 2160

gcaugguuga uuacccauac agacuuuggc acuaccccug cacuguuaac uuuuccaucu 2220

ucaaggucag gauguaugug gggggugugg agcacaggcu caccgccgcg ugcaauugga 2280

cucggggaga gcgcugcaac uuggaggaua gggacagauc ggagcucagc ccgcugcuac 2340

ugucuaccac agaguggcag guacugcccu guucuuucac caccuuaccg gcccugucca 2400

cugguuugau ccaccuccac cagaacaucg uggacgugca auaccuguac gguguggggu 2460

caucgguugu cuccauugca aucagguggg aguaugucgu gcugcucuuc cuccuccugg 2520

cggacgcgcg yguuugcgcc ugcuugugga ugaugcugcu gauagcccaa gcugaggccg 2580

ccuuagagaa ccuggugauc cucaaugcgg cgucuguggc cggagcgcau ggcguucucu 2640

cuuuccuugu guucuucugc gcugccuggu acaucaaggg caagcugguc cccggggcgg 2700

cauaugccuu cuauggugua uggccgcugc uccugcuucu gcugucauua ccaccacgag 2760

cauacgccuu ggaccgggag auggcugcau cgugcggagg cgcgguuuuc guaggucuga 2820

ugcuccugac cuugucacca cacuacaagg uguuucucgc uaggcucaua uggugguuac 2880

aguauuuuau caccagggcc gaggcgcacu ugcaggugug gguccccccc cucaacguuc 2940
gggggggccg cgaugccauc auccuccuca cguguguggu ccacccagag cuaauuuuug 3000
acaucaccaa aaucuugcuc gccaugcucg guccgcucau ggugcuccag gcuggccuaa 3060
cuagagugcc guacuucgua cgcgcucaag ggcucauccg ugcaugcaug uuagugcgga 3120
aagucgcugg gggccacuau guccaaaugg cccucaugaa acuggccgca cugacgggua 3180
cguacguuua ugaccaucuu acuccgcugc aggacugggc ccacgcgggc uugcgagacc 3240
uugcaguggc aguugagccc gucgucuucu cugacaugga gacuaagguc aucaccuggg 3300
gggcagacac cgcagcgugu ggggacauca ucucgggccu acccgucucc gcccgaaggg 3360
ggagggagau acuucugggc cccgccgaca gguuuggaga gcaggggugg cgacuccucg 3420
cgccuaucac ggcuuacgcu caacagacgc ggggccuacu uggcuguauc aucaccagcc 3480
ucacaggccg ggacaagaac caggucgagg gggagguuca gguggguucc accgcaacgc 3540
aaucuuuccu ggcgaccugc gucaacggcg uguguuggac ugucuaccau ggugccggcu 3600
cgaagacccu ggccggcccg aagggcccaa ucacccaaau guacaccaau guggaccaag 3660
accucgucgg cuggccggcg cccccggggg cgcgcucccu gacaccgugc accugcggca 3720
gcucggaccu cuaccugguc acgaggcaug cugaugucau uccggugcgc cggcggggcg 3780
acagcagggg gagucuacuc ucucccaggc ccaucuccua cuuaaagggc uccucaggug 3840
guccacugcu uugcccccug gggcacgcug ugggcaucuu ccgggccgcu gugugcaccc 3900
gggggguugc aaaggcggug gauuuuguac cuguugaguc uauggaaacc accaugcggu 3960
cuccggucuu uacggauaau ucaucucccc cggccguacc gcagacauuc caaguggccc 4020
aucuacacgc ucccacuggc aguggcaaga gcacuaaggu gccggcugcg uacgcagccc 4080
aaggguacaa gguacucguc uugaacccau ccguugccgc uaccuuaggg uuuggggcgu 4140
acaugucuaa agcacauggu guugagccua acaucagaac ugggguaagg accaucacca 4200
cgggcgcuuc caucacguau uccaccuacg guaaguuccu ugccgacggu gguugcucug 4260
ggggcgccua ugacaucaua auaugugaug agugccacuc aacugacucg acuuccaucu 4320
ugggcauugg cacaguccug gaccaagcgg agacggcugg agcgcggcuc gucgugcucg 4380
ccaccgcuac gccuccggga ucggucaccg ugccacaucc caauaucgag gagguggccu 4440
ugcccagcac cggagaaauu cccuucuacg gcaaagccau ccccauugag accaucaagg 4500
gggggaggca ccucaucuuc ugccacucca agaagaaaug ugacgagcuc gcugcaaagc 4560
uggugggccu cggaguuaac gcuguugcgu acuaccgggg ucuugaugug uccgucauac 4620
caacaagcgg agaugucguu gucguggcaa cagacgcucu aaugacgggc uucaccggcg 4680

acuuugacuc agugaucgac uguaauacuu gugucaccca gacaguugau uucagcuugg 4740

acccuaccuu caccauugag acgacaaccg ugccccaaga cgcggugucg cguucgcagc 4800

gacgaggcag gacuggcagg ggcaggaugg gcauauacag guuuguggcu ccaggggaac 4860

ggcccucggg cauguucgau ucuucggucc ugugugagug cuaugacgcg ggcugugcuu 4920

gguaugagcu cacgcccgcc gagaccucag ucagguugcg ggcuuaccua aauacaccag 4980

ggcugcccgu cugccaggac caccuggagu uuugggaggg ggucuucaca ggccucaccc 5040

acauagaugc ccauuucuug ucccagacua agcaggcagg agauaacuuc cccuaccugg 5100

uagcauacca ggcuacggug ugcgccaggg cccaggcucc cccuccaucg ugggaucaaa 5160

uguggaagug ucucauacgg cugaagccua cacuacacgg gccaacgccc cuguuguaua 5220

ggcuaggagc cguccagaau gaggucaucc ucacacaucc cauaaccaaa uacaucaugg 5280

caugcauguc ggcugaccua gaggucguca cuagcaccug ggugcugguc ggcggggucc 5340

uugcagcucu ggccgcguac ugccugacga cgggcagcgu ggucauugug ggcaggauca 5400

ucuuguccgg gaagccggcu aucauuccug acagggaagu ccucuaccgg gaguucgaug 5460

aaauggaaga gugugccuca caccuccccu acaucgaaca gggaaugcag cucgccgaac 5520

aauucaagca gaaggcgcuc ggguugcugc agacagccac caagcaagcg gaagccgcug 5580

cuccuguggu ggaguccaag uggcgagccc uugaggccuu cugggcgaag cacaugugga 5640

auuucaucag cgggauacag uacuuagcag gcuuguccac ucugccuggg aaccccgcga 5700

uagcaucacu gauggcauuc acagccucua ucaccagccc gcuuaccacc caacacaccc 5760

uccuguuuaa caucuuggga ggaugggugg ccgcccaacu ugcccccccc ggugcugccu 5820

cggcuuucgu gggcgccggc auugcuggcg cagcuguugg cagcauaggc cuugggaagg 5880

ugcuugugga cauccuggcg gguuauggag cagggguggc aggcgcgcuc guggccuuca 5940

aggucaugag cggcgagaug cccuccaccg aggaccuggu caacuuacuc ccugccaucc 6000

ucucuccugg ugcccuuguc gucggggucg ugugcgcagc aauacugcgu cggcaugugg 6060

gcccggggga gggggcugug caauggauga accggcugau agcguucgcc ucgcggggua 6120

accacgucuc ccccacgcac uaugugccug agagcgacgc ugcagcgcgu gucacacaga 6180

uccucucuag ccucaccauc acucagcuac ugaagaggcu ccaccagugg auuaaugagg 6240

acugcuccac accaugcucc ggcucguggc uuagggacgu uugggacugg auaugcacgg 6300

uuuugaguga cuucaagacc uggcuccagu ccaagcuccu gccacgguua ccgggaguuc 6360

cauuccuuuc augccaacgu ggguauaagg gggucuggcg gggagauggc aucaugcaga 6420

ccuccugccc auguggagca caaaucgccg gacaugucaa gaacgguucc augaggaucg 6480

uugggccuaa aaccuguagc aacacguggc acggaacauu ccccauuaac gcgcacacca 6540

cgggccccug cacacccucc ccagcgccga acuacucuaa ggcguugugg cggguggcug 6600

cugaggagua cguggaaguc acgcgggugg gggauuucca uuacgugacg ggcaugacca 6660

cugacaacgu aaaaugccca ugccagguuc cggcccccga guucuucaca gagguggaug 6720

ggguacggcu gcacagguac gcuccggcgu gcaaaccucu ccuacgggau gaggucacau 6780

uccaggucgg gcucaaccag uucccgguug ggucacagcu cccaugcgag cccgaaccgg 6840

auguaucagu gcucacuucc augcuuaccg acccuuccca caucacagca gagacggcua 6900

agcguaggcu ggccagaggg ucuucccccu cuuuggccag cucuucagcu agucaguugu 6960

cugcgcccuc auugaaggcg acaugcacca cccaucauga cuccccagac gcugaccuca 7020

uugaggccaa ccuccugugg cggcaggaga ugggagggaa caucacccgu guggagucag 7080

agaacaaggu gguaauccug gacucuuuug acccgcuucg agcggaggag gacgagaggg 7140

aggugucugu ugcggcggag auccugcgga aaaccaggaa guucccccca gcgaugccca 7200

uaugggcacg cccggacuac aacccaccgc ugcuagagac uuggaaggac ccggacuacg 7260

ucccuccagu ggugcacggg ugcccauugc caccuaccaa gaccccucca auaccaccuc 7320

cgcggaggaa aaagacaguu guccugacag aguccaccgu gucuucugcc cuggcggagc 7380

uugccacaaa gaccuuuggc agcuccggau cgucggccgu cgacagcggc acagcgaccg 7440

cccccccuaa ccagcucucc gacgaagugg auacaggauc cgacguugag ucguacuccu 7500

ccaugccccc ccuugaggga gagccggggg accccgaucu cagcgacggg ucuuggucua 7560

cuguaaguga ggaggcuggu gaggacgucg ucugcugcuc gauguccuac acauggacag 7620

gcgccuugau cacgccgugc gccgcggagg agagcaagcu gcccaucaau gcgcugagca 7680

acucuuugcu gcgccaccac aacauggucu augccacaac aucccgcagc gcaagccaac 7740

ggcagaaaaa ggucaccuuu gacagacugc aaguccugga cgaccauuac cgggacgugc 7800

ucaaggagau gaaggcgaag gcguccacag uuaaggcuaa acuucuaucc guagaagagg 7860

ccugcaagcu gacgccccca cacucagcca gguccaaauu uggcuauggg gcgaaggacg 7920

uccggaaccu auccagcaag gccguuaacc acaucaacuc cguguggaag gacuugcugg 7980

aagacacuga gacaccaauu gacaccacca ucauggcaaa aaaugagguc uucuguguuc 8040

aaccagagaa gggaggccgc aagccagcuc gccuuaucgu auacccagac uugggggugc 8100

gugugugcga gaaaauggcc cuuuacgacg uggucuccac ucuuccucag gccgugaugg 8160

gcuccucaua cggauuccag uacucuccug ggcagcgggu cgaguuccug gugaaugccu 8220

ggaaaucaaa gaagaacccu augggcuucg cauaugacac ccgcuguuuu gacucaacgg 8280

ucaccgagaa cgacauccgu guugaggagu caauuuacca auguugugac uuggcccccg 8340

aggccagaca ggugauaagg ucgcucacag agcggcuuua ugucgggggc ccccugacua 8400

auucaaaagg gcagaacugc gguuaucgcc ggugccgcgc cagcggcgug cugacgacua 8460

gcugcgguaa uacccucaca uguuacuuga aggccucugc agccugucga gcugcaaagc 8520

uccaggacug cacgaugcuc gugugcgggg acgaccuugu cguuaucugu gaaagcgcgg 8580

ggacccagga ggacgcggcg agccuacgag ucuucacgga ggcuaugacu agguacuccg 8640

ccccccccgg ggacccgccc cgaccggaau acgacuugga guugauaaca ucaugcuccu 8700

ccaacguguc ggucgcgcac gaugcaucug gcaaacgggu guauuaccuc acccgugacc 8760

ccaccacccc ccuugcgcgg gcugcguggg agacagcuaa acacacucca gucaacuccu 8820

ggcuaggcaa caucaucaug uaugcgccca cccucugggc aaggaugauu cugaugacuc 8880

acuucuucuc cauccuucua gcucaggagc agcuugaaaa agcccuggau ugucagaucu 8940

acggggccac uuacuccauu gaaccacuug accuaccuca gaucauucaa cgacuccaug 9000

gucuuagcgc auucucacuc cauaguuacu cuccagguga aaucaauagg guggcuucau 9060

gccucaggaa acuuggggua ccgcccuugc gagucuggag acaucgggcc agaagugucc 9120

gcgcuaagcu acugucccag gggggagggg cugccacuug uggcaaguac cucuucaacu 9180

gggcaguaag gaccaagcuc aaacucacuc caaucccggc ugcgucccag uuggacuugu 9240

ccggcugguu cauugcuggu uacagcgggg gagacauaua ucacagccug ucucgugccc 9300

gaccccgcug guuuauguug ugccuacucc uacuuucugu ggggguaggc aucuaccugc 9360

uccccaaucg augaacgggg ggcuaaacac uccaggccaa uaggccauuc uguuuuuuuu 9420

uuuuuuuuuu uuuuuuuuuu uuuuuuuuuu uuuuuuuuuu uuuuuuccuu uuuuuuuuuu 9480

uuuuucccuu ucuuuuggug gcuccaucuu agcccuaguc acggcuagcu gugaaagguc 9540

cgugagccgc augacugcag agagugcuga uacuggccuc ucugcagauc augu 9594

<210> 2
<211> 3010
<212> PRT
<213> Hepatitis C virus

<400> 2

Met Ser Thr Asn Pro Lys Pro Gln Arg Lys Thr Lys Arg Asn Thr Asn
1               5                   10                  15

Arg Arg Pro Gln Asp Val Lys Phe Pro Gly Gly Gly Gln Ile Val Gly
            20                  25                  30

Gly Val Tyr Leu Leu Pro Arg Arg Gly Pro Arg Leu Gly Val Arg Ala
        35                  40                  45

Thr Arg Lys Thr Ser Glu Arg Ser Gln Pro Arg Gly Arg Arg Gln Pro
    50                  55                  60

Ile Pro Lys Ala Arg Gln Pro Glu Gly Arg Ala Trp Ala Gln Pro Gly
65                  70                  75                  80

Tyr Pro Trp Pro Leu Tyr Gly Asn Glu Gly Leu Gly Trp Ala Gly Trp
                85                  90                  95

Leu Leu Ser Pro Arg Gly Ser Arg Pro Ser Trp Gly Pro Thr Asp Pro
            100                 105                 110

Arg Arg Arg Ser Arg Asn Leu Gly Lys Val Ile Asp Thr Leu Thr Cys
        115                 120                 125

Gly Phe Ala Asp Leu Met Gly Tyr Ile Pro Leu Val Gly Ala Pro Leu
    130                 135                 140

Gly Gly Ala Ala Arg Ala Leu Ala His Gly Val Arg Val Leu Glu Asp
145                 150                 155                 160

Gly Val Asn Tyr Ala Thr Gly Asn Leu Pro Gly Cys Pro Phe Ser Ile
                165                 170                 175

Phe Leu Leu Ala Leu Leu Ser Cys Leu Thr Ile Pro Ala Ser Ala His
            180                 185                 190

Glu Val Arg Asn Val Ser Gly Leu Tyr His Val Thr Asn Asp Cys Ser
        195                 200                 205

Asn Ser Ser Ile Val Tyr Glu Ala Ala Asp Met Ile Met His Thr Pro
    210                 215                 220

Gly Cys Val Pro Cys Val Arg Glu Gly Asn Ser Ser Arg Cys Trp Val
225                 230                 235                 240

Ala Leu Thr Pro Thr Leu Ala Ala Arg Asn Ser Ser Val Pro Thr Ala
                245                 250                 255

Thr Ile Arg Arg His Val Asp Leu Leu Val Gly Ala Ala Ala Phe Cys
            260                 265                 270

Ser Ala Met Tyr Val Gly Asp Leu Cys Gly Ser Val Phe Leu Val Ser
        275                 280                 285

Gln Leu Phe Thr Phe Ser Pro Arg Arg Tyr Glu Thr Val Gln Asp Cys
    290                 295                 300

Asn Cys Ser Leu Tyr Pro Gly His Val Ser Gly His Arg Met Ala Trp
305                 310                 315                 320

Asp Met Met Met Asn Trp Ser Pro Thr Thr Ala Leu Val Val Ser Gln
                325                 330                 335

Leu Leu Arg Ile Pro Gln Ala Val Val Asp Met Val Val Gly Ala His
            340                 345                 350

Trp Gly Val Leu Ala Gly Leu Ala Tyr Tyr Ser Met Val Gly Asn Trp
        355                 360                 365

Ala Lys Val Leu Ile Val Met Leu Leu Phe Ala Gly Val Asp Gly Lys
    370                 375                 380

Thr Tyr Val Thr Gly Gly Ala Gln Ser Arg Ala Thr Gln Gly Phe Ala
385                 390                 395                 400

Ser Leu Phe Thr Arg Gly Pro Ser Gln Lys Leu Gln Leu Val Asn Ser
                405                 410                 415

Asn Gly Ser Trp His Ile Asn Arg Thr Ala Leu Asn Cys Asn Asp Ser
            420                 425                 430

Phe Gln Thr Gly Phe Leu Ala Ala Leu Phe Tyr Ala His Arg Phe Asn
        435                 440                 445

Ser Ser Gly Cys Pro Glu Arg Met Ala Ser Cys Arg Pro Ile Asp Thr
    450                 455                 460

Phe Asp Gln Gly Trp Gly Pro Ile Thr His Val Ala Arg Arg Thr Ser
465                 470                 475                 480

Asp Gln Arg Pro Tyr Cys Trp His Tyr Ala Pro Gln Pro Cys Gly Ile
                485                 490                 495

Val Pro Ala Leu Gln Val Cys Gly Pro Val Tyr Cys Phe Thr Pro Ser
            500                 505                 510

Pro Val Val Val Gly Thr Thr Asp Arg Phe Gly Ala Pro Thr Tyr Asn
        515                 520                 525

Trp Gly Glu Asn Glu Thr Asp Val Leu Leu Leu Asn Asn Thr Arg Pro

530                 535                 540

Pro His Gly Asn Trp Phe Gly Cys Thr Trp Met Asn Ser Thr Gly Phe
545                 550                 555                 560

Thr Lys Thr Cys Gly Gly Pro Pro Cys Asn Ile Gly Gly Phe Gly Asn
                565                 570                 575

Asn Thr Leu Thr Cys Pro Thr Asp Cys Phe Arg Lys His Pro Glu Ala
            580                 585                 590

Thr Tyr Thr Lys Cys Gly Ser Gly Pro Trp Leu Thr Pro Arg Cys Met
        595                 600                 605

Val Asp Tyr Pro Tyr Arg Leu Trp His Tyr Pro Cys Thr Val Asn Phe
    610                 615                 620

Ser Ile Phe Lys Val Arg Met Tyr Val Gly Gly Val Glu His Arg Leu
625                 630                 635                 640

Thr Ala Ala Cys Asn Trp Thr Arg Gly Glu Arg Cys Asn Leu Glu Asp
                645                 650                 655

Arg Asp Arg Ser Glu Leu Ser Pro Leu Leu Leu Ser Thr Thr Glu Trp
            660                 665                 670

Gln Val Leu Pro Cys Ser Phe Thr Thr Leu Pro Ala Leu Ser Thr Gly
        675                 680                 685

Leu Ile His Leu His Gln Asn Ile Val Asp Val Gln Tyr Leu Tyr Gly
    690                 695                 700

Val Gly Ser Ser Val Val Ser Ile Ala Ile Arg Trp Glu Tyr Val Val
705                 710                 715                 720

Leu Leu Phe Leu Leu Leu Ala Asp Ala Arg Val Cys Ala Cys Leu Trp
                725                 730                 735

Met Met Leu Leu Ile Ala Gln Ala Glu Ala Ala Leu Glu Asn Leu Val
            740                 745                 750

Ile Leu Asn Ala Ala Ser Val Ala Gly Ala His Gly Val Leu Ser Phe
        755                 760                 765

Leu Val Phe Phe Cys Ala Ala Trp Tyr Ile Lys Gly Lys Leu Val Pro
    770                 775                 780

Gly Ala Ala Tyr Ala Phe Tyr Gly Val Trp Pro Leu Leu Leu Leu Leu
785                 790                 795                 800

Leu Ser Leu Pro Pro Arg Ala Tyr Ala Leu Asp Arg Glu Met Ala Ala
                805                 810                 815

Ser Cys Gly Gly Ala Val Phe Val Gly Leu Met Leu Leu Thr Leu Ser
            820                 825                 830

Pro His Tyr Lys Val Phe Leu Ala Arg Leu Ile Trp Trp Leu Gln Tyr
        835                 840                 845

Phe Ile Thr Arg Ala Glu Ala His Leu Gln Val Trp Val Pro Pro Leu
    850                 855                 860

Asn Val Arg Gly Gly Arg Asp Ala Ile Ile Leu Leu Thr Cys Val Val
865                 870                 875                 880

His Pro Glu Leu Ile Phe Asp Ile Thr Lys Ile Leu Leu Ala Met Leu
                885                 890                 895

Gly Pro Leu Met Val Leu Gln Ala Gly Leu Thr Arg Val Pro Tyr Phe
            900                 905                 910

Val Arg Ala Gln Gly Leu Ile Arg Ala Cys Met Leu Val Arg Lys Val
        915                 920                 925

Ala Gly Gly His Tyr Val Gln Met Ala Leu Met Lys Leu Ala Ala Leu
    930                 935                 940

Thr Gly Thr Tyr Val Tyr Asp His Leu Thr Pro Leu Gln Asp Trp Ala
945                 950                 955                 960

His Ala Gly Leu Arg Asp Leu Ala Val Ala Val Glu Pro Val Val Phe
                965                 970                 975

Ser Asp Met Glu Thr Lys Val Ile Thr Trp Gly Ala Asp Thr Ala Ala
            980                 985                 990

Cys Gly Asp Ile Ile Ser Gly Leu  Pro Val Ser Ala Arg  Arg Gly Arg
        995                 1000                 1005

Glu Ile  Leu Leu Gly Pro Ala  Asp Arg Phe Gly Glu  Gln Gly Trp
    1010                 1015                 1020

Arg Leu Leu Ala Pro Ile Thr Ala Tyr Ala Gln Gln Thr Arg Gly
    1025                1030                1035

Leu Leu Gly Cys Ile Ile Thr Ser Leu Thr Gly Arg Asp Lys Asn
    1040                1045                1050

Gln Val Glu Gly Glu Val Gln Val Val Ser Thr Ala Thr Gln Ser
    1055                1060                1065

Phe Leu Ala Thr Cys Val Asn Gly Val Cys Trp Thr Val Tyr His
    1070                1075                1080

Gly Ala Gly Ser Lys Thr Leu Ala Gly Pro Lys Gly Pro Ile Thr
    1085                1090                1095

Gln Met Tyr Thr Asn Val Asp Gln Asp Leu Val Gly Trp Pro Ala
    1100                1105                1110

Pro Pro Gly Ala Arg Ser Leu Thr Pro Cys Thr Cys Gly Ser Ser
    1115                1120                1125

Asp Leu Tyr Leu Val Thr Arg His Ala Asp Val Ile Pro Val Arg
    1130                1135                1140

Arg Arg Gly Asp Ser Arg Gly Ser Leu Leu Ser Pro Arg Pro Ile
    1145                1150                1155

Ser Tyr Leu Lys Gly Ser Ser Gly Gly Pro Leu Leu Cys Pro Leu
    1160                1165                1170

Gly His Ala Val Gly Ile Phe Arg Ala Ala Val Cys Thr Arg Gly
    1175                1180                1185

Val Ala Lys Ala Val Asp Phe Val Pro Val Glu Ser Met Glu Thr
    1190                1195                1200

Thr Met Arg Ser Pro Val Phe Thr Asp Asn Ser Ser Pro Pro Ala
    1205                1210                1215

Val Pro Gln Thr Phe Gln Val Ala His Leu His Ala Pro Thr Gly
    1220                1225                1230

Ser Gly Lys Ser Thr Lys Val Pro Ala Ala Tyr Ala Ala Gln Gly
    1235                1240                1245

Tyr Lys Val Leu Val Leu Asn Pro Ser Val Ala Ala Thr Leu Gly
    1250                1255                1260

Phe Gly Ala Tyr Met Ser Lys Ala His Gly Val Glu Pro Asn Ile
    1265                1270                1275

Arg Thr Gly Val Arg Thr Ile Thr Thr Gly Ala Ser Ile Thr Tyr
    1280                1285                1290

Ser Thr Tyr Gly Lys Phe Leu Ala Asp Gly Gly Cys Ser Gly Gly
    1295                1300                1305

Ala Tyr Asp Ile Ile Ile Cys Asp Glu Cys His Ser Thr Asp Ser
    1310                1315                1320

Thr Ser Ile Leu Gly Ile Gly Thr Val Leu Asp Gln Ala Glu Thr
    1325                1330                1335

Ala Gly Ala Arg Leu Val Val Leu Ala Thr Ala Thr Pro Pro Gly
    1340                1345                1350

Ser Val Thr Val Pro His Pro Asn Ile Glu Glu Val Ala Leu Pro
    1355                1360                1365

Ser Thr Gly Glu Ile Pro Phe Tyr Gly Lys Ala Ile Pro Ile Glu
    1370                1375                1380

Thr Ile Lys Gly Gly Arg His Leu Ile Phe Cys His Ser Lys Lys
    1385                1390                1395

Lys Cys Asp Glu Leu Ala Ala Lys Leu Val Gly Leu Gly Val Asn
    1400                1405                1410

Ala Val Ala Tyr Tyr Arg Gly Leu Asp Val Ser Val Ile Pro Thr
    1415                1420                1425

Ser Gly Asp Val Val Val Val Ala Thr Asp Ala Leu Met Thr Gly
    1430                1435                1440

Phe Thr Gly Asp Phe Asp Ser Val Ile Asp Cys Asn Thr Cys Val
    1445                1450                1455

Thr Gln Thr Val Asp Phe Ser Leu Asp Pro Thr Phe Thr Ile Glu
    1460                1465                1470

Thr Thr Thr Val Pro Gln Asp Ala Val Ser Arg Ser Gln Arg Arg

1475                 1480                 1485

Gly Arg Thr Gly Arg Gly Arg Met Gly Ile Tyr Arg Phe Val Ala
    1490                 1495                 1500

Pro Gly Glu Arg Pro Ser Gly Met Phe Asp Ser Ser Val Leu Cys
    1505                 1510                 1515

Glu Cys Tyr Asp Ala Gly Cys Ala Trp Tyr Glu Leu Thr Pro Ala
    1520                 1525                 1530

Glu Thr Ser Val Arg Leu Arg Ala Tyr Leu Asn Thr Pro Gly Leu
    1535                 1540                 1545

Pro Val Cys Gln Asp His Leu Glu Phe Trp Glu Gly Val Phe Thr
    1550                 1555                 1560

Gly Leu Thr His Ile Asp Ala His Phe Leu Ser Gln Thr Lys Gln
    1565                 1570                 1575

Ala Gly Asp Asn Phe Pro Tyr Leu Val Ala Tyr Gln Ala Thr Val
    1580                 1585                 1590

Cys Ala Arg Ala Gln Ala Pro Pro Pro Ser Trp Asp Gln Met Trp
    1595                 1600                 1605

Lys Cys Leu Ile Arg Leu Lys Pro Thr Leu His Gly Pro Thr Pro
    1610                 1615                 1620

Leu Leu Tyr Arg Leu Gly Ala Val Gln Asn Glu Val Ile Leu Thr
    1625                 1630                 1635

His Pro Ile Thr Lys Tyr Ile Met Ala Cys Met Ser Ala Asp Leu
    1640                 1645                 1650

Glu Val Val Thr Ser Thr Trp Val Leu Val Gly Gly Val Leu Ala
    1655                 1660                 1665

Ala Leu Ala Ala Tyr Cys Leu Thr Thr Gly Ser Val Val Ile Val
    1670                 1675                 1680

Gly Arg Ile Ile Leu Ser Gly Lys Pro Ala Ile Ile Pro Asp Arg
    1685                 1690                 1695

Glu Val Leu Tyr Arg Glu Phe Asp Glu Met Glu Glu Cys Ala Ser
    1700                 1705                 1710

His Leu Pro Tyr Ile Glu Gln Gly Met Gln Leu Ala Glu Gln Phe
    1715                1720                1725

Lys Gln Lys Ala Leu Gly Leu Leu Gln Thr Ala Thr Lys Gln Ala
    1730                1735                1740

Glu Ala Ala Ala Pro Val Val Glu Ser Lys Trp Arg Ala Leu Glu
    1745                1750                1755

Ala Phe Trp Ala Lys His Met Trp Asn Phe Ile Ser Gly Ile Gln
    1760                1765                1770

Tyr Leu Ala Gly Leu Ser Thr Leu Pro Gly Asn Pro Ala Ile Ala
    1775                1780                1785

Ser Leu Met Ala Phe Thr Ala Ser Ile Thr Ser Pro Leu Thr Thr
    1790                1795                1800

Gln His Thr Leu Leu Phe Asn Ile Leu Gly Gly Trp Val Ala Ala
    1805                1810                1815

Gln Leu Ala Pro Pro Gly Ala Ala Ser Ala Phe Val Gly Ala Gly
    1820                1825                1830

Ile Ala Gly Ala Ala Val Gly Ser Ile Gly Leu Gly Lys Val Leu
    1835                1840                1845

Val Asp Ile Leu Ala Gly Tyr Gly Ala Gly Val Ala Gly Ala Leu
    1850                1855                1860

Val Ala Phe Lys Val Met Ser Gly Glu Met Pro Ser Thr Glu Asp
    1865                1870                1875

Leu Val Asn Leu Leu Pro Ala Ile Leu Ser Pro Gly Ala Leu Val
    1880                1885                1890

Val Gly Val Val Cys Ala Ala Ile Leu Arg Arg His Val Gly Pro
    1895                1900                1905

Gly Glu Gly Ala Val Gln Trp Met Asn Arg Leu Ile Ala Phe Ala
    1910                1915                1920

Ser Arg Gly Asn His Val Ser Pro Thr His Tyr Val Pro Glu Ser
    1925                1930                1935

Asp Ala Ala Ala Arg Val Thr Gln Ile Leu Ser Ser Leu Thr Ile
    1940                1945                1950

Thr Gln Leu Leu Lys Arg Leu His Gln Trp Ile Asn Glu Asp Cys
    1955                1960                1965

Ser Thr Pro Cys Ser Gly Ser Trp Leu Arg Asp Val Trp Asp Trp
    1970                1975                1980

Ile Cys Thr Val Leu Ser Asp Phe Lys Thr Trp Leu Gln Ser Lys
    1985                1990                1995

Leu Leu Pro Arg Leu Pro Gly Val Pro Phe Leu Ser Cys Gln Arg
    2000                2005                2010

Gly Tyr Lys Gly Val Trp Arg Gly Asp Gly Ile Met Gln Thr Ser
    2015                2020                2025

Cys Pro Cys Gly Ala Gln Ile Ala Gly His Val Lys Asn Gly Ser
    2030                2035                2040

Met Arg Ile Val Gly Pro Lys Thr Cys Ser Asn Thr Trp His Gly
    2045                2050                2055

Thr Phe Pro Ile Asn Ala His Thr Thr Gly Pro Cys Thr Pro Ser
    2060                2065                2070

Pro Ala Pro Asn Tyr Ser Lys Ala Leu Trp Arg Val Ala Ala Glu
    2075                2080                2085

Glu Tyr Val Glu Val Thr Arg Val Gly Asp Phe His Tyr Val Thr
    2090                2095                2100

Gly Met Thr Thr Asp Asn Val Lys Cys Pro Cys Gln Val Pro Ala
    2105                2110                2115

Pro Glu Phe Phe Thr Glu Val Asp Gly Val Arg Leu His Arg Tyr
    2120                2125                2130

Ala Pro Ala Cys Lys Pro Leu Leu Arg Asp Glu Val Thr Phe Gln
    2135                2140                2145

Val Gly Leu Asn Gln Phe Pro Val Gly Ser Gln Leu Pro Cys Glu
    2150                2155                2160

Pro Glu Pro Asp Val Ser Val Leu Thr Ser Met Leu Thr Asp Pro
    2165                2170                2175

Ser His Ile Thr Ala Glu Thr Ala Lys Arg Arg Leu Ala Arg Gly
    2180                2185                2190

Ser Ser Pro Ser Leu Ala Ser Ser Ser Ala Ser Gln Leu Ser Ala
    2195                2200                2205

Pro Ser Leu Lys Ala Thr Cys Thr Thr His His Asp Ser Pro Asp
    2210                2215                2220

Ala Asp Leu Ile Glu Ala Asn Leu Leu Trp Arg Gln Glu Met Gly
    2225                2230                2235

Gly Asn Ile Thr Arg Val Glu Ser Glu Asn Lys Val Val Ile Leu
    2240                2245                2250

Asp Ser Phe Asp Pro Leu Arg Ala Glu Glu Asp Glu Arg Glu Val
    2255                2260                2265

Ser Val Ala Ala Glu Ile Leu Arg Lys Thr Arg Lys Phe Pro Pro
    2270                2275                2280

Ala Met Pro Ile Trp Ala Arg Pro Asp Tyr Asn Pro Pro Leu Leu
    2285                2290                2295

Glu Thr Trp Lys Asp Pro Asp Tyr Val Pro Pro Val Val His Gly
    2300                2305                2310

Cys Pro Leu Pro Pro Thr Lys Thr Pro Pro Ile Pro Pro Pro Arg
    2315                2320                2325

Arg Lys Lys Thr Val Val Leu Thr Glu Ser Thr Val Ser Ser Ala
    2330                2335                2340

Leu Ala Glu Leu Ala Thr Lys Thr Phe Gly Ser Ser Gly Ser Ser
    2345                2350                2355

Ala Val Asp Ser Gly Thr Ala Thr Ala Pro Pro Asn Gln Leu Ser
    2360                2365                2370

Asp Glu Val Asp Thr Gly Ser Asp Val Glu Ser Tyr Ser Ser Met
    2375                2380                2385

Pro Pro Leu Glu Gly Glu Pro Gly Asp Pro Asp Leu Ser Asp Gly

2390                2395                2400

Ser Trp Ser Thr Val Ser Glu Glu Ala Gly Glu Asp Val Val Cys
    2405                2410                2415

Cys Ser Met Ser Tyr Thr Trp Thr Gly Ala Leu Ile Thr Pro Cys
    2420                2425                2430

Ala Ala Glu Glu Ser Lys Leu Pro Ile Asn Ala Leu Ser Asn Ser
    2435                2440                2445

Leu Leu Arg His His Asn Met Val Tyr Ala Thr Thr Ser Arg Ser
    2450                2455                2460

Ala Ser Gln Arg Gln Lys Lys Val Thr Phe Asp Arg Leu Gln Val
    2465                2470                2475

Leu Asp Asp His Tyr Arg Asp Val Leu Lys Glu Met Lys Ala Lys
    2480                2485                2490

Ala Ser Thr Val Lys Ala Lys Leu Leu Ser Val Glu Glu Ala Cys
    2495                2500                2505

Lys Leu Thr Pro Pro His Ser Ala Arg Ser Lys Phe Gly Tyr Gly
    2510                2515                2520

Ala Lys Asp Val Arg Asn Leu Ser Ser Lys Ala Val Asn His Ile
    2525                2530                2535

Asn Ser Val Trp Lys Asp Leu Leu Glu Asp Thr Glu Thr Pro Ile
    2540                2545                2550

Asp Thr Thr Ile Met Ala Lys Asn Glu Val Phe Cys Val Gln Pro
    2555                2560                2565

Glu Lys Gly Gly Arg Lys Pro Ala Arg Leu Ile Val Tyr Pro Asp
    2570                2575                2580

Leu Gly Val Arg Val Cys Glu Lys Met Ala Leu Tyr Asp Val Val
    2585                2590                2595

Ser Thr Leu Pro Gln Ala Val Met Gly Ser Ser Tyr Gly Phe Gln
    2600                2605                2610

Tyr Ser Pro Gly Gln Arg Val Glu Phe Leu Val Asn Ala Trp Lys
    2615                2620                2625

Ser Lys Lys Asn Pro Met Gly Phe Ala Tyr Asp Thr Arg Cys Phe
    2630                2635                2640

Asp Ser Thr Val Thr Glu Asn Asp Ile Arg Val Glu Glu Ser Ile
    2645                2650                2655

Tyr Gln Cys Cys Asp Leu Ala Pro Glu Ala Arg Gln Val Ile Arg
    2660                2665                2670

Ser Leu Thr Glu Arg Leu Tyr Val Gly Gly Pro Leu Thr Asn Ser
    2675                2680                2685

Lys Gly Gln Asn Cys Gly Tyr Arg Arg Cys Arg Ala Ser Gly Val
    2690                2695                2700

Leu Thr Thr Ser Cys Gly Asn Thr Leu Thr Cys Tyr Leu Lys Ala
    2705                2710                2715

Ser Ala Ala Cys Arg Ala Ala Lys Leu Gln Asp Cys Thr Met Leu
    2720                2725                2730

Val Cys Gly Asp Asp Leu Val Val Ile Cys Glu Ser Ala Gly Thr
    2735                2740                2745

Gln Glu Asp Ala Ala Ser Leu Arg Val Phe Thr Glu Ala Met Thr
    2750                2755                2760

Arg Tyr Ser Ala Pro Pro Gly Asp Pro Pro Arg Pro Glu Tyr Asp
    2765                2770                2775

Leu Glu Leu Ile Thr Ser Cys Ser Ser Asn Val Ser Val Ala His
    2780                2785                2790

Asp Ala Ser Gly Lys Arg Val Tyr Tyr Leu Thr Arg Asp Pro Thr
    2795                2800                2805

Thr Pro Leu Ala Arg Ala Ala Trp Glu Thr Ala Lys His Thr Pro
    2810                2815                2820

Val Asn Ser Trp Leu Gly Asn Ile Ile Met Tyr Ala Pro Thr Leu
    2825                2830                2835

Trp Ala Arg Met Ile Leu Met Thr His Phe Phe Ser Ile Leu Leu
    2840                2845                2850

Ala Gln Glu Gln Leu Glu Lys Ala Leu Asp Cys Gln Ile Tyr Gly
    2855                2860                2865

Ala Thr Tyr Ser Ile Glu Pro Leu Asp Leu Pro Gln Ile Ile Gln
    2870                2875                2880

Arg Leu His Gly Leu Ser Ala Phe Ser Leu His Ser Tyr Ser Pro
    2885                2890                2895

Gly Glu Ile Asn Arg Val Ala Ser Cys Leu Arg Lys Leu Gly Val
    2900                2905                2910

Pro Pro Leu Arg Val Trp Arg His Arg Ala Arg Ser Val Arg Ala
    2915                2920                2925

Lys Leu Leu Ser Gln Gly Gly Arg Ala Ala Thr Cys Gly Lys Tyr
    2930                2935                2940

Leu Phe Asn Trp Ala Val Arg Thr Lys Leu Lys Leu Thr Pro Ile
    2945                2950                2955

Pro Ala Ala Ser Gln Leu Asp Leu Ser Gly Trp Phe Ile Ala Gly
    2960                2965                2970

Tyr Ser Gly Gly Asp Ile Tyr His Ser Leu Ser Arg Ala Arg Pro
    2975                2980                2985

Arg Trp Phe Met Leu Cys Leu Leu Leu Leu Ser Val Gly Val Gly
    2990                2995                3000

Ile Tyr Leu Leu Pro Asn Arg
    3005                3010

<210> 3
<211> 9594
<212> RNA
<213> Hepatitis C virus

<400> 3
gccagccccc ugaugggggc gacacuccac cauagaucac uccccuguga ggaacuacug 60

ucuucacgca gaaagcgucu agccauggcg uuaguaugag ugucgugcag ccuccaggac 120

cccccucccc gggagagcca uaguggucug cggaaccggu gaguacaccg gaauugccag 180

gacgaccggg uccuuucuug gaucaacccg cucaaugccu ggagauuugg gcgugccccc 240

gcgagacugc uagccgagua guguuggguc gcgaaaggcc uugugguacu gccugauagg 300

gugcuugcga gugccccggg aggucucgua gaccgugcau caugagcaca aauccuaaac 360
cucaaagaaa aaccaaacgu aacaccaacc gccgcccaca ggacgucaag uucccgggcg 420
guggccagau cguuggugga guuuaccugu ugccgcgcag gggccccagg uugggugugc 480
gcgcgacuag gaagacuucc gagcggucgc aaccucgugg aaggcgacaa ccuaucccca 540
aggcucgcca gcccgagggc agggccuggg cucagcccgg guauccuugg ccccucuaug 600
gcaacgaggg ucuggggugg gcaggauggc uccugucacc ccguggcucu cggccuaguu 660
ggggccccac ggacccccgg cguaggucgc guaauuuggg uaaggucauc gauacccuca 720
caugcggcuu cgccgaccuc augggguaca uuccgcucgu cggcgccccc cuaggaggcg 780
cugccagggc ccuggcgcau ggcguccggg uucuggagga cggcgugaac uaugcaacag 840
ggaaucugcc cgguugcccu uucucuaucu uccucuuagc uuugcugucc uguuugacca 900
ucccagcuuc cgcucacgaa gugcgcaacg uauccgggcu guaccauguc acgaacgacu 960
gcuccaacuc aagcauugug uaugaggcag cggacaugau caugcacacc cccgggugcg 1020
ugcccugcgu ccgggagggu aacuccuccc gcugcugggu agcgcucacu cccacgcucg 1080
cggccaggaa uagcagcguc cccacugcga caauacgacg ccaugucgau uugcucgucg 1140
gggcggcugc uuucuguucc gcuauguacg ugggggaucu uugcggaucu guuuuccucg 1200
ucucccagcu guucaccuuu ucaccucgcc gguacgagac gguacaggac ugcaauugcu 1260
cacucuaucc cggccacgua ucaggccauc gcauggcuug ggauaugaug augaacuggu 1320
caccuacaac agccuuagug guaucgcagu uacuccggau cccacaagcc gucguggaua 1380
uggugguagg ggcccacugg ggaguccugg cgggccuugc cuacuauucc auggugggga 1440
acugggcuaa ggucuugauu gugaugcuac ucuuugccgg cgucgacggg aagaccuacg 1500
ugacaggggg ggcgcagagc cgagccacuc aaggcuuugc gucccucuuu acacgggggc 1560
cgucucagaa acuccagcuu guaaauucca acggcagcug gcacauuaac aggacugccu 1620
ugaacugcaa ugacuccuuc cagacugggu uccuugccgc gcuguuuuac gcacaccguu 1680
ucaacucguc cggaugccca gagcgcaugg ccagcugccg ccccaucgac acguucgauc 1740
aggggugggg ccccaucacu caugucgcgc gucgcacauc ggaccagagg ccuuauugcu 1800
ggcacuacgc accucaaccg ugugguauug uacccgcguu gcagguaugu gguccagugu 1860
auugcuucac cccaagcccc gucguggugg ggacgaccga ucgcuucggc gcccccacgu 1920
acaacugggg ggagaaugag acggacgugc uacuccucaa caauacgcgg ccgccgcacg 1980
gcaacugguu cggcuguaca uggaugaaua guaccggguu caccaagacg ugugggggcc 2040
cccccugcaa caucgggggg uuuggcaaca acaccuugac cugcccuacg gauugcuucc 2100
ggaagcaccc cgaggccacu uacaccaaau gcggcucggg gcccugguug acgccuaggu 2160

gcaugguuga uuacccauac agacuuuggc acuaccccug cacuguuaac uuuuccaucu 2220

ucaaggucag gauguaugug gggggugugg agcacaggcu caccgccgcg ugcaauugga 2280

cucggggaga gcgcugcaac uuggaggaua gggacagauc ggagcucagc ccgcugcuac 2340

ugucuaccac agaguggcag guacugcccu guucuuucac caccuuaccg gcccugucca 2400

cugguuugau ccaccuccac cagaacaucg uggacgugca auaccuguac gguguggggu 2460

caucgguugu cuccauugca aucagguggg aguaugucgu gcugcucuuc cuccuccugg 2520

cggacgcgcg yguuugcgcc ugcuugugga ugaugcugcu gauagcccaa gcugaggccg 2580

ccuuagagaa ccuggugauc cucaaugcgg cgucuguggc cggagcgcau ggcguucucu 2640

cuuuccuugu guucuucugc gcugccuggu acaucaaggg caagcugguc cccggggcgg 2700

cauaugccuu cuauggugua uggccgcugc uccugcuucu gcugucauua ccaccacgag 2760

cauacgccuu ggaccgggag auggcugcau cgugcggagg cgcgguuuuc guaggucuga 2820

ugcuccugac cuugucacca cacuacaagg uguuucucgc uaggcucaua uggugguuac 2880

aguauuuuau caccagggcc gaggcgcacu ugcaggugug gguccccccc cucaacguuc 2940

gggggggccg cgaugccauc auccuccuca cguguguggu ccacccagag cuaauuuuug 3000

acaucaccaa aaucuugcuc gccaugcucg guccgcucau ggugcuccag gcuggccuaa 3060

cuagagugcc guacuucgua cgcgcucaag ggcucauccg ugcaugcaug uuagugcgga 3120

aagucgcugg gggccacuau guccaaaugg cccucaugaa acuggccgca cugacgggua 3180

cguacguuua ugaccaucuu acuccgcugc aggacugggc ccacgcgggc uugcgagacc 3240

uugcaguggc aguugagccc gucgucuucu cugacaugga gacuaagguc aucaccuggg 3300

gggcagacac cgcagcgugu ggggacauca ucucgggccu acccgucucc gcccgaaggg 3360

ggagggagau acuucugggc cccgccgaca gguuuggaga gcaggggugg cgacuccucg 3420

cgccuaucac ggcuuacgcu caacagacgc ggggccuacu uggcuguauc aucaccagcc 3480

ucacaggccg ggacaagaac caggucgagg gggagguuca ggugguuucc accgcaacgc 3540

aaucuuuccu ggcgaccugc gucaacggcg uguguuggac ugucuaccau ggugccggcu 3600

cgaagacccu ggccggcccg aagggcccaa ucacccaaau guacaccaau guggaccaag 3660

accucgucgg cuggccggcg ccccccgggg cgcgcucccu gacaccgugc accugcggca 3720

gcucggaccu cuaccugguc acgaggcaug cugaugucau uccggugcgc cggcggggcg 3780

acagcagggg gagucuacuc ucucccaggc ccaucuccua cuuaaagggc uccucaggug 3840

guccacugcu uugcccccug gggcacgcug ugggcaucuu ccgggccgcu gugugcaccc 3900

gggggguugc aaaggcggug gauuuuguac cuguugaguc uauggaaacc accaugcggu 3960

cuccggucuu uacggauaau ucaucucccc cggccguacc gcagacauuc caaguggccc 4020

aucuacacgc ucccacuggc aguggcaaga gcacuaaggu gccggcugcg uacgcagccc 4080

aaggguacaa gguacucguc uugaacccau ccguugccgc uaccuuaggg uuuggggcgu 4140

acaugucuaa agcacauggu guugagccua acaucagaac ugggguaagg accaucacca 4200

cgggcgcuuc caucacguau uccaccuacg guaaguuccu ugccgacggu gguugcucug 4260

ggggcgccua ugacaucaua auaugugaug agugccacuc aacugacucg acuuccaucu 4320

ugggcauugg cacaguccug gaccaagcgg agacggcugg agcgcggcuc gucgugcucg 4380

ccaccgcuac gccuccggga ucggucaccg ugccacaucc caauaucgag gagguggccu 4440

ugcccagcac cggagaaauu cccuucuacg gcaaagccau ccccauugag accaucaagg 4500

gggggaggca ccucaucuuc ugccacucca agaagaaaug ugacgagcuc gcugcaaagc 4560

uggugggccu cggaguuaac gcuguugcgu acuaccgggg ucuugaugug uccgucauac 4620

caacaagcgg agaugucguu gucguggcaa cagacgcucu aaugacgggc uucaccggcg 4680

acuuugacuc agugaucgac uguaauacuu gugucaccca gacaguugau uucagcuugg 4740

acccuaccuu caccauugag acgacaaccg ugccccaaga cgcggugucg cguucgcagc 4800

gacgaggcag gacuggcagg ggcaggaugg gcauauacag guuuguggcu ccaggggaac 4860

ggcccucggg cauguucgau ucuucggucc ugugugagug cuaugacgcg ggcugugcuu 4920

gguaugagcu cacgcccgcc gagaccucag ucagguugcg ggcuuaccua aauacaccag 4980

ggcugcccgu cugccaggac caccuggagu uuugggaggg ggucuucaca ggccucaccc 5040

acauagaugc ccauuucuug ucccagacua agcaggcagg agauaacuuc cccuaccugg 5100

uagcauacca ggcuacggug ugcgccaggg cccaggcucc cccuccaucg ugggaucaaa 5160

uguggaagug ucucauacgg cugaagccua cacuacacgg gccaacgccc cuguuguaua 5220

ggcuaggagc cguccagaau gaggucaucc ucacacaucc cauaaccaaa uacaucaugg 5280

caugcauguc ggcugaccua gaggucguca cuagcaccug ggugcugguc ggcggguccc 5340

uugcagcucu ggccgcguac ugccugacga cgggcagcgu ggucauugug ggcaggauca 5400

ucuuguccgg gaagccggcu aucauuccug acagggaagu ccucuaccgg gaguucgaug 5460

aaauggaaga gugugccuca caccuccccu acaucgaaca gggaaugcag cucgccgaac 5520

aauucaagca gaaggcgcuc ggguugcugc agacagccac caagcaagcg gaagccgcug 5580

cuccuguggu ggaguccaag uggcgagccc uugaggccuu cugggcgaag cacaugugga 5640

auuucaucag cgggauacag uacuuagcag gcuuguccac ucugccuggg aaccccgcga 5700

uagcaucacu gauggcauuc acagccucua ucaccagccc gcuuaccacc cuacacaccc 5760

uccuguuuaa caucuuggga ggaugggugg ccgcccaacu ugcccccccc ggugcugccu 5820

cggcuuucgu gggcgccggc auugcuggcg cagcuguugg cagcauaggc cuugggaagg 5880

ugcuugugga cauccuggcg gguuauggag cagggguggc aggcgcgcuc guggccuuca 5940

aggucaugag cggcgagaug cccuccaccg aggaccuggu caacuuacuc ccugccaucc 6000

ucucuccugg ugcccuuguc gucggggucg ugugcgcagc aauacugcgu cggcaugugg 6060

gcccggggga gggggcugug caauggauga accggcugau agcguucgcc ucgcggggua 6120

accacgucuc ccccacgcac uaugugccug agagcgacgc ugcagcgcgu gucacacaga 6180

uccucucuag ccucaccauc acucagcuac ugaagaggcu ccaccagugg auuaauaagg 6240

acugcuccac accaugcucc ggcucguggc uuagggacgu uugggacugg auaugcacgg 6300

uuuugaguga cuucaagacc uggcuccagu ccaagcuccu gccacgguua ccgggaguuc 6360

cauuccuuuc augccaacgu ggguauaagg gggucuggcg gggagauggc aucaugcaga 6420

ccuccugccc auguggagca caaaucgccg gacaugucaa gaacgguucc augaggaucg 6480

uugggccuaa aaccuguagc aacacguggc acggaacauu ccccauuaac gcgcacacca 6540

cgggccccug cacacccucc ccagcgccga acuacucuaa ggcguugugg cggguggcug 6600

cugaggagua cguggaaguc acgcgguggg gggauuucca uuacgugacg ggcaugacca 6660

cugacaacgu aaaaugccca ugccagguuc cggcccccga guucuucaca gagguggaug 6720

ggguacggcu gcacagguac gcuccggcgu gcaaaccucu ccuacgggau gaggucacau 6780

uccaggucgg gcucaaccag uucccgguug ggucacagcu cccaugcgag cccgaaccgg 6840

auguaucagu gcucacuucc augcuuaccg acccuuccca caucacagca gagacggcua 6900

agcguaggcu ggccagaggg ucuucccccu cuuuggccag cucuucagcu agucaguugu 6960

cugcgcccuc auugaaggcg acaugcacca cccaucauga cuccccagac gcugaccuca 7020

uugaggccaa ccuccugugg cggcaggaga ugggagggaa caucacccgu guggagucag 7080

agaacaaggu gguaauccug gacucuuuug acccgcuucg agcggaggag gacgagaggg 7140

aggugucugu ugcggcggag auccugcgga aaaccaggaa guucccccca gcgaugccca 7200

uaugggcacg cccggacuac aacccaccgc ugcuagagac uuggaaggac ccggacuacg 7260

ucccuccagu ggugcacggg ugcccauugc caccuaccaa gaccccucca auaccaccuc 7320

cgcggaggaa aaagacaguu guccugacag aguccaccgu gucuucugcc cuggcggagc 7380

uugccacaaa gaccuuuggc agcuccggau cgucggccgu cgacagcggc acagcgaccg 7440

cccccccuaa ccagcucucc gacgaagugg auacaggauc cgacguugag ucguacuccu 7500

ccaugccccc ccuugaggga gagccggggg accccgaucu cagcgacggg ucuuggucua 7560

cuguaaguga ggaggcuggu gaggacgucg ucugcugcuc gauguccuac acauggacag 7620

gcgccuugau cacgccgugc gccgcggagg agagcaagcu gcccaucaau gcgcugagca 7680
acucuuugcu gcgccaccac aacauggucu augccacaac aucccgcagc gcaagccaac 7740
ggcagaaaaa ggucaccuuu gacagacugc aaguccugga cgaccauuac cgggacgugc 7800
ucaaggagau gaaggcgaag gcguccacag uuaaggcuaa acuucuaucc guagaagagg 7860
ccugcaagcu gacgccccca cacucagcca gguccaaauu uggcuauggg gcgaaggacg 7920
uccggaaccu auccagcaag gccguuaacc acaucaacuc cguguggaag gacuugcugg 7980
aagacacuga gacaccaauu gacaccacca ucauggcaaa aaaugagguc uucuguguuc 8040
aaccagagaa gggaggccgc aagccagcuc gccuuaucgu auacccagac uugggggugc 8100
gugugugcga gaaaauggcc cuuuacgacg uggucuccac ucuuccucag gccgugaugg 8160
gcuccucaua cggauuccag uacucuccug ggcagcgggu cgaguuccug gugaaugccu 8220
ggaaaucaaa gaagaacccu augggcuucg cauaugacac ccgcuguuuu gacucaacgg 8280
ucaccgagaa cgacauccgu guugaggagu caauuuacca auguugugac uuggcccccg 8340
aggccagaca ggugauaagg ucgcucacag agcggcuuua ugucgggggc ccccugacua 8400
auucaaaagg gcagaacugc gguuaucgcc ggugccgcgc cagcggcgug cugacgacua 8460
gcugcgguaa uacccucaca uguuacuuga aggccucugc agccugucga gcugcaaagc 8520
uccaggacug cacgaugcuc gugugcgggg acgaccuugu cguuaucugu gaaagcgcgg 8580
ggacccagga ggacgcggcg agccuacgag ucuucacgga ggcuaugacu agguacuccg 8640
cccccccggg ggacccgccc cgaccggaau acgacuugga guugauaaca ucaugcuccu 8700
ccaacguguc ggucgcgcac gaugcaucug gcaaacgggu guauuaccuc acccgugacc 8760
ccaccacccc ccuugcgcgg gcugcguggg agacagcuaa acacacucca gucaacuccu 8820
ggcuaggcaa caucaucaug uaugcgccca cccucugggc aaggaugauu cugaugacuc 8880
acuucuucuc cauccuucua gcucaggagc agcuugaaaa agcccuggau ugucagaucu 8940
acggggccac uuacuccauu gaaccacuug accuaccuca gaucauucaa cgacuccaug 9000
gucuuagcgc auucucacuc cauaguuacu cuccagguga aaucaauagg guggcuucau 9060
gccucaggaa acuuggggua ccgcccuugc gagucuggag acaucgggcc agaagugucc 9120
gcgcuaagcu acugucccag gggggagggc ugccacuugu uggcaaguac cucuucaacu 9180
gggcaguaag gaccaagcuc aaacucacuc caaucccggc ugcgucccag uuggacuugu 9240
ccggcugguu cauugcuggu uacagcgggg gagacauaua ucacagccug ucucgugccc 9300
gaccccgcug guuuauguug ugccuacucc uacuuucugu ggggguaggc aucuaccugc 9360
uccccaaucg augaacgggg ggcuaaacac uccaggccaa uaggccauuc uguuuuuuuu 9420
uuuuuuuuuu uuuuuuuuuu uuuuuuuuuu uuuuuuuuuu uuuuuuccuu uuuuuuuuuu 9480

uuuuucccuu ucuuuuggug gcuccaucuu agcccuaguc acggcuagcu gugaaagguc 9540

cgugagccgc augacugcag agagugcuga uacuggccuc ucugcagauc augu 9594

<210> 4
<211> 3010
<212> PRT
<213> Hepatitis C virus

<400> 4

Met Ser Thr Asn Pro Lys Pro Gln Arg Lys Thr Lys Arg Asn Thr Asn
1               5                   10                  15

Arg Arg Pro Gln Asp Val Lys Phe Pro Gly Gly Gly Gln Ile Val Gly
            20                  25                  30

Gly Val Tyr Leu Leu Pro Arg Arg Gly Pro Arg Leu Gly Val Arg Ala
        35                  40                  45

Thr Arg Lys Thr Ser Glu Arg Ser Gln Pro Arg Gly Arg Arg Gln Pro
    50                  55                  60

Ile Pro Lys Ala Arg Gln Pro Glu Gly Arg Ala Trp Ala Gln Pro Gly
65                  70                  75                  80

Tyr Pro Trp Pro Leu Tyr Gly Asn Glu Gly Leu Gly Trp Ala Gly Trp
                85                  90                  95

Leu Leu Ser Pro Arg Gly Ser Arg Pro Ser Trp Gly Pro Thr Asp Pro
            100                 105                 110

Arg Arg Arg Ser Arg Asn Leu Gly Lys Val Ile Asp Thr Leu Thr Cys
        115                 120                 125

Gly Phe Ala Asp Leu Met Gly Tyr Ile Pro Leu Val Gly Ala Pro Leu
    130                 135                 140

Gly Gly Ala Ala Arg Ala Leu Ala His Gly Val Arg Val Leu Glu Asp
145                 150                 155                 160

Gly Val Asn Tyr Ala Thr Gly Asn Leu Pro Gly Cys Pro Phe Ser Ile
                165                 170                 175

Phe Leu Leu Ala Leu Leu Ser Cys Leu Thr Ile Pro Ala Ser Ala His
            180                 185                 190

Glu Val Arg Asn Val Ser Gly Leu Tyr His Val Thr Asn Asp Cys Ser
        195                 200                 205

Asn Ser Ser Ile Val Tyr Glu Ala Ala Asp Met Ile Met His Thr Pro
    210                 215                 220

Gly Cys Val Pro Cys Val Arg Glu Gly Asn Ser Ser Arg Cys Trp Val
225                 230                 235                 240

Ala Leu Thr Pro Thr Leu Ala Ala Arg Asn Ser Ser Val Pro Thr Ala
                245                 250                 255

Thr Ile Arg Arg His Val Asp Leu Leu Val Gly Ala Ala Ala Phe Cys
            260                 265                 270

Ser Ala Met Tyr Val Gly Asp Leu Cys Gly Ser Val Phe Leu Val Ser
        275                 280                 285

Gln Leu Phe Thr Phe Ser Pro Arg Arg Tyr Glu Thr Val Gln Asp Cys
    290                 295                 300

Asn Cys Ser Leu Tyr Pro Gly His Val Ser Gly His Arg Met Ala Trp
305                 310                 315                 320

Asp Met Met Met Asn Trp Ser Pro Thr Thr Ala Leu Val Val Ser Gln
                325                 330                 335

Leu Leu Arg Ile Pro Gln Ala Val Val Asp Met Val Val Gly Ala His
            340                 345                 350

Trp Gly Val Leu Ala Gly Leu Ala Tyr Tyr Ser Met Val Gly Asn Trp
        355                 360                 365

Ala Lys Val Leu Ile Val Met Leu Leu Phe Ala Gly Val Asp Gly Lys
    370                 375                 380

Thr Tyr Val Thr Gly Gly Ala Gln Ser Arg Ala Thr Gln Gly Phe Ala
385                 390                 395                 400

Ser Leu Phe Thr Arg Gly Pro Ser Gln Lys Leu Gln Leu Val Asn Ser
                405                 410                 415

Asn Gly Ser Trp His Ile Asn Arg Thr Ala Leu Asn Cys Asn Asp Ser
            420                 425                 430

Phe Gln Thr Gly Phe Leu Ala Ala Leu Phe Tyr Ala His Arg Phe Asn

435                 440                 445

Ser Ser Gly Cys Pro Glu Arg Met Ala Ser Cys Arg Pro Ile Asp Thr
    450                 455                 460

Phe Asp Gln Gly Trp Gly Pro Ile Thr His Val Ala Arg Arg Thr Ser
465                 470                 475                 480

Asp Gln Arg Pro Tyr Cys Trp His Tyr Ala Pro Gln Pro Cys Gly Ile
                485                 490                 495

Val Pro Ala Leu Gln Val Cys Gly Pro Val Tyr Cys Phe Thr Pro Ser
            500                 505                 510

Pro Val Val Val Gly Thr Thr Asp Arg Phe Gly Ala Pro Thr Tyr Asn
        515                 520                 525

Trp Gly Glu Asn Glu Thr Asp Val Leu Leu Leu Asn Asn Thr Arg Pro
    530                 535                 540

Pro His Gly Asn Trp Phe Gly Cys Thr Trp Met Asn Ser Thr Gly Phe
545                 550                 555                 560

Thr Lys Thr Cys Gly Gly Pro Pro Cys Asn Ile Gly Gly Phe Gly Asn
                565                 570                 575

Asn Thr Leu Thr Cys Pro Thr Asp Cys Phe Arg Lys His Pro Glu Ala
            580                 585                 590

Thr Tyr Thr Lys Cys Gly Ser Gly Pro Trp Leu Thr Pro Arg Cys Met
        595                 600                 605

Val Asp Tyr Pro Tyr Arg Leu Trp His Tyr Pro Cys Thr Val Asn Phe
    610                 615                 620

Ser Ile Phe Lys Val Arg Met Tyr Val Gly Gly Val Glu His Arg Leu
625                 630                 635                 640

Thr Ala Ala Cys Asn Trp Thr Arg Gly Glu Arg Cys Asn Leu Glu Asp
                645                 650                 655

Arg Asp Arg Ser Glu Leu Ser Pro Leu Leu Leu Ser Thr Thr Glu Trp
            660                 665                 670

Gln Val Leu Pro Cys Ser Phe Thr Thr Leu Pro Ala Leu Ser Thr Gly
        675                 680                 685

Leu Ile His Leu His Gln Asn Ile Val Asp Val Gln Tyr Leu Tyr Gly
    690                 695                 700

Val Gly Ser Ser Val Val Ser Ile Ala Ile Arg Trp Glu Tyr Val Val
705                 710                 715                 720

Leu Leu Phe Leu Leu Leu Ala Asp Ala Arg Val Cys Ala Cys Leu Trp
                725                 730                 735

Met Met Leu Leu Ile Ala Gln Ala Glu Ala Ala Leu Glu Asn Leu Val
            740                 745                 750

Ile Leu Asn Ala Ala Ser Val Ala Gly Ala His Gly Val Leu Ser Phe
        755                 760                 765

Leu Val Phe Phe Cys Ala Ala Trp Tyr Ile Lys Gly Lys Leu Val Pro
    770                 775                 780

Gly Ala Ala Tyr Ala Phe Tyr Gly Val Trp Pro Leu Leu Leu Leu Leu
785                 790                 795                 800

Leu Ser Leu Pro Pro Arg Ala Tyr Ala Leu Asp Arg Glu Met Ala Ala
                805                 810                 815

Ser Cys Gly Gly Ala Val Phe Val Gly Leu Met Leu Leu Thr Leu Ser
            820                 825                 830

Pro His Tyr Lys Val Phe Leu Ala Arg Leu Ile Trp Trp Leu Gln Tyr
        835                 840                 845

Phe Ile Thr Arg Ala Glu Ala His Leu Gln Val Trp Val Pro Pro Leu
    850                 855                 860

Asn Val Arg Gly Gly Arg Asp Ala Ile Ile Leu Leu Thr Cys Val Val
865                 870                 875                 880

His Pro Glu Leu Ile Phe Asp Ile Thr Lys Ile Leu Leu Ala Met Leu
                885                 890                 895

Gly Pro Leu Met Val Leu Gln Ala Gly Leu Thr Arg Val Pro Tyr Phe
            900                 905                 910

Val Arg Ala Gln Gly Leu Ile Arg Ala Cys Met Leu Val Arg Lys Val
        915                 920                 925

Ala Gly Gly His Tyr Val Gln Met Ala Leu Met Lys Leu Ala Ala Leu
    930                 935                 940

Thr Gly Thr Tyr Val Tyr Asp His Leu Thr Pro Leu Gln Asp Trp Ala
945                 950                 955                 960

His Ala Gly Leu Arg Asp Leu Ala Val Ala Val Glu Pro Val Val Phe
                965                 970                 975

Ser Asp Met Glu Thr Lys Val Ile Thr Trp Gly Ala Asp Thr Ala Ala
            980                 985                 990

Cys Gly Asp Ile Ile Ser Gly Leu  Pro Val Ser Ala Arg  Arg Gly Arg
        995                 1000                 1005

Glu Ile  Leu Leu Gly Pro Ala  Asp Arg Phe Gly Glu  Gln Gly Trp
    1010                 1015                 1020

Arg Leu  Leu Ala Pro Ile Thr  Ala Tyr Ala Gln Gln  Thr Arg Gly
    1025                 1030                 1035

Leu Leu  Gly Cys Ile Ile Thr  Ser Leu Thr Gly Arg  Asp Lys Asn
    1040                 1045                 1050

Gln Val  Glu Gly Glu Val Gln  Val Val Ser Thr Ala  Thr Gln Ser
    1055                 1060                 1065

Phe Leu  Ala Thr Cys Val Asn  Gly Val Cys Trp Thr  Val Tyr His
    1070                 1075                 1080

Gly Ala  Gly Ser Lys Thr Leu  Ala Gly Pro Lys Gly  Pro Ile Thr
    1085                 1090                 1095

Gln Met  Tyr Thr Asn Val Asp  Gln Asp Leu Val Gly  Trp Pro Ala
    1100                 1105                 1110

Pro Pro  Gly Ala Arg Ser Leu  Thr Pro Cys Thr Cys  Gly Ser Ser
    1115                 1120                 1125

Asp Leu  Tyr Leu Val Thr Arg  His Ala Asp Val Ile  Pro Val Arg
    1130                 1135                 1140

Arg Arg  Gly Asp Ser Arg Gly  Ser Leu Leu Ser Pro  Arg Pro Ile
    1145                 1150                 1155

Ser Tyr Leu Lys Gly Ser Ser Gly Gly Pro Leu Leu Cys Pro Leu
    1160                1165                1170

Gly His Ala Val Gly Ile Phe Arg Ala Ala Val Cys Thr Arg Gly
    1175                1180                1185

Val Ala Lys Ala Val Asp Phe Val Pro Val Glu Ser Met Glu Thr
    1190                1195                1200

Thr Met Arg Ser Pro Val Phe Thr Asp Asn Ser Ser Pro Pro Ala
    1205                1210                1215

Val Pro Gln Thr Phe Gln Val Ala His Leu His Ala Pro Thr Gly
    1220                1225                1230

Ser Gly Lys Ser Thr Lys Val Pro Ala Ala Tyr Ala Ala Gln Gly
    1235                1240                1245

Tyr Lys Val Leu Val Leu Asn Pro Ser Val Ala Ala Thr Leu Gly
    1250                1255                1260

Phe Gly Ala Tyr Met Ser Lys Ala His Gly Val Glu Pro Asn Ile
    1265                1270                1275

Arg Thr Gly Val Arg Thr Ile Thr Thr Gly Ala Ser Ile Thr Tyr
    1280                1285                1290

Ser Thr Tyr Gly Lys Phe Leu Ala Asp Gly Gly Cys Ser Gly Gly
    1295                1300                1305

Ala Tyr Asp Ile Ile Ile Cys Asp Glu Cys His Ser Thr Asp Ser
    1310                1315                1320

Thr Ser Ile Leu Gly Ile Gly Thr Val Leu Asp Gln Ala Glu Thr
    1325                1330                1335

Ala Gly Ala Arg Leu Val Val Leu Ala Thr Ala Thr Pro Pro Gly
    1340                1345                1350

Ser Val Thr Val Pro His Pro Asn Ile Glu Glu Val Ala Leu Pro
    1355                1360                1365

Ser Thr Gly Glu Ile Pro Phe Tyr Gly Lys Ala Ile Pro Ile Glu
    1370                1375                1380

Thr Ile Lys Gly Gly Arg His Leu Ile Phe Cys His Ser Lys Lys

1385                1390                1395

Lys Cys Asp Glu Leu Ala Ala Lys Leu Val Gly Leu Gly Val Asn
    1400                1405                1410

Ala Val Ala Tyr Tyr Arg Gly Leu Asp Val Ser Val Ile Pro Thr
    1415                1420                1425

Ser Gly Asp Val Val Val Val Ala Thr Asp Ala Leu Met Thr Gly
    1430                1435                1440

Phe Thr Gly Asp Phe Asp Ser Val Ile Asp Cys Asn Thr Cys Val
    1445                1450                1455

Thr Gln Thr Val Asp Phe Ser Leu Asp Pro Thr Phe Thr Ile Glu
    1460                1465                1470

Thr Thr Thr Val Pro Gln Asp Ala Val Ser Arg Ser Gln Arg Arg
    1475                1480                1485

Gly Arg Thr Gly Arg Gly Arg Met Gly Ile Tyr Arg Phe Val Ala
    1490                1495                1500

Pro Gly Glu Arg Pro Ser Gly Met Phe Asp Ser Ser Val Leu Cys
    1505                1510                1515

Glu Cys Tyr Asp Ala Gly Cys Ala Trp Tyr Glu Leu Thr Pro Ala
    1520                1525                1530

Glu Thr Ser Val Arg Leu Arg Ala Tyr Leu Asn Thr Pro Gly Leu
    1535                1540                1545

Pro Val Cys Gln Asp His Leu Glu Phe Trp Glu Gly Val Phe Thr
    1550                1555                1560

Gly Leu Thr His Ile Asp Ala His Phe Leu Ser Gln Thr Lys Gln
    1565                1570                1575

Ala Gly Asp Asn Phe Pro Tyr Leu Val Ala Tyr Gln Ala Thr Val
    1580                1585                1590

Cys Ala Arg Ala Gln Ala Pro Pro Pro Ser Trp Asp Gln Met Trp
    1595                1600                1605

Lys Cys Leu Ile Arg Leu Lys Pro Thr Leu His Gly Pro Thr Pro
    1610                1615                1620

Leu Leu Tyr Arg Leu Gly Ala Val Gln Asn Glu Val Ile Leu Thr
    1625                1630                1635

His Pro Ile Thr Lys Tyr Ile Met Ala Cys Met Ser Ala Asp Leu
    1640                1645                1650

Glu Val Val Thr Ser Thr Trp Val Leu Val Gly Gly Val Leu Ala
    1655                1660                1665

Ala Leu Ala Ala Tyr Cys Leu Thr Thr Gly Ser Val Val Ile Val
    1670                1675                1680

Gly Arg Ile Ile Leu Ser Gly Lys Pro Ala Ile Ile Pro Asp Arg
    1685                1690                1695

Glu Val Leu Tyr Arg Glu Phe Asp Glu Met Glu Glu Cys Ala Ser
    1700                1705                1710

His Leu Pro Tyr Ile Glu Gln Gly Met Gln Leu Ala Glu Gln Phe
    1715                1720                1725

Lys Gln Lys Ala Leu Gly Leu Leu Gln Thr Ala Thr Lys Gln Ala
    1730                1735                1740

Glu Ala Ala Ala Pro Val Val Glu Ser Lys Trp Arg Ala Leu Glu
    1745                1750                1755

Ala Phe Trp Ala Lys His Met Trp Asn Phe Ile Ser Gly Ile Gln
    1760                1765                1770

Tyr Leu Ala Gly Leu Ser Thr Leu Pro Gly Asn Pro Ala Ile Ala
    1775                1780                1785

Ser Leu Met Ala Phe Thr Ala Ser Ile Thr Ser Pro Leu Thr Thr
    1790                1795                1800

Leu His Thr Leu Leu Phe Asn Ile Leu Gly Gly Trp Val Ala Ala
    1805                1810                1815

Gln Leu Ala Pro Pro Gly Ala Ala Ser Ala Phe Val Gly Ala Gly
    1820                1825                1830

Ile Ala Gly Ala Ala Val Gly Ser Ile Gly Leu Gly Lys Val Leu
    1835                1840                1845

Val Asp Ile Leu Ala Gly Tyr Gly Ala Gly Val Ala Gly Ala Leu
    1850                1855                1860

Val Ala Phe Lys Val Met Ser Gly Glu Met Pro Ser Thr Glu Asp
    1865                1870                1875

Leu Val Asn Leu Leu Pro Ala Ile Leu Ser Pro Gly Ala Leu Val
    1880                1885                1890

Val Gly Val Val Cys Ala Ala Ile Leu Arg Arg His Val Gly Pro
    1895                1900                1905

Gly Glu Gly Ala Val Gln Trp Met Asn Arg Leu Ile Ala Phe Ala
    1910                1915                1920

Ser Arg Gly Asn His Val Ser Pro Thr His Tyr Val Pro Glu Ser
    1925                1930                1935

Asp Ala Ala Ala Arg Val Thr Gln Ile Leu Ser Ser Leu Thr Ile
    1940                1945                1950

Thr Gln Leu Leu Lys Arg Leu His Gln Trp Ile Asn Lys Asp Cys
    1955                1960                1965

Ser Thr Pro Cys Ser Gly Ser Trp Leu Arg Asp Val Trp Asp Trp
    1970                1975                1980

Ile Cys Thr Val Leu Ser Asp Phe Lys Thr Trp Leu Gln Ser Lys
    1985                1990                1995

Leu Leu Pro Arg Leu Pro Gly Val Pro Phe Leu Ser Cys Gln Arg
    2000                2005                2010

Gly Tyr Lys Gly Val Trp Arg Gly Asp Gly Ile Met Gln Thr Ser
    2015                2020                2025

Cys Pro Cys Gly Ala Gln Ile Ala Gly His Val Lys Asn Gly Ser
    2030                2035                2040

Met Arg Ile Val Gly Pro Lys Thr Cys Ser Asn Thr Trp His Gly
    2045                2050                2055

Thr Phe Pro Ile Asn Ala His Thr Thr Gly Pro Cys Thr Pro Ser
    2060                2065                2070

Pro Ala Pro Asn Tyr Ser Lys Ala Leu Trp Arg Val Ala Ala Glu
    2075                2080                2085

Glu Tyr Val Glu Val Thr Arg Val Gly Asp Phe His Tyr Val Thr
    2090                2095                2100

Gly Met Thr Thr Asp Asn Val Lys Cys Pro Cys Gln Val Pro Ala
    2105                2110                2115

Pro Glu Phe Phe Thr Glu Val Asp Gly Val Arg Leu His Arg Tyr
    2120                2125                2130

Ala Pro Ala Cys Lys Pro Leu Leu Arg Asp Glu Val Thr Phe Gln
    2135                2140                2145

Val Gly Leu Asn Gln Phe Pro Val Gly Ser Gln Leu Pro Cys Glu
    2150                2155                2160

Pro Glu Pro Asp Val Ser Val Leu Thr Ser Met Leu Thr Asp Pro
    2165                2170                2175

Ser His Ile Thr Ala Glu Thr Ala Lys Arg Arg Leu Ala Arg Gly
    2180                2185                2190

Ser Ser Pro Ser Leu Ala Ser Ser Ser Ala Ser Gln Leu Ser Ala
    2195                2200                2205

Pro Ser Leu Lys Ala Thr Cys Thr Thr His His Asp Ser Pro Asp
    2210                2215                2220

Ala Asp Leu Ile Glu Ala Asn Leu Leu Trp Arg Gln Glu Met Gly
    2225                2230                2235

Gly Asn Ile Thr Arg Val Glu Ser Glu Asn Lys Val Val Ile Leu
    2240                2245                2250

Asp Ser Phe Asp Pro Leu Arg Ala Glu Glu Asp Glu Arg Glu Val
    2255                2260                2265

Ser Val Ala Ala Glu Ile Leu Arg Lys Thr Arg Lys Phe Pro Pro
    2270                2275                2280

Ala Met Pro Ile Trp Ala Arg Pro Asp Tyr Asn Pro Pro Leu Leu
    2285                2290                2295

Glu Thr Trp Lys Asp Pro Asp Tyr Val Pro Pro Val Val His Gly

2300                2305                2310

Cys Pro Leu Pro Pro Thr Lys Thr Pro Pro Ile Pro Pro Pro Arg
    2315                2320                2325

Arg Lys Lys Thr Val Val Leu Thr Glu Ser Thr Val Ser Ser Ala
    2330                2335                2340

Leu Ala Glu Leu Ala Thr Lys Thr Phe Gly Ser Ser Gly Ser Ser
    2345                2350                2355

Ala Val Asp Ser Gly Thr Ala Thr Ala Pro Pro Asn Gln Leu Ser
    2360                2365                2370

Asp Glu Val Asp Thr Gly Ser Asp Val Glu Ser Tyr Ser Ser Met
    2375                2380                2385

Pro Pro Leu Glu Gly Glu Pro Gly Asp Pro Asp Leu Ser Asp Gly
    2390                2395                2400

Ser Trp Ser Thr Val Ser Glu Glu Ala Gly Glu Asp Val Val Cys
    2405                2410                2415

Cys Ser Met Ser Tyr Thr Trp Thr Gly Ala Leu Ile Thr Pro Cys
    2420                2425                2430

Ala Ala Glu Glu Ser Lys Leu Pro Ile Asn Ala Leu Ser Asn Ser
    2435                2440                2445

Leu Leu Arg His His Asn Met Val Tyr Ala Thr Thr Ser Arg Ser
    2450                2455                2460

Ala Ser Gln Arg Gln Lys Lys Val Thr Phe Asp Arg Leu Gln Val
    2465                2470                2475

Leu Asp Asp His Tyr Arg Asp Val Leu Lys Glu Met Lys Ala Lys
    2480                2485                2490

Ala Ser Thr Val Lys Ala Lys Leu Leu Ser Val Glu Glu Ala Cys
    2495                2500                2505

Lys Leu Thr Pro Pro His Ser Ala Arg Ser Lys Phe Gly Tyr Gly
    2510                2515                2520

Ala Lys Asp Val Arg Asn Leu Ser Ser Lys Ala Val Asn His Ile
    2525                2530                2535

Asn Ser Val Trp Lys Asp Leu Leu Glu Asp Thr Glu Thr Pro Ile
    2540                2545                2550

Asp Thr Thr Ile Met Ala Lys Asn Glu Val Phe Cys Val Gln Pro
    2555                2560                2565

Glu Lys Gly Gly Arg Lys Pro Ala Arg Leu Ile Val Tyr Pro Asp
    2570                2575                2580

Leu Gly Val Arg Val Cys Glu Lys Met Ala Leu Tyr Asp Val Val
    2585                2590                2595

Ser Thr Leu Pro Gln Ala Val Met Gly Ser Ser Tyr Gly Phe Gln
    2600                2605                2610

Tyr Ser Pro Gly Gln Arg Val Glu Phe Leu Val Asn Ala Trp Lys
    2615                2620                2625

Ser Lys Lys Asn Pro Met Gly Phe Ala Tyr Asp Thr Arg Cys Phe
    2630                2635                2640

Asp Ser Thr Val Thr Glu Asn Asp Ile Arg Val Glu Glu Ser Ile
    2645                2650                2655

Tyr Gln Cys Cys Asp Leu Ala Pro Glu Ala Arg Gln Val Ile Arg
    2660                2665                2670

Ser Leu Thr Glu Arg Leu Tyr Val Gly Gly Pro Leu Thr Asn Ser
    2675                2680                2685

Lys Gly Gln Asn Cys Gly Tyr Arg Arg Cys Arg Ala Ser Gly Val
    2690                2695                2700

Leu Thr Thr Ser Cys Gly Asn Thr Leu Thr Cys Tyr Leu Lys Ala
    2705                2710                2715

Ser Ala Ala Cys Arg Ala Ala Lys Leu Gln Asp Cys Thr Met Leu
    2720                2725                2730

Val Cys Gly Asp Asp Leu Val Val Ile Cys Glu Ser Ala Gly Thr
    2735                2740                2745

Gln Glu Asp Ala Ala Ser Leu Arg Val Phe Thr Glu Ala Met Thr
    2750                2755                2760

Arg Tyr Ser Ala Pro Pro Gly Asp Pro Pro Arg Pro Glu Tyr Asp
    2765                2770                2775

Leu Glu Leu Ile Thr Ser Cys Ser Ser Asn Val Ser Val Ala His
    2780                2785                2790

Asp Ala Ser Gly Lys Arg Val Tyr Tyr Leu Thr Arg Asp Pro Thr
    2795                2800                2805

Thr Pro Leu Ala Arg Ala Ala Trp Glu Thr Ala Lys His Thr Pro
    2810                2815                2820

Val Asn Ser Trp Leu Gly Asn Ile Ile Met Tyr Ala Pro Thr Leu
    2825                2830                2835

Trp Ala Arg Met Ile Leu Met Thr His Phe Phe Ser Ile Leu Leu
    2840                2845                2850

Ala Gln Glu Gln Leu Glu Lys Ala Leu Asp Cys Gln Ile Tyr Gly
    2855                2860                2865

Ala Thr Tyr Ser Ile Glu Pro Leu Asp Leu Pro Gln Ile Ile Gln
    2870                2875                2880

Arg Leu His Gly Leu Ser Ala Phe Ser Leu His Ser Tyr Ser Pro
    2885                2890                2895

Gly Glu Ile Asn Arg Val Ala Ser Cys Leu Arg Lys Leu Gly Val
    2900                2905                2910

Pro Pro Leu Arg Val Trp Arg His Arg Ala Arg Ser Val Arg Ala
    2915                2920                2925

Lys Leu Leu Ser Gln Gly Gly Arg Ala Ala Thr Cys Gly Lys Tyr
    2930                2935                2940

Leu Phe Asn Trp Ala Val Arg Thr Lys Leu Lys Leu Thr Pro Ile
    2945                2950                2955

Pro Ala Ala Ser Gln Leu Asp Leu Ser Gly Trp Phe Ile Ala Gly
    2960                2965                2970

Tyr Ser Gly Gly Asp Ile Tyr His Ser Leu Ser Arg Ala Arg Pro
    2975                2980                2985

Arg Trp Phe Met Leu Cys Leu Leu Leu Leu Ser Val Gly Val Gly
    2990                2995                3000

Ile Tyr Leu Leu Pro Asn Arg
    3005                3010

<210> 5
<211> 783
<212> RNA
<213> Hepatitis C virus

<400> 5
gccucacacc uccccuacau cgaacaggga augcagcucg ccgaacaauu caagcagaag 60

gcgcucgggu ugcugcagac agccaccaag caagcggaag ccgcugcucc ugugguggag 120

uccaaguggc gagcccuuga ggccuucugg gcgaagcaca uguggaauuu caucagcggg 180

auacaguacu uagcaggcuu guccacucug ccugggaacc ccgcgauagc aucacugaug 240

gcauucacag ccucuaucac cagcccgcuu accacccaac acacccuccu guuuaacauc 300

uugggaggau ggguggccgc ccaacuugcc ccccccggug cugccucggc uuucgugggc 360

gccggcauug cuggcgcagc uguuggcagc auaggccuug ggaaggugcu uguggacauc 420

cuggcggguu auggagcagg gguggcaggc gcgcucgugg ccuucaaggu caugagcggc 480

gagaugcccu ccaccgagga ccuggucaac uuacucccug ccauccucuc uccuggugcc 540

cuugucgucg gggucgugug cgcagcaaua cugcgucggc augugggccc gggggagggg 600

gcugugcaau ggaugaaccg gcugauagcg uucgccucgc gggguaacca cgucuccccc 660

acgcacuaug ugccugagag cgacgcugca gcgcguguca cacagauccu cucuagccuc 720

accaucacuc agcuacugaa gaggcuccac caguggauua augaggacug cuccacacca 780

ugc 783

<210> 6
<211> 261
<212> PRT
<213> Hepatitis C virus

<400> 6

Ala Ser His Leu Pro Tyr Ile Glu Gln Gly Met Gln Leu Ala Glu Gln
1               5                   10                  15

Phe Lys Gln Lys Ala Leu Gly Leu Leu Gln Thr Ala Thr Lys Gln Ala
            20                  25                  30

Glu Ala Ala Ala Pro Val Val Glu Ser Lys Trp Arg Ala Leu Glu Ala
        35                  40                  45

Phe Trp Ala Lys His Met Trp Asn Phe Ile Ser Gly Ile Gln Tyr Leu
    50                  55                  60

Ala Gly Leu Ser Thr Leu Pro Gly Asn Pro Ala Ile Ala Ser Leu Met
65                  70                  75                  80

Ala Phe Thr Ala Ser Ile Thr Ser Pro Leu Thr Thr Gln His Thr Leu
                85                  90                  95

Leu Phe Asn Ile Leu Gly Gly Trp Val Ala Ala Gln Leu Ala Pro Pro
            100                 105                 110

Gly Ala Ala Ser Ala Phe Val Gly Ala Gly Ile Ala Gly Ala Ala Val
        115                 120                 125

Gly Ser Ile Gly Leu Gly Lys Val Leu Val Asp Ile Leu Ala Gly Tyr
    130                 135                 140

Gly Ala Gly Val Ala Gly Ala Leu Val Ala Phe Lys Val Met Ser Gly
145                 150                 155                 160

Glu Met Pro Ser Thr Glu Asp Leu Val Asn Leu Leu Pro Ala Ile Leu
                165                 170                 175

Ser Pro Gly Ala Leu Val Val Gly Val Val Cys Ala Ala Ile Leu Arg
            180                 185                 190

Arg His Val Gly Pro Gly Glu Gly Ala Val Gln Trp Met Asn Arg Leu
        195                 200                 205

Ile Ala Phe Ala Ser Arg Gly Asn His Val Ser Pro Thr His Tyr Val
    210                 215                 220

Pro Glu Ser Asp Ala Ala Ala Arg Val Thr Gln Ile Leu Ser Ser Leu
225                 230                 235                 240

Thr Ile Thr Gln Leu Leu Lys Arg Leu His Gln Trp Ile Asn Glu Asp
                245                 250                 255

Cys Ser Thr Pro Cys
            260

<210>  7
<211>  783
<212>  RNA
<213>  Hepatitis C virus

<400> 7
gccucacacc uccccuacau cgaacaggga augcagcucg ccgaacaauu caagcagaag 60

gcgcucgggu ugcugcagac agccaccaag caagcggaag ccgcugcucc ugugguggag 120

uccaaguggc gagcccuuga ggccuucugg gcgaagcaca uguggaauuu caucagcggg 180

auacaguacu uagcaggcuu guccacucug ccugggaacc ccgcgauagc aucacugaug 240

gcauucacag ccucuaucac cagcccgcuu accacccuac acacccuccu guuuaacauc 300

uugggaggau ggguggccgc ccaacuugcc ccccccggug cugccucggc uuucgugggc 360

gccggcauug cuggcgcagc uguuggcagc auaggccuug ggaaggugcu uguggacauc 420

cuggcggguu auggagcagg gguggcaggc gcgcucgugg ccuucaaggu caugagcggc 480

gagaugcccu ccaccgagga ccuggucaac uuacucccug ccauccucuc uccuggugcc 540

cuugucgucg gggucgugug cgcagcaaua cugcgucggc augugggccc gggggagggg 600

gcugugcaau ggaugaaccg gcugauagcg uucgccucgc gggguaacca cgucuccccc 660

acgcacuaug ugccugagag cgacgcugca gcgcguguca cacagauccu cucuagccuc 720

accaucacuc agcuacugaa gaggcuccac caguggauua auaaggacug cuccacacca 780

ugc 783

<210> 8
<211> 261
<212> PRT
<213> Hepatitis C virus

<400> 8

Ala Ser His Leu Pro Tyr Ile Glu Gln Gly Met Gln Leu Ala Glu Gln
1               5                   10                  15

Phe Lys Gln Lys Ala Leu Gly Leu Leu Gln Thr Ala Thr Lys Gln Ala
            20                  25                  30

Glu Ala Ala Ala Pro Val Val Glu Ser Lys Trp Arg Ala Leu Glu Ala
        35                  40                  45

Phe Trp Ala Lys His Met Trp Asn Phe Ile Ser Gly Ile Gln Tyr Leu
    50                  55                  60

Ala Gly Leu Ser Thr Leu Pro Gly Asn Pro Ala Ile Ala Ser Leu Met
65                  70                  75                  80

Ala Phe Thr Ala Ser Ile Thr Ser Pro Leu Thr Thr Leu His Thr Leu
                85                  90                  95

Leu Phe Asn Ile Leu Gly Gly Trp Val Ala Ala Gln Leu Ala Pro Pro
            100                 105                 110

Gly Ala Ala Ser Ala Phe Val Gly Ala Gly Ile Ala Gly Ala Ala Val
        115                 120                 125

Gly Ser Ile Gly Leu Gly Lys Val Leu Val Asp Ile Leu Ala Gly Tyr
    130                 135                 140

Gly Ala Gly Val Ala Gly Ala Leu Val Ala Phe Lys Val Met Ser Gly
145                 150                 155                 160

Glu Met Pro Ser Thr Glu Asp Leu Val Asn Leu Leu Pro Ala Ile Leu
                165                 170                 175

Ser Pro Gly Ala Leu Val Val Gly Val Val Cys Ala Ala Ile Leu Arg
            180                 185                 190

Arg His Val Gly Pro Gly Glu Gly Ala Val Gln Trp Met Asn Arg Leu
        195                 200                 205

Ile Ala Phe Ala Ser Arg Gly Asn His Val Ser Pro Thr His Tyr Val
    210                 215                 220

Pro Glu Ser Asp Ala Ala Ala Arg Val Thr Gln Ile Leu Ser Ser Leu
225                 230                 235                 240

Thr Ile Thr Gln Leu Leu Lys Arg Leu His Gln Trp Ile Asn Lys Asp
                245                 250                 255

Cys Ser Thr Pro Cys
            260

<210>  9
<211>  8010
<212>  RNA
<213>  Artificial

<220>
<223>  subgenomic HCV RNA replicon

<400>  9
   uaauacgacu cacuauagcc agcccccgau ugggggcgac acuccaccau agaucacucc      60
   ccugugagga acuacugucu ucacgcagaa agcgucuagc cauggcguua guaugagugu     120
   cgugcagccu ccaggacccc cccucccggg agagccauag uggucugcgg aaccggugag     180
   uacaccggaa uugccaggac gaccgggucc uuucuuggau caacccgcuc aaugccugga     240
   gauuugggcg ugcccccgcg agacugcuag ccgaguagug uugggucgcg aaaggccuug 300
   ugguacugcc ugauagggug cuugcgagug ccccgggagg ucucguagac cgugcaccau 360
   gagcacgaau ccuaaaccuc aaagaaaaac caaacguaac accaacgggc gcgccaugau 420
   ugaacaagau ggauugcacg cagguucucc ggccgcuugg guggagaggc uauucggcua 480
   ugacugggca caacagacaa ucggcugcuc ugaugccgcc guguuccggc ugucagcgca 540
   ggggcgcccg guucuuuuug ucaagaccga ccuguccggu gcccugaaug aacugcagga 600
   cgaggcagcg cggcuaucgu ggcuggccac gacgggcguu ccuugcgcag cugugcucga 660
   cguugucacu gaagcgggaa gggacuggcu gcuauugggc gaagugccgg ggcaggaucu 720
   ccugucaucu caccuugcuc cugccgagaa aguauccauc auggcugaug caaugcggcg 780
   gcugcauacg cuugauccgg cuaccugccc auucgaccac caagcgaaac aucgcaucga 840
   gcgagcacgu acucggaugg aagccggucu ugucgaucag gaugaucugg acgaagagca 900
   ucaggggcuc gcgccagccg aacuguucgc caggcucaag gcgcgcaugc ccgacggcga 960
   ggaucucguc gugacccaug gcgaugccug cuugccgaau aucauggugg aaaauggccg 1020
   cuuuucugga uucaucgacu guggccggcu ggguguggcg gaccgcuauc aggacauagc 1080
   guuggcuacc cgugauauug cugaagagcu uggcggcgaa ugggcugacc gcuuccucgu 1140
   gcuuuacggu aucgccgcuc ccgauucgca gcgcaucgcc uucuaucgcc uucuugacga 1200
   guucuucuga guuuaaacag accacaacgg uuucccucua gcgggaucaa uuccgccccu 1260
   cucccucccc ccccccuaac guuacuggcc gaagccgcuu ggaauaaggc cggugugcgu 1320
   uugucuauau guuauuuucc accauauugc cgucuuuugg caaugugagg gcccggaaac 1380
   cuuggcccug ucuucuugac gagcauuccu aggggucuuu ccccucucgc caaaggaaug 1440
   caaggucugu uggaugucgu gaaggaagca guuccucugg gaagcuucuu gaagacaaac 1500
   aacgucugua gcgacccuuu gcaggcagcg gaacccccca ccuggcgaca ggugccucug 1560
   cggccaaaag ccacguguau aagauacacc ugcaaaggcg guacaacccc agugccacgu 1620
   ugugaguugg auaguugugg aaagagucaa auggcucucc ucaagcguau ucaacaaggg 1680
   gcugaaggau gcccagaagg uaccccauug uaugggaucu gaucuggggc cucggugcac 1740
   augcucuaca uguguuuagu cgagguuaaa aaaacgucua ggccccccga accacgggga 1800
   cgugguuuuc cuuugaaaaa cacgauaaua ccauggcgcc uaucacggcu uacgcucaac 1860
   agacgcgggg ccuacuuggc uguaucauca ccagccucac aggccgggac aagaaccagg 1920
   ucgaggggga gguucaggug guuuccaccg caacgcaauc uuuccuggcg accugcguca 1980
   acggcgugug uuggacuguc uaccauggug ccggcucgaa gacccuggcc ggcccgaagg 2040
   gcccaaucac ccaaauguac accaaugugg accaagaccu cgucggcugg ccggcgcccc 2100
   ccggggcgcg cucccugaca ccgugcaccu gcggcagcuc ggaccucuac cuggucacga 2160
   ggcaugcuga ugucauuccg gugcgccggc ggggcgacag cagggggagu cuacucucuc 2220
   ccaggcccau cuccuacuua aagggcuccu cagguggucc acugcuuugc ccccuggggc 2280
   acgcuguggg caucuuccgg gccgcugugu gcacccgggg gguugcaaag gcgguggauu 2340
   uuguaccugu ugagucuaug gaaaccacca ugcggucucc ggucuuuacg gauaauucau 2400
   cucccccggc cguaccgcag acauuccaag uggcccaucu acacgcuccc acuggcagug 2460
   gcaagagcac uaaggugccg gcugcguacg cagcccaagg guacaaggua cucgucuuga 2520
   acccauccgu ugccgcuacc uuagguuuug gggcguacau gucuaaagca caugguguug 2580
   agccuaacau cagaacuggg guaaggacca ucaccacggg cgcuuccauc acguauucca 2640
   ccuacgguaa guuccuugcc gacggugguu gcucuggggg cgccuaugac aucauaauau 2700
   gugaugagug ccacucaacu gacucgacuu ccaucuuggg cauuggcaca guccuggacc 2760
   aagcggagac ggcuggagcg cggcucgucg ugcucgccac cgcuacgccu ccgggaucgg 2820
   ucaccgugcc acaucccaau aucgaggagg uggccuugcc cagcaccgga gaaauucccu 2880
   ucuacggcaa agccaucccc auugagacca ucaagggggg gaggcaccuc aucuucugcc 2940
   acuccaagaa gaaaugugac gagcucgcug caaagcuggu gggccucgga guuaacgcug 3000
   uugcguacua ccggggucuu gauguguccg ucauaccaac aagcggagau gucguugucg 3060
   uggcaacaga cgcucuaaug acgggcuuca ccggcgacuu ugacucagug aucgacugua 3120
   auacuugugu cacccagaca guugauuuca gcuuggaccc uaccuucacc auugagacga 3180
   caaccgugcc ccaagacgcg gugucgcguu cgcagcgacg aggcaggacu ggcaggggca 3240
   ggaugggcau auacagguuu guggcuccag gggaacggcc cucgggcaug uucgauucuu 3300
   cgguccugug ugagugcuau gacgcgggcu gugcuuggua ugagcucacg cccgccgaga 3360
   ccucagucag guugcgggcu uaccuaaaua caccagggcu gcccgucugc caggaccacc 3420
   uggaguuuug ggaggggguc uucacaggcc ucacccacau agaugcccau uucuuguccc 3480
   agacuaagca ggcaggagau aacuuccccu accugguagc auaccaggcu acggugugcg 3540
   ccagggccca ggcucccccu ccaucguggg aucaaaugug gaagugucuc auacggcuga 3600
   agccuacacu acacgggcca acgccccugu uguauaggcu aggagccguc cagaaugagg 3660
   ucauccucac acaucccaua accaaauaca ucauggcaug caugucggcu gaccuagagg 3720
   ucgucacuag caccugggug cuggucggcg ggguccuugc agcucuggcc gcguacugcc 3780
   ugacgacggg cagcgugguc auugugggca ggaucaucuu guccgggaag ccggcuauca 3840
   uuccugacag ggaaguccuc uaccgggagu ucgaugaaau ggaagagugu gccucacacc 3900
   uccccuacau cgaacaggga augcagcucg ccgaacaauu caagcagaag gcgcucgggu 3960
   ugcugcagac agccaccaag caagcggaag ccgcugcucc ugugguggag uccaaguggc 4020
   gagcccuuga ggccuucugg gcgaagcaca uguggaauuu caucagcggg auacaguacu 4080
   uagcaggcuu guccacucug ccugggaacc ccgcgauagc aucacugaug gcauucacag 4140
   ccucuaucac cagcccgcuu accacccaac acacccuccu guuuaacauc uugggaggau 4200
   ggguggccgc ccaacuugcc ccccccggug cugccucggc uuucgugggc gccggcauug 4260
   cuggcgcagc uguuggcagc auaggccuug ggaaggugcu uguggacauc cuggcggguu 4320
   auggagcagg gguggcaggc gcgcucgugg ccuucaaggu caugagcggc gagaugcccu 4380
   ccaccgagga ccuggucaac uuacucccug ccauccucuc uccuggugcc cuugucgucg 4440
   gggucgugug cgcagcaaua cugcgucggc augugggccc gggggagggg gcugugcaau 4500
   ggaugaaccg gcugauagcg uucgccucgc gggguaacca cgucuccccc acgcacuaug 4560
   ugccugagag cgacgcugca gcgcguguca cacagauccu cucuagccuc accaucacuc 4620
   agcuacugaa gaggcuccac caguggauua augaggacug cuccacacca ugcuccggcu 4680
   cguggcuuag ggacguuugg gacuggauau gcacgguuuu gagugacuuc aagaccuggc 4740
   uccaguccaa gcuccugcca cgguuaccgg gaguuccauu ccuuucaugc caacgugggu 4800
   auaagggggu cuggcgggga gauggcauca ugcagaccuc cugcccaugu ggagcacaaa 4860
   ucgccggaca ugucaagaac gguuccauga ggaucguugg gccuaaaacc uguagcaaca 4920
   cguggcacgg aacauucccc auuaacgcgc acaccacggg ccccugcaca cccuccccag 4980
   cgccgaacua cucuaaggcg uuguggcggg uggcugcuga ggaguacgug gaagucacgc 5040
   ggguggggga uuuccauuac gugacgggca ugaccacuga caacguaaaa ugcccaugcc 5100
   agguuccggc ccccgaguuc uucacagagg uggauggggu acggcugcac agguacgcuc 5160
   cggcgugcaa accucuccua cgggaugagg ucacauucca ggucgggcuc aaccaguucc 5220
   cgguuggguc acagcuccca ugcgagcccg aaccggaugu aucagugcuc acuuccaugc 5280
   uuaccgaccc uucccacauc acagcagaga cggcuaagcg uaggcuggcc agagggucuu 5340
   cccccucuuu ggccagcucu ucagcuaguc aguugucugc gcccucauug aaggcgacau 5400
   gcaccaccca ucaugacucc ccagacgcug accucauuga ggccaaccuc cuguggcggc 5460
   aggagauggg agggaacauc acccgugugg agucagagaa caagguggua auccuggacu 5520
   cuuuugaccc gcuucgagcg gaggaggacg agagggaggu gucuguugcg gcggagaucc 5580
   ugcggaaaac caggaaguuc cccccagcga ugcccauaug ggcacgcccg gacuacaacc 5640
   caccgcugcu agagacuugg aaggacccgg acuacguccc uccaguggug cacgggugcc 5700
   cauugccacc uaccaagacc ccuccaauac caccuccgcg gaggaaaaag acaguugucc 5760
   ugacagaguc caccgugucu ucugcccugg cggagcuugc cacaaagacc uuuggcagcu 5820
   ccggaucguc ggccgucgac agcggcacag cgaccgcccc cccuaaccag cucuccgacg 5880
   aaguggauac aggauccgac guugagucgu acuccuccau gcccccccuu gagggagagc 5940
   cgggggaccc cgaucucagc gacgggucuu ggucuacugu aagugaggag gcuggugagg 6000
   acgucgucug cugcucgaug uccuacacau ggacaggcgc cuugaucacg ccgugcgccg 6060
   cggaggagag caagcugccc aucaaugcgc ugagcaacuc uuugcugcgc caccacaaca 6120
   uggucuaugc cacaacaucc cgcagcgcaa gccaacggca gaaaaagguc accuuugaca 6180
   gacugcaagu ccuggacgac cauuaccggg acgugcucaa ggagaugaag gcgaaggcgu 6240
   ccacaguuaa ggcuaaacuu cuauccguag aagaggccug caagcugacg cccccacacu 6300
   cagccagguc caaauuuggc uauggggcga aggacguccg gaaccuaucc agcaaggccg 6360
   uuaaccacau caacuccgug uggaaggacu ugcuggaaga cacugagaca ccaauugaca 6420
   ccaccaucau ggcaaaaaau gaggucuucu guguucaacc agagaaggga ggccgcaagc 6480
   cagcucgccu uaucguauac ccagacuugg gggugcgugu gugcgagaaa auggcccuuu 6540
   acgacguggu cuccacucuu ccucaggccg ugaugggcuc cucauacgga uuccaguacu 6600
   cuccugggca gcgggucgag uuccugguga augccuggaa aucaaagaag aacccuaugg 6660
   gcuucgcaua ugacacccgc uguuuugacu caacggucac cgagaacgac auccguguug 6720
   aggagucaau uuaccaaugu ugugacuugg cccccgaggc cagacaggug auaaggucgc 6780
   ucacagagcg gcuuuauguc gggggccccc ugacuaauuc aaaagggcag aacugcgguu 6840
   aucgccggug ccgcgccagc ggcgugcuga cgacuagcug cgguaauacc cucacauguu 6900
   acuugaaggc cucugcagcc ugucgagcug caaagcucca ggacugcacg augcucgugu 6960
   gcggggacga ccuugucguu aucugugaaa gcgcggggac ccaggaggac gcggcgagcc 7020
   uacgagucuu cacggaggcu augacuaggu acuccgcccc ccccggggac ccgccccgac 7080
   cggaauacga cuuggaguug auaacaucau gcuccuccaa cgugucgguc gcgcacgaug 7140
   caucuggcaa acggguguau uaccucaccc gugaccccac caccccccuu gcgcgggcug 7200
   cgugggagac agcuaaacac acuccaguca acuccuggcu aggcaacauc aucauguaug 7260
   cgcccacccu cugggcaagg augauucuga ugacucacuu cuucuccauc cuucuagcuc 7320
   aggagcagcu ugaaaaagcc cuggauuguc agaucuacgg ggccacuuac uccauugaac 7380
   cacuugaccu accucagauc auucaacgac uccauggucu uagcgcauuc ucacuccaua 7440
   guuacucucc aggugaaauc aauagguggg cuucaugccu caggaaacuu gggguaccgc 7500
   ccuugcgagu cuggagacau cgggccagaa guguccgcgc uaagcuacug ucccaggggg 7560
   ggagggcugc cacuuguggc aaguaccucu ucaacugggc aguaaggacc aagcucaaac 7620
   ucacuccaau cccggcugcg ucccaguugg acuuguccgg cugguucauu gcugguuaca 7680
   gcgggggaga cauauaucac agccugucuc gugcccgacc ccgcugguuu auguugugcc 7740
   uacuccuacu uucugugggg guaggcaucu accugcuccc caaucgauga acggggggcu 7800
   aaacacucca ggccaauagg ccauucuguu uuuuuuuuuu uuuuuuuuuu uuuuuuuuuu 7860
   uuuuuuuuuu uuuuuuuuuu uuccuuuuuu uuuuuuuuuu ucccuuucuu uugguggcuc 7920
   caucuuagcc cuagucacgg cuagcuguga aagguccgug agccgcauga cugcagagag 7980
   ugcugauacu ggccucucug cagaucaugu 8010

<210> 10
<211> 9594
<212> RNA
<213> Hepatitis C virus

<400> 10
gccagccccc ugaugggggc gacacuccac cauagaucac uccccuguga ggaacuacug 60

ucuucacgca gaaagcgucu agccauggcg uuaguaugag ugucgugcag ccuccaggac 120

ccccccuccc gggagagcca uaguggucug cggaaccggu gaguacaccg gaauugccag 180

gacgaccggg uccuuucuug gaucaacccg cucaaugccu ggagauuugg gcgugccccc 240

gcgagacugc uagccgagua guguuggguc gcgaaaggcc uugugguacu gccugauagg 300

gugcuugcga gugccccggg aggucucgua gaccgugcau caugagcaca aauccuaaac 360

cucaaagaaa aaccaaacgu aacaccaacc gccgcccaca ggacgucaag uucccgggcg 420

guggucagau cguuggugga guuuaccugu ugccgcgcag gggccccagg uugggugugc 480

gcgcgacuag gaagacuucc gagcggucgc aaccucguga aaggcggcaa ccuaucccca 540

aggcucgccg ccccgagggc agggccuggg cucagcccgg guacccuugg ccccucuaug 600

gcaaugaggg ucuggggugg gcaggguggc uccugucacc ccgcggcucc cggccuaauu 660

ggggccccac ggacccccgg cguaggucgc guaauuuggg uaaggucauc gauacccuca 720

caugcggcuu cgccgaccuc augggguaca uuccgcucgu cggcgccccc cuaggaggcg 780

uugccagggc ccuggcgcau ggcguccggg uucuggagga cggcgugaac uaugcaacag 840

ggaauuugcc cgguugcucu uucucuaucu uccucuuggc uuugcugucc uguuugacug 900

ucccagcuuc cgcuuaugaa gugcgcaacg uguccgggac auaccauguc acgaacgacu 960

gcgccaacuc aagcauugug uaugaggcag cggacauaau caugcacacc ccugggugcg 1020

ugcccugcgu ccgggagggc aauuccuccc gcugcugggu agcgcucacu cccacgcucg 1080

cggccaggaa caggagcauc cccacuacga cgauacgacg ccacgucgau uugcucguug 1140

gggcggcugc cuucugcucc gccauguacg ugggggaucu cugcgggucu guuuuccuag 1200
ucucccaacu guucaccuuc ucaccucgcc gguaugagac gguacaagaa ugcaauugcu 1260
caaucuaucc cggccacgua ucaggucacc gcauggcuug ggauaugaug augaauuggu 1320
cgcccacagc agcucuagug gugucacagu uacuccgaau cccacaagcc gucguggaua 1380
ugguggcagg ggcccacugg gggguucugg cgggucuugc cuacuauucc auggugggga 1440
acugggcuaa agucuugauu gugaugcuac ucuuugccgg cguugacggg accaccaccg 1500
ugacaggggg agcggcggcc uuuggcacca ggagccuugc guccuucuuu acauuagggc 1560
cgucucagaa aauccaacuu guaaacacca acggcaguug gcacaucaau aggacugccu 1620
ugaauuguaa ugacucccuc cagacugggu uccuugcugc gcuguucuau gcacacaaga 1680
ucaacacguc uggaugccca gagcgcaugg ccagcugccg cccuauugac aaguucgcuc 1740
aggggugggg ucccaucacc cauggugcgc cugacaccuc ggaccagagg cccuacugcu 1800
ggcacuacgc accucgaccg ugugguaucg uacccgcguc ggaggugugu ggcccagugu 1860
auuguuucac cccaagcccu guaguggugg ggacgaccga ucgcuucggc guccccacgu 1920
auagcugggg ggagaauaag acggacgugc ugcuccucaa caacacgcgg ccgccgcagg 1980
gcaauugguu cggcugcaca uggaugaaug gcaccggguu caccaagacg ugugggggcc 2040
ccccguguga uaucgggggg gucggcaaca acaccuugac cugccccacg gauugcuucc 2100
ggaagcaccc cgaggccacu uauaccaaau gcggcucggg gccuugguug acgccuaggu 2160
gucuaguuga cuacccauac aggcuuuggc acuaccccug cacugucaac uuuaccaucu 2220
ucaaggucag gauguaugug gggggcgugg agcacaggcu caacgcugcg ugcaacugga 2280
cucgaggaga gcgcugugac cuagaggaca gggacagauc agagcuuagc ccgcugcuac 2340
ugucuacaac agaguggcag auacugcccu guuccuucac cacccuaccg gcucugucca 2400
cuggcuugau ccaccuccau cagaacaucg uggacgugca auaccuguac gguguagggu 2460
cagcaguugu cuccguugug aucagauggg aguacguccu gcugcucuuc cuccuccugg 2520
cggacgcgcg cguuugugcc ugcuuaugga ugaugcuguu gauagcccag gcugaagccg 2580
ccuuggagaa ccugguaguc cucaacgcgg cguccguggc cggagcgcau ggcauccucu 2640
cuuuccuugu guucuucugc gcugccuggu acaucaaagg caagcuagug ccuggggugg 2700
cguaugcucu cuauggugua uggccgcugc uccugcuccu gcuggcguug ccacaacggg 2760
cguacgccau ggaccgggag auggcugcau cgugcggagg cgcgguuuuc gugggucugg 2820
uacucuugac cuugucacca cacuauaaag cauuccuugc caaguccaua uggugguuac 2880
aauauuuaau caccagggcc gaggcgcauu ugcaagugug gguccccccc cucaacguuc 2940

gggggggccg cgaugccauc auccuccuca cgugcgcggu ccacccagag cuaauuuuug 3000
acaucaccaa aauuuugcuc gccauauuug gcccgcucau ggugcuccag gcuggucuaa 3060
cuagagugcc guacuuugug cgcgcucaag ggcucauccg ugugugcaug uuggugcgga 3120
aagucgcugg gggucauuau auccaaaugg cucucaugag guuggccgca cugacgggca 3180
cguacguuua ugaucaucuu acuccgcugc gggacugggc ccaugcgggc uugcgagacc 3240
uugcgguggc aguugagccc gucguuuucu ccaacacaga gaccaagauu aucaccuggg 3300
gggcggacac cgcggcgugu ggggacauca ucuugggucu accugucucc gcccgaaggg 3360
ggagagaggu acuccuagga ccggccgaua acuuuggaga gcaagggugg cgacuccuug 3420
cgcccaucac ggccuacucc caacagacgc ggggcuuacu uggcuguauc aucaccggcc 3480
ucacaggucg agacaagaac caggucgaag gggagguuca ggugguuucc accgcaacac 3540
agucuuucuu ggcaaccugc gucaacggug uguguuggac ugucuaccau ggugccggcu 3600
caaagacccu agccggcccg aaggggccaa ucacccagau guacaccaac guagaccaag 3660
accucguugg cuggcaggcg cccccggggg cgcguuccau gacgccgugc accugcggca 3720
gcucggaccu uuacuugguc acgaggcaug cugaugucau uccggugcgc cggcggggug 3780
acagcagagg gagccuacuu ucccccaggc ccgucuccua cuugaagggc ucuucaggug 3840
guccacugcu cugccccuug gggcacgucg ugggcaucuu ucgggcugcc gugugcaccc 3900
gggggguugc gaaggcggug gacuucauac ccaucgaguc uauggaaacu accaugcggu 3960
cuccggucuu uacggauaau ucaucucccc cggccguacc gcagacuuuu caaguggccc 4020
aucugcacgc ccccacuggc agcggcaaga gcaccaaggu gccagcugcg uaugcagccc 4080
agggguacaa ggugcuuguc uugaauccau ccguugccgc caccuugggu uuuggggcgu 4140
auauguccaa ggcauacggu gucgacccua acauuagaac ugggguaagg accaucacca 4200
caggcgcucc caucacguac uccaccuacg gcaaguuccu ugccgacggu gguugcuccg 4260
ggggcgcuua cgacaucaua auaugcgaug agugccacuc aaccgacucg acuuccauuu 4320
ugggcauugg cacgguccug gaucaagcgg agacggcugg agcgcgacuc gucgugcucg 4380
ccaccgcuac gccuccggga ucggucacug ugccacaccc caacaucgag gagguggccu 4440
uguccaacac cggagagauu cccuucuaug gcaaagccau cccccucgag gucaucaagg 4500
gggggaggca ucucauuuuc ugucauucua agaagaagug ugaugagcuc gcugcaaagc 4560
ugucgggccu uggggucaac gcuguagcgu acuaccgggg ucuugaugug uccgucauac 4620
caacaagcgg ggacgucguu gucguggcaa cagacgcucu aaugacgggc uacaccggug 4680
acuuugacuc ugugaucgac uguaauacau gugucaccca gacagucgac uucagccugg 4740
accccaccuu caccauugag acgacgaccg ugccccaaga cgcagugucg cgcucgcagc 4800

ggcgagggag gacugguagg gguagagggg gcauauacag guuugugacu ccaggagagc 4860

ggcccucggg cauguucgau uccucggucc ugugugaaug cuaugacgcg ggcugugcuu 4920

gguacgagcu cacgcccgcc gagaccucgg uuaggcugcg ggcuuaccua aauacaccag 4980

gguugcccgu cugccaggac cauuuggagu ucugggaagg cgucuucaca ggccucacuc 5040

auauagaugc ccacuucuug ucucagacua agcaggcagg agacaacuuc cccuaccugg 5100

uggcauacca ggccacagug ugcgccaggg cccaggcacc accuccauca ugggaucaaa 5160

uguggaagug ucucauacgg cuaaaaccua cacuacacgg gccaacaccc cuguuguaca 5220

ggcuaggagc cguccaaaac gaggucaccc ucacacaccc caugaccaaa uacaucauga 5280

caugcauguc ggcugaccua gaggucguca ccagcacuug ggugcuggug ggcggggucc 5340

ucgcagcuuu ggccgcguac ugcuugacaa cgggcagcgu gguuaucaua ggcaggauca 5400

ucuuguccgg aaggccggcu aucauucccg auagggaagu ucucuaccag gaguucgaug 5460

aaauggaaga gugcgccuca caccuucccu acaucgaaca aggaaugcag cucgccgagc 5520

aauucaagca gaaggcgcuc ggguuguugc aaacggccac caagcaagcg gaggccgcug 5580

cucccguggu ggaguccaaa uggcaagccc uugaggccuu cugggcgaag cacaugugga 5640

acuucaucag cgggauacag uaucuagcag gcuuguccac ucugccugga aaccccgcga 5700

uagcaucgcu gauggcauuu acagccucua ucacuagccc gcucaccacc caacauaccc 5760

uucuauuuaa caucuugggg ggaugggugg ccgcccaacu cgcccccccc agcgcugcuu 5820

cagccuucgu aggcgccggc aucgccggcg cggcuguugg cagcauaggu cuugggaagg 5880

ugcucgugga cauccuagcg gguuauggag caggggugge aggcgcacuc guggccuuca 5940

aggucaugag cggcgaagug cccuccacug aggaccuggu caacuuacuc ccugccaucc 6000

ucuccccugg ugcccugguc gucggggucg ugugcgcagc gauacugcgu cggcaugugg 6060

gcccagggga gggggccgug caguggauga accggcugau agcguucgcu ucgcggggua 6120

accacgucuc ccccacgcac uaugugccug agagcgacgc cgcagcgcgu gucacccaga 6180

uccucuccag ccuuaccauc acucagcugc uaaagaggcu ccaccagugg auuaaugagg 6240

acuguuccac gccaugcucc gguucguggc ucagggaugu uugggacugg auaugcacgg 6300

uuuugaccga cuucaaaacc uggcuccagu ccaagcuccu gccacgguug ccgggacucc 6360

cuuucuuuuc augucaacgu ggauauaaag gagucuggcu gggagauggc guuaugcaaa 6420

cuaccugucc auguggugca caaaucagcg gacaugucaa aaacggcucc augaagaucg 6480

uggggccuaa aaccugcagc aacacguggc acgggacguu ccccaucaac gcauacacca 6540

caggccccug cacacccucc ccggcgccga acuauuccaa ggcguugugg cgaguggcug 6600

cugaggagua uguggagguc acgcgguggg gggauuucca cuacgugacg ggcaugacca 6660
cugacaacgu aaaaugccca ugccaggucc cggcccccga auucuucacg gaguuggaug 6720
gggugcggcu gcacagguac gcuccggcgu gcaagccucu cuuacgggau gaggucacau 6780
uccaggucgg gcucaaccag uauccgguug gaucacagcu uccaugugag cccgagccgg 6840
auguaacagu gcucacuucc augcucaccg accccuccca uauuacagca gagacggcua 6900
agcguaggcu ggccagaggg uccccccccu cuuuggccag cuccucagcu agccaguugu 6960
cugcgccuuc cuugaaggcg acaugcacua cccaccauga cuccccagau gcugaccuca 7020
ucgaggccaa ccuccugugg cggcaggaga ugggcgggaa caucacccgc guggagucag 7080
agaacaaggu aguaauucug gacucuuuug aaccgcuucg agcggaggag gaugauaggg 7140
aaguguccgu agcggcggag auccugcgga gaaccaggaa auuccccgca gcgaugccca 7200
uaugggcacg gccggacuac aacccaccac uccuagaguc uuggaagaac ccagacuacg 7260
ucccuccagu gguacacggg ugcccauuac caccuauuaa ggccccuccg auaccaccuc 7320
cacggagaaa gaggacgguc auccugacag aauccaccgu gucuucugcc uuggcggagc 7380
uugcuacaaa gaccuuuggc agcuccggau cgucggccgu ugacagcggc acagcgacug 7440
gcccuccuga ucaguccucc ggugacggag auacaggauc cgacgcugag ucgugcuccu 7500
ccaugccccc ccuugagggg gagccggggg accccgaucu cagcgacggg ucuuggucua 7560
ccgugagcga ggaggccggu gaggacgucg ucugcugcuc gauguccuac acauggacag 7620
gcgccuuaau cacaccgugc gccgcagagg agagcaagcu gccuaucaac gcguugagca 7680
acucuuugcu gcgccaccac aacauggucu augccacaac aucccgcagc gcgggccaac 7740
ggcagaagaa ggucacuuuu gacagagugc agguccugga cgaccauuac cgggacgugc 7800
ucaaggagau gaaggcgaag gcguccacag uuaaggcuaa acuucuaccu guagaagaag 7860
ccugcaggcu gacgccccca cacucggcca gauccaaguu uggcuauggg gcgaaggacg 7920
uccggaaccu auccagcaag gccgucaacc acauccacuc cguguggaag gacuugcugg 7980
aagacucuga gacgccgauu gacaccacca ucauggcaaa aaaugagguc uuuuguguuc 8040
aaccagagaa aggaggccgc aagucagcuc gucuuaucgu auucccagac uuggggguuc 8100
gugugugcga gaaaauggcc cuuuacgacg ugguuuccac ucuuccucag gccgugaugg 8160
gcuccucaua cggguuccag uacucuccug gacagcgggu cgaguuccug gugaaugccu 8220
ggaaaucaaa gaagaacccu augggcuucg cauaugacac ccgcuguuuu gacucaacgg 8280
ucacugagag ugacauccgu guugaggagu caauuuacca auguugugac uuggcccccg 8340
aggccagaca ggucauaagg ucgcucacgg agcggcuuua uaucgggggc ccccugacua 8400
auucaaaagg gcagagcugc ggcuaucgcc ggugccgcgc cagcggugug cugacgacca 8460

gcugcgguaa uacccucaca uguuacuuga aggcuucugc agccugucga gcugcaaagc 8520

uccaggacug cacaauguua gugugcggag acgaccuugu cguuaucugu gaaagugcgg 8580

gaacccagga ggacgcggcg agccuacgag ucuucacgga ggcuaugacu agguacucug 8640

ccccccccgg ggacccgccc cagccagaau acgacuugga gcugauaaca ucaugcuccu 8700

ccaacgucuc ggucgcgcac gaugcacuug gcaagcgggu guauuaucug acccgcgacc 8760

ccaccacccc ccuugcgcgg gcugcguggg agacagcaag acacacucca guuaacuccu 8820

ggcuaggcaa caucaucaug uaugcgccca cccuaugggc aaggaugauu cugaugaccc 8880

acuucuuuuc cauccuucua gcucaggaac aacuugaaaa agcccuagau ugucagaucu 8940

acggggccac uuacuccauu gagccacuug accuaccuca gaucauucag cgacuccacg 9000

gccuuagcgc auuuucacuc cauagcuacu cuccagguga gaucaauagg guggcuucau 9060

gccucaggaa acuuggggua ccacccuugc gagucuggag acaucgggcc agaagugucc 9120

gcgcuaagcu acugucccag ggggggaggg ccgccacuug uggcaaauac cucuucaacu 9180

gggcaguaag gaccaagcuc aaacucacuc caauuccggc ugcgucccag uuggacuugu 9240

ccggcugguu cauugcuggu uacagcgggg gagacauaua ucacagccug ucucgcgccc 9300

gaccccgcug guucaugugg ugccuacucc uacuuuccgu agggguaggc aucuaucugc 9360

uccccaaucg augaacgggg ggcuaaacac uccaggccaa uaggccauuc uguuuuuuuu 9420

uuuuuuuuuu uuuuuuuuuu uuuuuuuuuu uuuuuuuuuu uuuuuuccuu uuuuuuuuuu 9480

uuuuucccuu ucuuuuggug gcuccaucuu agcccuaguc acggcuagcu gugaaagguc 9540

cgugagccgc augacugcag agagugcuga uacuggccuc ucugcagauc augu 9594

<210> 11
<211> 3010
<212> PRT
<213> Hepatitis C virus

<400> 11

Met Ser Thr Asn Pro Lys Pro Gln Arg Lys Thr Lys Arg Asn Thr Asn
1               5                   10                  15

Arg Arg Pro Gln Asp Val Lys Phe Pro Gly Gly Gly Gln Ile Val Gly
            20                  25                  30

Gly Val Tyr Leu Leu Pro Arg Arg Gly Pro Arg Leu Gly Val Arg Ala
        35                  40                  45

Thr Arg Lys Thr Ser Glu Arg Ser Gln Pro Arg Glu Arg Arg Gln Pro
    50                  55                  60

Ile Pro Lys Ala Arg Arg Pro Glu Gly Arg Ala Trp Ala Gln Pro Gly
65                  70                  75                  80

Tyr Pro Trp Pro Leu Tyr Gly Asn Glu Gly Leu Gly Trp Ala Gly Trp
                85                  90                  95

Leu Leu Ser Pro Arg Gly Ser Arg Pro Asn Trp Gly Pro Thr Asp Pro
            100                 105                 110

Arg Arg Arg Ser Arg Asn Leu Gly Lys Val Ile Asp Thr Leu Thr Cys
        115                 120                 125

Gly Phe Ala Asp Leu Met Gly Tyr Ile Pro Leu Val Gly Ala Pro Leu
    130                 135                 140

Gly Gly Val Ala Arg Ala Leu Ala His Gly Val Arg Val Leu Glu Asp
145                 150                 155                 160

Gly Val Asn Tyr Ala Thr Gly Asn Leu Pro Gly Cys Ser Phe Ser Ile
                165                 170                 175

Phe Leu Leu Ala Leu Leu Ser Cys Leu Thr Val Pro Ala Ser Ala Tyr
            180                 185                 190

Glu Val Arg Asn Val Ser Gly Thr Tyr His Val Thr Asn Asp Cys Ala
        195                 200                 205

Asn Ser Ser Ile Val Tyr Glu Ala Ala Asp Ile Ile Met His Thr Pro
    210                 215                 220

Gly Cys Val Pro Cys Val Arg Glu Gly Asn Ser Ser Arg Cys Trp Val
225                 230                 235                 240

Ala Leu Thr Pro Thr Leu Ala Ala Arg Asn Arg Ser Ile Pro Thr Thr
                245                 250                 255

Thr Ile Arg Arg His Val Asp Leu Leu Val Gly Ala Ala Ala Phe Cys
            260                 265                 270

Ser Ala Met Tyr Val Gly Asp Leu Cys Gly Ser Val Phe Leu Val Ser
        275                 280                 285

Gln Leu Phe Thr Phe Ser Pro Arg Arg Tyr Glu Thr Val Gln Glu Cys
    290                 295                 300

Asn Cys Ser Ile Tyr Pro Gly His Val Ser Gly His Arg Met Ala Trp
305                 310                 315                 320

Asp Met Met Met Asn Trp Ser Pro Thr Ala Ala Leu Val Val Ser Gln
                325                 330                 335

Leu Leu Arg Ile Pro Gln Ala Val Val Asp Met Val Ala Gly Ala His
            340                 345                 350

Trp Gly Val Leu Ala Gly Leu Ala Tyr Tyr Ser Met Val Gly Asn Trp
        355                 360                 365

Ala Lys Val Leu Ile Val Met Leu Leu Phe Ala Gly Val Asp Gly Thr
    370                 375                 380

Thr Thr Val Thr Gly Gly Ala Ala Ala Phe Gly Thr Arg Ser Leu Ala
385                 390                 395                 400

Ser Phe Phe Thr Leu Gly Pro Ser Gln Lys Ile Gln Leu Val Asn Thr
                405                 410                 415

Asn Gly Ser Trp His Ile Asn Arg Thr Ala Leu Asn Cys Asn Asp Ser
            420                 425                 430

Leu Gln Thr Gly Phe Leu Ala Ala Leu Phe Tyr Ala His Lys Ile Asn
        435                 440                 445

Thr Ser Gly Cys Pro Glu Arg Met Ala Ser Cys Arg Pro Ile Asp Lys
    450                 455                 460

Phe Ala Gln Gly Trp Gly Pro Ile Thr His Gly Ala Pro Asp Thr Ser
465                 470                 475                 480

Asp Gln Arg Pro Tyr Cys Trp His Tyr Ala Pro Arg Pro Cys Gly Ile
                485                 490                 495

Val Pro Ala Ser Glu Val Cys Gly Pro Val Tyr Cys Phe Thr Pro Ser
            500                 505                 510

Pro Val Val Val Gly Thr Thr Asp Arg Phe Gly Val Pro Thr Tyr Ser
        515                 520                 525

Trp Gly Glu Asn Lys Thr Asp Val Leu Leu Leu Asn Asn Thr Arg Pro
    530                 535                 540

Pro Gln Gly Asn Trp Phe Gly Cys Thr Trp Met Asn Gly Thr Gly Phe
545                 550                 555                 560

Thr Lys Thr Cys Gly Gly Pro Pro Cys Asp Ile Gly Gly Val Gly Asn
                565                 570                 575

Asn Thr Leu Thr Cys Pro Thr Asp Cys Phe Arg Lys His Pro Glu Ala
            580                 585                 590

Thr Tyr Thr Lys Cys Gly Ser Gly Pro Trp Leu Thr Pro Arg Cys Leu
        595                 600                 605

Val Asp Tyr Pro Tyr Arg Leu Trp His Tyr Pro Cys Thr Val Asn Phe
    610                 615                 620

Thr Ile Phe Lys Val Arg Met Tyr Val Gly Gly Val Glu His Arg Leu
625                 630                 635                 640

Asn Ala Ala Cys Asn Trp Thr Arg Gly Glu Arg Cys Asp Leu Glu Asp
                645                 650                 655

Arg Asp Arg Ser Glu Leu Ser Pro Leu Leu Leu Ser Thr Thr Glu Trp
            660                 665                 670

Gln Ile Leu Pro Cys Ser Phe Thr Thr Leu Pro Ala Leu Ser Thr Gly
        675                 680                 685

Leu Ile His Leu His Gln Asn Ile Val Asp Val Gln Tyr Leu Tyr Gly
    690                 695                 700

Val Gly Ser Ala Val Val Ser Val Val Ile Arg Trp Glu Tyr Val Leu
705                 710                 715                 720

Leu Leu Phe Leu Leu Leu Ala Asp Ala Arg Val Cys Ala Cys Leu Trp
                725                 730                 735

Met Met Leu Leu Ile Ala Gln Ala Glu Ala Ala Leu Glu Asn Leu Val
            740                 745                 750

Val Leu Asn Ala Ala Ser Val Ala Gly Ala His Gly Ile Leu Ser Phe
        755                 760                 765

Leu Val Phe Phe Cys Ala Ala Trp Tyr Ile Lys Gly Lys Leu Val Pro
    770                 775                 780

Gly Val Ala Tyr Ala Leu Tyr Gly Val Trp Pro Leu Leu Leu Leu Leu

785                 790                 795                 800

Leu Ala Leu Pro Gln Arg Ala Tyr Ala Met Asp Arg Glu Met Ala Ala
                805                 810                 815

Ser Cys Gly Gly Ala Val Phe Val Gly Leu Val Leu Leu Thr Leu Ser
            820                 825                 830

Pro His Tyr Lys Ala Phe Leu Ala Lys Ser Ile Trp Trp Leu Gln Tyr
        835                 840                 845

Leu Ile Thr Arg Ala Glu Ala His Leu Gln Val Trp Val Pro Pro Leu
    850                 855                 860

Asn Val Arg Gly Gly Arg Asp Ala Ile Ile Leu Leu Thr Cys Ala Val
865                 870                 875                 880

His Pro Glu Leu Ile Phe Asp Ile Thr Lys Ile Leu Leu Ala Ile Phe
                885                 890                 895

Gly Pro Leu Met Val Leu Gln Ala Gly Leu Thr Arg Val Pro Tyr Phe
            900                 905                 910

Val Arg Ala Gln Gly Leu Ile Arg Val Cys Met Leu Val Arg Lys Val
        915                 920                 925

Ala Gly Gly His Tyr Ile Gln Met Ala Leu Met Arg Leu Ala Ala Leu
    930                 935                 940

Thr Gly Thr Tyr Val Tyr Asp His Leu Thr Pro Leu Arg Asp Trp Ala
945                 950                 955                 960

His Ala Gly Leu Arg Asp Leu Ala Val Ala Val Glu Pro Val Val Phe
                965                 970                 975

Ser Asn Thr Glu Thr Lys Ile Ile Thr Trp Gly Ala Asp Thr Ala Ala
            980                 985                 990

Cys Gly Asp Ile Ile Leu Gly Leu  Pro Val Ser Ala Arg  Arg Gly Arg
        995                 1000                 1005

Glu Val  Leu Leu Gly Pro Ala  Asp Asn Phe Gly Glu  Gln Gly Trp
    1010                 1015                 1020

Arg Leu  Leu Ala Pro Ile Thr  Ala Tyr Ser Gln Gln  Thr Arg Gly
    1025                 1030                 1035

Leu Leu Gly Cys Ile Ile Thr Gly Leu Thr Gly Arg Asp Lys Asn
    1040                1045                1050

Gln Val Glu Gly Glu Val Gln Val Val Ser Thr Ala Thr Gln Ser
    1055                1060                1065

Phe Leu Ala Thr Cys Val Asn Gly Val Cys Trp Thr Val Tyr His
    1070                1075                1080

Gly Ala Gly Ser Lys Thr Leu Ala Gly Pro Lys Gly Pro Ile Thr
    1085                1090                1095

Gln Met Tyr Thr Asn Val Asp Gln Asp Leu Val Gly Trp Gln Ala
    1100                1105                1110

Pro Pro Gly Ala Arg Ser Met Thr Pro Cys Thr Cys Gly Ser Ser
    1115                1120                1125

Asp Leu Tyr Leu Val Thr Arg His Ala Asp Val Ile Pro Val Arg
    1130                1135                1140

Arg Arg Gly Asp Ser Arg Gly Ser Leu Leu Ser Pro Arg Pro Val
    1145                1150                1155

Ser Tyr Leu Lys Gly Ser Ser Gly Gly Pro Leu Leu Cys Pro Leu
    1160                1165                1170

Gly His Val Val Gly Ile Phe Arg Ala Ala Val Cys Thr Arg Gly
    1175                1180                1185

Val Ala Lys Ala Val Asp Phe Ile Pro Ile Glu Ser Met Glu Thr
    1190                1195                1200

Thr Met Arg Ser Pro Val Phe Thr Asp Asn Ser Ser Pro Pro Ala
    1205                1210                1215

Val Pro Gln Thr Phe Gln Val Ala His Leu His Ala Pro Thr Gly
    1220                1225                1230

Ser Gly Lys Ser Thr Lys Val Pro Ala Ala Tyr Ala Ala Gln Gly
    1235                1240                1245

Tyr Lys Val Leu Val Leu Asn Pro Ser Val Ala Ala Thr Leu Gly
    1250                1255                1260

Phe Gly Ala Tyr Met Ser Lys Ala Tyr Gly Val Asp Pro Asn Ile
    1265                1270                1275

Arg Thr Gly Val Arg Thr Ile Thr Thr Gly Ala Pro Ile Thr Tyr
    1280                1285                1290

Ser Thr Tyr Gly Lys Phe Leu Ala Asp Gly Gly Cys Ser Gly Gly
    1295                1300                1305

Ala Tyr Asp Ile Ile Ile Cys Asp Glu Cys His Ser Thr Asp Ser
    1310                1315                1320

Thr Ser Ile Leu Gly Ile Gly Thr Val Leu Asp Gln Ala Glu Thr
    1325                1330                1335

Ala Gly Ala Arg Leu Val Val Leu Ala Thr Ala Thr Pro Pro Gly
    1340                1345                1350

Ser Val Thr Val Pro His Pro Asn Ile Glu Glu Val Ala Leu Ser
    1355                1360                1365

Asn Thr Gly Glu Ile Pro Phe Tyr Gly Lys Ala Ile Pro Leu Glu
    1370                1375                1380

Val Ile Lys Gly Gly Arg His Leu Ile Phe Cys His Ser Lys Lys
    1385                1390                1395

Lys Cys Asp Glu Leu Ala Ala Lys Leu Ser Gly Leu Gly Val Asn
    1400                1405                1410

Ala Val Ala Tyr Tyr Arg Gly Leu Asp Val Ser Val Ile Pro Thr
    1415                1420                1425

Ser Gly Asp Val Val Val Val Ala Thr Asp Ala Leu Met Thr Gly
    1430                1435                1440

Tyr Thr Gly Asp Phe Asp Ser Val Ile Asp Cys Asn Thr Cys Val
    1445                1450                1455

Thr Gln Thr Val Asp Phe Ser Leu Asp Pro Thr Phe Thr Ile Glu
    1460                1465                1470

Thr Thr Thr Val Pro Gln Asp Ala Val Ser Arg Ser Gln Arg Arg
    1475                1480                1485

Gly Arg Thr Gly Arg Gly Arg Gly Gly Ile Tyr Arg Phe Val Thr
    1490                1495                1500

Pro Gly Glu Arg Pro Ser Gly Met Phe Asp Ser Ser Val Leu Cys
    1505                1510                1515

Glu Cys Tyr Asp Ala Gly Cys Ala Trp Tyr Glu Leu Thr Pro Ala
    1520                1525                1530

Glu Thr Ser Val Arg Leu Arg Ala Tyr Leu Asn Thr Pro Gly Leu
    1535                1540                1545

Pro Val Cys Gln Asp His Leu Glu Phe Trp Glu Gly Val Phe Thr
    1550                1555                1560

Gly Leu Thr His Ile Asp Ala His Phe Leu Ser Gln Thr Lys Gln
    1565                1570                1575

Ala Gly Asp Asn Phe Pro Tyr Leu Val Ala Tyr Gln Ala Thr Val
    1580                1585                1590

Cys Ala Arg Ala Gln Ala Pro Pro Pro Ser Trp Asp Gln Met Trp
    1595                1600                1605

Lys Cys Leu Ile Arg Leu Lys Pro Thr Leu His Gly Pro Thr Pro
    1610                1615                1620

Leu Leu Tyr Arg Leu Gly Ala Val Gln Asn Glu Val Thr Leu Thr
    1625                1630                1635

His Pro Met Thr Lys Tyr Ile Met Thr Cys Met Ser Ala Asp Leu
    1640                1645                1650

Glu Val Val Thr Ser Thr Trp Val Leu Val Gly Gly Val Leu Ala
    1655                1660                1665

Ala Leu Ala Ala Tyr Cys Leu Thr Thr Gly Ser Val Val Ile Ile
    1670                1675                1680

Gly Arg Ile Ile Leu Ser Gly Arg Pro Ala Ile Ile Pro Asp Arg
    1685                1690                1695

Glu Val Leu Tyr Gln Glu Phe Asp Glu Met Glu Glu Cys Ala Ser
    1700                1705                1710

His Leu Pro Tyr Ile Glu Gln Gly Met Gln Leu Ala Glu Gln Phe

1715                 1720                 1725

Lys Gln Lys Ala Leu Gly Leu Leu Gln Thr Ala Thr Lys Gln Ala
    1730                 1735                 1740

Glu Ala Ala Ala Pro Val Val Glu Ser Lys Trp Gln Ala Leu Glu
    1745                 1750                 1755

Ala Phe Trp Ala Lys His Met Trp Asn Phe Ile Ser Gly Ile Gln
    1760                 1765                 1770

Tyr Leu Ala Gly Leu Ser Thr Leu Pro Gly Asn Pro Ala Ile Ala
    1775                 1780                 1785

Ser Leu Met Ala Phe Thr Ala Ser Ile Thr Ser Pro Leu Thr Thr
    1790                 1795                 1800

Gln His Thr Leu Leu Phe Asn Ile Leu Gly Gly Trp Val Ala Ala
    1805                 1810                 1815

Gln Leu Ala Pro Pro Ser Ala Ala Ser Ala Phe Val Gly Ala Gly
    1820                 1825                 1830

Ile Ala Gly Ala Ala Val Gly Ser Ile Gly Leu Gly Lys Val Leu
    1835                 1840                 1845

Val Asp Ile Leu Ala Gly Tyr Gly Ala Gly Val Ala Gly Ala Leu
    1850                 1855                 1860

Val Ala Phe Lys Val Met Ser Gly Glu Val Pro Ser Thr Glu Asp
    1865                 1870                 1875

Leu Val Asn Leu Leu Pro Ala Ile Leu Ser Pro Gly Ala Leu Val
    1880                 1885                 1890

Val Gly Val Val Cys Ala Ala Ile Leu Arg Arg His Val Gly Pro
    1895                 1900                 1905

Gly Glu Gly Ala Val Gln Trp Met Asn Arg Leu Ile Ala Phe Ala
    1910                 1915                 1920

Ser Arg Gly Asn His Val Ser Pro Thr His Tyr Val Pro Glu Ser
    1925                 1930                 1935

Asp Ala Ala Ala Arg Val Thr Gln Ile Leu Ser Ser Leu Thr Ile
    1940                 1945                 1950

Thr Gln Leu Leu Lys Arg Leu His Gln Trp Ile Asn Glu Asp Cys
    1955                1960                1965

Ser Thr Pro Cys Ser Gly Ser Trp Leu Arg Asp Val Trp Asp Trp
    1970                1975                1980

Ile Cys Thr Val Leu Thr Asp Phe Lys Thr Trp Leu Gln Ser Lys
    1985                1990                1995

Leu Leu Pro Arg Leu Pro Gly Leu Pro Phe Phe Ser Cys Gln Arg
    2000                2005                2010

Gly Tyr Lys Gly Val Trp Leu Gly Asp Gly Val Met Gln Thr Thr
    2015                2020                2025

Cys Pro Cys Gly Ala Gln Ile Ser Gly His Val Lys Asn Gly Ser
    2030                2035                2040

Met Lys Ile Val Gly Pro Lys Thr Cys Ser Asn Thr Trp His Gly
    2045                2050                2055

Thr Phe Pro Ile Asn Ala Tyr Thr Thr Gly Pro Cys Thr Pro Ser
    2060                2065                2070

Pro Ala Pro Asn Tyr Ser Lys Ala Leu Trp Arg Val Ala Ala Glu
    2075                2080                2085

Glu Tyr Val Glu Val Thr Arg Val Gly Asp Phe His Tyr Val Thr
    2090                2095                2100

Gly Met Thr Thr Asp Asn Val Lys Cys Pro Cys Gln Val Pro Ala
    2105                2110                2115

Pro Glu Phe Phe Thr Glu Leu Asp Gly Val Arg Leu His Arg Tyr
    2120                2125                2130

Ala Pro Ala Cys Lys Pro Leu Leu Arg Asp Glu Val Thr Phe Gln
    2135                2140                2145

Val Gly Leu Asn Gln Tyr Pro Val Gly Ser Gln Leu Pro Cys Glu
    2150                2155                2160

Pro Glu Pro Asp Val Thr Val Leu Thr Ser Met Leu Thr Asp Pro
    2165                2170                2175

Ser His Ile Thr Ala Glu Thr Ala Lys Arg Arg Leu Ala Arg Gly
    2180                2185                2190

Ser Pro Pro Ser Leu Ala Ser Ser Ser Ala Ser Gln Leu Ser Ala
    2195                2200                2205

Pro Ser Leu Lys Ala Thr Cys Thr Thr His His Asp Ser Pro Asp
    2210                2215                2220

Ala Asp Leu Ile Glu Ala Asn Leu Leu Trp Arg Gln Glu Met Gly
    2225                2230                2235

Gly Asn Ile Thr Arg Val Glu Ser Glu Asn Lys Val Val Ile Leu
    2240                2245                2250

Asp Ser Phe Glu Pro Leu Arg Ala Glu Glu Asp Asp Arg Glu Val
    2255                2260                2265

Ser Val Ala Ala Glu Ile Leu Arg Arg Thr Arg Lys Phe Pro Ala
    2270                2275                2280

Ala Met Pro Ile Trp Ala Arg Pro Asp Tyr Asn Pro Pro Leu Leu
    2285                2290                2295

Glu Ser Trp Lys Asn Pro Asp Tyr Val Pro Pro Val Val His Gly
    2300                2305                2310

Cys Pro Leu Pro Pro Ile Lys Ala Pro Pro Ile Pro Pro Pro Arg
    2315                2320                2325

Arg Lys Arg Thr Val Ile Leu Thr Glu Ser Thr Val Ser Ser Ala
    2330                2335                2340

Leu Ala Glu Leu Ala Thr Lys Thr Phe Gly Ser Ser Gly Ser Ser
    2345                2350                2355

Ala Val Asp Ser Gly Thr Ala Thr Gly Pro Pro Asp Gln Ser Ser
    2360                2365                2370

Gly Asp Gly Asp Thr Gly Ser Asp Ala Glu Ser Cys Ser Ser Met
    2375                2380                2385

Pro Pro Leu Glu Gly Glu Pro Gly Asp Pro Asp Leu Ser Asp Gly
    2390                2395                2400

Ser Trp Ser Thr Val Ser Glu Glu Ala Gly Glu Asp Val Val Cys
    2405                2410                2415

Cys Ser Met Ser Tyr Thr Trp Thr Gly Ala Leu Ile Thr Pro Cys
    2420                2425                2430

Ala Ala Glu Glu Ser Lys Leu Pro Ile Asn Ala Leu Ser Asn Ser
    2435                2440                2445

Leu Leu Arg His His Asn Met Val Tyr Ala Thr Thr Ser Arg Ser
    2450                2455                2460

Ala Gly Gln Arg Gln Lys Lys Val Thr Phe Asp Arg Val Gln Val
    2465                2470                2475

Leu Asp Asp His Tyr Arg Asp Val Leu Lys Glu Met Lys Ala Lys
    2480                2485                2490

Ala Ser Thr Val Lys Ala Lys Leu Leu Pro Val Glu Glu Ala Cys
    2495                2500                2505

Arg Leu Thr Pro Pro His Ser Ala Arg Ser Lys Phe Gly Tyr Gly
    2510                2515                2520

Ala Lys Asp Val Arg Asn Leu Ser Ser Lys Ala Val Asn His Ile
    2525                2530                2535

His Ser Val Trp Lys Asp Leu Leu Glu Asp Ser Glu Thr Pro Ile
    2540                2545                2550

Asp Thr Thr Ile Met Ala Lys Asn Glu Val Phe Cys Val Gln Pro
    2555                2560                2565

Glu Lys Gly Gly Arg Lys Ser Ala Arg Leu Ile Val Phe Pro Asp
    2570                2575                2580

Leu Gly Val Arg Val Cys Glu Lys Met Ala Leu Tyr Asp Val Val
    2585                2590                2595

Ser Thr Leu Pro Gln Ala Val Met Gly Ser Ser Tyr Gly Phe Gln
    2600                2605                2610

Tyr Ser Pro Gly Gln Arg Val Glu Phe Leu Val Asn Ala Trp Lys
    2615                2620                2625

Ser Lys Lys Asn Pro Met Gly Phe Ala Tyr Asp Thr Arg Cys Phe

2630                2635                2640

Asp Ser Thr Val Thr Glu Ser Asp Ile Arg Val Glu Glu Ser Ile
    2645                2650                2655

Tyr Gln Cys Cys Asp Leu Ala Pro Glu Ala Arg Gln Val Ile Arg
    2660                2665                2670

Ser Leu Thr Glu Arg Leu Tyr Ile Gly Gly Pro Leu Thr Asn Ser
    2675                2680                2685

Lys Gly Gln Ser Cys Gly Tyr Arg Arg Cys Arg Ala Ser Gly Val
    2690                2695                2700

Leu Thr Thr Ser Cys Gly Asn Thr Leu Thr Cys Tyr Leu Lys Ala
    2705                2710                2715

Ser Ala Ala Cys Arg Ala Ala Lys Leu Gln Asp Cys Thr Met Leu
    2720                2725                2730

Val Cys Gly Asp Asp Leu Val Val Ile Cys Glu Ser Ala Gly Thr
    2735                2740                2745

Gln Glu Asp Ala Ala Ser Leu Arg Val Phe Thr Glu Ala Met Thr
    2750                2755                2760

Arg Tyr Ser Ala Pro Pro Gly Asp Pro Pro Gln Pro Glu Tyr Asp
    2765                2770                2775

Leu Glu Leu Ile Thr Ser Cys Ser Ser Asn Val Ser Val Ala His
    2780                2785                2790

Asp Ala Leu Gly Lys Arg Val Tyr Tyr Leu Thr Arg Asp Pro Thr
    2795                2800                2805

Thr Pro Leu Ala Arg Ala Ala Trp Glu Thr Ala Arg His Thr Pro
    2810                2815                2820

Val Asn Ser Trp Leu Gly Asn Ile Ile Met Tyr Ala Pro Thr Leu
    2825                2830                2835

Trp Ala Arg Met Ile Leu Met Thr His Phe Phe Ser Ile Leu Leu
    2840                2845                2850

Ala Gln Glu Gln Leu Glu Lys Ala Leu Asp Cys Gln Ile Tyr Gly
    2855                2860                2865

Ala Thr Tyr Ser Ile Glu Pro Leu Asp Leu Pro Gln Ile Ile Gln
    2870                2875                2880

Arg Leu His Gly Leu Ser Ala Phe Ser Leu His Ser Tyr Ser Pro
    2885                2890                2895

Gly Glu Ile Asn Arg Val Ala Ser Cys Leu Arg Lys Leu Gly Val
    2900                2905                2910

Pro Pro Leu Arg Val Trp Arg His Arg Ala Arg Ser Val Arg Ala
    2915                2920                2925

Lys Leu Leu Ser Gln Gly Gly Arg Ala Ala Thr Cys Gly Lys Tyr
    2930                2935                2940

Leu Phe Asn Trp Ala Val Arg Thr Lys Leu Lys Leu Thr Pro Ile
    2945                2950                2955

Pro Ala Ala Ser Gln Leu Asp Leu Ser Gly Trp Phe Ile Ala Gly
    2960                2965                2970

Tyr Ser Gly Gly Asp Ile Tyr His Ser Leu Ser Arg Ala Arg Pro
    2975                2980                2985

Arg Trp Phe Met Trp Cys Leu Leu Leu Leu Ser Val Gly Val Gly
    2990                2995                3000

Ile Tyr Leu Leu Pro Asn Arg
    3005                3010

<210>  12
<211>  30
<212>  DNA
<213>  Artificial

<220>
<223>  primer for HCV

<400>  12
tcatgcggct cacggacctt tcacagctag                                        30

<210>  13
<211>  30
<212>  DNA
<213>  Artificial

<220>
<223>  primer for HCV

<400>  13
atcgtcttca cgcagaaagc gtctagccat                                        30


<210>  14
<211>  26
<212>  DNA
<213>  Artificial

<220>
<223>  primer for HCV

<400>  14
gcctattggc ctggagtgtt tagctc                                            26


<210>  15
<211>  29
<212>  DNA
<213>  Artificial

<220>
<223>  primer for HCV

<400>  15
atggcgttag tatgagtgtc gtgcagcct                                         29


<210>  16
<211>  31
<212>  DNA
<213>  Artificial

<220>
<223>  primer for HCV

<400>  16
tcgggcacga gacaggctgt gatatatgtc t                                      31


<210>  17
<211>  19
<212>  DNA
<213>  Artificial

<220>
<223>  primer for HCV

<400>  17
tgcacggtct acgagacct                                                    19


<210>  18
<211>  24
<212>  DNA
<213>  Artificial

<220>
<223>  primer for HCV

<400>  18
ctcgcaagca ccctatcagc cagt                                              24


<210>  19
<211>  19
<212>  DNA
<213>  Artificial

<220>
<223>  primer for HCV

<400>  19
aggcattgag cgggtttat                                                    19


<210>  20
<211>  38
<212>  DNA
<213>  Artificial

<220>
<223>  primer for RACE

<400>  20
ctagactcga gtcgacatcg tttttttttt tttttttt                               38


<210>  21
<211>  20
<212>  DNA
<213>  Artificial

<220>
<223>  primer for HCV

<400>  21
atcttagccc tagtcacggc                                                   20


<210>  22
<211>  20
<212>  DNA
<213>  Artificial

<220>
<223>  primer for RACE

<400>  22
ctagactcga gtcgacatcg                                                   20


<210>  23
<211>  29
<212>  DNA
<213>  Artificial

<220>
<223>  primer for HCV

<400>  23

ctagctgtaa aggtccgtga gccgcatga                                    29

<210>  24
<211>  17
<212>  DNA
<213>  Artificial

<220>
<223>  primer

<400>  24
gtaaaacgac ggccagt                                                17

<210>  25
<211>  17
<212>  DNA
<213>  Artificial

<220>
<223>  primer

<400>  25
caggaaacag ctatgac                                                17

<210>  26
<211>  20
<212>  DNA
<213>  Artificial

<220>
<223>  primer for HCV

<400>  26
aggaagactt ccgagcggtc                                             20

<210>  27
<211>  32
<212>  DNA
<213>  Artificial

<220>
<223>  primer for HCV

<400>  27
ggaacttgcc cggttgctct ttctctatct tc                               32

<210>  28
<211>  25
<212>  DNA
<213>  Artificial

<220>
<223>  primer for HCV

<400>  28
attccatggt ggggaactgg gctaa                                       25

<210>  29
<211>  30
<212>  DNA
<213>  Artificial

<220>
<223>  primer for HCV

<400>  29
taacaatacc ttgacctgcc ccacggactg                                        30


<210>  30
<211>  29
<212>  DNA
<213>  Artificial

<220>
<223>  primer for HCV

<400>  30
aacatcgtgg acgtgcaata cctgtacgg                                         29


<210>  31
<211>  20
<212>  DNA
<213>  Artificial

<220>
<223>  primer for HCV

<400>  31
gaccctacac cgtacaggta                                                   20


<210>  32
<211>  26
<212>  DNA
<213>  Artificial

<220>
<223>  primer for HCV

<400>  32
ttggaccggg agatggctgc atcgtg                                            26


<210>  33
<211>  21
<212>  DNA
<213>  Artificial

<220>
<223>  primer for HCV

<400>  33
cacccaaatg tacaccaatg t                                                 21

<210>  34
<211>  22
<212>  DNA
<213>  Artificial

<220>
<223>  primer for HCV

<400>  34
tacccgttga gtctatggaa ac                                                22


<210>  35
<211>  20
<212>  DNA
<213>  Artificial

<220>
<223>  primer for HCV

<400>  35
cacttggaat gtctgcggta                                                   20


<210>  36
<211>  25
<212>  DNA
<213>  Artificial

<220>
<223>  primer for HCV

<400>  36
agggggggag gcatctcatt ttctg                                             25


<210>  37
<211>  26
<212>  DNA
<213>  Artificial

<220>
<223>  primer for HCV

<400>  37
tgctatgacg cgggctgtgc ttggta                                            26


<210>  38
<211>  21
<212>  DNA
<213>  Artificial

<220>
<223>  primer for HCV

<400>  38
ggtcattgtg ggcaggatca t                                                 21

<210>  39
<211>  20
<212>  DNA
<213>  Artificial

<220>
<223>  primer for HCV

<400>  39
ctgcctggaa accccgcgat                                                    20


<210>  40
<211>  24
<212>  DNA
<213>  Artificial

<220>
<223>  primer for HCV

<400>  40
tggcagcata ggccttggga aggt                                               24


<210>  41
<211>  21
<212>  DNA
<213>  Artificial

<220>
<223>  primer for HCV

<400>  41
aagacctggc tccagtccaa g                                                  21


<210>  42
<211>  21
<212>  DNA
<213>  Artificial

<220>
<223>  primer for HCV

<400>  42
ttccatgctc accgacccct c                                                  21


<210>  43
<211>  20
<212>  DNA
<213>  Artificial

<220>
<223>  primer for HCV

<400>  43
accttattct ctgactccac                                                    20


<210>  44

<211>  21
<212>  DNA
<213>  Artificial

<220>
<223>  primer for HCV

<400>  44
cagaagaagg tcacctttga c                                                21


<210>  45
<211>  25
<212>  DNA
<213>  Artificial

<220>
<223>  primer for HCV

<400>  45
gcagcgggtc gagttcctgg tgaat                                            25


<210>  46
<211>  26
<212>  DNA
<213>  Artificial

<220>
<223>  primer for HCV

<400>  46
ctacggggcc tgttactcca ttgaac                                           26


<210>  47
<211>  26
<212>  DNA
<213>  Artificial

<220>
<223>  primer for HCV

<400>  47
cttgaaaaag ccctggattg tcagat                                           26


<210>  48
<211>  35
<212>  DNA
<213>  Artificial

<220>
<223>  primer for HCV

<400>  48
acatgatctg cagagaggcc agtatcagca ctctc                                 35


<210>  49
<211>  31

<212>  DNA
<213>  Artificial

<220>
<223>  primer for HCV

<400>  49
gccagccccc tgatgggggc gacactccac c                                    31


<210>  50
<211>  25
<212>  DNA
<213>  Artificial

<220>
<223>  primer for HCV

<400>  50
agccgcatgt aagggtatcg atgac                                           25


<210>  51
<211>  20
<212>  DNA
<213>  Artificial

<220>
<223>  primer for HCV

<400>  51
acatgatctg cagagaggcc                                                 20


<210>  52
<211>  30
<212>  DNA
<213>  Artificial

<220>
<223>  primer for HCV

<400>  52
gagtacaccg gaattgccag gacgaccggg                                      30


<210>  53
<211>  25
<212>  DNA
<213>  Artificial

<220>
<223>  primer for HCV

<400>  53
attccatggt ggggaactgg gccaa                                           25


<210>  54
<211>  24
<212>  DNA

<213>  Artificial

<220>
<223>  primer for HCV

<400>  54
catccatgtg cagccgaacc aatt                                              24


<210>  55
<211>  30
<212>  DNA
<213>  Artificial

<220>
<223>  primer for HCV

<400>  55
aggggtagtg ccaaagcctg tatgggtagt                                        30


<210>  56
<211>  31
<212>  DNA
<213>  Artificial

<220>
<223>  primer for HCV

<400>  56
ataatgaccc ccggcgactt tccgcactaa c                                      31


<210>  57
<211>  20
<212>  DNA
<213>  Artificial

<220>
<223>  primer for HCV

<400>  57
catggtagac agtccagcac                                                   20


<210>  58
<211>  24
<212>  DNA
<213>  Artificial

<220>
<223>  primer for HCV

<400>  58
gacatgcatg tcatgatgta tttg                                              24


<210>  59
<211>  20
<212>  DNA
<213>  Artificial

<220>
<223>  primer for HCV

<400>  59
ctcatgacct taaaggccac                                                    20


<210>  60
<211>  30
<212>  DNA
<213>  Artificial

<220>
<223>  primer for ECMV IRES

<400>  60
tgcacatgct ctacatgtgt ttagtcgagg                                         30


<210>  61
<211>  9594
<212>  RNA
<213>  Hepatitis C virus

<400>  61
gccagccccc ugaugggggc gacacuccac cauagaucac uccccuguga ggaacuacug      60

ucuucacgca gaaagcgucu agccauggcg uuaguaugag ugucgugcag ccuccaggac     120

cccccucccc gggagagcca uaguggucug cggaaccggu gaguacaccg gaauugccag     180

gacgaccggg uccuuucuug gaucaacccg cucaaugccu ggagauuugg gcgugccccc     240

gcgagacugc uagccgagua guguuggguc gcgaaaggcc uugugguacu gccugauagg     300

gugcuugcga gugccccggg aggucucgua gaccgugcau caugagcaca aauccuaaac     360

cucaaagaaa aaccaaacgu aacaccaacc gccgcccaca ggacgucaag uucccgggcg     420

guggucagau cguuggugga guuuaccugu ugccgcgcag gggccccagg uugggugugc     480

gcgcgacuag gaagacuucc gagcggucgc aaccucguga aaggcggcaa ccuaucccca     540

aggcucgccg ccccgagggc agggccuggg cucagcccgg guacccuugg ccccucuaug     600

gcaaugaggg ucuggggugg gcagggugge uccugucacc ccgcggcucc cggccuaauu     660

ggggccccac ggacccccgg cguaggucgc guaauuuggg uaaggucauc gauacccuca     720

caugcggcuu cgccgaccuc augggguaca uuccgcucgu cggcgccccc cuaggaggcg     780

uugccagggc ccuggcgcau ggcguccggg uucuggagga cggcgugaac uaugcaacag     840

ggaauuugcc cgguugcucu uucucuaucu uccucuuggc uuugcugucc uguuugacug     900

ucccagcuuc cgcuuaugaa gugcgcaacg uguccgggac auaccauguc acgaacgacu     960

gcgccaacuc aagcauugug uaugaggcag cggacauaau caugcacacc ccugggugcg    1020

ugcccugcgu ccgggagggc aauuccuccc gcugcugggu agcgcucacu cccacgcucg    1080

cggccaggaa caggagcauc cccacuacga cgauacgacg ccacgucgau uugcucguug 1140
gggcggcugc cuucugcucc gccauguacg ugggggaucu cugcgggucu guuuuccuag 1200
ucucccaacu guucaccuuc ucaccucgcc gguaugagac gguacaagaa ugcaauugcu 1260
caaucuaucc cggccacgua ucaggucacc gcauggcuug ggauaugaug augaauuggu 1320
cgcccacagc agcucuagug gugucacagu uacuccgaau cccacaagcc gucguggaua 1380
ugguggcagg ggcccacugg gggguucugg cgggucuugc cuacuauucc auggugggga 1440
acugggcuaa agucuugauu gugaugcuac ucuuugccgg cguugacggg accaccaccg 1500
ugacaggggg agcggcggcc uuuggcacca ggagccuugc guccuucuuu acauuagggc 1560
cgucucagaa aauccaacuu guaaacacca acggcaguug gcacaucaau aggacugccu 1620
ugaauuguaa ugacucccuc cagacugggu uccuugcugc gcuguucuau gcacacaaga 1680
ucaacacguc uggaugccca gagcgcaugg ccagcugccg cccuauugac aaguucgcuc 1740
aggggugggg ucccaucacc cauggugcgc cugacaccuc ggaccagagg cccuacugcu 1800
ggcacuacgc accucgaccg ugugguaucg uacccgcguc ggaggugugu ggcccagugu 1860
auuguuucac cccaagcccu guaguggugg ggacgaccga ucgcuucggc guccccacgu 1920
auagcugggg ggagaauaag acggacgugc ugcuccucaa caacacgcgg ccgccgcagg 1980
gcaauugguu cggcugcaca uggaugaaug gcaccggguu caccaagacg ugugggggcc 2040
ccccguguga uaucgggggg gucggcaaca acaccuugac cugccccacg gauugcuucc 2100
ggaagcaccc cgaggccacu uauaccaaau gcggcucggg gccuugguug acgccuaggu 2160
gucuaguuga cuacccauac aggcuuuggc acuaccccug cacugucaac uuuaccaucu 2220
ucaaggucag gauguaugug gggggcgugg agcacaggcu caacgcugcg ugcaacugga 2280
cucgaggaga gcgcugugac cuagaggaca gggacagauc agagcuuagc ccgcugcuac 2340
ugucuacaac agaguggcag auacugcccu guuccuucac cacccuaccg gcucugucca 2400
cuggcuugau ccaccuccau cagaacaucg uggacgugca auaccuguac gguguagggu 2460
cagcaguugu cuccguugug aucagauggg aguacguccu gcugcucuuc cuccuccugg 2520
cggacgcgcg cguuugugcc ugcuuaugga ugaugcuguu gauagcccag gcugaagccg 2580
ccuuggagaa ccugguaguc cucaacgcgg cguccguggc cggagcgcau ggcauccucu 2640
cuuuccuugu guucuucugc gcugccuggu acaucaaagg caagcuagug ccuggggugg 2700
cguaugcucu cuauggugua uggccgcugc uccugcuccu gcuggcguug ccacaacggg 2760
cguacgccau ggaccgggag auggcugcau cgugcggagg cgcgguuuuc gugggucugg 2820
uacucuugac cuugucacca cacuauaaag cauuccuugc caaguccaua uggugguuac 2880

aauauuuaau caccagggcc gaggcgcauu ugcaagugug gguccccccc cucaacguuc 2940

gggggggccg cgaugccauc auccuccuca cgugcgcggu ccacccagag cuaauuuuug 3000

acaucaccaa aauuuugcuc gccauauuug gcccgcucau ggugcuccag gcuggucuaa 3060

cuagagugcc guacuuugug cgcgcucaag ggcucauccg ugugugcaug uuggugcgga 3120

aagucgcugg gggucauuau auccaaaugg cucucaugag guuggccgca cugacgggca 3180

cguacguuua ugaucaucuu acuccgcugc gggacugggc ccaugcgggc uugcgagacc 3240

uugcggugge aguugagccc gucguuuucu ccaacacaga gaccaagauu aucaccuggg 3300

gggcggacac cgcggcgugu ggggacauca ucuugggucu accugucucc gcccgaaggg 3360

ggagagaggu acuccuagga ccggccgaua acuuuggaga gcaagggugg cgacuccuug 3420

cgcccaucac ggccuacucc caacagacgc ggggcuuacu uggcuguauc aucaccggcc 3480

ucacaggucg agacaagaac caggucgaag gggagguuca gguggguucc accgcaacac 3540

agucuuucuu ggcaaccugc gucaacggug uguguuggac ugucuaccau ggugccggcu 3600

caaagacccu agccggcccg aaggggccaa ucacccagau guacaccaac guagaccaag 3660

accucguugg cuggcaggcg ccccccgggg cgcguuccau gacgccgugc accugcggca 3720

gcucggaccu uuacuugguc acgaggcaug cugaugucau uccggugcgc cggcggggug 3780

acagcagagg gagccuacuu ucccccaggc ccgucuccua cuugaagggc ucuucaggug 3840

guccacugcu cugccccuug gggcacgucg ugggcaucuu ucgggcugcc gugugcaccc 3900

gggggguugc gaaggcggug gacuucauac ccaucgaguc uauggaaacu accaugcggu 3960

cuccggucuu uacggauaau ucaucucccc cggccguacc gcagacuuuu caaguggccc 4020

aucugcacgc ccccacuggc agcggcaaga gcaccaaggu gccagcugcg uaugcagccc 4080

agggguacaa ggugcuuguc uugaauccau ccguugccgc caccuugggu uuuggggcgu 4140

auauguccaa ggcauacggu gucgacccua acauuagaac ugggguaagg accaucacca 4200

caggcgcucc caucacguac uccaccuacg gcaaguuccu ugccgacggu gguugcuccg 4260

ggggcgcuua cgacaucaua auaugcgaug agugccacuc aaccgacucg acuuccauuu 4320

ugggcauugg cacgguccug gaucaagcgg agacggcugg agcgcgacuc gucgugcucg 4380

ccaccgcuac gccuccggga ucggucacug ugccacaccc caacaucgag gagguggccu 4440

uguccaacac cggagagauu cccuucuaug gcaaagccau cccccucgag gucaucaagg 4500

gggggaggca ucucauuuuc ugucauucua agaagaagug ugaugagcuc gcugcaaagc 4560

ugucgggccu uggggucaac gcuguagcgu acuaccgggg ucuugaugug uccgucauac 4620

caacaagcgg ggacgucguu gucguggcaa cagacgcucu aaugacgggc uacaccggug 4680

acuuugacuc ugugaucgac uguaauacau gugucaccca gacagucgac uucagccugg 4740

accccaccuu caccauugag acgacgaccg ugccccaaga cgcagugucg cgcucgcagc 4800
ggcgagggag gacugguagg gguagagggg gcauauacag guuugugacu ccaggagagc 4860
ggcccucggg cauguucgau ucccucgguc ugugugaaug cuaugacgcg ggcugugcuu 4920
gguacgagcu cacgcccgcc gagaccucgg uuaggcugcg ggcuuaccua aauacaccag 4980
gguugcccgu cugccaggac cauuuggagu ucugggaagg cgucuucaca ggccucacuc 5040
auauagaugc ccacuucuug ucucagacua agcaggcagg agacaacuuc cccuaccugg 5100
uggcauacca ggccacagug ugcgccaggg cccaggcacc accuccauca ugggaucaaa 5160
uguggaagug ucucauacgg cuaaaaccua cacuacacgg gccaacaccc cuguuguaca 5220
ggcuaggagc cguccaaaac gaggucaccc ucacacaccc caugaccaaa uacaucauga 5280
caugcauguc ggcugaccua gaggucguca ccagcacuug ggugcuggug ggcggggucc 5340
ucgcagcuuu ggccgcguac ugcuugacaa cgggcagcgu gguuaucaua ggcaggauca 5400
ucuuguccgg aaggccggcu aucauucccg auagggaagu ucucuaccag gaguucgaug 5460
aaauggaaga gugcgccuca caccuucccu acaucgaaca aggaaugcag cucgccgagc 5520
aauucaagca gaaggcgcuc ggguuguugc aaacggccac caagcaagcg gaggccgcug 5580
cucccguggu ggaguccaaa uggcaagccc uugaggccuu cugggcgaag cacaugugga 5640
acuucaucag cgggauacag uaucuagcag gcuuguccac ucugccugga aaccccgcga 5700
uagcaucgcu gauggcauuu acagccucua ucacuagccc gcucaccacc cuacauaccc 5760
uucuauuuaa caucuugggg ggaugggugg ccgcccaacu cgcccccccc agcgcugcuu 5820
cagccuucgu aggcgccggc aucgccggcg cggcuguugg cagcauaggu cuugggaagg 5880
ugcucgugga cauccuagcg gguuauggag cagggguggc aggcgcacuc guggccuuca 5940
aggucaugag cggcgaagug cccuccacug aggaccuggu caacuuacuc ccugccaucc 6000
ucuccccugg ugcccugguc gucggggucg ugugcgcagc gauacugcgu cggcaugugg 6060
gcccagggga gggggccgug caguggauga accggcugau agcguucgcu ucgcggggua 6120
accacgucuc ccccacgcac uaugugccug agagcgacgc cgcagcgcgu gucacccaga 6180
uccucuccag ccuuaccauc acucagcugc uaaagaggcu ccaccagugg auuaauaagg 6240
acuguuccac gccaugcucc gguucguggc ucagggaugu uugggacugg auaugcacgg 6300
uuuugaccga cuucaaaacc uggcuccagu ccaagcuccu gccacgguug ccgggacucc 6360
cuuucuuuuc augucaacgu ggauauaaag gagucuggcu gggagauggc guuaugcaaa 6420
cuaccugucc auguggugca caaaucagcg gacaugucaa aaacggcucc augaagaucg 6480
uggggccuaa aaccugcagc aacacguggc acgggacguu ccccaucaac gcauacacca 6540

cagggcccug cacacccucc ccggcgccga acuauuccaa ggcguugugg cgaguggcug 6600
cugaggagua uguggagguc acgcgggugg gggauuucca cuacgugacg ggcaugacca 6660
cugacaacgu aaaaugccca ugccaggucc cggcccccga auucuucacg gaguuggaug 6720
gggugcggcu gcacagguac gcuccggcgu gcaagccucu cuuacgggau gaggucacau 6780
uccaggucgg gcucaaccag uauccgguug gaucacagcu uccaugugag cccgagccgg 6840
auguaacagu gcucacuucc augcucaccg accccuccca uauuacagca gagacggcua 6900
agcguaggcu ggccagaggg uccccccccu cuuuggccag cuccucagcu agccaguugu 6960
cugcgccuuc cuugaaggcg acaugcacua cccaccauga cuccccagau gcugaccuca 7020
ucgaggccaa ccuccugugg cggcaggaga ugggcgggaa caucacccgc guggagucag 7080
agaacaaggu aguaauucug gacucuuuug aaccgcuucg agcggaggag gaugauaggg 7140
aaguguccgu agcggcggag auccugcgga gaaccaggaa auuccccgca gcgaugccca 7200
uaugggcacg gccggacuac aacccaccac uccuagaguc uuggaagaac ccagacuacg 7260
ucccuccagu gguacacggg ugcccauuac caccuauuaa ggccccuccg auaccaccuc 7320
cacggagaaa gaggacgguc auccugacag aauccaccgu gucuucugcc uuggcggagc 7380
uugcuacaaa gaccuuuggc agcuccggau cgucggccgu ugacagcggc acagcgacug 7440
gcccuccuga ucaguccucc ggugacggag auacaggauc cgacgcugag ucgugcuccu 7500
ccaugccccc ccuugagggg gagccggggg accccgaucu cagcgacggg ucuuggucua 7560
ccgugagcga ggaggccggu gaggacgucg ucugcugcuc gauguccuac acauggacag 7620
gcgccuuaau cacaccgugc gccgcagagg agagcaagcu gccuaucaac gcguugagca 7680
acucuuugcu gcgccaccac aacauggucu augccacaac aucccgcagc gcgggccaac 7740
ggcagaagaa ggucacuuuu gacagagugc agguccugga cgaccauuac cgggacgugc 7800
ucaaggagau gaaggcgaag gcguccacag uuaaggcuaa acuucuaccu guagaagaag 7860
ccugcaggcu gacgccccca cacucggcca gauccaaguu uggcuauggg gcgaaggacg 7920
uccggaaccu auccagcaag gccgucaacc acauccacuc cguguggaag gacuugcugg 7980
aagacucuga gacgccgauu gacaccacca ucauggcaaa aaaugagguc uuuuguguuc 8040
aaccagagaa aggaggccgc aagucagcuc gucuuaucgu auucccagac uuggggguuc 8100
gugugugcga gaaaauggcc cuuuacgacg ugguuuccac ucuuccucag gccgugaugg 8160
gcuccucaua cggguuccag uacucuccug gacagcgggu cgaguuccug gugaaugccu 8220
ggaaaucaaa gaagaacccu augggcuucg cauaugacac ccgcuguuuu gacucaacgg 8280
ucacugagag ugacauccgu guugaggagu caauuuacca auguugugac uuggcccccg 8340
aggccagaca ggucauaagg ucgcucacgg agcggcuuua uaucgggggc ccccugacua 8400

auucaaaagg gcagagcugc ggcuaucgcc ggugccgcgc cagcggugug cugacgacca 8460

gcugcgguaa uacccucaca uguuacuuga aggcuucugc agccugucga gcugcaaagc 8520

uccaggacug cacaauguua gugugcggag acgaccuugu cguuaucugu gaaagugcgg 8580

gaacccagga ggacgcggcg agccuacgag ucuucacgga ggcuaugacu agguacucug 8640

cccccccegg ggacccgccc cagccagaau acgacuugga gcugauaaca ucaugcuccu 8700

ccaacgucuc ggucgcgcac gaugcacuug gcaagcgggu guauuaucug acccgcgacc 8760

ccaccacccc ccuugcgcgg gcugcguggg agacagcaag acacacucca guuaacuccu 8820

ggcuaggcaa caucaucaug uaugcgccca cccuaugggc aaggaugauu cugaugaccc 8880

acuucuuuuc cauccuucua gcucaggaac aacuugaaaa agcccuagau ugucagaucu 8940

acggggccac uuacuccauu gagccacuug accuaccuca gaucauucag cgacuccacg 9000

gccuuagcgc auuuucacuc cauagcuacu cuccagguga gaucaauagg guggcuucau 9060

gccucaggaa acuuggggua ccacccuugc gagucuggag acaucgggcc agaagugucc 9120

gcgcuaagcu acugucccag ggggggaggg ccgccacuug uggcaaauac cucuucaacu 9180

gggcaguaag gaccaagcuc aaacucacuc caauuccggc ugcgucccag uuggacuugu 9240

ccggcugguu cauugcuggu uacagcgggg gagacauaua ucacagccug ucucgcgccc 9300

gaccccgcug guucaugugg ugccuacucc uacuuuccgu agggguaggc aucuaucugc 9360

uccccaaucg augaacgggg ggcuaaacac uccaggccaa uaggccauuc uguuuuuuuu 9420

uuuuuuuuuu uuuuuuuuuu uuuuuuuuuu uuuuuuuuuu uuuuuuccuu uuuuuuuuuu 9480

uuuuucccuu ucuuuuggug gcuccaucuu agcccuaguc acggcuagcu gugaaagguc 9540

cgugagccgc augacugcag agagugcuga uacuggccuc ucugcagauc augu 9594

<210> 62
<211> 3010
<212> PRT
<213> Hepatitis C virus

<400> 62

Met Ser Thr Asn Pro Lys Pro Gln Arg Lys Thr Lys Arg Asn Thr Asn
1               5                   10                  15

Arg Arg Pro Gln Asp Val Lys Phe Pro Gly Gly Gly Gln Ile Val Gly
            20                  25                  30

Gly Val Tyr Leu Leu Pro Arg Arg Gly Pro Arg Leu Gly Val Arg Ala
        35                  40                  45

Thr Arg Lys Thr Ser Glu Arg Ser Gln Pro Arg Glu Arg Arg Gln Pro
    50                  55                  60

Ile Pro Lys Ala Arg Arg Pro Glu Gly Arg Ala Trp Ala Gln Pro Gly
65                  70                  75                  80

Tyr Pro Trp Pro Leu Tyr Gly Asn Glu Gly Leu Gly Trp Ala Gly Trp
                85                  90                  95

Leu Leu Ser Pro Arg Gly Ser Arg Pro Asn Trp Gly Pro Thr Asp Pro
            100                 105                 110

Arg Arg Arg Ser Arg Asn Leu Gly Lys Val Ile Asp Thr Leu Thr Cys
        115                 120                 125

Gly Phe Ala Asp Leu Met Gly Tyr Ile Pro Leu Val Gly Ala Pro Leu
    130                 135                 140

Gly Gly Val Ala Arg Ala Leu Ala His Gly Val Arg Val Leu Glu Asp
145                 150                 155                 160

Gly Val Asn Tyr Ala Thr Gly Asn Leu Pro Gly Cys Ser Phe Ser Ile
                165                 170                 175

Phe Leu Leu Ala Leu Leu Ser Cys Leu Thr Val Pro Ala Ser Ala Tyr
            180                 185                 190

Glu Val Arg Asn Val Ser Gly Thr Tyr His Val Thr Asn Asp Cys Ala
        195                 200                 205

Asn Ser Ser Ile Val Tyr Glu Ala Ala Asp Ile Ile Met His Thr Pro
    210                 215                 220

Gly Cys Val Pro Cys Val Arg Glu Gly Asn Ser Ser Arg Cys Trp Val
225                 230                 235                 240

Ala Leu Thr Pro Thr Leu Ala Ala Arg Asn Arg Ser Ile Pro Thr Thr
                245                 250                 255

Thr Ile Arg Arg His Val Asp Leu Leu Val Gly Ala Ala Ala Phe Cys
            260                 265                 270

Ser Ala Met Tyr Val Gly Asp Leu Cys Gly Ser Val Phe Leu Val Ser
        275                 280                 285

Gln Leu Phe Thr Phe Ser Pro Arg Arg Tyr Glu Thr Val Gln Glu Cys

290                 295                 300

Asn Cys Ser Ile Tyr Pro Gly His Val Ser Gly His Arg Met Ala Trp
305                 310                 315                 320

Asp Met Met Met Asn Trp Ser Pro Thr Ala Ala Leu Val Val Ser Gln
                325                 330                 335

Leu Leu Arg Ile Pro Gln Ala Val Val Asp Met Val Ala Gly Ala His
            340                 345                 350

Trp Gly Val Leu Ala Gly Leu Ala Tyr Tyr Ser Met Val Gly Asn Trp
        355                 360                 365

Ala Lys Val Leu Ile Val Met Leu Leu Phe Ala Gly Val Asp Gly Thr
    370                 375                 380

Thr Thr Val Thr Gly Gly Ala Ala Ala Phe Gly Thr Arg Ser Leu Ala
385                 390                 395                 400

Ser Phe Phe Thr Leu Gly Pro Ser Gln Lys Ile Gln Leu Val Asn Thr
                405                 410                 415

Asn Gly Ser Trp His Ile Asn Arg Thr Ala Leu Asn Cys Asn Asp Ser
            420                 425                 430

Leu Gln Thr Gly Phe Leu Ala Ala Leu Phe Tyr Ala His Lys Ile Asn
        435                 440                 445

Thr Ser Gly Cys Pro Glu Arg Met Ala Ser Cys Arg Pro Ile Asp Lys
    450                 455                 460

Phe Ala Gln Gly Trp Gly Pro Ile Thr His Gly Ala Pro Asp Thr Ser
465                 470                 475                 480

Asp Gln Arg Pro Tyr Cys Trp His Tyr Ala Pro Arg Pro Cys Gly Ile
                485                 490                 495

Val Pro Ala Ser Glu Val Cys Gly Pro Val Tyr Cys Phe Thr Pro Ser
            500                 505                 510

Pro Val Val Val Gly Thr Thr Asp Arg Phe Gly Val Pro Thr Tyr Ser
        515                 520                 525

Trp Gly Glu Asn Lys Thr Asp Val Leu Leu Leu Asn Asn Thr Arg Pro
    530                 535                 540

Pro Gln Gly Asn Trp Phe Gly Cys Thr Trp Met Asn Gly Thr Gly Phe
545                 550                 555                 560

Thr Lys Thr Cys Gly Gly Pro Pro Cys Asp Ile Gly Gly Val Gly Asn
                565                 570                 575

Asn Thr Leu Thr Cys Pro Thr Asp Cys Phe Arg Lys His Pro Glu Ala
            580                 585                 590

Thr Tyr Thr Lys Cys Gly Ser Gly Pro Trp Leu Thr Pro Arg Cys Leu
        595                 600                 605

Val Asp Tyr Pro Tyr Arg Leu Trp His Tyr Pro Cys Thr Val Asn Phe
    610                 615                 620

Thr Ile Phe Lys Val Arg Met Tyr Val Gly Gly Val Glu His Arg Leu
625                 630                 635                 640

Asn Ala Ala Cys Asn Trp Thr Arg Gly Glu Arg Cys Asp Leu Glu Asp
                645                 650                 655

Arg Asp Arg Ser Glu Leu Ser Pro Leu Leu Leu Ser Thr Thr Glu Trp
            660                 665                 670

Gln Ile Leu Pro Cys Ser Phe Thr Thr Leu Pro Ala Leu Ser Thr Gly
        675                 680                 685

Leu Ile His Leu His Gln Asn Ile Val Asp Val Gln Tyr Leu Tyr Gly
    690                 695                 700

Val Gly Ser Ala Val Val Ser Val Val Ile Arg Trp Glu Tyr Val Leu
705                 710                 715                 720

Leu Leu Phe Leu Leu Leu Ala Asp Ala Arg Val Cys Ala Cys Leu Trp
                725                 730                 735

Met Met Leu Leu Ile Ala Gln Ala Glu Ala Ala Leu Glu Asn Leu Val
            740                 745                 750

Val Leu Asn Ala Ala Ser Val Ala Gly Ala His Gly Ile Leu Ser Phe
        755                 760                 765

Leu Val Phe Phe Cys Ala Ala Trp Tyr Ile Lys Gly Lys Leu Val Pro
    770                 775                 780

Gly Val Ala Tyr Ala Leu Tyr Gly Val Trp Pro Leu Leu Leu Leu Leu
785                 790                 795                 800

Leu Ala Leu Pro Gln Arg Ala Tyr Ala Met Asp Arg Glu Met Ala Ala
                805                 810                 815

Ser Cys Gly Gly Ala Val Phe Val Gly Leu Val Leu Leu Thr Leu Ser
            820                 825                 830

Pro His Tyr Lys Ala Phe Leu Ala Lys Ser Ile Trp Trp Leu Gln Tyr
        835                 840                 845

Leu Ile Thr Arg Ala Glu Ala His Leu Gln Val Trp Val Pro Pro Leu
    850                 855                 860

Asn Val Arg Gly Gly Arg Asp Ala Ile Ile Leu Leu Thr Cys Ala Val
865                 870                 875                 880

His Pro Glu Leu Ile Phe Asp Ile Thr Lys Ile Leu Leu Ala Ile Phe
                885                 890                 895

Gly Pro Leu Met Val Leu Gln Ala Gly Leu Thr Arg Val Pro Tyr Phe
            900                 905                 910

Val Arg Ala Gln Gly Leu Ile Arg Val Cys Met Leu Val Arg Lys Val
        915                 920                 925

Ala Gly Gly His Tyr Ile Gln Met Ala Leu Met Arg Leu Ala Ala Leu
    930                 935                 940

Thr Gly Thr Tyr Val Tyr Asp His Leu Thr Pro Leu Arg Asp Trp Ala
945                 950                 955                 960

His Ala Gly Leu Arg Asp Leu Ala Val Ala Val Glu Pro Val Val Phe
                965                 970                 975

Ser Asn Thr Glu Thr Lys Ile Ile Thr Trp Gly Ala Asp Thr Ala Ala
            980                 985                 990

Cys Gly Asp Ile Ile Leu Gly Leu  Pro Val Ser Ala Arg  Arg Gly Arg
        995                 1000                 1005

Glu Val  Leu Leu Gly Pro Ala  Asp Asn Phe Gly Glu  Gln Gly Trp
    1010                 1015                 1020

Arg Leu Leu Ala Pro Ile Thr Ala Tyr Ser Gln Gln Thr Arg Gly
    1025                1030                1035

Leu Leu Gly Cys Ile Ile Thr Gly Leu Thr Gly Arg Asp Lys Asn
    1040                1045                1050

Gln Val Glu Gly Glu Val Gln Val Val Ser Thr Ala Thr Gln Ser
    1055                1060                1065

Phe Leu Ala Thr Cys Val Asn Gly Val Cys Trp Thr Val Tyr His
    1070                1075                1080

Gly Ala Gly Ser Lys Thr Leu Ala Gly Pro Lys Gly Pro Ile Thr
    1085                1090                1095

Gln Met Tyr Thr Asn Val Asp Gln Asp Leu Val Gly Trp Gln Ala
    1100                1105                1110

Pro Pro Gly Ala Arg Ser Met Thr Pro Cys Thr Cys Gly Ser Ser
    1115                1120                1125

Asp Leu Tyr Leu Val Thr Arg His Ala Asp Val Ile Pro Val Arg
    1130                1135                1140

Arg Arg Gly Asp Ser Arg Gly Ser Leu Leu Ser Pro Arg Pro Val
    1145                1150                1155

Ser Tyr Leu Lys Gly Ser Ser Gly Gly Pro Leu Leu Cys Pro Leu
    1160                1165                1170

Gly His Val Val Gly Ile Phe Arg Ala Ala Val Cys Thr Arg Gly
    1175                1180                1185

Val Ala Lys Ala Val Asp Phe Ile Pro Ile Glu Ser Met Glu Thr
    1190                1195                1200

Thr Met Arg Ser Pro Val Phe Thr Asp Asn Ser Ser Pro Pro Ala
    1205                1210                1215

Val Pro Gln Thr Phe Gln Val Ala His Leu His Ala Pro Thr Gly
    1220                1225                1230

Ser Gly Lys Ser Thr Lys Val Pro Ala Ala Tyr Ala Ala Gln Gly
    1235                1240                1245

Tyr Lys Val Leu Val Leu Asn Pro Ser Val Ala Ala Thr Leu Gly

1250                1255                1260

Phe Gly Ala Tyr Met Ser Lys Ala Tyr Gly Val Asp Pro Asn Ile
    1265                1270                1275

Arg Thr Gly Val Arg Thr Ile Thr Thr Gly Ala Pro Ile Thr Tyr
    1280                1285                1290

Ser Thr Tyr Gly Lys Phe Leu Ala Asp Gly Gly Cys Ser Gly Gly
    1295                1300                1305

Ala Tyr Asp Ile Ile Ile Cys Asp Glu Cys His Ser Thr Asp Ser
    1310                1315                1320

Thr Ser Ile Leu Gly Ile Gly Thr Val Leu Asp Gln Ala Glu Thr
    1325                1330                1335

Ala Gly Ala Arg Leu Val Val Leu Ala Thr Ala Thr Pro Pro Gly
    1340                1345                1350

Ser Val Thr Val Pro His Pro Asn Ile Glu Glu Val Ala Leu Ser
    1355                1360                1365

Asn Thr Gly Glu Ile Pro Phe Tyr Gly Lys Ala Ile Pro Leu Glu
    1370                1375                1380

Val Ile Lys Gly Gly Arg His Leu Ile Phe Cys His Ser Lys Lys
    1385                1390                1395

Lys Cys Asp Glu Leu Ala Ala Lys Leu Ser Gly Leu Gly Val Asn
    1400                1405                1410

Ala Val Ala Tyr Tyr Arg Gly Leu Asp Val Ser Val Ile Pro Thr
    1415                1420                1425

Ser Gly Asp Val Val Val Val Ala Thr Asp Ala Leu Met Thr Gly
    1430                1435                1440

Tyr Thr Gly Asp Phe Asp Ser Val Ile Asp Cys Asn Thr Cys Val
    1445                1450                1455

Thr Gln Thr Val Asp Phe Ser Leu Asp Pro Thr Phe Thr Ile Glu
    1460                1465                1470

Thr Thr Thr Val Pro Gln Asp Ala Val Ser Arg Ser Gln Arg Arg
    1475                1480                1485

Gly Arg Thr Gly Arg Gly Arg Gly Gly Ile Tyr Arg Phe Val Thr
    1490                1495                1500

Pro Gly Glu Arg Pro Ser Gly Met Phe Asp Ser Ser Val Leu Cys
    1505                1510                1515

Glu Cys Tyr Asp Ala Gly Cys Ala Trp Tyr Glu Leu Thr Pro Ala
    1520                1525                1530

Glu Thr Ser Val Arg Leu Arg Ala Tyr Leu Asn Thr Pro Gly Leu
    1535                1540                1545

Pro Val Cys Gln Asp His Leu Glu Phe Trp Glu Gly Val Phe Thr
    1550                1555                1560

Gly Leu Thr His Ile Asp Ala His Phe Leu Ser Gln Thr Lys Gln
    1565                1570                1575

Ala Gly Asp Asn Phe Pro Tyr Leu Val Ala Tyr Gln Ala Thr Val
    1580                1585                1590

Cys Ala Arg Ala Gln Ala Pro Pro Pro Ser Trp Asp Gln Met Trp
    1595                1600                1605

Lys Cys Leu Ile Arg Leu Lys Pro Thr Leu His Gly Pro Thr Pro
    1610                1615                1620

Leu Leu Tyr Arg Leu Gly Ala Val Gln Asn Glu Val Thr Leu Thr
    1625                1630                1635

His Pro Met Thr Lys Tyr Ile Met Thr Cys Met Ser Ala Asp Leu
    1640                1645                1650

Glu Val Val Thr Ser Thr Trp Val Leu Val Gly Gly Val Leu Ala
    1655                1660                1665

Ala Leu Ala Ala Tyr Cys Leu Thr Thr Gly Ser Val Val Ile Ile
    1670                1675                1680

Gly Arg Ile Ile Leu Ser Gly Arg Pro Ala Ile Ile Pro Asp Arg
    1685                1690                1695

Glu Val Leu Tyr Gln Glu Phe Asp Glu Met Glu Glu Cys Ala Ser
    1700                1705                1710

His Leu Pro Tyr Ile Glu Gln Gly Met Gln Leu Ala Glu Gln Phe
    1715                1720                1725

Lys Gln Lys Ala Leu Gly Leu Leu Gln Thr Ala Thr Lys Gln Ala
    1730                1735                1740

Glu Ala Ala Ala Pro Val Val Glu Ser Lys Trp Gln Ala Leu Glu
    1745                1750                1755

Ala Phe Trp Ala Lys His Met Trp Asn Phe Ile Ser Gly Ile Gln
    1760                1765                1770

Tyr Leu Ala Gly Leu Ser Thr Leu Pro Gly Asn Pro Ala Ile Ala
    1775                1780                1785

Ser Leu Met Ala Phe Thr Ala Ser Ile Thr Ser Pro Leu Thr Thr
    1790                1795                1800

Leu His Thr Leu Leu Phe Asn Ile Leu Gly Gly Trp Val Ala Ala
    1805                1810                1815

Gln Leu Ala Pro Pro Ser Ala Ala Ser Ala Phe Val Gly Ala Gly
    1820                1825                1830

Ile Ala Gly Ala Ala Val Gly Ser Ile Gly Leu Gly Lys Val Leu
    1835                1840                1845

Val Asp Ile Leu Ala Gly Tyr Gly Ala Gly Val Ala Gly Ala Leu
    1850                1855                1860

Val Ala Phe Lys Val Met Ser Gly Glu Val Pro Ser Thr Glu Asp
    1865                1870                1875

Leu Val Asn Leu Leu Pro Ala Ile Leu Ser Pro Gly Ala Leu Val
    1880                1885                1890

Val Gly Val Val Cys Ala Ala Ile Leu Arg Arg His Val Gly Pro
    1895                1900                1905

Gly Glu Gly Ala Val Gln Trp Met Asn Arg Leu Ile Ala Phe Ala
    1910                1915                1920

Ser Arg Gly Asn His Val Ser Pro Thr His Tyr Val Pro Glu Ser
    1925                1930                1935

Asp Ala Ala Ala Arg Val Thr Gln Ile Leu Ser Ser Leu Thr Ile
    1940                1945                1950

Thr Gln Leu Leu Lys Arg Leu His Gln Trp Ile Asn Lys Asp Cys
    1955                1960                1965

Ser Thr Pro Cys Ser Gly Ser Trp Leu Arg Asp Val Trp Asp Trp
    1970                1975                1980

Ile Cys Thr Val Leu Thr Asp Phe Lys Thr Trp Leu Gln Ser Lys
    1985                1990                1995

Leu Leu Pro Arg Leu Pro Gly Leu Pro Phe Phe Ser Cys Gln Arg
    2000                2005                2010

Gly Tyr Lys Gly Val Trp Leu Gly Asp Gly Val Met Gln Thr Thr
    2015                2020                2025

Cys Pro Cys Gly Ala Gln Ile Ser Gly His Val Lys Asn Gly Ser
    2030                2035                2040

Met Lys Ile Val Gly Pro Lys Thr Cys Ser Asn Thr Trp His Gly
    2045                2050                2055

Thr Phe Pro Ile Asn Ala Tyr Thr Thr Gly Pro Cys Thr Pro Ser
    2060                2065                2070

Pro Ala Pro Asn Tyr Ser Lys Ala Leu Trp Arg Val Ala Ala Glu
    2075                2080                2085

Glu Tyr Val Glu Val Thr Arg Val Gly Asp Phe His Tyr Val Thr
    2090                2095                2100

Gly Met Thr Thr Asp Asn Val Lys Cys Pro Cys Gln Val Pro Ala
    2105                2110                2115

Pro Glu Phe Phe Thr Glu Leu Asp Gly Val Arg Leu His Arg Tyr
    2120                2125                2130

Ala Pro Ala Cys Lys Pro Leu Leu Arg Asp Glu Val Thr Phe Gln
    2135                2140                2145

Val Gly Leu Asn Gln Tyr Pro Val Gly Ser Gln Leu Pro Cys Glu
    2150                2155                2160

Pro Glu Pro Asp Val Thr Val Leu Thr Ser Met Leu Thr Asp Pro

2165                    2170                    2175

Ser His Ile Thr Ala Glu Thr Ala Lys Arg Arg Leu Ala Arg Gly
    2180                    2185                    2190

Ser Pro Pro Ser Leu Ala Ser Ser Ser Ala Ser Gln Leu Ser Ala
    2195                    2200                    2205

Pro Ser Leu Lys Ala Thr Cys Thr Thr His His Asp Ser Pro Asp
    2210                    2215                    2220

Ala Asp Leu Ile Glu Ala Asn Leu Leu Trp Arg Gln Glu Met Gly
    2225                    2230                    2235

Gly Asn Ile Thr Arg Val Glu Ser Glu Asn Lys Val Val Ile Leu
    2240                    2245                    2250

Asp Ser Phe Glu Pro Leu Arg Ala Glu Glu Asp Asp Arg Glu Val
    2255                    2260                    2265

Ser Val Ala Ala Glu Ile Leu Arg Arg Thr Arg Lys Phe Pro Ala
    2270                    2275                    2280

Ala Met Pro Ile Trp Ala Arg Pro Asp Tyr Asn Pro Pro Leu Leu
    2285                    2290                    2295

Glu Ser Trp Lys Asn Pro Asp Tyr Val Pro Pro Val Val His Gly
    2300                    2305                    2310

Cys Pro Leu Pro Pro Ile Lys Ala Pro Pro Ile Pro Pro Pro Arg
    2315                    2320                    2325

Arg Lys Arg Thr Val Ile Leu Thr Glu Ser Thr Val Ser Ser Ala
    2330                    2335                    2340

Leu Ala Glu Leu Ala Thr Lys Thr Phe Gly Ser Ser Gly Ser Ser
    2345                    2350                    2355

Ala Val Asp Ser Gly Thr Ala Thr Gly Pro Pro Asp Gln Ser Ser
    2360                    2365                    2370

Gly Asp Gly Asp Thr Gly Ser Asp Ala Glu Ser Cys Ser Ser Met
    2375                    2380                    2385

Pro Pro Leu Glu Gly Glu Pro Gly Asp Pro Asp Leu Ser Asp Gly
    2390                    2395                    2400

Ser Trp Ser Thr Val Ser Glu Glu Ala Gly Glu Asp Val Val Cys
    2405                2410                2415

Cys Ser Met Ser Tyr Thr Trp Thr Gly Ala Leu Ile Thr Pro Cys
    2420                2425                2430

Ala Ala Glu Glu Ser Lys Leu Pro Ile Asn Ala Leu Ser Asn Ser
    2435                2440                2445

Leu Leu Arg His His Asn Met Val Tyr Ala Thr Thr Ser Arg Ser
    2450                2455                2460

Ala Gly Gln Arg Gln Lys Lys Val Thr Phe Asp Arg Val Gln Val
    2465                2470                2475

Leu Asp Asp His Tyr Arg Asp Val Leu Lys Glu Met Lys Ala Lys
    2480                2485                2490

Ala Ser Thr Val Lys Ala Lys Leu Leu Pro Val Glu Glu Ala Cys
    2495                2500                2505

Arg Leu Thr Pro Pro His Ser Ala Arg Ser Lys Phe Gly Tyr Gly
    2510                2515                2520

Ala Lys Asp Val Arg Asn Leu Ser Ser Lys Ala Val Asn His Ile
    2525                2530                2535

His Ser Val Trp Lys Asp Leu Leu Glu Asp Ser Glu Thr Pro Ile
    2540                2545                2550

Asp Thr Thr Ile Met Ala Lys Asn Glu Val Phe Cys Val Gln Pro
    2555                2560                2565

Glu Lys Gly Gly Arg Lys Ser Ala Arg Leu Ile Val Phe Pro Asp
    2570                2575                2580

Leu Gly Val Arg Val Cys Glu Lys Met Ala Leu Tyr Asp Val Val
    2585                2590                2595

Ser Thr Leu Pro Gln Ala Val Met Gly Ser Ser Tyr Gly Phe Gln
    2600                2605                2610

Tyr Ser Pro Gly Gln Arg Val Glu Phe Leu Val Asn Ala Trp Lys
    2615                2620                2625

Ser Lys Lys Asn Pro Met Gly Phe Ala Tyr Asp Thr Arg Cys Phe
    2630                2635                2640

Asp Ser Thr Val Thr Glu Ser Asp Ile Arg Val Glu Glu Ser Ile
    2645                2650                2655

Tyr Gln Cys Cys Asp Leu Ala Pro Glu Ala Arg Gln Val Ile Arg
    2660                2665                2670

Ser Leu Thr Glu Arg Leu Tyr Ile Gly Gly Pro Leu Thr Asn Ser
    2675                2680                2685

Lys Gly Gln Ser Cys Gly Tyr Arg Arg Cys Arg Ala Ser Gly Val
    2690                2695                2700

Leu Thr Thr Ser Cys Gly Asn Thr Leu Thr Cys Tyr Leu Lys Ala
    2705                2710                2715

Ser Ala Ala Cys Arg Ala Ala Lys Leu Gln Asp Cys Thr Met Leu
    2720                2725                2730

Val Cys Gly Asp Asp Leu Val Val Ile Cys Glu Ser Ala Gly Thr
    2735                2740                2745

Gln Glu Asp Ala Ala Ser Leu Arg Val Phe Thr Glu Ala Met Thr
    2750                2755                2760

Arg Tyr Ser Ala Pro Pro Gly Asp Pro Pro Gln Pro Glu Tyr Asp
    2765                2770                2775

Leu Glu Leu Ile Thr Ser Cys Ser Ser Asn Val Ser Val Ala His
    2780                2785                2790

Asp Ala Leu Gly Lys Arg Val Tyr Tyr Leu Thr Arg Asp Pro Thr
    2795                2800                2805

Thr Pro Leu Ala Arg Ala Ala Trp Glu Thr Ala Arg His Thr Pro
    2810                2815                2820

Val Asn Ser Trp Leu Gly Asn Ile Ile Met Tyr Ala Pro Thr Leu
    2825                2830                2835

Trp Ala Arg Met Ile Leu Met Thr His Phe Phe Ser Ile Leu Leu
    2840                2845                2850

Ala Gln Glu Gln Leu Glu Lys Ala Leu Asp Cys Gln Ile Tyr Gly
    2855                2860                2865

Ala Thr Tyr Ser Ile Glu Pro Leu Asp Leu Pro Gln Ile Ile Gln
    2870                2875                2880

Arg Leu His Gly Leu Ser Ala Phe Ser Leu His Ser Tyr Ser Pro
    2885                2890                2895

Gly Glu Ile Asn Arg Val Ala Ser Cys Leu Arg Lys Leu Gly Val
    2900                2905                2910

Pro Pro Leu Arg Val Trp Arg His Arg Ala Arg Ser Val Arg Ala
    2915                2920                2925

Lys Leu Leu Ser Gln Gly Gly Arg Ala Ala Thr Cys Gly Lys Tyr
    2930                2935                2940

Leu Phe Asn Trp Ala Val Arg Thr Lys Leu Lys Leu Thr Pro Ile
    2945                2950                2955

Pro Ala Ala Ser Gln Leu Asp Leu Ser Gly Trp Phe Ile Ala Gly
    2960                2965                2970

Tyr Ser Gly Gly Asp Ile Tyr His Ser Leu Ser Arg Ala Arg Pro
    2975                2980                2985

Arg Trp Phe Met Trp Cys Leu Leu Leu Leu Ser Val Gly Val Gly
    2990                2995                3000

Ile Tyr Leu Leu Pro Asn Arg
    3005                3010

<210> 63
<211> 9594
<212> RNA
<213> Hepatitis C virus

<400> 63
gccagccccc ugaugggggc gacacuccac cauagaucac uccccuguga ggaacuacug 60

ucuucacgca gaaagcgucu agccauggcg uuaguaugag ugucgugcag ccuccaggac 120

cccccucccc gggagagcca uaguggucug cggaaccggu gaguacaccg gaauugccag 180

gacgaccggg uccuuucuug gaucaacccg cucaaugccu ggagauuugg gcgugccccc 240

gcgagacugc uagccgagua guguuggguc gcgaaaggcc uugugguacu gccugauagg 300

gugcuugcga gugccccggg aggucucgua gaccgugcau caugagcaca aauccuaaac 360

cucaaagaaa aaccaaacgu aacaccaacc gccgcccaca ggacgucaag uucccgggcg 420
guggucagau cguuggugga guuuaccugu ugccgcgcag gggccccagg uuggguguge 480
gcgcgacuag gaagacuucc gagcggucgc aaccucguga aaggcggcaa ccuaucccca 540
aggcucgccg ccccgagggc agggccuggg cucagcccgg guacccuugg ccccucuaug 600
gcaaugaggg ucuggggugg gcagggugge uccugucacc ccgcggcucc cggccuaauu 660
ggggccccac ggacccccgg cguaggucgc guaauuuggg uaaggucauc gauacccuca 720
caugcggcuu cgccgaccuc augggguaca uuccgcucgu cggcgccccc cuaggaggcg 780
uugccagggc ccuggcgcau ggcguccggg uucuggagga cggcgugaac uaugcaacag 840
ggaauuugcc cgguugcucu uucucuaucu uccucuuggc uuugcugucc uguuugacug 900
ucccagcuuc cgcuuaugaa gugcgcaacg uguccgggac auaccauguc acgaacgacu 960
gcgccaacuc aagcauugug uaugaggcag cggacauaau caugcacacc ccugggugcg 1020
ugcccugcgu ccgggagggc aauuccuccc gcugcugggu agcgcucacu cccacgcucg 1080
cggccaggaa caggagcauc cccacuacga cgauacgacg ccacgucgau uugcucguug 1140
gggcggcugc cuucugcucc gccauguacg ugggggaucu cugcgggucu guuuuccuag 1200
ucucccaacu guucaccuuc ucaccucgcc gguaugagac gguacaagaa ugcaauugcu 1260
caaucuaucc cggccacgua ucaggucacc gcauggcuug ggauaugaug augaauuggu 1320
cgcccacagc agcucuagug gugucacagu uacuccgaau cccacaagcc gucguggaua 1380
ugguggcagg ggcccacugg gggguucugg cgggucuugc cuacuauucc auggugggga 1440
acugggcuaa agucuugauu gugaugcuac ucuuugccgg cguugacggg accaccaccg 1500
ugacaggggg agcggcggcc uuuggcacca ggagccuugc guccuucuuu acauuagggc 1560
cgucucagaa aauccaacuu guaaacacca acggcaguug gcacaucaau aggacugccu 1620
ugaauuguaa ugacucccuc cagacugggu uccuugcugc gcuguucuau gcacacaaga 1680
ucaacacguc uggaugccca gagcgcaugg ccagcugccg cccuauugac aaguucgcuc 1740
aggggugggg ucccaucacc cauggugcgc cugacaccuc ggaccagagg cccuacugcu 1800
ggcacuacgc accucgaccg ugugguaucg uacccgcguc ggaggugugu ggcccagugu 1860
auuguuucac cccaagcccu guaguggugg ggacgaccga ucgcuucggc guccccacgu 1920
auagcugggg ggagaauaag acggacgugc ugcuccucaa caacacgcgg ccgccgcagg 1980
gcaauugguu cggcugcaca uggaugaaug gcaccggguu caccaagacg ugugggggcc 2040
ccccguguga uaucgggggg gucggcaaca acaccuugac cugccccacg gauugcuucc 2100
ggaagcaccc cgaggccacu uauaccaaau gcggcucggg gccuugguug acgccuaggu 2160

gucuaguuga cuacccauac aggcuuuggc acuaccccug cacugucaac uuuaccaucu 2220
ucaaggucag gauguaugug gggggcgugg agcacaggcu caacgcugcg ugcaacugga 2280
cucgaggaga gcgcugugac cuagaggaca gggacagauc agagcuuagc ccgcugcuac 2340
ugucuacaac agaguggcag auacugcccu guuccuucac cacccuaccg gcucugucca 2400
cuggcuugau ccaccuccau cagaacaucg uggacgugca auaccuguac gguguagggu 2460
cagcaguugu cuccguugug aucagauggg aguacguccu gcugcucuuc cuccuccugg 2520
cggacgcgcg cguuugugcc ugcuuaugga ugaugcuguu gauagcccag gcugaagccg 2580
ccuuggagaa ccugguaguc cucaacgcgg cguccguggc cggagcgcau ggcauccucu 2640
cuuuccuugu guucuucugc gcugccuggu acaucaaagg caagcuagug ccuggggugg 2700
cguaugcucu cuauggugua uggccgcugc uccugcuccu gcuggcguug ccacaacggg 2760
cguacgccau ggaccgggag auggcugcau cgugcggagg cgcgguuuuc gugggucugg 2820
uacucuugac cuugucacca cacuauaaag cauuccuugc caaguccaua uggugguuac 2880
aauauuuaau caccagggcc gaggcgcauu ugcaagugug gguccccccc cucaacguuc 2940
gggggggccg cgaugccauc auccuccuca cgugcgcggu ccacccagag cuaauuuuug 3000
acaucaccaa aauuuugcuc gccauauuug gcccgcucau ggugcuccag gcuggucuaa 3060
cuagagugcc guacuuugug cgcgcucaag ggcucauccg ugugugcaug uuggugcgga 3120
aagucgcugg gggucauuau auccaaaugg cucucaugag guuggccgca cugacgggca 3180
cguacguuua ugaucaucuu acuccgcugc gggacugggc ccaugcgggc uugcgagacc 3240
uugcgguggc aguugagccc gucguuuucu ccaacacaga gaccaagauu aucaccuggg 3300
gggcggacac cgcggcgugu ggggacauca ucuugggucu accugucucc gcccgaaggg 3360
ggagagaggu acuccuagga ccggccgaua acuuuggaga gcaagggugg cgacuccuug 3420
cgcccaucac ggccuacucc caacagacgc ggggcuuacu uggcuguauc aucaccggcc 3480
ucacaggucg agacaagaac caggucgaag gggagguuca ggugguuucc accgcaacac 3540
agucuuucuu ggcaaccugc gucaacggug uguguuggac ugucuaccau ggugccggcu 3600
caaagacccu agccggcccg aaggggccaa ucacccagau guacaccaac guagaccaag 3660
accucguugg cuggcaggcg ccccucgggg cgcguuccau gacgccgugc accugcggca 3720
gcucggaccu uuacuugguc acgaggcaug cugaugucau uccggugcgc cggcggggug 3780
acagcagagg gagccuacuu ucccccaggc ccgucuccua cuugaagggc ucuucaggug 3840
guccacugcu cugccccuug gggcacgucg ugggcaucuu ucgggcugcc gugugcaccc 3900
gggggguugc gaaggcggug gacuucauac ccaucgaguc uauggaaacu accaugcggu 3960
cuccggucuu uacggauaau ucaucucccc cggccguacc gcagacuuuu caaguggccc 4020

aucugcacgc ccccacuggc agcggcaaga gcaccaaggu gccagcugcg uaugcagccc 4080

agggguacaa ggugcuuguc uugaauccau ccguugccgc caccuugggu uuuggggcgu 4140

auauguccaa ggcauacggu gucgacccua acauuagaac ugggguaagg accaucacca 4200

caggcgcucc caucacguac uccaccuacg gcaaguuccu ugccgacggu gguugcuccg 4260

ggggcgcuua cgacaucaua auaugcgaug agugccacuc aaccgacucg acuuccauuu 4320

ugggcauugg cacgguccug gaucaagcgg agacggcugg agcgcgacuc gucgugcucg 4380

ccaccgcuac gccuccggga ucggucacug ugccacaccc caacaucgag gagguggccu 4440

uguccaacac cggagagauu cccuucuaug gcaaagccau cccccucgag gucaucaagg 4500

gggggaggca ucucauuuuc ugucauucua agaagaagug ugaugagcuc gcugcaaagc 4560

ugucgggccu uggggucaac gcuguagcgu acuaccgggg ucuugaugug uccgucauac 4620

caacaagcgg ggacgucguu gucguggcaa cagacgcucu aaugacgggc uacaccggug 4680

acuuugacuc ugugaucgac uguaauacau gugucaccca gacagucgac uucagccugg 4740

accccaccuu caccauugag acgacgaccg ugccccaaga cgcagugucg cgcucgcagc 4800

ggcgagggag gacugguagg gguagagggg gcauauacag guuugugacu ccaggagagc 4860

ggcccucggg cauguucgau uccucggucc ugugugaaug cuaugacgcg ggcugugcuu 4920

gguacgagcu cacgcccgcc gagaccucgg uuaggcugcg ggcuuaccua aauacaccag 4980

gguugcccgu cugccaggac cauuuggagu ucugggaagg cgucuucaca ggccucacuc 5040

auauagaugc ccacuucuug ucucagacua agcaggcagg agacaacuuc cccuaccugg 5100

uggcauacca ggccacagug ugcgccaggg cccaggcacc accuccauca ugggaucaaa 5160

uguggaagug ucucauacgg cuaaaaccua cacuacacgg gccaacaccc cuguuguaca 5220

ggcuaggagc cguccaaaac gaggucaccc ucacacaccc caugaccaaa uacaucauga 5280

caugcauguc ggcugaccua gaggucguca ccagcacuug ggugcuggug ggcggggucc 5340

ucgcagcuuu ggccgcguac ugcuugacaa cgggcagcgu gguuaucaua ggcaggauca 5400

ucuuguccgg aaggccggcu aucauucccg auagggaagu ucucuaccag gaguucgaug 5460

aaauggaaga gugcgccuca caccuucccu acaucgaaca aggaaugcag cucgccgagc 5520

aauucaagca gaaggcgcuc ggguuguugc aaacggccac caagcaagcg gaggccgcug 5580

cucccguggu ggaguccaaa uggcaagccc uugaggccuu cugggcgaag cacaugugga 5640

acuucaucag cgggauacag uaucuagcag gcuuguccac ucugccugga aaccccgcga 5700

uagcaucgcu gauggcauuu acagccucua ucacuagccc gcucaccacc cuacauaccc 5760

uucuauuuaa caucuugggg ggaugggugg ccgcccaacu cgcccccccc agcgcugcuu 5820

cagccuucgu aggcgccggc aucgccggcg cggcuguugg cagcauaggu cuugggaagg 5880

ugcucgugga cauccuagcg gguuauggag cagggguggc aggcgcacuc guggccuuca 5940

aggucaugag cggcgaagug cccuccacug aggaccuggu caacuuacuc ccugccaucc 6000

ucuccccugg ugcccugguc gucggggucg ugugcgcagc gauacugcgu cggcaugugg 6060

gcccagggga gggggccgug caguggauga accggcugau agcguucgcu ucgcggggua 6120

accacgucuc ccccacgcac uaugugccug agagcgacgc cgcagcgcgu gucacccaga 6180

uccucuccag ccuuaccauc acucagcugc uaaagaggcu ccaccagugg auuaauaagg 6240

acuguuccac gccaugcucc gguucguggc ucagggaugu uugggacugg auaugcacgg 6300

uuuugaccga cuucaaaacc uggcuccagu ccaagcuccu gccacgguug ccgggacucc 6360

cuuucuuuuc augucaacgu ggauauaaag gagucuggcu gggagauggc guuaugcaaa 6420

cuaccugucc augguggca caaaucagcg gacaugucaa aaacggcucc augaagaucg 6480

uggggccuaa aaccugcagc aacacguggc acgggacguu ccccaucaac gcauacacca 6540

caggccccug cacacccucc ccggcgccga acuauuccaa ggcguugugg cgaguggcug 6600

cugaggagua uguggagguc acgcgggugg gggauuucca cuacgugacg ggcaugacca 6660

cugacaacgu aaaaugccca ugccaggucc cggcccccga auucuucacg gaguuggaug 6720

gggugcggcu gcacagguac gcuccggcgu gcaagccucu cuuacgggau gaggucacau 6780

uccaggucgg gcucaaccag uauccgguug gaucacagcu uccaugugag cccgagccgg 6840

auguaacagu gcucacuucc augcucaccg accccuccca uauuacagca gagacggcua 6900

agcguaggcu ggccagaggg uccccccccu cuuuggccag cuccucagcu agccaguugu 6960

cugcgccuuc cuugaaggcg acaugcacua cccaccauga cuccccagau gcugaccuca 7020

ucgaggccaa ccuccugugg cggcaggaga ugggcgggaa caucacccgc guggagucag 7080

agaacaaggu aguaauucug gacucuuuug aaccgcuucg agcggaggag gaugauaggg 7140

aaguguccgu agcggcggag auccugcgga gaaccaggaa auuccccgca gcgaugccca 7200

uaugggcacg gccggacuac aacccaccac uccuagaguc uuggaagaac ccagacuacg 7260

ucccuccagu gguacacggg ugcccauuac caccuauuaa ggccccuccg auaccaccuc 7320

cacggagaaa gaggacgguc auccugacag aauccaccgu gucuucugcc uuggcggagc 7380

uugcuacaaa gaccuuuggc agcuccggau cgucggccgu ugacagcggc acagcgacug 7440

gcccuccuga ucaguccucc ggugacggag auacaggauc cgacgcugag ucgugcuccu 7500

ccaugccccc ccuugagggg gagccggggg accccgaucu cagcgacggg ucuuggucua 7560

ccgugagcga ggaggccggu gaggacgucg ucugcugcuc gauguccuac acauggacag 7620

gcgccuuaau cacaccgugc gccgcagagg agagcaagcu gccuaucaac gcguugagca 7680

acucuuugcu gcgccaccac aacauggucu augccacaac aucccgcagc gcgggccaac 7740
ggcagaagaa ggucacuuuu gacagagugc agguccugga cgaccauuac cgggacgugc 7800
ucaaggagau gaaggcgaag gcguccacag uuaaggcuaa acuucuaccu guagaagaag 7860
ccugcaggcu gacgccccca cacucggcca gauccaaguu uggcuauggg gcgaaggacg 7920
uccggaaccu auccagcaag gccgucaacc acauccacuc cguguggaag gacuugcugg 7980
aagacucuga gacgccgauu gacaccacca ucauggcaaa aaaugagguc uuuuguguuc 8040
aaccagagaa aggaggccgc aagucagcuc gucuuaucgu auucccagac uuggggguuc 8100
gugugugcga gaaaauggcc cuuuacgacg ugguuuccac ucuuccucag gccgugaugg 8160
gcuccucaua cggguuccag uacucuccug gacagcgggu cgaguuccug gugaaugccu 8220
ggaaaucaaa gaagaacccu augggcuucg cauaugacac ccgcuguuuu gacucaacgg 8280
ucacugagag ugacauccgu guugaggagu caauuuacca auguugugac uuggcccccg 8340
aggccagaca ggucauaagg ucgcucacgg agcggcuuua uaucgggggc ccccugacua 8400
auucaaaagg gcagagcugc ggcuaucgcc ggugccgcgc cagcggugug cugacgacca 8460
gcugcgguaa uacccucaca uguuacuuga aggcuucugc agccugucga gcugcaaagc 8520
uccaggacug cacaauguua gugugcggag acgaccuugu cguuaucugu gaaagugcgg 8580
gaacccagga ggacgcggcg agccuacgag ucuucacgga ggcuaugacu agguacucug 8640
ccccccccgg ggacccgccc cagccagaau acgacuugga gcugauaaca ucaugcuccu 8700
ccaacgucuc ggucgcgcac gaugcacuug gcaagcgggu guauuaucug acccgcgacc 8760
ccaccacccc ccuugcgcgg gcugcguggg agacagcaag acacacucca guuaacuccu 8820
ggcuaggcaa caucaucaug uaugcgccca cccuaugggc aaggaugauu cugaugaccc 8880
acuucuuuuc cauccuucua gcucaggaac aacuugaaaa agcccuagau ugucagaucu 8940
acggggccac uuacuccauu gagccacuug accuaccuca gaucauucag cgacuccacg 9000
gccuuagcgc auuuucacuc cauagcuacu cuccagguga gaucaauagg guggcuucau 9060
gccucaggaa acuuggggua ccacccuugc gagucuggag acaucgggcc agaagugucc 9120
gcgcuaagcu acugucccag gggggggagg ccgccacuug uggcaaauac cucuucaacu 9180
gggcaguaag gaccaagcuc aaacucacuc caauuccggc ugcgucccag uuggacuugu 9240
ccggcugguu cauugcuggu uacagcgggg gagacauaua ucacagccug ucucgcgccc 9300
gaccccgcug guucaugugg ugccuacucc uacuuuccgu agggguaggc aucuaucugc 9360
uccccaaucg augaacgggg ggcuaaacac uccaggccaa uaggccauuc uguuuuuuuu 9420
uuuuuuuuuu uuuuuuuuuu uuuuuuuuuu uuuuuuuuuu uuuuuuccuu uuuuuuuuuu 9480

uuuuucccuu ucuuuuggug gcuccaucuu agcccuaguc acggcuagcu gugaaagguc    9540

cgugagccgc augacugcag agagugcuga uacuggccuc ucugcagauc augu          9594

<210>  64
<211>  3010
<212>  PRT
<213>  Hepatitis C virus

<400>  64

Met Ser Thr Asn Pro Lys Pro Gln Arg Lys Thr Lys Arg Asn Thr Asn
1               5                   10                  15

Arg Arg Pro Gln Asp Val Lys Phe Pro Gly Gly Gly Gln Ile Val Gly
            20                  25                  30

Gly Val Tyr Leu Leu Pro Arg Arg Gly Pro Arg Leu Gly Val Arg Ala
        35                  40                  45

Thr Arg Lys Thr Ser Glu Arg Ser Gln Pro Arg Glu Arg Arg Gln Pro
    50                  55                  60

Ile Pro Lys Ala Arg Arg Pro Glu Gly Arg Ala Trp Ala Gln Pro Gly
65                  70                  75                  80

Tyr Pro Trp Pro Leu Tyr Gly Asn Glu Gly Leu Gly Trp Ala Gly Trp
                85                  90                  95

Leu Leu Ser Pro Arg Gly Ser Arg Pro Asn Trp Gly Pro Thr Asp Pro
            100                 105                 110

Arg Arg Arg Ser Arg Asn Leu Gly Lys Val Ile Asp Thr Leu Thr Cys
        115                 120                 125

Gly Phe Ala Asp Leu Met Gly Tyr Ile Pro Leu Val Gly Ala Pro Leu
    130                 135                 140

Gly Gly Val Ala Arg Ala Leu Ala His Gly Val Arg Val Leu Glu Asp
145                 150                 155                 160

Gly Val Asn Tyr Ala Thr Gly Asn Leu Pro Gly Cys Ser Phe Ser Ile
                165                 170                 175

Phe Leu Leu Ala Leu Leu Ser Cys Leu Thr Val Pro Ala Ser Ala Tyr
            180                 185                 190

Glu Val Arg Asn Val Ser Gly Thr Tyr His Val Thr Asn Asp Cys Ala

195                 200                 205

Asn Ser Ser Ile Val Tyr Glu Ala Ala Asp Ile Ile Met His Thr Pro
    210                 215                 220

Gly Cys Val Pro Cys Val Arg Glu Gly Asn Ser Ser Arg Cys Trp Val
225                 230                 235                 240

Ala Leu Thr Pro Thr Leu Ala Ala Arg Asn Arg Ser Ile Pro Thr Thr
                245                 250                 255

Thr Ile Arg Arg His Val Asp Leu Leu Val Gly Ala Ala Ala Phe Cys
            260                 265                 270

Ser Ala Met Tyr Val Gly Asp Leu Cys Gly Ser Val Phe Leu Val Ser
        275                 280                 285

Gln Leu Phe Thr Phe Ser Pro Arg Arg Tyr Glu Thr Val Gln Glu Cys
    290                 295                 300

Asn Cys Ser Ile Tyr Pro Gly His Val Ser Gly His Arg Met Ala Trp
305                 310                 315                 320

Asp Met Met Met Asn Trp Ser Pro Thr Ala Ala Leu Val Val Ser Gln
                325                 330                 335

Leu Leu Arg Ile Pro Gln Ala Val Val Asp Met Val Ala Gly Ala His
            340                 345                 350

Trp Gly Val Leu Ala Gly Leu Ala Tyr Tyr Ser Met Val Gly Asn Trp
        355                 360                 365

Ala Lys Val Leu Ile Val Met Leu Leu Phe Ala Gly Val Asp Gly Thr
    370                 375                 380

Thr Thr Val Thr Gly Gly Ala Ala Ala Phe Gly Thr Arg Ser Leu Ala
385                 390                 395                 400

Ser Phe Phe Thr Leu Gly Pro Ser Gln Lys Ile Gln Leu Val Asn Thr
                405                 410                 415

Asn Gly Ser Trp His Ile Asn Arg Thr Ala Leu Asn Cys Asn Asp Ser
            420                 425                 430

Leu Gln Thr Gly Phe Leu Ala Ala Leu Phe Tyr Ala His Lys Ile Asn
        435                 440                 445

Thr Ser Gly Cys Pro Glu Arg Met Ala Ser Cys Arg Pro Ile Asp Lys
    450                 455                 460

Phe Ala Gln Gly Trp Gly Pro Ile Thr His Gly Ala Pro Asp Thr Ser
465                 470                 475                 480

Asp Gln Arg Pro Tyr Cys Trp His Tyr Ala Pro Arg Pro Cys Gly Ile
                485                 490                 495

Val Pro Ala Ser Glu Val Cys Gly Pro Val Tyr Cys Phe Thr Pro Ser
            500                 505                 510

Pro Val Val Val Gly Thr Thr Asp Arg Phe Gly Val Pro Thr Tyr Ser
        515                 520                 525

Trp Gly Glu Asn Lys Thr Asp Val Leu Leu Leu Asn Asn Thr Arg Pro
    530                 535                 540

Pro Gln Gly Asn Trp Phe Gly Cys Thr Trp Met Asn Gly Thr Gly Phe
545                 550                 555                 560

Thr Lys Thr Cys Gly Gly Pro Pro Cys Asp Ile Gly Gly Val Gly Asn
                565                 570                 575

Asn Thr Leu Thr Cys Pro Thr Asp Cys Phe Arg Lys His Pro Glu Ala
            580                 585                 590

Thr Tyr Thr Lys Cys Gly Ser Gly Pro Trp Leu Thr Pro Arg Cys Leu
        595                 600                 605

Val Asp Tyr Pro Tyr Arg Leu Trp His Tyr Pro Cys Thr Val Asn Phe
    610                 615                 620

Thr Ile Phe Lys Val Arg Met Tyr Val Gly Gly Val Glu His Arg Leu
625                 630                 635                 640

Asn Ala Ala Cys Asn Trp Thr Arg Gly Glu Arg Cys Asp Leu Glu Asp
                645                 650                 655

Arg Asp Arg Ser Glu Leu Ser Pro Leu Leu Leu Ser Thr Thr Glu Trp
            660                 665                 670

Gln Ile Leu Pro Cys Ser Phe Thr Thr Leu Pro Ala Leu Ser Thr Gly
        675                 680                 685

Leu Ile His Leu His Gln Asn Ile Val Asp Val Gln Tyr Leu Tyr Gly
    690                 695                 700

Val Gly Ser Ala Val Val Ser Val Val Ile Arg Trp Glu Tyr Val Leu
705                 710                 715                 720

Leu Leu Phe Leu Leu Leu Ala Asp Ala Arg Val Cys Ala Cys Leu Trp
                725                 730                 735

Met Met Leu Leu Ile Ala Gln Ala Glu Ala Ala Leu Glu Asn Leu Val
            740                 745                 750

Val Leu Asn Ala Ala Ser Val Ala Gly Ala His Gly Ile Leu Ser Phe
        755                 760                 765

Leu Val Phe Phe Cys Ala Ala Trp Tyr Ile Lys Gly Lys Leu Val Pro
    770                 775                 780

Gly Val Ala Tyr Ala Leu Tyr Gly Val Trp Pro Leu Leu Leu Leu Leu
785                 790                 795                 800

Leu Ala Leu Pro Gln Arg Ala Tyr Ala Met Asp Arg Glu Met Ala Ala
                805                 810                 815

Ser Cys Gly Gly Ala Val Phe Val Gly Leu Val Leu Leu Thr Leu Ser
            820                 825                 830

Pro His Tyr Lys Ala Phe Leu Ala Lys Ser Ile Trp Trp Leu Gln Tyr
        835                 840                 845

Leu Ile Thr Arg Ala Glu Ala His Leu Gln Val Trp Val Pro Pro Leu
    850                 855                 860

Asn Val Arg Gly Gly Arg Asp Ala Ile Ile Leu Leu Thr Cys Ala Val
865                 870                 875                 880

His Pro Glu Leu Ile Phe Asp Ile Thr Lys Ile Leu Leu Ala Ile Phe
                885                 890                 895

Gly Pro Leu Met Val Leu Gln Ala Gly Leu Thr Arg Val Pro Tyr Phe
            900                 905                 910

Val Arg Ala Gln Gly Leu Ile Arg Val Cys Met Leu Val Arg Lys Val
        915                 920                 925

Ala Gly Gly His Tyr Ile Gln Met Ala Leu Met Arg Leu Ala Ala Leu
    930                 935                 940

Thr Gly Thr Tyr Val Tyr Asp His Leu Thr Pro Leu Arg Asp Trp Ala
945                 950                 955                 960

His Ala Gly Leu Arg Asp Leu Ala Val Ala Val Glu Pro Val Val Phe
                965                 970                 975

Ser Asn Thr Glu Thr Lys Ile Ile Thr Trp Gly Ala Asp Thr Ala Ala
            980                 985                 990

Cys Gly Asp Ile Ile Leu Gly Leu  Pro Val Ser Ala Arg  Arg Gly Arg
        995                 1000                 1005

Glu Val  Leu Leu Gly Pro Ala  Asp Asn Phe Gly Glu  Gln Gly Trp
    1010                 1015                 1020

Arg Leu  Leu Ala Pro Ile Thr  Ala Tyr Ser Gln Gln  Thr Arg Gly
    1025                 1030                 1035

Leu Leu  Gly Cys Ile Ile Thr  Gly Leu Thr Gly Arg  Asp Lys Asn
    1040                 1045                 1050

Gln Val  Glu Gly Glu Val Gln  Val Val Ser Thr Ala  Thr Gln Ser
    1055                 1060                 1065

Phe Leu  Ala Thr Cys Val Asn  Gly Val Cys Trp Thr  Val Tyr His
    1070                 1075                 1080

Gly Ala  Gly Ser Lys Thr Leu  Ala Gly Pro Lys Gly  Pro Ile Thr
    1085                 1090                 1095

Gln Met  Tyr Thr Asn Val Asp  Gln Asp Leu Val Gly  Trp Gln Ala
    1100                 1105                 1110

Pro Leu  Gly Ala Arg Ser Met  Thr Pro Cys Thr Cys  Gly Ser Ser
    1115                 1120                 1125

Asp Leu  Tyr Leu Val Thr Arg  His Ala Asp Val Ile  Pro Val Arg
    1130                 1135                 1140

Arg Arg  Gly Asp Ser Arg Gly  Ser Leu Leu Ser Pro  Arg Pro Val
    1145                 1150                 1155

Ser Tyr  Leu Lys Gly Ser Ser  Gly Gly Pro Leu Leu  Cys Pro Leu

1160                 1165                 1170

Gly His  Val Val Gly Ile Phe  Arg Ala Ala Val Cys  Thr Arg Gly
    1175                 1180                 1185

Val Ala  Lys Ala Val Asp Phe  Ile Pro Ile Glu Ser  Met Glu Thr
    1190                 1195                 1200

Thr Met  Arg Ser Pro Val Phe  Thr Asp Asn Ser Ser  Pro Pro Ala
    1205                 1210                 1215

Val Pro  Gln Thr Phe Gln Val  Ala His Leu His Ala  Pro Thr Gly
    1220                 1225                 1230

Ser Gly  Lys Ser Thr Lys Val  Pro Ala Ala Tyr Ala  Ala Gln Gly
    1235                 1240                 1245

Tyr Lys  Val Leu Val Leu Asn  Pro Ser Val Ala Ala  Thr Leu Gly
    1250                 1255                 1260

Phe Gly  Ala Tyr Met Ser Lys  Ala Tyr Gly Val Asp  Pro Asn Ile
    1265                 1270                 1275

Arg Thr  Gly Val Arg Thr Ile  Thr Thr Gly Ala Pro  Ile Thr Tyr
    1280                 1285                 1290

Ser Thr  Tyr Gly Lys Phe Leu  Ala Asp Gly Gly Cys  Ser Gly Gly
    1295                 1300                 1305

Ala Tyr  Asp Ile Ile Ile Cys  Asp Glu Cys His Ser  Thr Asp Ser
    1310                 1315                 1320

Thr Ser  Ile Leu Gly Ile Gly  Thr Val Leu Asp Gln  Ala Glu Thr
    1325                 1330                 1335

Ala Gly  Ala Arg Leu Val Val  Leu Ala Thr Ala Thr  Pro Pro Gly
    1340                 1345                 1350

Ser Val  Thr Val Pro His Pro  Asn Ile Glu Glu Val  Ala Leu Ser
    1355                 1360                 1365

Asn Thr  Gly Glu Ile Pro Phe  Tyr Gly Lys Ala Ile  Pro Leu Glu
    1370                 1375                 1380

Val Ile  Lys Gly Gly Arg His  Leu Ile Phe Cys His  Ser Lys Lys
    1385                 1390                 1395

Lys Cys Asp Glu Leu Ala Ala Lys Leu Ser Gly Leu Gly Val Asn
    1400                1405                1410

Ala Val Ala Tyr Tyr Arg Gly Leu Asp Val Ser Val Ile Pro Thr
    1415                1420                1425

Ser Gly Asp Val Val Val Val Ala Thr Asp Ala Leu Met Thr Gly
    1430                1435                1440

Tyr Thr Gly Asp Phe Asp Ser Val Ile Asp Cys Asn Thr Cys Val
    1445                1450                1455

Thr Gln Thr Val Asp Phe Ser Leu Asp Pro Thr Phe Thr Ile Glu
    1460                1465                1470

Thr Thr Thr Val Pro Gln Asp Ala Val Ser Arg Ser Gln Arg Arg
    1475                1480                1485

Gly Arg Thr Gly Arg Gly Arg Gly Gly Ile Tyr Arg Phe Val Thr
    1490                1495                1500

Pro Gly Glu Arg Pro Ser Gly Met Phe Asp Ser Ser Val Leu Cys
    1505                1510                1515

Glu Cys Tyr Asp Ala Gly Cys Ala Trp Tyr Glu Leu Thr Pro Ala
    1520                1525                1530

Glu Thr Ser Val Arg Leu Arg Ala Tyr Leu Asn Thr Pro Gly Leu
    1535                1540                1545

Pro Val Cys Gln Asp His Leu Glu Phe Trp Glu Gly Val Phe Thr
    1550                1555                1560

Gly Leu Thr His Ile Asp Ala His Phe Leu Ser Gln Thr Lys Gln
    1565                1570                1575

Ala Gly Asp Asn Phe Pro Tyr Leu Val Ala Tyr Gln Ala Thr Val
    1580                1585                1590

Cys Ala Arg Ala Gln Ala Pro Pro Pro Ser Trp Asp Gln Met Trp
    1595                1600                1605

Lys Cys Leu Ile Arg Leu Lys Pro Thr Leu His Gly Pro Thr Pro
    1610                1615                1620

Leu Leu Tyr Arg Leu Gly Ala Val Gln Asn Glu Val Thr Leu Thr
    1625                1630                1635

His Pro Met Thr Lys Tyr Ile Met Thr Cys Met Ser Ala Asp Leu
    1640                1645                1650

Glu Val Val Thr Ser Thr Trp Val Leu Val Gly Gly Val Leu Ala
    1655                1660                1665

Ala Leu Ala Ala Tyr Cys Leu Thr Thr Gly Ser Val Val Ile Ile
    1670                1675                1680

Gly Arg Ile Ile Leu Ser Gly Arg Pro Ala Ile Ile Pro Asp Arg
    1685                1690                1695

Glu Val Leu Tyr Gln Glu Phe Asp Glu Met Glu Glu Cys Ala Ser
    1700                1705                1710

His Leu Pro Tyr Ile Glu Gln Gly Met Gln Leu Ala Glu Gln Phe
    1715                1720                1725

Lys Gln Lys Ala Leu Gly Leu Leu Gln Thr Ala Thr Lys Gln Ala
    1730                1735                1740

Glu Ala Ala Ala Pro Val Val Glu Ser Lys Trp Gln Ala Leu Glu
    1745                1750                1755

Ala Phe Trp Ala Lys His Met Trp Asn Phe Ile Ser Gly Ile Gln
    1760                1765                1770

Tyr Leu Ala Gly Leu Ser Thr Leu Pro Gly Asn Pro Ala Ile Ala
    1775                1780                1785

Ser Leu Met Ala Phe Thr Ala Ser Ile Thr Ser Pro Leu Thr Thr
    1790                1795                1800

Leu His Thr Leu Leu Phe Asn Ile Leu Gly Gly Trp Val Ala Ala
    1805                1810                1815

Gln Leu Ala Pro Pro Ser Ala Ala Ser Ala Phe Val Gly Ala Gly
    1820                1825                1830

Ile Ala Gly Ala Ala Val Gly Ser Ile Gly Leu Gly Lys Val Leu
    1835                1840                1845

Val Asp Ile Leu Ala Gly Tyr Gly Ala Gly Val Ala Gly Ala Leu
    1850                1855                1860

Val Ala Phe Lys Val Met Ser Gly Glu Val Pro Ser Thr Glu Asp
    1865                1870                1875

Leu Val Asn Leu Leu Pro Ala Ile Leu Ser Pro Gly Ala Leu Val
    1880                1885                1890

Val Gly Val Val Cys Ala Ala Ile Leu Arg Arg His Val Gly Pro
    1895                1900                1905

Gly Glu Gly Ala Val Gln Trp Met Asn Arg Leu Ile Ala Phe Ala
    1910                1915                1920

Ser Arg Gly Asn His Val Ser Pro Thr His Tyr Val Pro Glu Ser
    1925                1930                1935

Asp Ala Ala Ala Arg Val Thr Gln Ile Leu Ser Ser Leu Thr Ile
    1940                1945                1950

Thr Gln Leu Leu Lys Arg Leu His Gln Trp Ile Asn Lys Asp Cys
    1955                1960                1965

Ser Thr Pro Cys Ser Gly Ser Trp Leu Arg Asp Val Trp Asp Trp
    1970                1975                1980

Ile Cys Thr Val Leu Thr Asp Phe Lys Thr Trp Leu Gln Ser Lys
    1985                1990                1995

Leu Leu Pro Arg Leu Pro Gly Leu Pro Phe Phe Ser Cys Gln Arg
    2000                2005                2010

Gly Tyr Lys Gly Val Trp Leu Gly Asp Gly Val Met Gln Thr Thr
    2015                2020                2025

Cys Pro Cys Gly Ala Gln Ile Ser Gly His Val Lys Asn Gly Ser
    2030                2035                2040

Met Lys Ile Val Gly Pro Lys Thr Cys Ser Asn Thr Trp His Gly
    2045                2050                2055

Thr Phe Pro Ile Asn Ala Tyr Thr Thr Gly Pro Cys Thr Pro Ser
    2060                2065                2070

Pro Ala Pro Asn Tyr Ser Lys Ala Leu Trp Arg Val Ala Ala Glu

2075                2080                2085

Glu Tyr Val Glu Val Thr Arg Val Gly Asp Phe His Tyr Val Thr
    2090                2095                2100

Gly Met Thr Thr Asp Asn Val Lys Cys Pro Cys Gln Val Pro Ala
    2105                2110                2115

Pro Glu Phe Phe Thr Glu Leu Asp Gly Val Arg Leu His Arg Tyr
    2120                2125                2130

Ala Pro Ala Cys Lys Pro Leu Leu Arg Asp Glu Val Thr Phe Gln
    2135                2140                2145

Val Gly Leu Asn Gln Tyr Pro Val Gly Ser Gln Leu Pro Cys Glu
    2150                2155                2160

Pro Glu Pro Asp Val Thr Val Leu Thr Ser Met Leu Thr Asp Pro
    2165                2170                2175

Ser His Ile Thr Ala Glu Thr Ala Lys Arg Arg Leu Ala Arg Gly
    2180                2185                2190

Ser Pro Pro Ser Leu Ala Ser Ser Ser Ala Ser Gln Leu Ser Ala
    2195                2200                2205

Pro Ser Leu Lys Ala Thr Cys Thr Thr His His Asp Ser Pro Asp
    2210                2215                2220

Ala Asp Leu Ile Glu Ala Asn Leu Leu Trp Arg Gln Glu Met Gly
    2225                2230                2235

Gly Asn Ile Thr Arg Val Glu Ser Glu Asn Lys Val Val Ile Leu
    2240                2245                2250

Asp Ser Phe Glu Pro Leu Arg Ala Glu Glu Asp Asp Arg Glu Val
    2255                2260                2265

Ser Val Ala Ala Glu Ile Leu Arg Arg Thr Arg Lys Phe Pro Ala
    2270                2275                2280

Ala Met Pro Ile Trp Ala Arg Pro Asp Tyr Asn Pro Pro Leu Leu
    2285                2290                2295

Glu Ser Trp Lys Asn Pro Asp Tyr Val Pro Pro Val Val His Gly
    2300                2305                2310

Cys Pro Leu Pro Pro Ile Lys Ala Pro Pro Ile Pro Pro Pro Arg
    2315                2320                2325

Arg Lys Arg Thr Val Ile Leu Thr Glu Ser Thr Val Ser Ser Ala
    2330                2335                2340

Leu Ala Glu Leu Ala Thr Lys Thr Phe Gly Ser Ser Gly Ser Ser
    2345                2350                2355

Ala Val Asp Ser Gly Thr Ala Thr Gly Pro Pro Asp Gln Ser Ser
    2360                2365                2370

Gly Asp Gly Asp Thr Gly Ser Asp Ala Glu Ser Cys Ser Ser Met
    2375                2380                2385

Pro Pro Leu Glu Gly Glu Pro Gly Asp Pro Asp Leu Ser Asp Gly
    2390                2395                2400

Ser Trp Ser Thr Val Ser Glu Glu Ala Gly Glu Asp Val Val Cys
    2405                2410                2415

Cys Ser Met Ser Tyr Thr Trp Thr Gly Ala Leu Ile Thr Pro Cys
    2420                2425                2430

Ala Ala Glu Glu Ser Lys Leu Pro Ile Asn Ala Leu Ser Asn Ser
    2435                2440                2445

Leu Leu Arg His His Asn Met Val Tyr Ala Thr Thr Ser Arg Ser
    2450                2455                2460

Ala Gly Gln Arg Gln Lys Lys Val Thr Phe Asp Arg Val Gln Val
    2465                2470                2475

Leu Asp Asp His Tyr Arg Asp Val Leu Lys Glu Met Lys Ala Lys
    2480                2485                2490

Ala Ser Thr Val Lys Ala Lys Leu Leu Pro Val Glu Glu Ala Cys
    2495                2500                2505

Arg Leu Thr Pro Pro His Ser Ala Arg Ser Lys Phe Gly Tyr Gly
    2510                2515                2520

Ala Lys Asp Val Arg Asn Leu Ser Ser Lys Ala Val Asn His Ile
    2525                2530                2535

His Ser Val Trp Lys Asp Leu Leu Glu Asp Ser Glu Thr Pro Ile
    2540                2545                2550

Asp Thr Thr Ile Met Ala Lys Asn Glu Val Phe Cys Val Gln Pro
    2555                2560                2565

Glu Lys Gly Gly Arg Lys Ser Ala Arg Leu Ile Val Phe Pro Asp
    2570                2575                2580

Leu Gly Val Arg Val Cys Glu Lys Met Ala Leu Tyr Asp Val Val
    2585                2590                2595

Ser Thr Leu Pro Gln Ala Val Met Gly Ser Ser Tyr Gly Phe Gln
    2600                2605                2610

Tyr Ser Pro Gly Gln Arg Val Glu Phe Leu Val Asn Ala Trp Lys
    2615                2620                2625

Ser Lys Lys Asn Pro Met Gly Phe Ala Tyr Asp Thr Arg Cys Phe
    2630                2635                2640

Asp Ser Thr Val Thr Glu Ser Asp Ile Arg Val Glu Glu Ser Ile
    2645                2650                2655

Tyr Gln Cys Cys Asp Leu Ala Pro Glu Ala Arg Gln Val Ile Arg
    2660                2665                2670

Ser Leu Thr Glu Arg Leu Tyr Ile Gly Gly Pro Leu Thr Asn Ser
    2675                2680                2685

Lys Gly Gln Ser Cys Gly Tyr Arg Arg Cys Arg Ala Ser Gly Val
    2690                2695                2700

Leu Thr Thr Ser Cys Gly Asn Thr Leu Thr Cys Tyr Leu Lys Ala
    2705                2710                2715

Ser Ala Ala Cys Arg Ala Ala Lys Leu Gln Asp Cys Thr Met Leu
    2720                2725                2730

Val Cys Gly Asp Asp Leu Val Val Ile Cys Glu Ser Ala Gly Thr
    2735                2740                2745

Gln Glu Asp Ala Ala Ser Leu Arg Val Phe Thr Glu Ala Met Thr
    2750                2755                2760

Arg Tyr Ser Ala Pro Pro Gly Asp Pro Pro Gln Pro Glu Tyr Asp
    2765                2770                2775

Leu Glu Leu Ile Thr Ser Cys Ser Ser Asn Val Ser Val Ala His
    2780                2785                2790

Asp Ala Leu Gly Lys Arg Val Tyr Tyr Leu Thr Arg Asp Pro Thr
    2795                2800                2805

Thr Pro Leu Ala Arg Ala Ala Trp Glu Thr Ala Arg His Thr Pro
    2810                2815                2820

Val Asn Ser Trp Leu Gly Asn Ile Ile Met Tyr Ala Pro Thr Leu
    2825                2830                2835

Trp Ala Arg Met Ile Leu Met Thr His Phe Phe Ser Ile Leu Leu
    2840                2845                2850

Ala Gln Glu Gln Leu Glu Lys Ala Leu Asp Cys Gln Ile Tyr Gly
    2855                2860                2865

Ala Thr Tyr Ser Ile Glu Pro Leu Asp Leu Pro Gln Ile Ile Gln
    2870                2875                2880

Arg Leu His Gly Leu Ser Ala Phe Ser Leu His Ser Tyr Ser Pro
    2885                2890                2895

Gly Glu Ile Asn Arg Val Ala Ser Cys Leu Arg Lys Leu Gly Val
    2900                2905                2910

Pro Pro Leu Arg Val Trp Arg His Arg Ala Arg Ser Val Arg Ala
    2915                2920                2925

Lys Leu Leu Ser Gln Gly Gly Arg Ala Ala Thr Cys Gly Lys Tyr
    2930                2935                2940

Leu Phe Asn Trp Ala Val Arg Thr Lys Leu Lys Leu Thr Pro Ile
    2945                2950                2955

Pro Ala Ala Ser Gln Leu Asp Leu Ser Gly Trp Phe Ile Ala Gly
    2960                2965                2970

Tyr Ser Gly Gly Asp Ile Tyr His Ser Leu Ser Arg Ala Arg Pro
    2975                2980                2985

Arg Trp Phe Met Trp Cys Leu Leu Leu Leu Ser Val Gly Val Gly

2990                 2995                3000

Ile Tyr  Leu Leu Pro Asn Arg
    3005                3010

<210>  65
<211>  783
<212>  RNA
<213>  Hepatitis C virus

<400>  65
gccucacacc uucccuacau cgaacaagga augcagcucg ccgagcaauu caagcagaag      60

gcgcucgggu uguugcaaac ggccaccaag caagcggagg ccgcugcucc cgugguggag     120

uccaaauggc aagcccuuga ggccuucugg gcgaagcaca uguggaacuu caucagcggg     180

auacaguauc uagcaggcuu guccacucug ccuggaaacc ccgcgauagc aucgcugaug     240

gcauuuacag ccucuaucac uagcccgcuc accacccuac auacccuucu auuuaacauc     300

uuggggggau ggguggccgc ccaacucgcc ccccccagcg cugcuucagc cuucguaggc     360

gccggcaucg ccggcgcggc uguuggcagc auaggucuug ggaaggugcu cguggacauc     420

cuagcggguu auggagcagg gguggcaggc gcacucgugg ccuucaaggu caugagcggc     480

gaagugcccu ccacugagga ccuggucaac uuacucccug ccauccucuc cccuggugcc     540

cuggucgucg gggucgugug cgcagcgaua cugcgucggc augugggccc aggggagggg     600

gccgugcagu ggaugaaccg gcugauagcg uucgcuucgc gggguaacca cgucuccccc     660

acgcacuaug ugccugagag cgacgccgca gcgcguguca cccagauccu cuccagccuu     720

accaucacuc agcugcuaaa gaggcuccac caguggauua auaaggacug uuccacgcca     780

ugc                                                                   783

<210>  66
<211>  261
<212>  PRT
<213>  Hepatitis C virus

<400>  66

Ala Ser His Leu Pro Tyr Ile Glu Gln Gly Met Gln Leu Ala Glu Gln
1               5                   10                  15

Phe Lys Gln Lys Ala Leu Gly Leu Leu Gln Thr Ala Thr Lys Gln Ala
            20                  25                  30

Glu Ala Ala Ala Pro Val Val Glu Ser Lys Trp Gln Ala Leu Glu Ala
        35                  40                  45

Phe Trp Ala Lys His Met Trp Asn Phe Ile Ser Gly Ile Gln Tyr Leu
    50                  55                  60

Ala Gly Leu Ser Thr Leu Pro Gly Asn Pro Ala Ile Ala Ser Leu Met
65                  70                  75                  80

Ala Phe Thr Ala Ser Ile Thr Ser Pro Leu Thr Thr Leu His Thr Leu
                85                  90                  95

Leu Phe Asn Ile Leu Gly Gly Trp Val Ala Ala Gln Leu Ala Pro Pro
            100                 105                 110

Ser Ala Ala Ser Ala Phe Val Gly Ala Gly Ile Ala Gly Ala Ala Val
        115                 120                 125

Gly Ser Ile Gly Leu Gly Lys Val Leu Val Asp Ile Leu Ala Gly Tyr
    130                 135                 140

Gly Ala Gly Val Ala Gly Ala Leu Val Ala Phe Lys Val Met Ser Gly
145                 150                 155                 160

Glu Val Pro Ser Thr Glu Asp Leu Val Asn Leu Leu Pro Ala Ile Leu
                165                 170                 175

Ser Pro Gly Ala Leu Val Val Gly Val Val Cys Ala Ala Ile Leu Arg
            180                 185                 190

Arg His Val Gly Pro Gly Glu Gly Ala Val Gln Trp Met Asn Arg Leu
        195                 200                 205

Ile Ala Phe Ala Ser Arg Gly Asn His Val Ser Pro Thr His Tyr Val
    210                 215                 220

Pro Glu Ser Asp Ala Ala Ala Arg Val Thr Gln Ile Leu Ser Ser Leu
225                 230                 235                 240

Thr Ile Thr Gln Leu Leu Lys Arg Leu His Gln Trp Ile Asn Lys Asp
                245                 250                 255

Cys Ser Thr Pro Cys
            260

<210>  67
<211>  8010
<212>  RNA
<213>  Artificial

<220>
<223>  subgenomic HCV RNA replicon

<400>  67
uaauacgacu cacuauagcc agcccccgau ugggggcgac acuccaccau agaucacucc      60

ccugugagga acuacugucu ucacgcagaa agcgucuagc cauggcguua guaugagugu     120

cgugcagccu ccaggacccc cccucccggg agagccauag uggucugcgg aaccggugag     180

uacaccggaa uugccaggac gaccgggucc uuucuuggau caacccgcuc aaugccugga     240

gauuugggcg ugcccccgcg agacugcuag ccgaguagug uugggucgcg aaaggccuug     300

ugguacugcc ugauagggug cuugcgagug ccccgggagg ucucguagac cgugcaccau     360

gagcacgaau ccuaaaccuc aaagaaaaac caaacguaac accaacgggc gcgccaugau     420

ugaacaagau ggauugcacg cagguucucc ggccgcuugg guggagaggc uauucggcua     480

ugacugggca caacagacaa ucggcugcuc ugaugccgcc guguuccggc ugucagcgca     540

ggggcgcccg guucuuuuug ucaagaccga ccuguccggu gcccugaaug aacugcagga     600

cgaggcagcg cggcuaucgu ggcuggccac gacgggcguu ccuugcgcag cugugcucga     660

cguugucacu gaagcgggaa gggacuggcu gcuauugggc gaagugccgg ggcaggaucu     720

ccugucaucu caccuugcuc cugccgagaa aguauccauc auggcugaug caaugcggcg     780

gcugcauacg cuugauccgg cuaccugccc auucgaccac caagcgaaac aucgcaucga     840

gcgagcacgu acucggaugg aagccggucu ugucgaucag gaugaucugg acgaagagca     900

ucaggggcuc gcgccagccg aacuguucgc caggcucaag gcgcgcaugc ccgacggcga     960

ggaucucguc gugacccaug gcgaugccug cuugccgaau aucauggugg aaaauggccg    1020

cuuuucugga uucaucgacu guggccggcu ggguguggcg gaccgcuauc aggacauagc    1080

guuggcuacc cgugauauug cugaagagcu uggcggcgaa ugggcugacc gcuuccucgu    1140

gcuuuacggu aucgccgcuc ccgauucgca gcgcaucgcc uucuaucgcc uucuugacga    1200

guucuucuga guuuaaacag accacaacgg uuucccucua gcgggaucaa uuccgccccu    1260

cucccucccc ccccccuaac guuacuggcc gaagccgcuu ggaauaaggc cggugugcgu    1320

uugucuauau guuauuuucc accauauugc cgucuuuugg caaugugagg gcccggaaac    1380

cuuggcccug ucuucuugac gagcauuccu aggggucuuu ccccucucgc caaaggaaug    1440

caaggucugu uggaugucgu gaaggaagca guuccucugg gaagcuucuu gaagacaaac    1500

aacgucugua gcgacccuuu gcaggcagcg gaacccccca ccuggcgaca ggugccucug    1560

cggccaaaag ccacguguau aagauacacc ugcaaaggcg guacaacccc agugccacgu    1620

ugugaguugg auaguugugg aaagagucaa auggcucucc ucaagcguau ucaacaaggg    1680

gcugaaggau gcccagaagg uaccccauug uaugggaucu gaucuggggc cucggugcac    1740

augcucuaca uguguuuagu cgagguuaaa aaaacgucua ggccccccga accacgggga 1800
cgugguuuuc cuuugaaaaa cacgauaaua ccauggcgcc caucacggcc uacucccaac 1860
agacgcgggg cuuacuuggc uguaucauca ccggccucac aggucgagac aagaaccagg 1920
ucgaagggga gguucaggug guuuccaccg caacacaguc uuucuuggca accugcguca 1980
acggugugug uuggacuguc uaccauggug ccggcucaaa gacccuagcc ggcccgaagg 2040
ggccaaucac ccagauguac accaacguag accaagaccu cguuggcugg caggcgcccc 2100
ccggggcgcg uuccaugacg ccgugcaccu gcggcagcuc ggaccuuuac uuggucacga 2160
ggcaugcuga ugucauuccg gugcgccggc ggggugacag cagagggagc cuacuuuccc 2220
ccaggcccgu cuccuacuug aagggcucuu cagguggucc acugcucugc cccuuggggc 2280
acgucguggg caucuuucgg gcugccgugu gcacccgggg gguugcgaag gcgguggacu 2340
ucauacccau cgagucuaug gaaacuacca ugcggucucc ggucuuuacg gauaauucau 2400
cucccccggc cguaccgcag acuuuucaag uggcccaucu gcacgccccc acuggcagcg 2460
gcaagagcac caaggugcca gcugcguaug cagcccaggg guacaaggug cuugucuuga 2520
auccauccgu ugccgccacc uugggguuug gggcguauau guccaaggca uacggugucg 2580
acccuaacau uagaacuggg guaaggacca ucaccacagg cgcucccauc acguacucca 2640
ccuacggcaa guuccuugcc gacggugguu gcuccggggg cgcuuacgac aucauaauau 2700
gcgaugagug ccacucaacc gacucgacuu ccauuuggg cauuggcacg guccuggauc 2760
aagcggagac ggcuggagcg cgacucgucg ugcucgccac cgcuacgccu ccgggaucgg 2820
ucacugugcc acaccccaac aucgaggagg uggccuuguc caacaccgga gagauucccu 2880
ucuauggcaa agccaucccc cucgagguca ucaagggggg gaggcaucuc auuuucuguc 2940
auucuaagaa gaagugugau gagcucgcug caaagcuguc gggccuuggg gucaacgcug 3000
uagcguacua ccggggucuu gauguguccg ucauaccaac aagcggggac gucguugucg 3060
uggcaacaga cgcucuaaug acgggcuaca ccggugacuu ugacucugug aucgacugua 3120
auacaugugu cacccagaca gucgacuuca gccuggaccc caccuucacc auugagacga 3180
cgaccgugcc ccaagacgca gugucgcgcu cgcagcggcg agggaggacu gguaggggua 3240
gagggggcau auacagguuu gugacuccag gagagcggcc cucgggcaug uucgauuccu 3300
cgguccugug ugaaugcuau gacgcgggcu gugcuuggua cgagcucacg cccgccgaga 3360
ccucgguuag gcugcgggcu uaccuaaaua caccaggguu gcccgucugc caggaccauu 3420
uggaguucug ggaaggcguc uucacaggcc ucacucauau agaugcccac uucuugucuc 3480
agacuaagca ggcaggagac aacuuccccu accugguggc auaccaggcc acagugugcg 3540

ccagggccca ggcaccaccu ccaucauggg aucaaaugug gaagugucuc auacggcuaa 3600
aaccuacacu acacgggcca acaccccugu uguacaggcu aggagccguc caaaacgagg 3660
ucacccucac acaccccaug accaaauaca ucaugacaug caugucggcu gaccuagagg 3720
ucgucaccag cacuugggug cuggugggcg ggguccucgc agcuuuggcc gcguacugcu 3780
ugacaacggg cagcgugguu aucauaggca ggaucaucuu guccggaagg ccggcuauca 3840
uucccgauag ggaaguucuc uaccaggagu ucgaugaaau ggaagagugc gccucacacc 3900
uucccuacau cgaacaagga augcagcucg ccgagcaauu caagcagaag gcgcucgggu 3960
uguugcaaac ggccaccaag caagcggagg ccgcugcucc cgugguggag uccaaauggc 4020
aagcccuuga ggccuucugg gcgaagcaca uguggaacuu caucagcggg auacaguauc 4080
uagcaggcuu guccacucug ccuggaaacc ccgcgauagc aucgcugaug gcauuuacag 4140
ccucuaucac uagcccgcuc accacccuac auacccuucu auuuaacauc uuggggggau 4200
ggguggccgc ccaacucgcc ccccccagcg cugcuucagc cuucguaggc gccggcaucg 4260
ccggcgcggc uguuggcagc auaggucuug ggaaggugcu cguggacauc cuagcggguu 4320
auggagcagg gguggcaggc gcacucgugg ccuucaaggu caugagcggc gaagugcccu 4380
ccacugagga ccuggucaac uuacucccug ccauccucuc cccuggugcc cuggucgucg 4440
gggucgugug cgcagcgaua cugcgucggc augugggccc aggggagggg gccgugcagu 4500
ggaugaaccg gcugauagcg uucgcuucgc gggguaacca cgucuccccc acgcacuaug 4560
ugccugagag cgacgccgca gcgcguguca cccagauccu cuccagccuu accaucacuc 4620
agcugcuaaa gaggcuccac caguggauua auaaggacug uuccacgcca ugcuccgguu 4680
cguggcucag ggauguuugg gacuggauau gcacgguuuu gaccgacuuc aaaaccuggc 4740
uccaguccaa gcuccugcca cgguugccgg gacucccuuu cuuuucaugu caacguggau 4800
auaaaggagu cuggcuggga gauggcguua ugcaaacuac cuguccaugu ggugcacaaa 4860
ucagcggaca ugucaaaaac ggcuccauga agaucguggg gccuaaaacc ugcagcaaca 4920
cguggcacgg gacguucccc aucaacgcau acaccacagg ccccugcaca cccuccccgg 4980
cgccgaacua uuccaaggcg uuguggcgag uggcugcuga ggaguaugug gaggucacgc 5040
ggguggggga uuuccacuac gugacgggca ugaccacuga caacguaaaa ugcccaugcc 5100
aggucccggc ccccgaauuc uucacggagu uggauggggu gcggcugcac agguacgcuc 5160
cggcgugcaa gccucucuua cgggaugagg ucacauucca ggucgggcuc aaccaguauc 5220
cgguuggauc acagcuucca ugugagcccg agccggaugu aacagugcuc acuuccaugc 5280
ucaccgaccc cucccauauu acagcagaga cggcuaagcg uaggcuggcc agaggguccc 5340
cccccucuuu ggccagcucc ucagcuagcc aguugucugc gccuuccuug aaggcgacau 5400

gcacuaccca ccaugacucc ccagaugcug accucaucga ggccaaccuc cuguggcggc 5460
aggagauggg cgggaacauc acccgcgugg agucagagaa caagguagua auucuggacu 5520
cuuuugaacc gcuucgagcg gaggaggaug auagggaagu guccguagcg gcggagaucc 5580
ugcggagaac caggaaauuc cccgcagcga ugcccauaug ggcacggccg gacuacaacc 5640
caccacuccu agagucuugg aagaacccag acuacguccc uccaguggua cacgggugcc 5700
cauuaccacc uauuaaggcc ccuccgauac caccuccacg gagaaagagg acggucaucc 5760
ugacagaauc caccgugucu ucugccuugg cggagcuugc uacaaagacc uuuggcagcu 5820
ccggaucguc ggccguugac agcggcacag cgacuggccc uccugaucag uccuccggug 5880
acggagauac aggauccgac gcugagucgu gcuccuccau gcccccccuu gagggggagc 5940
cgggggaccc cgaucucagc gacgggucuu ggucuaccgu gagcgaggag gccggugagg 6000
acgucgucug cugcucgaug uccuacacau ggacaggcgc cuuaaucaca ccgugcgccg 6060
cagaggagag caagcugccu aucaacgcgu ugagcaacuc uuugcugcgc caccacaaca 6120
uggucuaugc cacaacaucc cgcagcgcgg gccaacggca gaagaagguc acuuuugaca 6180
gagugcaggu ccuggacgac cauuaccggg acgugcucaa ggagaugaag gcgaaggcgu 6240
ccacaguuaa ggcuaaacuu cuaccuguag aagaagccug caggcugacg cccccacacu 6300
cggccagauc caaguuuggc uauggggcga aggacguccg gaaccuaucc agcaaggccg 6360
ucaaccacau ccacuccgug uggaaggacu ugcuggaaga cucugagacg ccgauugaca 6420
ccaccaucau ggcaaaaaau gaggucuuuu guguucaacc agagaaagga ggccgcaagu 6480
cagcucgucu uaucguauuc ccagacuugg ggguucgugu gugcgagaaa auggcccuuu 6540
acgacguggu uuccacucuu ccucaggccg ugaugggcuc cucauacggg uuccaguacu 6600
cuccuggaca gcgggucgag uuccugguga augccuggaa aucaaagaag aacccuaugg 6660
gcuucgcaua ugacacccgc uguuuugacu caacggucac ugagagugac auccguguug 6720
aggagucaau uuaccaaugu ugugacuugg cccccgaggc cagacagguc auaaggucgc 6780
ucacggagcg gcuuuauauc gggggccccc ugacuaauuc aaaagggcag agcugcggcu 6840
aucgccggug ccgcgccagc ggugugcuga cgaccagcug cgguaauacc cucacauguu 6900
acuugaaggc uucugcagcc ugucgagcug caaagcucca ggacugcaca auguuagugu 6960
gcggagacga ccuugucguu aucugugaaa gugcgggaac ccaggaggac gcggcgagcc 7020
uacgagucuu cacggaggcu augacuaggu acucugcccc ccccggggac ccgccccagc 7080
cagaauacga cuuggagcug auaacaucau gcuccuccaa cgucucgguc gcgcacgaug 7140
cacuuggcaa gcggguguau uaucugaccc gcgaccccac caccccccuu gcgcgggcug 7200

cgugggagac agcaagacac acuccaguua acuccuggcu aggcaacauc aucauguaug 7260

cgcccacccu augggcaagg augauucuga ugacccacuu cuuuuccauc cuucuagcuc 7320

aggaacaacu ugaaaaagcc cuagauuguc agaucuacgg ggccacuuac uccauugagc 7380

cacuugaccu accucagauc auucagcgac uccacggccu uagcgcauuu ucacuccaua 7440

gcuacucucc aggugagauc aauagggugg cuucaugccu caggaaacuu gggguaccac 7500

ccuugcgagu cuggagacau cgggccagaa guguccgcgc uaagcuacug ucccaggggg 7560

ggagggccgc cacuuguggc aaauaccucu ucaacugggc aguaaggacc aagcucaaac 7620

ucacuccaau uccggcugcg ucccaguugg acuuguccgg cugguucauu gcugguuaca 7680

gcgggggaga cauauaucac agccugucuc gcgcccgacc ccgcugguuc auguggugcc 7740

uacuccuacu uuccguaggg guaggcaucu aucugcuccc caaucgauga acggggggcu 7800

aaacacucca ggccaauagg ccauucuguu uuuuuuuuuu uuuuuuuuuu uuuuuuuuuu 7860

uuuuuuuuuu uuuuuuuuuu uuccuuuuuu uuuuuuuuuu ucccuuucuu uugguggcuc 7920

caucuuagcc cuagucacgg cuagcuguga aagguccgug agccgcauga cugcagagag 7980

ugcugauacu ggccucucug cagaucaugu 8010

<210> 68
<211> 8010
<212> RNA
<213> Artificial

<220>
<223> subgenomic HCV RNA replicon

<400> 68
uaauacgacu cacuauagcc agcccccgau ugggggcgac acuccaccau agaucacucc 60

ccugugagga acuacugucu ucacgcagaa agcgucuagc cauggcguua guaugagugu 120

cgugcagccu ccaggacccc cccucccggg agagccauag uggucugcgg aaccggugag 180

uacaccggaa uugccaggac gaccgggucc uuucuuggau caacccgcuc aaugccugga 240

gauuugggcg ugcccccgcg agacugcuag ccgaguagug uugggucgcg aaaggccuug 300

ugguacugcc ugauagggug cuugcgagug ccccgggagg ucucguagac cgugcaccau 360

gagcacgaau ccuaaaccuc aaagaaaaac caaacguaac accaacgggc gcgccaugau 420

ugaacaagau ggauugcacg cagguucucc ggccgcuugg guggagaggc uauucggcua 480

ugacugggca caacagacaa ucggcugcuc ugaugccgcc guguuccggc ugucagcgca 540

ggggcgcccg guucuuuuug ucaagaccga ccuguccggu gcccugaaug aacugcagga 600

cgaggcagcg cggcuaucgu ggcuggccac gacgggcguu ccuugcgcag cugugcucga 660

cguugucacu gaagcgggaa gggacuggcu gcuauugggc gaagugccgg ggcaggaucu 720

ccugucaucu caccuugcuc cugccgagaa aguauccauc auggcugaug caaugcggcg 780
gcugcauacg cuugauccgg cuaccugccc auucgaccac caagcgaaac aucgcaucga 840
gcgagcacgu acucggaugg aagccggucu ugucgaucag gaugaucugg acgaagagca 900
ucaggggcuc gcgccagccg aacuguucgc caggcucaag gcgcgcaugc ccgacggcga 960
ggaucucguc gugacccaug gcgaugccug cuugccgaau aucauggugg aaaauggccg 1020
cuuuucugga uucaucgacu guggccggcu ggguguggcg gaccgcuauc aggacauagc 1080
guuggcuacc cgugauauug cugaagagcu uggcggcgaa ugggcugacc gcuuccucgu 1140
gcuuuacggu aucgccgcuc ccgauucgca gcgcaucgcc uucuaucgcc uucuugacga 1200
guucuucuga guuuaaacag accacaacgg uuucccucua gcgggaucaa uuccgccccu 1260
cucccucccc ccccccuaac guuacuggcc gaagccgcuu ggaauaaggc cggugugcgu 1320
uugucuauau guuauuuucc accauauugc cgucuuuugg caaugugagg gcccggaaac 1380
cuggcccugu cuucuugacg agcauuccua ggggucuuuc cccucucgcc aaaggaaugc 1440
aaggucuguu gaaugucgug aaggaagcag uuccucugga agcuucuuga agacaaacaa 1500
cgucuguagc gacccuuugc aggcagcgga accccccacc uggcgacagg ugccucugcg 1560
gccaaaagcc acguguauaa gauacaccug caaaggcggc acaaccccag ugccacguug 1620
ugaguuggau aguuguggaa agagucaaau ggcucuccuc aagcguauuc aacaaggggc 1680
ugaaggaugc ccagaaggua ccccauugua ugggaucuga ucuggggccu cggugcacau 1740
gcucuacaug uguuuagucg agguuaaaaa aacgucuagg ccccccgaac cacggggacg 1800
ugguuuuccu uugaaaaaca cgauaauacc auggcgccca ucacggccua cucccaacag 1860
acgcggggcu uacuuggcug uaucaucacc ggccucacag gucgagacaa gaaccagguc 1920
gaaggggagg uucagguggu uuccaccgca acacagucuu ucuuggcaac cugcgucaac 1980
gguguguguu ggacugucua ccauggugcc ggcucaaaga cccuagccgg cccgaagggc 2040
caaucaccca gauguacacc aacguagacc aagaccucgu uggcuggcag gcgccccucg 2100
gggcgcguuc caugacgccg ugcaccugcg gcagcucgga ccuuuacuug gucacgaggc 2160
augcugaugu cauuccggug cgccggcggg gugacagcag agggagccua cuuuccccca 2220
ggcccgucuc cuacuugaag ggcucuucag gugguccacu gcucugcccc uuggggcacg 2280
ucgugggcau cuuucggcug ccgugugcac ccgggggguu gcgaaggcgg uggacuucau 2340
acccaucgag ucuauggaaa cuaccaugcg gucuccgguc uuuacggaua auucaucucc 2400
cccggccgua ccgcagacuu uucaaguggc ccaucugcac gcccccacug gcagcggcaa 2460
gagcaccaag gugccagcug cguaugcagc ccaggguaca aggugcuugu cuuga 2520

auccauccgu ugccgccacc uuggguuuug gggcguauau guccaaggca uacggugucg 2580
acccuaacau uagaacuggg guaaggacca ucaccacagg cgcucccauc acguacucca 2640
ccuacggcaa guuccuugcc gacggugguu gcuccggggg cgcuuacgac aucauaauau 2700
gcgaugagug ccacucaacc gacucgacuu ccauuuuggg cauuggcacg guccuggauc 2760
aagcggagac ggcuggagcg cgacucgucg ugcucgccac cgcuacgccu ccgggaucgg 2820
ucacugugcc acaccccaac aucgaggagg uggccuuguc caacaccgga gagauucccu 2880
ucuauggcaa agccaucccc cucgagguca ucaagggggg gaggcaucuc auuuucuguc 2940
auucuaagaa gaagugugau gagcucgcug caaagcuguc gggccuuggg gucaacgcug 3000
uagcguacua ccggggucuu gauguguccg ucauaccaac aagcggggac gucguugucg 3060
uggcaacaga cgcucuaaug acgggcuaca ccggugacuu ugacucugug aucgacugua 3120
auacaugugu cacccagaca gucgacuuca gccuggaccc caccuucacc auugagacga 3180
cgaccgugcc ccaagacgca gugucgcgcu cgcagcggcg agggaggacu gguaggggua 3240
gagggggcau auacagguuu gugacuccag gagagcggcc cucgggcaug uucgauuccu 3300
cgguccugug ugaaugcuau gacgcgggcu gugcuuggua cgagcucacg cccgccgaga 3360
ccucgguuag gcugcgggcu uaccuaaaua caccaggguu gcccgucugc caggaccauu 3420
uggaguucug ggaaggcguc uucacaggcc ucacucauau agaugcccac uucuugucuc 3480
agacuaagca ggcaggagac aacuuccccu accugguggc auaccaggcc acagugugcg 3540
ccagggccca ggcaccaccu ccaucauggg aucaaaugug gaagugucuc auacggcuaa 3600
aaccuacacu acacgggcca acaccccugu uguacaggcu aggagccguc caaaacgagg 3660
ucacccucac acaccccaug accaaauaca ucaugacaug caugucggcu gaccuagagg 3720
ucgucaccag cacuugggug cuggugggcg ggguccucgc agcuuuggcc gcguacugcu 3780
ugacaacggg cagcgugguu aucauaggca ggaucaucuu guccggaagg ccggcuauca 3840
uucccgauag ggaaguucuc uaccaggagu ucgaugaaau ggaagagugc gccucacacc 3900
uucccuacau cgaacaagga augcagcucg ccgagcaauu caagcagaag gcgcucgggu 3960
uguugcaaac ggccaccaag caagcggagg ccgcugcucc cgugguggag uccaaauggc 4020
aagcccuuga ggccuucugg gcgaagcaca uguggaacuu caucagcggg auacaguauc 4080
uagcaggcuu guccacucug ccuggaaacc ccgcgauagc aucgcugaug gcauuuacag 4140
ccucuaucac uagcccgcuc accacccuac auacccuucu auuuaacauc uuggggggau 4200
ggguggccgc ccaacucgcc ccccccagcg cugcuucagc cuucguaggc gccggcaucg 4260
ccggcgcggc uguuggcagc auaggucuug ggaaggugcu cguggacauc cuagcggguu 4320
auggagcagg gguggcaggc gcacucgugg ccuucaaggu caugagcggc gaagugcccu 4380

ccacugagga ccuggucaac uuacucccug ccauccucuc cccuggugcc cuggucgucg 4440
gggucgugug cgcagcgaua cugcgucggc augugggccc aggggagggg gccgugcagu 4500
ggaugaaccg gcugauagcg uucgcuucgc gggguaacca cgucuccccc acgcacuaug 4560
ugccugagag cgacgccgca gcgcguguca cccagauccu cuccagccuu accaucacuc 4620
agcugcuaaa gaggcuccac caguggauua auaaggacug uuccacgcca ugcuccgguu 4680
cguggcucag ggauguuugg gacuggauau gcacgguuuu gaccgacuuc aaaaccuggc 4740
uccaguccaa gcuccugcca cgguugccgg gacucccuuu cuuuucaugu caacguggau 4800
auaaaggagu cuggcuggga gauggcguua ugcaaacuac cuguccaugu ggugcacaaa 4860
ucagcggaca ugucaaaaac ggcuccauga agaucguggg gccuaaaacc ugcagcaaca 4920
cguggcacgg gacguucccc aucaacgcau acaccacagg ccccugcaca cccuccccgg 4980
cgccgaacua uuccaaggcg uuguggcgag uggcugcuga ggaguaugug gaggucacgc 5040
ggguggggga uuuccacuac gugacgggca ugaccacuga caacguaaaa ugcccaugcc 5100
aggucccggc ccccgaauuc uucacggagu uggauggggu gcggcugcac agguacgcuc 5160
cggcgugcaa gccucucuua cgggaugagg ucacauucca ggucgggcuc aaccaguauc 5220
cgguuggauc acagcuucca ugugagcccg agccggaugu aacagugcuc acuuccaugc 5280
ucaccgaccc cucccauauu acagcagaga cggcuaagcg uaggcuggcc agaggguccc 5340
cccccucuuu ggccagcucc ucagcuagcc aguugucugc gccuuccuug aaggcgacau 5400
gcacuaccca ccaugacucc ccagaugcug accucaucga ggccaaccuc cuguggcggc 5460
aggagauggg cgggaacauc acccgcgugg agucagagaa caagguagua auucuggacu 5520
cuuuugaacc gcuucgagcg gaggaggaug auagggaagu guccguagcg gcggagaucc 5580
ugcggagaac caggaaauuc cccgcagcga ugcccauaug ggcacggccg gacuacaacc 5640
caccacuccu agagucuugg aagaacccag acuacguccc uccaguggua cacgggugcc 5700
cauuaccacc uauuaaggcc ccuccgauac caccuccacg gagaaagagg acggucaucc 5760
ugacagaauc caccgugucu ucugccuugg cggagcuugc uacaaagacc uuuggcagcu 5820
ccggaucguc ggccguugac agcggcacag cgacuggccc uccugaucag uccuccggug 5880
acggagauac aggauccgac gcugagucgu gcuccuccau gcccccccuu gagggggagc 5940
cgggggaccc cgaucucagc gacgggucuu ggucuaccgu gagcgaggag gccggugagg 6000
acgucgucug cugcucgaug uccuacacau ggacaggcgc cuuaaucaca ccgugcgccg 6060
cagaggagag caagcugccu aucaacgcgu ugagcaacuc uuugcugcgc caccacaaca 6120
uggucuaugc cacaacaucc cgcagcgcgg gccaacggca gaagaagguc acuuuugaca 6180

gagugcaggu ccuggacgac cauuaccggg acgugcucaa ggagaugaag gcgaaggcgu 6240
ccacaguuaa ggcuaaacuu cuaccuguag aagaagccug caggcugacg cccccacacu 6300
cggccagauc caaguuuggc uauggggcga aggacguccg gaaccuaucc agcaaggccg 6360
ucaaccacau ccacuccgug uggaaggacu ugcuggaaga cucugagacg ccgauugaca 6420
ccaccaucau ggcaaaaaau gaggucuuuu guguucaacc agagaaagga ggccgcaagu 6480
cagcucgucu uaucguauuc ccagacuugg ggguucgugu gugcgagaaa auggcccuuu 6540
acgacguggu uuccacucuu ccucaggccg ugaugggcuc cucauacggg uuccaguacu 6600
cuccuggaca gcgggucgag uuccugguga augccuggaa aucaaagaag aacccuaugg 6660
gcuucgcaua ugacacccgc uguuuugacu caacggucac ugagagugac auccguguug 6720
aggagucaau uuaccaaugu ugugacuugg cccccgaggc cagacagguc auaaggucgc 6780
ucacggagcg gcuuuauauc gggggccccc ugacuaauuc aaaagggcag agcugcggcu 6840
aucgccggug ccgcgccagc ggugugcuga cgaccagcug cgguaauacc cucacauguu 6900
acuugaaggc uucugcagcc ugucgagcug caaagcucca ggacugcaca auguuagugu 6960
gcggagacga ccuugucguu aucugugaaa gugcgggaac ccaggaggac gcggcgagcc 7020
uacgagucuu cacggaggcu augacuaggu acucugcccc ccccggggac ccgccccagc 7080
cagaauacga cuuggagcug auaacaucau gcuccuccaa cgucucgguc gcgcacgaug 7140
cacuuggcaa gcggguguau uaucugaccc gcgaccccac caccccccuu gcgcgggcug 7200
cgugggagac agcaagacac acuccaguua acuccuggcu aggcaacauc aucauguaug 7260
cgcccacccu augggcaagg augauucuga ugacccacuu cuuuuccauc cuucuagcuc 7320
aggaacaacu ugaaaaagcc cuagauuguc agaucuacgg ggccacuuac uccauugagc 7380
cacuugaccu accucagauc auucagcgac uccacggccu uagcgcauuu ucacuccaua 7440
gcuacucucc aggugagauc aauagggugg cuucaugccu caggaaacuu gggguaccac 7500
ccuugcgagu cuggagacau cgggccagaa guguccgcgc uaagcuacug ucccaggggg 7560
ggagggccgc cacuuguggc aaauaccucu ucaacugggc aguaaggacc aagcucaaac 7620
ucacuccaau uccggcugcg ucccaguugg acuuguccgg cugguucauu gcugguuaca 7680
gcgggggaga cauauaucac agccugucuc gcgcccgacc ccgcugguuc auguggugcc 7740
uacuccuacu uuccguaggg guaggcaucu aucugcuccc caaucgauga acggggggcu 7800
aaacacucca ggccaauagg ccauucuguu uuuuuuuuuu uuuuuuuuuu uuuuuuuuuu 7860
uuuuuuuuu uuuuuuuuu uuccuuuuu uuuuuuuuu ucccuuucuu uugguggcuc 7920
caucuuagcc cuagucacgg cuagcuguga aagguccgug agccgcauga cugcagagag 7980
ugcugauacu ggccucucug cagaucaugu 8010

SEQUENCE LISTING

<110> Advanced Life Science Institute, Inc.

<120> HCV genome

<130> ALS-805

<150> JP2007-119667
<151> 2007-04-27

<160> 68

<170> PatentIn version 3.1

<210> 1
<211> 9594
<212> RNA
<213> Hepatitis C virus

<400> 1
gccagccccc ugaugggggc gacacuccac cauagaucac uccccuguga ggaacuacug 60

ucuucacgca gaaagcgucu agccauggcg uuaguaugag ugucgugcag ccuccaggac 120

cccccucccc gggagagcca uaguggucug cggaaccggu gaguacaccg gaauugccag 180

gacgaccggg uccuuucuug gaucaacccg cucaaugccu ggagauuugg gcgugccccc 240

gcgagacugc uagccgagua guguuggguc gcgaaaggcc uugugguacu gccugauagg 300

gugcuugcga gugccccggg aggucucgua gaccgugcau caugagcaca aauccuaaac 360

cucaaagaaa aaccaaacgu aacaccaacc gccgcccaca ggacgucaag uucccgggcg 420

guggccagau cguuggugga guuuaccugu ugccgcgcag gggccccagg uugggugugc 480

gcgcgacuag gaagacuucc gagcggucgc aaccucgugg aaggcgacaa ccuaucccca 540

aggcucgcca gcccgagggc agggccuggg cucagcccgg guauccuugg ccccucuaug 600

gcaacgaggg ucuggggugg gcaggauggc uccugucacc ccguggcucu cggccuaguu 660

ggggccccac ggacccccgg cguaggucgc guaauuuggg uaaggucauc gauacccuca 720

caugcggcuu cgccgaccuc augggguaca uuccgcucgu cggcgccccc cuaggaggcg 780

cugccagggc ccuggcgcau ggcguccggg uucuggagga cggcgugaac uaugcaacag 840

ggaaucugcc cgguugcccu uucucuaucu uccucuuagc uuugcugucc uguuugacca 900

ucccagcuuc cgcucacgaa gugcgcaacg uauccgggcu guaccauguc acgaacgacu 960

gcuccaacuc aagcauugug uaugaggcag cggacaugau caugcacacc cccgggugcg 1020

ugcccugcgu ccgggagggu aacuccuccc gcugcugggu agcgcucacu cccacgcucg 1080

cggccaggaa uagcagcguc cccacugcga caauacgacg ccaugucgau uugcucgucg 1140

gggcggcugc uuucuguucc gcuauguacg ugggggaucu uugcggaucu guuuuccucg 1200

ucucccagcu guucaccuuu ucaccucgcc gguacgagac gguacaggac ugcaauugcu 1260

cacucuaucc cggccacgua ucaggccauc gcauggcuug ggauaugaug augaacuggu 1320

caccuacaac agccuuagug guaucgcagu uacuccggau cccacaagcc gucguggaua 1380

uggugguagg ggcccacugg ggaguccugg cgggccuugc cuacuauucc auggugggga 1440

acugggcuaa ggucuugauu gugaugcuac ucuuugccgg cgucgacggg aagaccuacg 1500
ugacaggggg ggcgcagagc cgagccacuc aaggcuuugc gucccucuuu acacgggggc 1560
cgucucagaa acuccagcuu guaaauucca acggcagcug gcacauuaac aggacugccu 1620
ugaacugcaa ugacuccuuc cagacugggu uccuugccgc gcuguuuuac gcacaccguu 1680
ucaacucguc cggaugccca gagcgcaugg ccagcugccg ccccaucgac acguucgauc 1740
aggggugggg ccccaucacu caugucgcgc gucgcacauc ggaccagagg ccuuauugcu 1800
ggcacuacgc accucaaccg ugugguauug uacccgcguu gcagguaugu gguccagugu 1860
auugcuucac cccaagcccc gucguggugg ggacgaccga ucgcuucggc gcccccacgu 1920
acaacugggg ggagaaugag acggacgugc uacuccucaa caauacgcgg ccgccgcacg 1980
gcaacugguu cggcuguaca uggaugaaua guaccggguu caccaagacg ugugggggcc 2040
cccccugcaa caucgggggg uuuggcaaca acaccuugac cugcccuacg gauugcuucc 2100
ggaagcaccc cgaggccacu uacaccaaau gcggcucggg gcccugguug acgccuaggu 2160
gcaugguuga uuacccauac agacuuuggc acuaccccug cacuguuaac uuuuccaucu 2220
ucaaggucag gauguaugug gggggugugg agcacaggcu caccgccgcg ugcaauugga 2280
cucggggaga gcgcugcaac uuggaggaua gggacagauc ggagcucagc ccgcugcuac 2340
ugucuaccac agaguggcag guacugcccu guucuuucac caccuuaccg gcccugucca 2400
cugguuugau ccaccuccac cagaacaucg uggacgugca auaccuguac gguguggggu 2460
caucgguugu cuccauugca aucagguggg aguaugucgu gcugcucuuc cuccuccugg 2520
cggacgcgcg yguuugcgcc ugcuugugga ugaugcugcu gauagcccaa gcugaggccg 2580
ccuuagagaa ccuggugauc cucaaugcgg cgucuguggc cggagcgcau ggcguucucu 2640
cuuuccuugu guucuucugc gcugccuggu acaucaaggg caagcugguc cccggggcgg 2700
cauaugccuu cuauggugua uggccgcugc uccugcuucu gcugucauua ccaccacgag 2760
cauacgccuu ggaccgggag auggcugcau cgugcggagg cgcgguuuuc guaggucuga 2820
ugcuccugac cuugucacca cacuacaagg uguuucucgc uaggcucaua uggugguuac 2880
aguauuuuau caccagggcc gaggcgcacu ugcaggugug gguccccccc cucaacguuc 2940
gggggggccg cgaugccauc auccuccuca cguguguggu ccacccagag cuaauuuuug 3000
acaucaccaa aaucuugcuc gccaugcucg guccgcucau ggugcuccag gcuggccuaa 3060
cuagagugcc guacuucgua cgcgcucaag ggcucauccg ugcaugcaug uuagugcgga 3120
aagucgcugg gggccacuau guccaaaugg cccucaugaa acuggccgca cugacgggua 3180
cguacguuua ugaccaucuu acuccgcugc aggacugggc ccacgcgggc uugcgagacc 3240
uugcaguggc aguugagccc gucgucuucu cugacaugga gacuaagguc aucaccuggg 3300
gggcagacac cgcagcgugu ggggacauca ucucgggccu acccgucucc gcccgaaggg 3360
ggagggagau acuucugggc cccgccgaca gguuuggaga gcaggggugg cgacuccucg 3420
cgccuaucac ggcuuacgcu caacagacgc ggggccuacu uggcuguauc aucaccagcc 3480
ucacaggccg ggacaagaac caggucgagg gggagguuca ggugguuucc accgcaacgc 3540

aaucuuuccu ggcgaccugc gucaacggcg uguguuggac ugucuaccau ggugccggcu 3600
cgaagacccu ggccggcccg aagggcccaa ucacccaaau guacaccaau guggaccaag 3660
accucgucgg cuggccggcg ccccccgggg cgcgcucccu gacaccgugc accugcggca 3720
gcucggaccu cuaccugguc acgaggcaug cugaugucau uccggugcgc cggcggggcg 3780
acagcagggg gagucuacuc ucucccaggc ccaucuccua cuuaaagggc uccucaggug 3840
guccacugcu uugcccccug gggcacgcug ugggcaucuu ccgggccgcu gugugcaccc 3900
gggggguugc aaaggcggug gauuuuguac cuguugaguc uauggaaacc accaugcggu 3960
cuccggucuu uacggauaau ucaucucccc cggccguacc gcagacauuc caaguggccc 4020
aucuacacgc ucccacuggc aguggcaaga gcacuaaggu gccggcugcg uacgcagccc 4080
aaggguacaa gguacucguc uugaacccau ccguugccgc uaccuuaggg uuuggggcgu 4140
acaugucuaa agcacauggu guugagccua acaucagaac ugggguaagg accaucacca 4200
cgggcgcuuc caucacguau uccaccuacg guaaguuccu ugccgacggu gguugcucug 4260
ggggcgccua ugacaucaua auaugugaug agugccacuc aacugacucg acuuccaucu 4320
ugggcauugg cacaguccug gaccaagcgg agacggcugg agcgcggcuc gucgugcucg 4380
ccaccgcuac gccuccggga ucggucaccg ugccacaucc caauaucgag gagguggccu 4440
ugcccagcac cggagaaauu cccuucuacg gcaaagccau ccccauugag accaucaagg 4500
gggggaggca ccucaucuuc ugccacucca agaagaaaug ugacgagcuc gcugcaaagc 4560
uggugggccu cggaguuaac gcuguugcgu acuaccgggg ucuugaugug uccgucauac 4620
caacaagcgg agaugucguu gucguggcaa cagacgcucu aaugacgggc uucaccggcg 4680
acuuugacuc agugaucgac uguaauacuu gugucaccca gacaguugau uucagcuugg 4740
acccuaccuu caccauugag acgacaaccg ugccccaaga cgcggugucg cguucgcagc 4800
gacgaggcag gacuggcagg ggcaggaugg gcauauacag guuuguggcu ccaggggaac 4860
ggcccucggg cauguucgau ucuucggucc ugugugagug cuaugacgcg ggcugugcuu 4920
gguaugagcu cacgcccgcc gagaccucag ucagguugcg ggcuuaccua aauacaccag 4980
ggcugcccgu cugccaggac caccuggagu uuugggaggg ggucuucaca ggccucaccc 5040
acauagaugc ccauuucuug ucccagacua agcaggcagg agauaacuuc cccuaccugg 5100
uagcauacca ggcuacggug ugcgccaggg cccaggcucc cccuccaucg ugggaucaaa 5160
uguggaagug ucucauacgg cugaagccua cacuacacgg gccaacgccc cuguuguaua 5220
ggcuaggagc cguccagaau gaggucaucc ucacacaucc cauaaccaaa uacaucaugg 5280
caugcauguc ggcugaccua gaggucguca cuagcaccug ggugcugguc ggcggggucc 5340
uugcagcucu ggccgcguac ugccugacga cgggcagcgu ggucauugug ggcaggauca 5400
ucuuguccgg gaagccggcu aucauuccug acagggaagu ccucuaccgg gaguucgaug 5460
aaauggaaga gugugccuca caccuccccu acaucgaaca gggaaugcag cucgccgaac 5520
aauucaagca gaaggcgcuc ggguugcugc agacagccac caagcaagcg gaagccgcug 5580

cuccugugguggaguccaag uggcgagccc uugaggccuu cugggcgaag cacaugugga 5640
auuucaucag cgggauacag uacuuagcag gcuuguccac ucugccuggg aaccccgcga 5700
uagcaucacu gauggcauuc acagccucua ucaccagccc gcuuaccacc caacacaccc 5760
uccuguuuaa caucuuggga ggaugggugg ccgcccaacu ugcccccccc ggugcugccu 5820
cggcuuucgu gggcgccggc auugcuggcg cagcuguugg cagcauaggc cuugggaagg 5880
ugcuugugga cauccuggcg gguuauggag cagggguggc aggcgcgcuc guggccuuca 5940
aggucaugag cggcgagaug cccuccaccg aggaccuggu caacuuacuc ccugccaucc 6000
ucucuccugg ugcccuuguc gucggggucg ugugcgcagc aauacugcgu cggcaugugg 6060
gcccggggga gggggcugug caauggauga accggcugau agcguucgcc ucgcggggua 6120
accacgucuc ccccacgcac uaugugccug agagcgacgc ugcagcgcgu gucacacaga 6180
uccucucuag ccucaccauc acucagcuac ugaagaggcu ccaccagugg auuaaugagg 6240
acugcuccac accaugcucc ggcucguggc uuagggacgu uugggacugg auaugcacgg 6300
uuuugaguga cuucaagacc uggcuccagu ccaagcuccu gccacgguua ccgggaguuc 6360
cauuccuuuc augccaacgu ggguauaagg gggucuggcg gggagauggc aucaugcaga 6420
ccuccugccc auguggagca caaaucgccg gacaugucaa gaacgguucc augaggaucg 6480
uugggccuaa aaccuguagc aacacguggc acggaacauu ccccauuaac gcgcacacca 6540
cgggccccug cacacccucc ccagcgccga acuacucuaa ggcguugugg cggguggcug 6600
cugaggagua cguggaaguc acgcgggugg gggauuucca uuacgugacg ggcaugacca 6660
cugacaacgu aaaaugccca ugccagguuc cggcccccga guucuucaca gagguggaug 6720
ggguacggcu gcacagguac gcuccggcgu gcaaaccucu ccuacgggau gaggucacau 6780
uccaggucgg gcucaaccag uucccgguug ggucacagcu cccaugcgag cccgaaccgg 6840
auguaucagu gcucacuucc augcuuaccg acccuuccca caucacagca gagacggcua 6900
agcguaggcu ggccagaggg ucuucccccu cuuuggccag cucuucagcu agucaguugu 6960
cugcgcccuc auugaaggcg acaugcacca cccaucauga cuccccagac gcugaccuca 7020
uugaggccaa ccuccugugg cggcaggaga ugggagggaa caucacccgu guggagucag 7080
agaacaaggu gguaauccug gacucuuuug acccgcuucg agcggaggag gacgagaggg 7140
aggugucugu ugcggcggag auccugcgga aaaccaggaa guucccccca gcgaugccca 7200
uaugggcacg cccggacuac aacccaccgc ugcuagagac uuggaaggac ccggacuacg 7260
ucccuccagu ggugcacggg ugcccauugc caccuaccaa gaccccucca auaccaccuc 7320
cgcggaggaa aaagacaguu guccugacag aguccaccgu gucuucugcc cuggcggagc 7380
uugccacaaa gaccuuuggc agcuccggau cgucggccgu cgacagcggc acagcgaccg 7440
cccccccuaa ccagcucucc gacgaagugg auacaggauc cgacguugag ucguacuccu 7500
ccaugccccc ccuugaggga gagccggggg accccgaucu cagcgacggg ucuuggucua 7560
cuguaaguga ggaggcuggu gaggacgucg ucugcugcuc gauguccuac acauggacag 7620
gcgccuugau cacgccgugc gccgcggagg agagcaagcu gcccaucaau gcgcugagca 7680

acucuuugcu gcgccaccac aacauggucu augccacaac aucccgcagc gcaagccaac 7740
ggcagaaaaa ggucaccuuu gacagacugc aaguccugga cgaccauuac cgggacgugc 7800
ucaaggagau gaaggcgaag gcguccacag uuaaggcuaa acuucuaucc guagaagagg 7860
ccugcaagcu gacgccccca cacucagcca gguccaaauu uggcuauggg gcgaaggacg 7920
uccggaaccu auccagcaag gccguuaacc acaucaacuc cguguggaag gacuugcugg 7980
aagacacuga gacaccaauu gacaccacca ucauggcaaa aaaugagguc uucuguguuc 8040
aaccagagaa gggaggccgc aagccagcuc gccuuaucgu auacccagac uugggggugc 8100
gugugugcga gaaaauggcc cuuuacgacg uggucuccac ucuuccucag gccgugaugg 8160
gcuccucaua cggauuccag uacucuccug ggcagcgggu cgaguuccug gugaaugccu 8220
ggaaaucaaa gaagaacccu augggcuucg cauaugacac ccgcuguuuu gacucaacgg 8280
ucaccgagaa cgacauccgu guugaggagu caauuuacca auguugugac uuggcccccg 8340
aggccagaca ggugauaagg ucgcucacag agcggcuuua ugucgggggc ccccugacua 8400
auucaaaagg gcagaacugc gguuaucgcc ggugccgcgc cagcggcgug cugacgacua 8460
gcugcgguaa uacccucaca uguuacuuga aggccucugc agccugucga gcugcaaagc 8520
uccaggacug cacgaugcuc gugugcgggg acgaccuugu cguuaucugu gaaagcgcgg 8580
ggacccagga ggacgcggcg agccuacgag ucuucacgga ggcuaugacu agguacuccg 8640
ccccccccgg ggacccgccc cgaccggaau acgacuugga guugauaaca ucaugcuccu 8700
ccaacguguc ggucgcgcac gaugcaucug gcaaacgggu guauuaccuc acccgugacc 8760
ccaccacccc ccuugcgcgg gcugcguggg agacagcuaa acacacucca gucaacuccu 8820
ggcuaggcaa caucaucaug uaugcgccca cccucugggc aaggaugauu cugaugacuc 8880
acuucuucuc cauccuucua gcucaggagc agcuugaaaa agcccuggau ugucagaucu 8940
acggggccac uuacuccauu gaaccacuug accuaccuca gaucauucaa cgacuccaug 9000
gucuuagcgc auucucacuc cauaguuacu cuccagguga aaucaauagg guggcuucau 9060
gccucaggaa acuuggggua ccgcccuugc gagucuggag acaucgggcc agaagugucc 9120
gcgcuaagcu acugucccag ggggggaggg cugccacuug uggcaaguac cucuucaacu 9180
gggcaguaag gaccaagcuc aaacucacuc caaucccggc ugcgucccag uuggacuugu 9240
ccggcugguu cauugcuggu uacagcgggg gagacauaua ucacagccug ucucgugccc 9300
gaccccgcug guuuauguug ugccuacucc uacuuucugu ggggguaggc aucuaccugc 9360
uccccaaucg augaacgggg ggcuaaacac uccaggccaa uaggccauuc uguuuuuuuu 9420
uuuuuuuuuu uuuuuuuuuu uuuuuuuuuu uuuuuuuuuu uuuuuuccuu uuuuuuuuuu 9480
uuuuucccuu ucuuuuggug gcuccaucuu agcccuaguc acggcuagcu gugaaagguc 9540
cgugagccgc augacugcag agagugcuga uacuggccuc ucugcagauc augu 9594

<210> 2
<211> 3010
<212> PRT

<213>  Hepatitis C virus

<400>  2

Met Ser Thr Asn Pro Lys Pro Gln Arg Lys Thr Lys Arg Asn Thr Asn
1               5                   10                  15

Arg Arg Pro Gln Asp Val Lys Phe Pro Gly Gly Gly Gln Ile Val Gly
            20                  25                  30

Gly Val Tyr Leu Leu Pro Arg Arg Gly Pro Arg Leu Gly Val Arg Ala
        35                  40                  45

Thr Arg Lys Thr Ser Glu Arg Ser Gln Pro Arg Gly Arg Arg Gln Pro
    50                  55                  60

Ile Pro Lys Ala Arg Gln Pro Glu Gly Arg Ala Trp Ala Gln Pro Gly
65                  70                  75                  80

Tyr Pro Trp Pro Leu Tyr Gly Asn Glu Gly Leu Gly Trp Ala Gly Trp
                85                  90                  95

Leu Leu Ser Pro Arg Gly Ser Arg Pro Ser Trp Gly Pro Thr Asp Pro
            100                 105                 110

Arg Arg Arg Ser Arg Asn Leu Gly Lys Val Ile Asp Thr Leu Thr Cys
        115                 120                 125

Gly Phe Ala Asp Leu Met Gly Tyr Ile Pro Leu Val Gly Ala Pro Leu
    130                 135                 140

Gly Gly Ala Ala Arg Ala Leu Ala His Gly Val Arg Val Leu Glu Asp
145                 150                 155                 160

Gly Val Asn Tyr Ala Thr Gly Asn Leu Pro Gly Cys Pro Phe Ser Ile
                165                 170                 175

Phe Leu Leu Ala Leu Leu Ser Cys Leu Thr Ile Pro Ala Ser Ala His
            180                 185                 190

Glu Val Arg Asn Val Ser Gly Leu Tyr His Val Thr Asn Asp Cys Ser
        195                 200                 205

Asn Ser Ser Ile Val Tyr Glu Ala Ala Asp Met Ile Met His Thr Pro
    210                 215                 220

Gly Cys Val Pro Cys Val Arg Glu Gly Asn Ser Ser Arg Cys Trp Val
225                 230                 235                 240

Ala Leu Thr Pro Thr Leu Ala Ala Arg Asn Ser Ser Val Pro Thr Ala
                245                 250                 255

Thr Ile Arg Arg His Val Asp Leu Leu Val Gly Ala Ala Ala Phe Cys

            260                 265                 270

Ser Ala Met Tyr Val Gly Asp Leu Cys Gly Ser Val Phe Leu Val Ser
        275                 280                 285

Gln Leu Phe Thr Phe Ser Pro Arg Arg Tyr Glu Thr Val Gln Asp Cys
    290                 295                 300

Asn Cys Ser Leu Tyr Pro Gly His Val Ser Gly His Arg Met Ala Trp
305                 310                 315                 320

Asp Met Met Met Asn Trp Ser Pro Thr Thr Ala Leu Val Val Ser Gln
                325                 330                 335

Leu Leu Arg Ile Pro Gln Ala Val Val Asp Met Val Val Gly Ala His
            340                 345                 350

Trp Gly Val Leu Ala Gly Leu Ala Tyr Tyr Ser Met Val Gly Asn Trp
        355                 360                 365

Ala Lys Val Leu Ile Val Met Leu Leu Phe Ala Gly Val Asp Gly Lys
    370                 375                 380

Thr Tyr Val Thr Gly Gly Ala Gln Ser Arg Ala Thr Gln Gly Phe Ala
385                 390                 395                 400

Ser Leu Phe Thr Arg Gly Pro Ser Gln Lys Leu Gln Leu Val Asn Ser
                405                 410                 415

Asn Gly Ser Trp His Ile Asn Arg Thr Ala Leu Asn Cys Asn Asp Ser
            420                 425                 430

Phe Gln Thr Gly Phe Leu Ala Ala Leu Phe Tyr Ala His Arg Phe Asn
        435                 440                 445

Ser Ser Gly Cys Pro Glu Arg Met Ala Ser Cys Arg Pro Ile Asp Thr
    450                 455                 460

Phe Asp Gln Gly Trp Gly Pro Ile Thr His Val Ala Arg Arg Thr Ser
465                 470                 475                 480

Asp Gln Arg Pro Tyr Cys Trp His Tyr Ala Pro Gln Pro Cys Gly Ile
                485                 490                 495

Val Pro Ala Leu Gln Val Cys Gly Pro Val Tyr Cys Phe Thr Pro Ser
            500                 505                 510

Pro Val Val Val Gly Thr Thr Asp Arg Phe Gly Ala Pro Thr Tyr Asn
        515                 520                 525

Trp Gly Glu Asn Glu Thr Asp Val Leu Leu Leu Asn Asn Thr Arg Pro
    530                 535                 540

Pro His Gly Asn Trp Phe Gly Cys Thr Trp Met Asn Ser Thr Gly Phe
545                 550                 555                 560

Thr Lys Thr Cys Gly Gly Pro Pro Cys Asn Ile Gly Gly Phe Gly Asn
                565                 570                 575

Asn Thr Leu Thr Cys Pro Thr Asp Cys Phe Arg Lys His Pro Glu Ala
            580                 585                 590

Thr Tyr Thr Lys Cys Gly Ser Gly Pro Trp Leu Thr Pro Arg Cys Met
        595                 600                 605

Val Asp Tyr Pro Tyr Arg Leu Trp His Tyr Pro Cys Thr Val Asn Phe
    610                 615                 620

Ser Ile Phe Lys Val Arg Met Tyr Val Gly Gly Val Glu His Arg Leu
625                 630                 635                 640

Thr Ala Ala Cys Asn Trp Thr Arg Gly Glu Arg Cys Asn Leu Glu Asp
                645                 650                 655

Arg Asp Arg Ser Glu Leu Ser Pro Leu Leu Leu Ser Thr Thr Glu Trp
            660                 665                 670

Gln Val Leu Pro Cys Ser Phe Thr Thr Leu Pro Ala Leu Ser Thr Gly
        675                 680                 685

Leu Ile His Leu His Gln Asn Ile Val Asp Val Gln Tyr Leu Tyr Gly
    690                 695                 700

Val Gly Ser Ser Val Val Ser Ile Ala Ile Arg Trp Glu Tyr Val Val
705                 710                 715                 720

Leu Leu Phe Leu Leu Leu Ala Asp Ala Arg Val Cys Ala Cys Leu Trp
                725                 730                 735

Met Met Leu Leu Ile Ala Gln Ala Glu Ala Ala Leu Glu Asn Leu Val
            740                 745                 750

Ile Leu Asn Ala Ala Ser Val Ala Gly Ala His Gly Val Leu Ser Phe
        755                 760                 765

Leu Val Phe Phe Cys Ala Ala Trp Tyr Ile Lys Gly Lys Leu Val Pro
    770                 775                 780

Gly Ala Ala Tyr Ala Phe Tyr Gly Val Trp Pro Leu Leu Leu Leu Leu
785                 790                 795                 800

Leu Ser Leu Pro Pro Arg Ala Tyr Ala Leu Asp Arg Glu Met Ala Ala
                805                 810                 815

Ser Cys Gly Gly Ala Val Phe Val Gly Leu Met Leu Leu Thr Leu Ser
            820                 825                 830

Pro His Tyr Lys Val Phe Leu Ala Arg Leu Ile Trp Trp Leu Gln Tyr
        835                 840                 845

Phe Ile Thr Arg Ala Glu Ala His Leu Gln Val Trp Val Pro Pro Leu
    850                 855                 860

Asn Val Arg Gly Gly Arg Asp Ala Ile Ile Leu Leu Thr Cys Val Val
865                 870                 875                 880

His Pro Glu Leu Ile Phe Asp Ile Thr Lys Ile Leu Leu Ala Met Leu
                885                 890                 895

Gly Pro Leu Met Val Leu Gln Ala Gly Leu Thr Arg Val Pro Tyr Phe
            900                 905                 910

Val Arg Ala Gln Gly Leu Ile Arg Ala Cys Met Leu Val Arg Lys Val
        915                 920                 925

Ala Gly Gly His Tyr Val Gln Met Ala Leu Met Lys Leu Ala Ala Leu
    930                 935                 940

Thr Gly Thr Tyr Val Tyr Asp His Leu Thr Pro Leu Gln Asp Trp Ala
945                 950                 955                 960

His Ala Gly Leu Arg Asp Leu Ala Val Ala Val Glu Pro Val Val Phe
                965                 970                 975

Ser Asp Met Glu Thr Lys Val Ile Thr Trp Gly Ala Asp Thr Ala Ala
            980                 985                 990

Cys Gly Asp Ile Ile Ser Gly Leu Pro Val Ser Ala Arg Arg Gly Arg
        995                 1000                1005

Glu Ile Leu Leu Gly Pro Ala Asp Arg Phe Gly Glu Gln Gly Trp
    1010                1015                1020

Arg Leu Leu Ala Pro Ile Thr Ala Tyr Ala Gln Gln Thr Arg Gly
    1025                1030                1035

Leu Leu Gly Cys Ile Ile Thr Ser Leu Thr Gly Arg Asp Lys Asn
    1040                1045                1050

Gln Val Glu Gly Glu Val Gln Val Val Ser Thr Ala Thr Gln Ser
    1055                1060                1065

Phe Leu Ala Thr Cys Val Asn Gly Val Cys Trp Thr Val Tyr His
    1070                1075                1080

Gly Ala Gly Ser Lys Thr Leu Ala Gly Pro Lys Gly Pro Ile Thr
    1085                1090                1095

Gln Met Tyr Thr Asn Val Asp Gln Asp Leu Val Gly Trp Pro Ala
    1100                1105                1110

Pro Pro Gly Ala Arg Ser Leu Thr Pro Cys Thr Cys Gly Ser Ser
    1115                1120                1125

Asp Leu Tyr Leu Val Thr Arg His Ala Asp Val Ile Pro Val Arg
    1130                1135                1140

Arg Arg Gly Asp Ser Arg Gly Ser Leu Leu Ser Pro Arg Pro Ile
    1145                1150                1155

Ser Tyr Leu Lys Gly Ser Ser Gly Gly Pro Leu Leu Cys Pro Leu
    1160                1165                1170

Gly His Ala Val Gly Ile Phe Arg Ala Ala Val Cys Thr Arg Gly
    1175                1180                1185

Val Ala Lys Ala Val Asp Phe Val Pro Val Glu Ser Met Glu Thr
    1190                1195                1200

Thr Met Arg Ser Pro Val Phe Thr Asp Asn Ser Ser Pro Pro Ala
    1205                1210                1215

Val Pro Gln Thr Phe Gln Val Ala His Leu His Ala Pro Thr Gly
    1220                1225                1230

Ser Gly Lys Ser Thr Lys Val Pro Ala Ala Tyr Ala Ala Gln Gly
    1235                1240                1245

Tyr Lys Val Leu Val Leu Asn Pro Ser Val Ala Ala Thr Leu Gly
    1250                1255                1260

Phe Gly Ala Tyr Met Ser Lys Ala His Gly Val Glu Pro Asn Ile
    1265                1270                1275

Arg Thr Gly Val Arg Thr Ile Thr Thr Gly Ala Ser Ile Thr Tyr
    1280                1285                1290

Ser Thr Tyr Gly Lys Phe Leu Ala Asp Gly Gly Cys Ser Gly Gly
    1295                1300                1305

Ala Tyr Asp Ile Ile Ile Cys Asp Glu Cys His Ser Thr Asp Ser
    1310                1315                1320

Thr Ser Ile Leu Gly Ile Gly Thr Val Leu Asp Gln Ala Glu Thr
    1325                1330                1335

Ala Gly Ala Arg Leu Val Val Leu Ala Thr Ala Thr Pro Pro Gly

1340                1345                1350

Ser Val Thr Val Pro His Pro Asn Ile Glu Glu Val Ala Leu Pro
    1355                1360                1365

Ser Thr Gly Glu Ile Pro Phe Tyr Gly Lys Ala Ile Pro Ile Glu
    1370                1375                1380

Thr Ile Lys Gly Gly Arg His Leu Ile Phe Cys His Ser Lys Lys
    1385                1390                1395

Lys Cys Asp Glu Leu Ala Ala Lys Leu Val Gly Leu Gly Val Asn
    1400                1405                1410

Ala Val Ala Tyr Tyr Arg Gly Leu Asp Val Ser Val Ile Pro Thr
    1415                1420                1425

Ser Gly Asp Val Val Val Val Ala Thr Asp Ala Leu Met Thr Gly
    1430                1435                1440

Phe Thr Gly Asp Phe Asp Ser Val Ile Asp Cys Asn Thr Cys Val
    1445                1450                1455

Thr Gln Thr Val Asp Phe Ser Leu Asp Pro Thr Phe Thr Ile Glu
    1460                1465                1470

Thr Thr Thr Val Pro Gln Asp Ala Val Ser Arg Ser Gln Arg Arg
    1475                1480                1485

Gly Arg Thr Gly Arg Gly Arg Met Gly Ile Tyr Arg Phe Val Ala
    1490                1495                1500

Pro Gly Glu Arg Pro Ser Gly Met Phe Asp Ser Ser Val Leu Cys
    1505                1510                1515

Glu Cys Tyr Asp Ala Gly Cys Ala Trp Tyr Glu Leu Thr Pro Ala
    1520                1525                1530

Glu Thr Ser Val Arg Leu Arg Ala Tyr Leu Asn Thr Pro Gly Leu
    1535                1540                1545

Pro Val Cys Gln Asp His Leu Glu Phe Trp Glu Gly Val Phe Thr
    1550                1555                1560

Gly Leu Thr His Ile Asp Ala His Phe Leu Ser Gln Thr Lys Gln
    1565                1570                1575

Ala Gly Asp Asn Phe Pro Tyr Leu Val Ala Tyr Gln Ala Thr Val
    1580                1585                1590

Cys Ala Arg Ala Gln Ala Pro Pro Pro Ser Trp Asp Gln Met Trp
    1595                1600                1605

Lys Cys Leu Ile Arg Leu Lys Pro Thr Leu His Gly Pro Thr Pro
    1610                1615                1620

Leu Leu Tyr Arg Leu Gly Ala Val Gln Asn Glu Val Ile Leu Thr
    1625                1630                1635

His Pro Ile Thr Lys Tyr Ile Met Ala Cys Met Ser Ala Asp Leu
    1640                1645                1650

Glu Val Val Thr Ser Thr Trp Val Leu Val Gly Gly Val Leu Ala
    1655                1660                1665

Ala Leu Ala Ala Tyr Cys Leu Thr Thr Gly Ser Val Val Ile Val
    1670                1675                1680

Gly Arg Ile Ile Leu Ser Gly Lys Pro Ala Ile Ile Pro Asp Arg
    1685                1690                1695

Glu Val Leu Tyr Arg Glu Phe Asp Glu Met Glu Glu Cys Ala Ser
    1700                1705                1710

His Leu Pro Tyr Ile Glu Gln Gly Met Gln Leu Ala Glu Gln Phe
    1715                1720                1725

Lys Gln Lys Ala Leu Gly Leu Leu Gln Thr Ala Thr Lys Gln Ala
    1730                1735                1740

Glu Ala Ala Ala Pro Val Val Glu Ser Lys Trp Arg Ala Leu Glu
    1745                1750                1755

Ala Phe Trp Ala Lys His Met Trp Asn Phe Ile Ser Gly Ile Gln
    1760                1765                1770

Tyr Leu Ala Gly Leu Ser Thr Leu Pro Gly Asn Pro Ala Ile Ala
    1775                1780                1785

Ser Leu Met Ala Phe Thr Ala Ser Ile Thr Ser Pro Leu Thr Thr
    1790                1795                1800

Gln His Thr Leu Leu Phe Asn Ile Leu Gly Gly Trp Val Ala Ala
    1805                1810                1815

Gln Leu Ala Pro Pro Gly Ala Ala Ser Ala Phe Val Gly Ala Gly
    1820                1825                1830

Ile Ala Gly Ala Ala Val Gly Ser Ile Gly Leu Gly Lys Val Leu
    1835                1840                1845

Val Asp Ile Leu Ala Gly Tyr Gly Ala Gly Val Ala Gly Ala Leu
    1850                1855                1860

Val Ala Phe Lys Val Met Ser Gly Glu Met Pro Ser Thr Glu Asp
    1865                1870                1875

Leu Val Asn Leu Leu Pro Ala Ile Leu Ser Pro Gly Ala Leu Val
    1880                1885                1890

Val Gly Val Val Cys Ala Ala Ile Leu Arg Arg His Val Gly Pro
    1895                1900                1905

Gly Glu Gly Ala Val Gln Trp Met Asn Arg Leu Ile Ala Phe Ala
    1910                1915                1920

Ser Arg Gly Asn His Val Ser Pro Thr His Tyr Val Pro Glu Ser
    1925                1930                1935

Asp Ala Ala Ala Arg Val Thr Gln Ile Leu Ser Ser Leu Thr Ile
    1940                1945                1950

Thr Gln Leu Leu Lys Arg Leu His Gln Trp Ile Asn Glu Asp Cys
    1955                1960                1965

Ser Thr Pro Cys Ser Gly Ser Trp Leu Arg Asp Val Trp Asp Trp
    1970                1975                1980

Ile Cys Thr Val Leu Ser Asp Phe Lys Thr Trp Leu Gln Ser Lys
    1985                1990                1995

Leu Leu Pro Arg Leu Pro Gly Val Pro Phe Leu Ser Cys Gln Arg
    2000                2005                2010

Gly Tyr Lys Gly Val Trp Arg Gly Asp Gly Ile Met Gln Thr Ser
    2015                2020                2025

Cys Pro Cys Gly Ala Gln Ile Ala Gly His Val Lys Asn Gly Ser
    2030                2035                2040

Met Arg Ile Val Gly Pro Lys Thr Cys Ser Asn Thr Trp His Gly
    2045                2050                2055

Thr Phe Pro Ile Asn Ala His Thr Thr Gly Pro Cys Thr Pro Ser
    2060                2065                2070

Pro Ala Pro Asn Tyr Ser Lys Ala Leu Trp Arg Val Ala Ala Glu
    2075                2080                2085

Glu Tyr Val Glu Val Thr Arg Val Gly Asp Phe His Tyr Val Thr
    2090                2095                2100

Gly Met Thr Thr Asp Asn Val Lys Cys Pro Cys Gln Val Pro Ala
    2105                2110                2115

Pro Glu Phe Phe Thr Glu Val Asp Gly Val Arg Leu His Arg Tyr
    2120                2125                2130

Ala Pro Ala Cys Lys Pro Leu Leu Arg Asp Glu Val Thr Phe Gln
    2135                2140                2145

Val Gly Leu Asn Gln Phe Pro Val Gly Ser Gln Leu Pro Cys Glu
    2150                2155                2160

Pro Glu Pro Asp Val Ser Val Leu Thr Ser Met Leu Thr Asp Pro
    2165                2170                2175

Ser His Ile Thr Ala Glu Thr Ala Lys Arg Arg Leu Ala Arg Gly
    2180                2185                2190

Ser Ser Pro Ser Leu Ala Ser Ser Ser Ala Ser Gln Leu Ser Ala
    2195                2200                2205

Pro Ser Leu Lys Ala Thr Cys Thr Thr His His Asp Ser Pro Asp
    2210                2215                2220

Ala Asp Leu Ile Glu Ala Asn Leu Leu Trp Arg Gln Glu Met Gly
    2225                2230                2235

Gly Asn Ile Thr Arg Val Glu Ser Glu Asn Lys Val Val Ile Leu
    2240                2245                2250

Asp Ser Phe Asp Pro Leu Arg Ala Glu Glu Asp Glu Arg Glu Val
    2255                2260                2265

Ser Val Ala Ala Glu Ile Leu Arg Lys Thr Arg Lys Phe Pro Pro
    2270                2275                2280

Ala Met Pro Ile Trp Ala Arg Pro Asp Tyr Asn Pro Pro Leu Leu
    2285                2290                2295

Glu Thr Trp Lys Asp Pro Asp Tyr Val Pro Pro Val Val His Gly
    2300                2305                2310

Cys Pro Leu Pro Pro Thr Lys Thr Pro Pro Ile Pro Pro Pro Arg
    2315                2320                2325

Arg Lys Lys Thr Val Val Leu Thr Glu Ser Thr Val Ser Ser Ala
    2330                2335                2340

Leu Ala Glu Leu Ala Thr Lys Thr Phe Gly Ser Ser Gly Ser Ser
    2345                2350                2355

Ala Val Asp Ser Gly Thr Ala Thr Ala Pro Pro Asn Gln Leu Ser
    2360                2365                2370

Asp Glu Val Asp Thr Gly Ser Asp Val Glu Ser Tyr Ser Ser Met

    2375                2380                2385

Pro Pro Leu Glu Gly Glu Pro Gly Asp Pro Asp Leu Ser Asp Gly
    2390                2395                2400

Ser Trp Ser Thr Val Ser Glu Glu Ala Gly Glu Asp Val Val Cys
    2405                2410                2415

Cys Ser Met Ser Tyr Thr Trp Thr Gly Ala Leu Ile Thr Pro Cys
    2420                2425                2430

Ala Ala Glu Glu Ser Lys Leu Pro Ile Asn Ala Leu Ser Asn Ser
    2435                2440                2445

Leu Leu Arg His His Asn Met Val Tyr Ala Thr Thr Ser Arg Ser
    2450                2455                2460

Ala Ser Gln Arg Gln Lys Lys Val Thr Phe Asp Arg Leu Gln Val
    2465                2470                2475

Leu Asp Asp His Tyr Arg Asp Val Leu Lys Glu Met Lys Ala Lys
    2480                2485                2490

Ala Ser Thr Val Lys Ala Lys Leu Leu Ser Val Glu Glu Ala Cys
    2495                2500                2505

Lys Leu Thr Pro Pro His Ser Ala Arg Ser Lys Phe Gly Tyr Gly
    2510                2515                2520

Ala Lys Asp Val Arg Asn Leu Ser Ser Lys Ala Val Asn His Ile
    2525                2530                2535

Asn Ser Val Trp Lys Asp Leu Leu Glu Asp Thr Glu Thr Pro Ile
    2540                2545                2550

Asp Thr Thr Ile Met Ala Lys Asn Glu Val Phe Cys Val Gln Pro
    2555                2560                2565

Glu Lys Gly Gly Arg Lys Pro Ala Arg Leu Ile Val Tyr Pro Asp
    2570                2575                2580

Leu Gly Val Arg Val Cys Glu Lys Met Ala Leu Tyr Asp Val Val
    2585                2590                2595

Ser Thr Leu Pro Gln Ala Val Met Gly Ser Ser Tyr Gly Phe Gln
    2600                2605                2610

Tyr Ser Pro Gly Gln Arg Val Glu Phe Leu Val Asn Ala Trp Lys
    2615                2620                2625

Ser Lys Lys Asn Pro Met Gly Phe Ala Tyr Asp Thr Arg Cys Phe
    2630                2635                2640

Asp Ser Thr Val Thr Glu Asn Asp Ile Arg Val Glu Glu Ser Ile
    2645                2650                2655

Tyr Gln Cys Cys Asp Leu Ala Pro Glu Ala Arg Gln Val Ile Arg
    2660                2665                2670

Ser Leu Thr Glu Arg Leu Tyr Val Gly Gly Pro Leu Thr Asn Ser
    2675                2680                2685

Lys Gly Gln Asn Cys Gly Tyr Arg Arg Cys Arg Ala Ser Gly Val
    2690                2695                2700

Leu Thr Thr Ser Cys Gly Asn Thr Leu Thr Cys Tyr Leu Lys Ala
    2705                2710                2715

Ser Ala Ala Cys Arg Ala Ala Lys Leu Gln Asp Cys Thr Met Leu
    2720                2725                2730

Val Cys Gly Asp Asp Leu Val Val Ile Cys Glu Ser Ala Gly Thr
    2735                2740                2745

Gln Glu Asp Ala Ala Ser Leu Arg Val Phe Thr Glu Ala Met Thr
    2750                2755                2760

Arg Tyr Ser Ala Pro Pro Gly Asp Pro Pro Arg Pro Glu Tyr Asp
    2765                2770                2775

Leu Glu Leu Ile Thr Ser Cys Ser Ser Asn Val Ser Val Ala His
    2780                2785                2790

Asp Ala Ser Gly Lys Arg Val Tyr Tyr Leu Thr Arg Asp Pro Thr
    2795                2800                2805

Thr Pro Leu Ala Arg Ala Ala Trp Glu Thr Ala Lys His Thr Pro
    2810                2815                2820

Val Asn Ser Trp Leu Gly Asn Ile Ile Met Tyr Ala Pro Thr Leu
    2825                2830                2835

Trp Ala Arg Met Ile Leu Met Thr His Phe Phe Ser Ile Leu Leu
    2840                2845                2850

Ala Gln Glu Gln Leu Glu Lys Ala Leu Asp Cys Gln Ile Tyr Gly
    2855                2860                2865

Ala Thr Tyr Ser Ile Glu Pro Leu Asp Leu Pro Gln Ile Ile Gln
    2870                2875                2880

Arg Leu His Gly Leu Ser Ala Phe Ser Leu His Ser Tyr Ser Pro
    2885                2890                2895

Gly Glu Ile Asn Arg Val Ala Ser Cys Leu Arg Lys Leu Gly Val
    2900                2905                2910

Pro Pro Leu Arg Val Trp Arg His Arg Ala Arg Ser Val Arg Ala
    2915                2920                2925

Lys Leu Leu Ser Gln Gly Gly Arg Ala Ala Thr Cys Gly Lys Tyr
    2930                2935                2940

Leu Phe Asn Trp Ala Val Arg Thr Lys Leu Lys Leu Thr Pro Ile
    2945                2950                2955

Pro Ala Ala Ser Gln Leu Asp Leu Ser Gly Trp Phe Ile Ala Gly
    2960                2965                2970

Tyr Ser Gly Gly Asp Ile Tyr His Ser Leu Ser Arg Ala Arg Pro
    2975                2980                2985

Arg Trp Phe Met Leu Cys Leu Leu Leu Leu Ser Val Gly Val Gly
    2990                2995                3000

Ile Tyr Leu Leu Pro Asn Arg
    3005                3010

<210> 3
<211> 9594
<212> RNA
<213> Hepatitis C virus

<400> 3
gccagccccc ugaugggggc gacacuccac cauagaucac uccccuguga ggaacuacug 60

ucuucacgca gaaagcgucu agccauggcg uuaguaugag ugucgugcag ccuccaggac 120

cccccucccc gggagagcca uaguggucug cggaaccggu gaguacaccg gaauugccag 180

gacgaccggg uccuuucuug gaucaacccg cucaaugccu ggagauuugg gcgugccccc 240

gcgagacugc uagccgagua guguuggguc gcgaaaggcc uugugguacu gccugauagg 300

gugcuugcga gugccccggg aggucucgua gaccgugcau caugagcaca aauccuaaac 360

cucaaagaaa aaccaaacgu aacaccaacc gccgcccaca ggacgucaag uucccgggcg 420

guggccagau cguuggugga guuuaccugu ugccgcgcag gggccccagg uugggugugc 480

gcgcgacuag gaagacuucc gagcggucgc aaccucgugg aaggcgacaa ccuaucccca 540

aggcucgcca gcccgagggc agggccuggg cucagcccgg guauccuugg ccccucuaug 600

gcaacgaggg ucuggggugg gcaggauggc uccugucacc ccguggcucu cggccuaguu 660

ggggccccac ggacccccgg cguaggucgc guaauuuggg uaaggucauc gauacccuca 720

caugcggcuu cgccgaccuc augggguaca uuccgcucgu cggcgccccc cuaggaggcg 780

cugccagggc ccuggcgcau ggcguccggg uucuggagga cggcgugaac uaugcaacag 840

ggaaucugcc cgguugcccu uucucuaucu ucccucuagc uuugcugucc uguuugacca 900

ucccagcuuc cgcucacgaa gugcgcaacg uauccgggcu guaccauguc acgaacgacu 960
gcuccaacuc aagcauugug uaugaggcag cggacaugau caugcacacc cccgggugcg 1020
ugcccugcgu ccgggagggu aacuccuccc gcugcugggu agcgcucacu cccacgcucg 1080
cggccaggaa uagcagcguc cccacugcga caauacgacg ccaugucgau uugcucgucg 1140
gggcggcugc uuucuguucc gcuauguacg ugggggaucu uugcggaucu guuuuccucg 1200
ucucccagcu guucaccuuu ucaccucgcc gguacgagac gguacaggac ugcaauugcu 1260
cacucuaucc cggccacgua ucaggccauc gcauggcuug ggauaugaug augaacuggu 1320
caccuacaac agccuuagug guaucgcagu uacuccggau cccacaagcc gucguggaua 1380
uggugguagg ggcccacugg ggaguccugg cgggccuugc cuacuauucc auggugggga 1440
acugggcuaa ggucuugauu gugaugcuac ucuuugccgg cgucgacggg aagaccuacg 1500
ugacaggggg ggcgcagagc cgagccacuc aaggcuuugc gucccucuuu acacgggggc 1560
cgucucagaa acuccagcuu guaaauucca acggcagcug gcacauuaac aggacugccu 1620
ugaacugcaa ugacuccuuc cagacugggu uccuugccgc gcuguuuuac gcacaccguu 1680
ucaacucguc cggaugccca gagcgcaugg ccagcugccg ccccaucgac acguucgauc 1740
aggggugggg ccccaucacu caugucgcgc gucgcacauc ggaccagagg ccuuauugcu 1800
ggcacuacgc accucaaccg ugugguauug uacccgcguu gcagguaugu gguccagugu 1860
auugcuucac cccaagcccc gucguggugg ggacgaccga ucgcuucggc gcccccacgu 1920
acaacugggg ggagaaugag acggacgugc uacuccucaa caauacgcgg ccgccgcacg 1980
gcaacugguu cggcuguaca uggaugaaua guaccggguu caccaagacg ugugggggcc 2040
cccccugcaa caucgggggg uuuggcaaca acaccuugac cugcccuacg gauugcuucc 2100
ggaagcaccc cgaggccacu uacaccaaau gcggcucggg gcccugguug acgccuaggu 2160
gcaugguuga uuacccauac agacuuuggc acuaccccug cacuguuaac uuuuccaucu 2220
ucaaggucag gauguaugug gggggugugg agcacaggcu caccgccgcg ugcaauugga 2280
cucggggaga gcgcugcaac uuggaggaua gggacagauc ggagcucagc ccgcugcuac 2340
ugucuaccac agaguggcag guacugcccu guucuuucac caccuuaccg gcccugucca 2400
cugguuugau ccaccuccac cagaacaucg uggacgugca auaccuguac gguguggggu 2460
caucgguugu cuccauugca aucagguggg aguaugucgu gcugcucuuc cuccuccugg 2520
cggacgcgcg yguuugcgcc ugcuugugga ugaugcugcu gauagcccaa gcugaggccg 2580
ccuuagagaa ccuggugauc cucaaugcgg cgucuguggc cggagcgcau ggcguucucu 2640
cuuuccuugu guucuucugc gcugccuggu acaucaaggg caagcugguc cccggggcgg 2700
cauaugccuu cuauggugua uggccgcugc uccugcuucu gcugucauua ccaccacgag 2760
cauacgccuu ggaccgggag auggcugcau cgugcggagg cgcgguuuuc guaggucuga 2820
ugcuccugac cuugucacca cacuacaagg uguuucucgc uaggcucaua uggugguuac 2880
aguauuuuau caccagggcc gaggcgcacu ugcaggugug gguccccccc cucaacguuc 2940
gggggggccg cgaugccauc auccuccuca cguguguggu ccacccagag cuaauuuuug 3000

acaucaccaa aaucuugcuc gccaugcucg guccgcucau ggugcuccag gcuggccuaa 3060
cuagagugcc guacuucgua cgcgcucaag ggcucauccg ugcaugcaug uuagugcgga 3120
aagucgcugg gggccacuau guccaaaugg cccucaugaa acuggccgca cugacgggua 3180
cguacguuua ugaccaucuu acuccgcugc aggacugggc ccacgcgggc uugcgagacc 3240
uugcaguggc aguugagccc gucgucuucu cugacaugga gacuaagguc aucaccuggg 3300
gggcagacac cgcagcgugu ggggacauca ucucgggccu acccgucucc gcccgaaggg 3360
ggagggagau acuucugggc cccgccgaca gguuuggaga gcaggggugg cgacuccucg 3420
cgccuaucac ggcuuacgcu caacagacgc ggggccuacu uggcuguauc aucaccagcc 3480
ucacaggccg ggacaagaac caggucgagg gggagguuca ggugguuucc accgcaacgc 3540
aaucuuuccu ggcgaccugc gucaacggcg uguguuggac ugucuaccau ggugccggcu 3600
cgaagacccu ggccggcccg aagggcccaa ucacccaaau guacaccaau guggaccaag 3660
accucgucgg cuggccggcg cccccggggg cgcgcucccu gacaccgugc accugcggca 3720
gcucggaccu cuaccugguc acgaggcaug cugaugucau uccggugcgc cggcggggcg 3780
acagcagggg gagucuacuc ucucccaggc ccaucuccua cuuaaagggc uccucaggug 3840
guccacugcu uugcccccug gggcacgcug ugggcaucuu ccgggccgcu gugugcaccc 3900
gggggguugc aaaggcggug gauuuuguac cuguugaguc uauggaaacc accaugcggu 3960
cuccggucuu uacggauaau ucaucucccc cggccguacc gcagacauuc caaguggccc 4020
aucuacacgc ucccacuggc aguggcaaga gcacuaaggu gccggcugcg uacgcagccc 4080
aaggguacaa gguacucguc uugaacccau ccguugccgc uaccuuaggg uuuggggcgu 4140
acaugucuaa agcacauggu guugagccua acaucagaac ugggguaagg accaucacca 4200
cgggcgcuuc caucacguau uccaccuacg guaaguuccu ugccgacggu gguugcucug 4260
ggggcgccua ugacaucaua auaugugaug agugccacuc aacugacucg acuuccaucu 4320
ugggcauugg cacaguccug gaccaagcgg agacggcugg agcgcggcuc gucgugcucg 4380
ccaccgcuac gccuccggga ucggucaccg ugccacaucc caauaucgag gagguggccu 4440
ugcccagcac cggagaaauu cccuucuacg gcaaagccau ccccauugag accaucaagg 4500
gggggaggca ccucaucuuc ugccacucca agaagaaaug ugacgagcuc gcugcaaagc 4560
uggugggccu cggaguuaac gcuguugcgu acuaccgggg ucuugaugug uccgucauac 4620
caacaagcgg agaugucguu gucguggcaa cagacgcucu aaugacgggc uucaccggcg 4680
acuuugacuc agugaucgac uguaauacuu gugucaccca gacaguugau uucagcuugg 4740
acccuaccuu caccauugag acgacaaccg ugccccaaga cgcggugucg cguucgcagc 4800
gacgaggcag gacuggcagg ggcaggaugg gcauauacag guuuguggcu ccaggggaac 4860
ggcccucggg cauguucgau ucuucggucc ugugugagug cuaugacgcg ggcugugcuu 4920
gguaugagcu cacgcccgcc gagaccucag ucagguugcg ggcuuaccua aauacaccag 4980
ggcugcccgu cugccaggac caccuggagu uuugggaggg ggucuucaca ggccucaccc 5040

acauagaugc ccauuucuug ucccagacua agcaggcagg agauaacuuc cccuaccugg 5100
uagcauacca ggcuacggug ugcgccaggg cccaggcucc cccuccaucg ugggaucaaa 5160
uguggaagug ucucauacgg cugaagccua cacuacacgg gccaacgccc cuguuguaua 5220
ggcuaggagc cguccagaau gaggucaucc ucacacaucc cauaaccaaa uacaucaugg 5280
caugcauguc ggcugaccua gaggucguca cuagcaccug ggugcugguc ggcggggucc 5340
uugcagcucu ggccgcguac ugccugacga cgggcagcgu ggucauugug ggcaggauca 5400
ucuuguccgg gaagccggcu aucauuccug acagggaagu ccucuaccgg gaguucgaug 5460
aaauggaaga gugugccuca caccuccccu acaucgaaca gggaaugcag cucgccgaac 5520
aauucaagca gaaggcgcuc ggguugcugc agacagccac caagcaagcg gaagccgcug 5580
cuccuguggu ggaguccaag uggcgagccc uugaggccuu cugggcgaag cacaugugga 5640
auuucaucag cgggauacag uacuuagcag gcuuguccac ucugccuggg aaccccgcga 5700
uagcaucacu gauggcauuc acagccucua ucaccagccc gcuuaccacc cuacacaccc 5760
uccuguuuaa caucuuggga ggaugggugg ccgcccaacu ugcccccccc ggugcugccu 5820
cggcuuucgu gggcgccggc auugcuggcg cagcuguugg cagcauaggc cuugggaagg 5880
ugcuugugga cauccuggcg gguuauggag cagggguggc aggcgcgcuc guggccuuca 5940
aggucaugag cggcgagaug cccuccaccg aggaccuggu caacuuacuc ccugccaucc 6000
ucucuccugg ugcccuuguc gucggggucg ugugcgcagc aauacugcgu cggcaugugg 6060
gcccggggga gggggcugug caauggauga accggcugau agcguucgcc ucgcggggua 6120
accacgucuc ccccacgcac uaugugccug agagcgacgc ugcagcgcgu gucacacaga 6180
uccucucuag ccucaccauc acucagcuac ugaagaggcu ccaccagugg auuaauaagg 6240
acugcuccac accaugcucc ggcucguggc uuagggacgu uugggacugg auaugcacgg 6300
uuuugaguga cuucaagacc uggcuccagu ccaagcuccu gccacgguua ccgggaguuc 6360
cauuccuuuc augccaacgu ggguauaagg gggucuggcg gggagauggc aucaugcaga 6420
ccuccugccc auguggagca caaaucgccg gacaugucaa gaacgguucc augaggaucg 6480
uugggccuaa aaccuguagc aacacguggc acggaacauu ccccauuaac gcgcacacca 6540
cgggccccug cacacccucc ccagcgccga acuacucuaa ggcguugugg cggguggcug 6600
cugaggagua cguggaaguc acgcgggugg gggauuucca uuacgugacg ggcaugacca 6660
cugacaacgu aaaaugccca ugccagguuc cggcccccga guucuucaca gagguggaug 6720
ggguacggcu gcacagguac gcuccggcgu gcaaaccucu ccuacgggau gaggucacau 6780
uccaggucgg gcucaaccag uucccgguug ggucacagcu cccaugcgag cccgaaccgg 6840
auguaucagu gcucacuucc augcuuaccg acccuuccca caucacagca gagacggcua 6900
agcguaggcu ggccagaggg ucuucccccu cuuuggccag cucuucagcu agucaguugu 6960
cugcgcccuc auugaaggcg acaugcacca cccaucauga cuccccagac gcugaccuca 7020
uugaggccaa ccuccugugg cggcaggaga ugggagggaa caucacccgu guggagucag 7080
agaacaaggu gguaauccug gacucuuuug acccgcuucg agcggaggag gacgagaggg 7140

aggugucugu ugcggcggag auccugcgga aaaccaggaa guucccccca gcgaugccca 7200
uaugggcacg cccggacuac aacccaccgc ugcuagagac uuggaaggac ccggacuacg 7260
ucccuccagu ggugcacggg ugcccauugc caccuaccaa gaccccucca auaccaccuc 7320
cgcggaggaa aaagacaguu guccugacag aguccaccgu gucuucugcc cuggcggagc 7380
uugccacaaa gaccuuuggc agcuccggau cgucggccgu cgacagcggc acagcgaccg 7440
cccccccuaa ccagcucucc gacgaagugg auacaggauc cgacguugag ucguacuccu 7500
ccaugccccc ccuugaggga gagccggggg accccgaucu cagcgacggg ucuuggucua 7560
cuguaaguga ggaggcuggu gaggacgucg ucugcugcuc gauguccuac acauggacag 7620
gcgccuugau cacgccgugc gccgcggagg agagcaagcu gcccaucaau gcgcugagca 7680
acucuuugcu gcgccaccac aacauggucu augccacaac aucccgcagc gcaagccaac 7740
ggcagaaaaa ggucaccuuu gacagacugc aaguccugga cgaccauuac cgggacgugc 7800
ucaaggagau gaaggcgaag gcguccacag uuaaggcuaa acuucuaucc guagaagagg 7860
ccugcaagcu gacgccccca cacucagcca gguccaaauu uggcuauggg gcgaaggacg 7920
uccggaaccu auccagcaag gccguuaacc acaucaacuc cguguggaag gacuugcugg 7980
aagacacuga gacaccaauu gacaccacca ucauggcaaa aaaugagguc uucuguguuc 8040
aaccagagaa gggaggccgc aagccagcuc gccuuaucgu auacccagac uugggggugc 8100
gugugugcga gaaaauggcc cuuuacgacg uggucuccac ucuuccucag gccgugaugg 8160
gcuccucaua cggauuccag uacucuccug ggcagcgggu cgaguuccug gugaaugccu 8220
ggaaaucaaa gaagaacccu augggcuucg cauaugacac ccgcuguuuu gacucaacgg 8280
ucaccgagaa cgacauccgu guugaggagu caauuuacca auguugugac uuggcccccg 8340
aggccagaca ggugauaagg ucgcucacag agcggcuuua ugucgggggc ccccugacua 8400
auucaaaagg gcagaacugc gguuaucgcc ggugccgcgc cagcggcgug cugacgacua 8460
gcugcgguaa uacccucaca uguuacuuga aggccucugc agccugucga gcugcaaagc 8520
uccaggacug cacgaugcuc gugugcgggg acgaccuugu cguuaucugu gaaagcgcgg 8580
ggacccagga ggacgcggcg agccuacgag ucuucacgga ggcuaugacu agguacuccg 8640
ccccccccgg ggacccgccc cgaccggaau acgacuugga guugauaaca ucaugcuccu 8700
ccaacguguc ggucgcgcac gaugcaucug gcaaacgggu guauuaccuc acccgugacc 8760
ccaccacccc ccuugcgcgg gcugcguggg agacagcuaa acacacucca gucaacuccu 8820
ggcuaggcaa caucaucaug uaugcgccca cccucugggc aaggaugauu cugaugacuc 8880
acuucuucuc cauccuucua gcucaggagc agcuugaaaa agcccuggau ugucagaucu 8940
acggggccac uuacuccauu gaaccacuug accuaccuca gaucauucaa cgacuccaug 9000
gucuuagcgc auucucacuc cauaguuacu cuccagguga aaucaauagg guggcuucau 9060
gccucaggaa acuuggggua ccgcccuugc gagucuggag acaucgggcc agaagugucc 9120
gcgcuaagcu acugucccag gggggagggc ugccacuugu ggcaaguacc cucuucaacu 9180

gggcaguaag gaccaagcuc aaacucacuc caaucccggc ugcgucccag uuggacuugu 9240

ccggcugguu cauugcuggu uacagcgggg gagacauaua ucacagccug ucucgugccc 9300

gaccccgcug guuuauguug ugccuacucc uacuuucugu ggggguaggc aucuaccugc 9360

uccccaaucg augaacgggg ggcuaaacac uccaggccaa uaggccauuc uguuuuuuuu 9420

uuuuuuuuuu uuuuuuuuuu uuuuuuuuuu uuuuuuuuuu uuuuuuccuu uuuuuuuuuu 9480

uuuuucccuu ucuuuuggug gcuccaucuu agcccuaguc acggcuagcu gugaaagguc 9540

cgugagccgc augacugcag agagugcuga uacuggccuc ucugcagauc augu 9594

<210> 4
<211> 3010
<212> PRT
<213> Hepatitis C virus

<400> 4

Met Ser Thr Asn Pro Lys Pro Gln Arg Lys Thr Lys Arg Asn Thr Asn
1               5                   10                  15

Arg Arg Pro Gln Asp Val Lys Phe Pro Gly Gly Gly Gln Ile Val Gly
            20                  25                  30

Gly Val Tyr Leu Leu Pro Arg Arg Gly Pro Arg Leu Gly Val Arg Ala
        35                  40                  45

Thr Arg Lys Thr Ser Glu Arg Ser Gln Pro Arg Gly Arg Arg Gln Pro
    50                  55                  60

Ile Pro Lys Ala Arg Gln Pro Glu Gly Arg Ala Trp Ala Gln Pro Gly
65                  70                  75                  80

Tyr Pro Trp Pro Leu Tyr Gly Asn Glu Gly Leu Gly Trp Ala Gly Trp
                85                  90                  95

Leu Leu Ser Pro Arg Gly Ser Arg Pro Ser Trp Gly Pro Thr Asp Pro
            100                 105                 110

Arg Arg Arg Ser Arg Asn Leu Gly Lys Val Ile Asp Thr Leu Thr Cys
        115                 120                 125

Gly Phe Ala Asp Leu Met Gly Tyr Ile Pro Leu Val Gly Ala Pro Leu
    130                 135                 140

Gly Gly Ala Ala Arg Ala Leu Ala His Gly Val Arg Val Leu Glu Asp
145                 150                 155                 160

Gly Val Asn Tyr Ala Thr Gly Asn Leu Pro Gly Cys Pro Phe Ser Ile
                165                 170                 175

Phe Leu Leu Ala Leu Leu Ser Cys Leu Thr Ile Pro Ala Ser Ala His
            180                 185                 190

Glu Val Arg Asn Val Ser Gly Leu Tyr His Val Thr Asn Asp Cys Ser
        195                 200                 205

Asn Ser Ser Ile Val Tyr Glu Ala Ala Asp Met Ile Met His Thr Pro
    210                 215                 220

Gly Cys Val Pro Cys Val Arg Glu Gly Asn Ser Ser Arg Cys Trp Val
225                 230                 235                 240

Ala Leu Thr Pro Thr Leu Ala Ala Arg Asn Ser Ser Val Pro Thr Ala
                245                 250                 255

Thr Ile Arg Arg His Val Asp Leu Leu Val Gly Ala Ala Ala Phe Cys
            260                 265                 270

Ser Ala Met Tyr Val Gly Asp Leu Cys Gly Ser Val Phe Leu Val Ser
        275                 280                 285

Gln Leu Phe Thr Phe Ser Pro Arg Arg Tyr Glu Thr Val Gln Asp Cys
    290                 295                 300

Asn Cys Ser Leu Tyr Pro Gly His Val Ser Gly His Arg Met Ala Trp
305                 310                 315                 320

Asp Met Met Met Asn Trp Ser Pro Thr Thr Ala Leu Val Val Ser Gln
                325                 330                 335

Leu Leu Arg Ile Pro Gln Ala Val Val Asp Met Val Val Gly Ala His
            340                 345                 350

Trp Gly Val Leu Ala Gly Leu Ala Tyr Tyr Ser Met Val Gly Asn Trp
        355                 360                 365

Ala Lys Val Leu Ile Val Met Leu Leu Phe Ala Gly Val Asp Gly Lys
    370                 375                 380

Thr Tyr Val Thr Gly Gly Ala Gln Ser Arg Ala Thr Gln Gly Phe Ala
385                 390                 395                 400

Ser Leu Phe Thr Arg Gly Pro Ser Gln Lys Leu Gln Leu Val Asn Ser
                405                 410                 415

Asn Gly Ser Trp His Ile Asn Arg Thr Ala Leu Asn Cys Asn Asp Ser
            420                 425                 430

Phe Gln Thr Gly Phe Leu Ala Ala Leu Phe Tyr Ala His Arg Phe Asn
        435                 440                 445

Ser Ser Gly Cys Pro Glu Arg Met Ala Ser Cys Arg Pro Ile Asp Thr
    450                 455                 460

Phe Asp Gln Gly Trp Gly Pro Ile Thr His Val Ala Arg Arg Thr Ser
465                 470                 475                 480

Asp Gln Arg Pro Tyr Cys Trp His Tyr Ala Pro Gln Pro Cys Gly Ile
                485                 490                 495

Val Pro Ala Leu Gln Val Cys Gly Pro Val Tyr Cys Phe Thr Pro Ser
            500                 505                 510

Pro Val Val Val Gly Thr Thr Asp Arg Phe Gly Ala Pro Thr Tyr Asn
        515                 520                 525

Trp Gly Glu Asn Glu Thr Asp Val Leu Leu Leu Asn Asn Thr Arg Pro
    530                 535                 540

Pro His Gly Asn Trp Phe Gly Cys Thr Trp Met Asn Ser Thr Gly Phe
545                 550                 555                 560

Thr Lys Thr Cys Gly Gly Pro Pro Cys Asn Ile Gly Gly Phe Gly Asn
                565                 570                 575

Asn Thr Leu Thr Cys Pro Thr Asp Cys Phe Arg Lys His Pro Glu Ala
            580                 585                 590

Thr Tyr Thr Lys Cys Gly Ser Gly Pro Trp Leu Thr Pro Arg Cys Met
        595                 600                 605

Val Asp Tyr Pro Tyr Arg Leu Trp His Tyr Pro Cys Thr Val Asn Phe
    610                 615                 620

Ser Ile Phe Lys Val Arg Met Tyr Val Gly Gly Val Glu His Arg Leu
625                 630                 635                 640

Thr Ala Ala Cys Asn Trp Thr Arg Gly Glu Arg Cys Asn Leu Glu Asp
                645                 650                 655

Arg Asp Arg Ser Glu Leu Ser Pro Leu Leu Leu Ser Thr Thr Glu Trp
            660                 665                 670

Gln Val Leu Pro Cys Ser Phe Thr Thr Leu Pro Ala Leu Ser Thr Gly
        675                 680                 685

Leu Ile His Leu His Gln Asn Ile Val Asp Val Gln Tyr Leu Tyr Gly
    690                 695                 700

Val Gly Ser Ser Val Val Ser Ile Ala Ile Arg Trp Glu Tyr Val Val
705                 710                 715                 720

Leu Leu Phe Leu Leu Leu Ala Asp Ala Arg Val Cys Ala Cys Leu Trp
                725                 730                 735

Met Met Leu Leu Ile Ala Gln Ala Glu Ala Ala Leu Glu Asn Leu Val

            740                 745                 750

Ile Leu Asn Ala Ala Ser Val Ala Gly Ala His Gly Val Leu Ser Phe
        755                 760                 765

Leu Val Phe Phe Cys Ala Ala Trp Tyr Ile Lys Gly Lys Leu Val Pro
    770                 775                 780

Gly Ala Ala Tyr Ala Phe Tyr Gly Val Trp Pro Leu Leu Leu Leu Leu
785                 790                 795                 800

Leu Ser Leu Pro Pro Arg Ala Tyr Ala Leu Asp Arg Glu Met Ala Ala
                805                 810                 815

Ser Cys Gly Gly Ala Val Phe Val Gly Leu Met Leu Leu Thr Leu Ser
            820                 825                 830

Pro His Tyr Lys Val Phe Leu Ala Arg Leu Ile Trp Trp Leu Gln Tyr
        835                 840                 845

Phe Ile Thr Arg Ala Glu Ala His Leu Gln Val Trp Val Pro Pro Leu
    850                 855                 860

Asn Val Arg Gly Gly Arg Asp Ala Ile Ile Leu Leu Thr Cys Val Val
865                 870                 875                 880

His Pro Glu Leu Ile Phe Asp Ile Thr Lys Ile Leu Leu Ala Met Leu
                885                 890                 895

Gly Pro Leu Met Val Leu Gln Ala Gly Leu Thr Arg Val Pro Tyr Phe
            900                 905                 910

Val Arg Ala Gln Gly Leu Ile Arg Ala Cys Met Leu Val Arg Lys Val
        915                 920                 925

Ala Gly Gly His Tyr Val Gln Met Ala Leu Met Lys Leu Ala Ala Leu
    930                 935                 940

Thr Gly Thr Tyr Val Tyr Asp His Leu Thr Pro Leu Gln Asp Trp Ala
945                 950                 955                 960

His Ala Gly Leu Arg Asp Leu Ala Val Ala Val Glu Pro Val Val Phe
                965                 970                 975

Ser Asp Met Glu Thr Lys Val Ile Thr Trp Gly Ala Asp Thr Ala Ala
            980                 985                 990

Cys Gly Asp Ile Ile Ser Gly Leu Pro Val Ser Ala Arg Arg Gly Arg
        995                 1000                1005

Glu Ile Leu Leu Gly Pro Ala Asp Arg Phe Gly Glu Gln Gly Trp
    1010                1015                1020

Arg Leu Leu Ala Pro Ile Thr Ala Tyr Ala Gln Gln Thr Arg Gly
    1025                1030                1035

Leu Leu Gly Cys Ile Ile Thr Ser Leu Thr Gly Arg Asp Lys Asn
    1040                1045                1050

Gln Val Glu Gly Glu Val Gln Val Val Ser Thr Ala Thr Gln Ser
    1055                1060                1065

Phe Leu Ala Thr Cys Val Asn Gly Val Cys Trp Thr Val Tyr His
    1070                1075                1080

Gly Ala Gly Ser Lys Thr Leu Ala Gly Pro Lys Gly Pro Ile Thr
    1085                1090                1095

Gln Met Tyr Thr Asn Val Asp Gln Asp Leu Val Gly Trp Pro Ala
    1100                1105                1110

Pro Pro Gly Ala Arg Ser Leu Thr Pro Cys Thr Cys Gly Ser Ser
    1115                1120                1125

Asp Leu Tyr Leu Val Thr Arg His Ala Asp Val Ile Pro Val Arg
    1130                1135                1140

Arg Arg Gly Asp Ser Arg Gly Ser Leu Leu Ser Pro Arg Pro Ile
    1145                1150                1155

Ser Tyr Leu Lys Gly Ser Ser Gly Gly Pro Leu Leu Cys Pro Leu
    1160                1165                1170

Gly His Ala Val Gly Ile Phe Arg Ala Ala Val Cys Thr Arg Gly
    1175                1180                1185

Val Ala Lys Ala Val Asp Phe Val Pro Val Glu Ser Met Glu Thr
    1190                1195                1200

Thr Met Arg Ser Pro Val Phe Thr Asp Asn Ser Ser Pro Pro Ala
    1205                1210                1215

Val Pro Gln Thr Phe Gln Val Ala His Leu His Ala Pro Thr Gly
    1220                1225                1230

Ser Gly Lys Ser Thr Lys Val Pro Ala Ala Tyr Ala Ala Gln Gly
    1235                1240                1245

Tyr Lys Val Leu Val Leu Asn Pro Ser Val Ala Ala Thr Leu Gly
    1250                1255                1260

Phe Gly Ala Tyr Met Ser Lys Ala His Gly Val Glu Pro Asn Ile
    1265                1270                1275

Arg Thr Gly Val Arg Thr Ile Thr Thr Gly Ala Ser Ile Thr Tyr
    1280                1285                1290

Ser Thr Tyr Gly Lys Phe Leu Ala Asp Gly Gly Cys Ser Gly Gly
    1295                1300                1305

Ala Tyr Asp Ile Ile Ile Cys Asp Glu Cys His Ser Thr Asp Ser
    1310                1315                1320

Thr Ser Ile Leu Gly Ile Gly Thr Val Leu Asp Gln Ala Glu Thr
    1325                1330                1335

Ala Gly Ala Arg Leu Val Val Leu Ala Thr Ala Thr Pro Pro Gly
    1340                1345                1350

Ser Val Thr Val Pro His Pro Asn Ile Glu Glu Val Ala Leu Pro
    1355                1360                1365

Ser Thr Gly Glu Ile Pro Phe Tyr Gly Lys Ala Ile Pro Ile Glu
    1370                1375                1380

Thr Ile Lys Gly Gly Arg His Leu Ile Phe Cys His Ser Lys Lys
    1385                1390                1395

Lys Cys Asp Glu Leu Ala Ala Lys Leu Val Gly Leu Gly Val Asn
    1400                1405                1410

Ala Val Ala Tyr Tyr Arg Gly Leu Asp Val Ser Val Ile Pro Thr
    1415                1420                1425

Ser Gly Asp Val Val Val Val Ala Thr Asp Ala Leu Met Thr Gly
    1430                1435                1440

Phe Thr Gly Asp Phe Asp Ser Val Ile Asp Cys Asn Thr Cys Val
    1445                1450                1455

Thr Gln Thr Val Asp Phe Ser Leu Asp Pro Thr Phe Thr Ile Glu
    1460                1465                1470

Thr Thr Thr Val Pro Gln Asp Ala Val Ser Arg Ser Gln Arg Arg
    1475                1480                1485

Gly Arg Thr Gly Arg Gly Arg Met Gly Ile Tyr Arg Phe Val Ala
    1490                1495                1500

Pro Gly Glu Arg Pro Ser Gly Met Phe Asp Ser Ser Val Leu Cys
    1505                1510                1515

Glu Cys Tyr Asp Ala Gly Cys Ala Trp Tyr Glu Leu Thr Pro Ala
    1520                1525                1530

Glu Thr Ser Val Arg Leu Arg Ala Tyr Leu Asn Thr Pro Gly Leu
    1535                1540                1545

Pro Val Cys Gln Asp His Leu Glu Phe Trp Glu Gly Val Phe Thr
    1550                1555                1560

Gly Leu Thr His Ile Asp Ala His Phe Leu Ser Gln Thr Lys Gln
    1565                1570                1575

Ala Gly Asp Asn Phe Pro Tyr Leu Val Ala Tyr Gln Ala Thr Val
    1580                1585                1590

Cys Ala Arg Ala Gln Ala Pro Pro Pro Ser Trp Asp Gln Met Trp
    1595                1600                1605

Lys Cys Leu Ile Arg Leu Lys Pro Thr Leu His Gly Pro Thr Pro
    1610                1615                1620

Leu Leu Tyr Arg Leu Gly Ala Val Gln Asn Glu Val Ile Leu Thr
    1625                1630                1635

His Pro Ile Thr Lys Tyr Ile Met Ala Cys Met Ser Ala Asp Leu
    1640                1645                1650

Glu Val Val Thr Ser Thr Trp Val Leu Val Gly Gly Val Leu Ala
    1655                1660                1665

Ala Leu Ala Ala Tyr Cys Leu Thr Thr Gly Ser Val Val Ile Val
    1670                1675                1680

Gly Arg Ile Ile Leu Ser Gly Lys Pro Ala Ile Ile Pro Asp Arg
    1685                1690                1695

Glu Val Leu Tyr Arg Glu Phe Asp Glu Met Glu Glu Cys Ala Ser
    1700                1705                1710

His Leu Pro Tyr Ile Glu Gln Gly Met Gln Leu Ala Glu Gln Phe
    1715                1720                1725

Lys Gln Lys Ala Leu Gly Leu Leu Gln Thr Ala Thr Lys Gln Ala
    1730                1735                1740

Glu Ala Ala Ala Pro Val Val Glu Ser Lys Trp Arg Ala Leu Glu
    1745                1750                1755

Ala Phe Trp Ala Lys His Met Trp Asn Phe Ile Ser Gly Ile Gln
    1760                1765                1770

Tyr Leu Ala Gly Leu Ser Thr Leu Pro Gly Asn Pro Ala Ile Ala
    1775                1780                1785

Ser Leu Met Ala Phe Thr Ala Ser Ile Thr Ser Pro Leu Thr Thr

1790                    1795                    1800

Leu His Thr Leu Leu Phe Asn Ile Leu Gly Gly Trp Val Ala Ala
    1805                    1810                    1815

Gln Leu Ala Pro Pro Gly Ala Ala Ser Ala Phe Val Gly Ala Gly
    1820                    1825                    1830

Ile Ala Gly Ala Ala Val Gly Ser Ile Gly Leu Gly Lys Val Leu
    1835                    1840                    1845

Val Asp Ile Leu Ala Gly Tyr Gly Ala Gly Val Ala Gly Ala Leu
    1850                    1855                    1860

Val Ala Phe Lys Val Met Ser Gly Glu Met Pro Ser Thr Glu Asp
    1865                    1870                    1875

Leu Val Asn Leu Leu Pro Ala Ile Leu Ser Pro Gly Ala Leu Val
    1880                    1885                    1890

Val Gly Val Val Cys Ala Ala Ile Leu Arg Arg His Val Gly Pro
    1895                    1900                    1905

Gly Glu Gly Ala Val Gln Trp Met Asn Arg Leu Ile Ala Phe Ala
    1910                    1915                    1920

Ser Arg Gly Asn His Val Ser Pro Thr His Tyr Val Pro Glu Ser
    1925                    1930                    1935

Asp Ala Ala Ala Arg Val Thr Gln Ile Leu Ser Ser Leu Thr Ile
    1940                    1945                    1950

Thr Gln Leu Leu Lys Arg Leu His Gln Trp Ile Asn Lys Asp Cys
    1955                    1960                    1965

Ser Thr Pro Cys Ser Gly Ser Trp Leu Arg Asp Val Trp Asp Trp
    1970                    1975                    1980

Ile Cys Thr Val Leu Ser Asp Phe Lys Thr Trp Leu Gln Ser Lys
    1985                    1990                    1995

Leu Leu Pro Arg Leu Pro Gly Val Pro Phe Leu Ser Cys Gln Arg
    2000                    2005                    2010

Gly Tyr Lys Gly Val Trp Arg Gly Asp Gly Ile Met Gln Thr Ser
    2015                    2020                    2025

Cys Pro Cys Gly Ala Gln Ile Ala Gly His Val Lys Asn Gly Ser
    2030                    2035                    2040

Met Arg Ile Val Gly Pro Lys Thr Cys Ser Asn Thr Trp His Gly
    2045                    2050                    2055

Thr Phe Pro Ile Asn Ala His Thr Thr Gly Pro Cys Thr Pro Ser
    2060                2065                2070

Pro Ala Pro Asn Tyr Ser Lys Ala Leu Trp Arg Val Ala Ala Glu
    2075                2080                2085

Glu Tyr Val Glu Val Thr Arg Val Gly Asp Phe His Tyr Val Thr
    2090                2095                2100

Gly Met Thr Thr Asp Asn Val Lys Cys Pro Cys Gln Val Pro Ala
    2105                2110                2115

Pro Glu Phe Phe Thr Glu Val Asp Gly Val Arg Leu His Arg Tyr
    2120                2125                2130

Ala Pro Ala Cys Lys Pro Leu Leu Arg Asp Glu Val Thr Phe Gln
    2135                2140                2145

Val Gly Leu Asn Gln Phe Pro Val Gly Ser Gln Leu Pro Cys Glu
    2150                2155                2160

Pro Glu Pro Asp Val Ser Val Leu Thr Ser Met Leu Thr Asp Pro
    2165                2170                2175

Ser His Ile Thr Ala Glu Thr Ala Lys Arg Arg Leu Ala Arg Gly
    2180                2185                2190

Ser Ser Pro Ser Leu Ala Ser Ser Ser Ala Ser Gln Leu Ser Ala
    2195                2200                2205

Pro Ser Leu Lys Ala Thr Cys Thr Thr His His Asp Ser Pro Asp
    2210                2215                2220

Ala Asp Leu Ile Glu Ala Asn Leu Leu Trp Arg Gln Glu Met Gly
    2225                2230                2235

Gly Asn Ile Thr Arg Val Glu Ser Glu Asn Lys Val Val Ile Leu
    2240                2245                2250

Asp Ser Phe Asp Pro Leu Arg Ala Glu Glu Asp Glu Arg Glu Val
    2255                2260                2265

Ser Val Ala Ala Glu Ile Leu Arg Lys Thr Arg Lys Phe Pro Pro
    2270                2275                2280

Ala Met Pro Ile Trp Ala Arg Pro Asp Tyr Asn Pro Pro Leu Leu
    2285                2290                2295

Glu Thr Trp Lys Asp Pro Asp Tyr Val Pro Pro Val Val His Gly
    2300                2305                2310

Cys Pro Leu Pro Pro Thr Lys Thr Pro Pro Ile Pro Pro Pro Arg
    2315                2320                2325

Arg Lys Lys Thr Val Val Leu Thr Glu Ser Thr Val Ser Ser Ala
    2330                2335                2340

Leu Ala Glu Leu Ala Thr Lys Thr Phe Gly Ser Ser Gly Ser Ser
    2345                2350                2355

Ala Val Asp Ser Gly Thr Ala Thr Ala Pro Pro Asn Gln Leu Ser
    2360                2365                2370

Asp Glu Val Asp Thr Gly Ser Asp Val Glu Ser Tyr Ser Ser Met
    2375                2380                2385

Pro Pro Leu Glu Gly Glu Pro Gly Asp Pro Asp Leu Ser Asp Gly
    2390                2395                2400

Ser Trp Ser Thr Val Ser Glu Glu Ala Gly Glu Asp Val Val Cys
    2405                2410                2415

Cys Ser Met Ser Tyr Thr Trp Thr Gly Ala Leu Ile Thr Pro Cys
    2420                2425                2430

Ala Ala Glu Glu Ser Lys Leu Pro Ile Asn Ala Leu Ser Asn Ser
    2435                2440                2445

Leu Leu Arg His His Asn Met Val Tyr Ala Thr Thr Ser Arg Ser
    2450                2455                2460

Ala Ser Gln Arg Gln Lys Lys Val Thr Phe Asp Arg Leu Gln Val
    2465                2470                2475

Leu Asp Asp His Tyr Arg Asp Val Leu Lys Glu Met Lys Ala Lys
    2480                2485                2490

Ala Ser Thr Val Lys Ala Lys Leu Leu Ser Val Glu Glu Ala Cys
    2495                2500                2505

Lys Leu Thr Pro Pro His Ser Ala Arg Ser Lys Phe Gly Tyr Gly
    2510                2515                2520

Ala Lys Asp Val Arg Asn Leu Ser Ser Lys Ala Val Asn His Ile
    2525                2530                2535

Asn Ser Val Trp Lys Asp Leu Leu Glu Asp Thr Glu Thr Pro Ile
    2540                2545                2550

Asp Thr Thr Ile Met Ala Lys Asn Glu Val Phe Cys Val Gln Pro
    2555                2560                2565

Glu Lys Gly Gly Arg Lys Pro Ala Arg Leu Ile Val Tyr Pro Asp
    2570                2575                2580

Leu Gly Val Arg Val Cys Glu Lys Met Ala Leu Tyr Asp Val Val
    2585                2590                2595

Ser Thr Leu Pro Gln Ala Val Met Gly Ser Ser Tyr Gly Phe Gln
    2600                2605                2610

Tyr Ser Pro Gly Gln Arg Val Glu Phe Leu Val Asn Ala Trp Lys
    2615                2620                2625

Ser Lys Lys Asn Pro Met Gly Phe Ala Tyr Asp Thr Arg Cys Phe
    2630                2635                2640

Asp Ser Thr Val Thr Glu Asn Asp Ile Arg Val Glu Glu Ser Ile
    2645                2650                2655

Tyr Gln Cys Cys Asp Leu Ala Pro Glu Ala Arg Gln Val Ile Arg
    2660                2665                2670

Ser Leu Thr Glu Arg Leu Tyr Val Gly Gly Pro Leu Thr Asn Ser
    2675                2680                2685

Lys Gly Gln Asn Cys Gly Tyr Arg Arg Cys Arg Ala Ser Gly Val
    2690                2695                2700

Leu Thr Thr Ser Cys Gly Asn Thr Leu Thr Cys Tyr Leu Lys Ala
    2705                2710                2715

Ser Ala Ala Cys Arg Ala Ala Lys Leu Gln Asp Cys Thr Met Leu
    2720                2725                2730

Val Cys Gly Asp Asp Leu Val Val Ile Cys Glu Ser Ala Gly Thr
    2735                2740                2745

Gln Glu Asp Ala Ala Ser Leu Arg Val Phe Thr Glu Ala Met Thr
    2750                2755                2760

Arg Tyr Ser Ala Pro Pro Gly Asp Pro Pro Arg Pro Glu Tyr Asp
    2765                2770                2775

Leu Glu Leu Ile Thr Ser Cys Ser Ser Asn Val Ser Val Ala His
    2780                2785                2790

Asp Ala Ser Gly Lys Arg Val Tyr Tyr Leu Thr Arg Asp Pro Thr
    2795                2800                2805

Thr Pro Leu Ala Arg Ala Ala Trp Glu Thr Ala Lys His Thr Pro
    2810                2815                2820

Val Asn Ser Trp Leu Gly Asn Ile Ile Met Tyr Ala Pro Thr Leu

2825                    2830                    2835

Trp Ala Arg Met Ile Leu Met Thr His Phe Phe Ser Ile Leu Leu
    2840                2845                2850

Ala Gln Glu Gln Leu Glu Lys Ala Leu Asp Cys Gln Ile Tyr Gly
    2855                2860                2865

Ala Thr Tyr Ser Ile Glu Pro Leu Asp Leu Pro Gln Ile Ile Gln
    2870                2875                2880

Arg Leu His Gly Leu Ser Ala Phe Ser Leu His Ser Tyr Ser Pro
    2885                2890                2895

Gly Glu Ile Asn Arg Val Ala Ser Cys Leu Arg Lys Leu Gly Val
    2900                2905                2910

Pro Pro Leu Arg Val Trp Arg His Arg Ala Arg Ser Val Arg Ala
    2915                2920                2925

Lys Leu Leu Ser Gln Gly Gly Arg Ala Ala Thr Cys Gly Lys Tyr
    2930                2935                2940

Leu Phe Asn Trp Ala Val Arg Thr Lys Leu Lys Leu Thr Pro Ile
    2945                2950                2955

Pro Ala Ala Ser Gln Leu Asp Leu Ser Gly Trp Phe Ile Ala Gly
    2960                2965                2970

Tyr Ser Gly Gly Asp Ile Tyr His Ser Leu Ser Arg Ala Arg Pro
    2975                2980                2985

Arg Trp Phe Met Leu Cys Leu Leu Leu Leu Ser Val Gly Val Gly
    2990                2995                3000

Ile Tyr Leu Leu Pro Asn Arg
    3005                3010

<210>  5
<211>  783
<212>  RNA
<213>  Hepatitis C virus

<400>  5
gccucacacc uccccuacau cgaacaggga augcagcucg ccgaacaauu caagcagaag      60

gcgcucgggu ugcugcagac agccaccaag caagcggaag ccgcugcucc ugugguggag     120

uccaaguggc gagcccuuga ggccuucugg gcgaagcaca uguggaauuu caucagcggg     180

auacaguacu uagcaggcuu guccacucug ccugggaacc ccgcgauagc aucacugaug     240

gcauucacag ccucuaucac cagcccgcuu accacccaac acacccuccu guuuaacauc     300

uugggaggau ggguggccgc ccaacuugcc ccccccggug cugccucggc uuucgugggc     360

gccggcauug cuggcgcagc uguuggcagc auaggccuug ggaaggugcu uguggacauc 420

cuggcggguu auggagcagg gguggcaggc gcgcucgugg ccuucaaggu caugagcggc 480

gagaugcccu ccaccgagga ccuggucaac uuacucccug ccauccucuc uccuggugcc 540

cuugucgucg gggucgugug cgcagcaaua cugcgucggc augugggccc gggggagggg 600

gcugugcaau ggaugaaccg gcugauagcg uucgccucgc gggguaacca cgucuccccc 660

acgcacuaug ugccugagag cgacgcugca gcgcguguca cacagauccu cucuagccuc 720

accaucacuc agcuacugaa gaggcuccac caguggauua augaggacug cuccacacca 780

ugc 783

<210> 6
<211> 261
<212> PRT
<213> Hepatitis C virus

<400> 6

Ala Ser His Leu Pro Tyr Ile Glu Gln Gly Met Gln Leu Ala Glu Gln
1               5                   10                  15

Phe Lys Gln Lys Ala Leu Gly Leu Leu Gln Thr Ala Thr Lys Gln Ala
            20                  25                  30

Glu Ala Ala Ala Pro Val Val Glu Ser Lys Trp Arg Ala Leu Glu Ala
        35                  40                  45

Phe Trp Ala Lys His Met Trp Asn Phe Ile Ser Gly Ile Gln Tyr Leu
    50                  55                  60

Ala Gly Leu Ser Thr Leu Pro Gly Asn Pro Ala Ile Ala Ser Leu Met
65                  70                  75                  80

Ala Phe Thr Ala Ser Ile Thr Ser Pro Leu Thr Thr Gln His Thr Leu
                85                  90                  95

Leu Phe Asn Ile Leu Gly Gly Trp Val Ala Ala Gln Leu Ala Pro Pro
            100                 105                 110

Gly Ala Ala Ser Ala Phe Val Gly Ala Gly Ile Ala Gly Ala Ala Val
        115                 120                 125

Gly Ser Ile Gly Leu Gly Lys Val Leu Val Asp Ile Leu Ala Gly Tyr
    130                 135                 140

Gly Ala Gly Val Ala Gly Ala Leu Val Ala Phe Lys Val Met Ser Gly
145                 150                 155                 160

Glu Met Pro Ser Thr Glu Asp Leu Val Asn Leu Leu Pro Ala Ile Leu
                165                 170                 175

Ser Pro Gly Ala Leu Val Val Gly Val Val Cys Ala Ala Ile Leu Arg

            180                 185                 190

Arg His Val Gly Pro Gly Glu Gly Ala Val Gln Trp Met Asn Arg Leu
        195                 200                 205

Ile Ala Phe Ala Ser Arg Gly Asn His Val Ser Pro Thr His Tyr Val
    210                 215                 220

Pro Glu Ser Asp Ala Ala Ala Arg Val Thr Gln Ile Leu Ser Ser Leu
225                 230                 235                 240

Thr Ile Thr Gln Leu Leu Lys Arg Leu His Gln Trp Ile Asn Glu Asp
                245                 250                 255

Cys Ser Thr Pro Cys
            260

<210> 7
<211> 783
<212> RNA
<213> Hepatitis C virus

<400> 7
gccucacacc uccccuacau cgaacaggga augcagcucg ccgaacaauu caagcagaag 60

gcgcucgggu ugcugcagac agccaccaag caagcggaag ccgcugcucc ugugguggag 120

uccaaguggc gagcccuuga ggccuucugg gcgaagcaca uguggaauuu caucagcggg 180

auacaguacu uagcaggcuu guccacucug ccugggaacc ccgcgauagc aucacugaug 240

gcauucacag ccucuaucac cagcccgcuu accacccuac acacccuccu guuuaacauc 300

uugggaggau ggguggccgc ccaacuugcc ccccccggug cugccucggc uuucgugggc 360

gccggcauug cuggcgcagc uguuggcagc auaggccuug ggaaggugcu uguggacauc 420

cuggcggguu auggagcagg gguggcaggc gcgcucgugg ccuucaaggu caugagcggc 480

gagaugcccu ccaccgagga ccuggucaac uuacucccug ccauccucuc uccuggugcc 540

cuugucgucg gggucgugug cgcagcaaua cugcgucggc augugggccc gggggagggg 600

gcugugcaau ggaugaaccg gcugauagcg uucgccucgc gggguaacca cgucuccccc 660

acgcacuaug ugccugagag cgacgcugca gcgcguguca cacagauccu cucuagccuc 720

accaucacuc agcuacugaa gaggcuccac caguggauua auaaggacug cuccacacca 780

ugc 783

<210> 8
<211> 261
<212> PRT
<213> Hepatitis C virus

<400> 8

Ala Ser His Leu Pro Tyr Ile Glu Gln Gly Met Gln Leu Ala Glu Gln
1               5                   10                  15

Phe Lys Gln Lys Ala Leu Gly Leu Leu Gln Thr Ala Thr Lys Gln Ala
            20                  25                  30

Glu Ala Ala Ala Pro Val Val Glu Ser Lys Trp Arg Ala Leu Glu Ala
        35                  40                  45

Phe Trp Ala Lys His Met Trp Asn Phe Ile Ser Gly Ile Gln Tyr Leu
    50                  55                  60

Ala Gly Leu Ser Thr Leu Pro Gly Asn Pro Ala Ile Ala Ser Leu Met
65                  70                  75                  80

Ala Phe Thr Ala Ser Ile Thr Ser Pro Leu Thr Thr Leu His Thr Leu
                85                  90                  95

Leu Phe Asn Ile Leu Gly Gly Trp Val Ala Ala Gln Leu Ala Pro Pro
            100                 105                 110

Gly Ala Ala Ser Ala Phe Val Gly Ala Gly Ile Ala Gly Ala Ala Val
        115                 120                 125

Gly Ser Ile Gly Leu Gly Lys Val Leu Val Asp Ile Leu Ala Gly Tyr
    130                 135                 140

Gly Ala Gly Val Ala Gly Ala Leu Val Ala Phe Lys Val Met Ser Gly
145                 150                 155                 160

Glu Met Pro Ser Thr Glu Asp Leu Val Asn Leu Leu Pro Ala Ile Leu
                165                 170                 175

Ser Pro Gly Ala Leu Val Val Gly Val Val Cys Ala Ala Ile Leu Arg
            180                 185                 190

Arg His Val Gly Pro Gly Glu Gly Ala Val Gln Trp Met Asn Arg Leu
        195                 200                 205

Ile Ala Phe Ala Ser Arg Gly Asn His Val Ser Pro Thr His Tyr Val
    210                 215                 220

Pro Glu Ser Asp Ala Ala Ala Arg Val Thr Gln Ile Leu Ser Ser Leu
225                 230                 235                 240

Thr Ile Thr Gln Leu Leu Lys Arg Leu His Gln Trp Ile Asn Lys Asp
                245                 250                 255

Cys Ser Thr Pro Cys
            260

<210> 9
<211> 8010
<212> RNA
<213> Artificial

<220>
<223>  subgenomic HCV RNA replicon

<400>  9
uaauacgacu cacuauagcc agcccccgau ugggggcgac acuccaccau agaucacucc      60

ccugugagga acuacugucu ucacgcagaa agcgucuagc cauggcguua guaugagugu     120

cgugcagccu ccaggacccc cccucccggg agagccauag uggucugcgg aaccggugag     180

uacaccggaa uugccaggac gaccgggucc uuucuuggau caacccgcuc aaugccugga     240

gauuugggcg ugcccccgcg agacugcuag ccgaguagug uugggucgcg aaaggccuug     300

ugguacugcc ugauagggug cuugcgagug ccccgggagg ucucguagac cgugcaccau     360

gagcacgaau ccuaaaccuc aaagaaaaac caaacguaac accaacgggc gcgccaugau     420

ugaacaagau ggauugcacg cagguucucc ggccgcuugg guggagaggc uauucggcua     480

ugacugggca caacagacaa ucggcugcuc ugaugccgcc guguuccggc ugucagcgca     540

ggggcgcccg guucuuuuug ucaagaccga ccuguccggu gcccugaaug aacugcagga     600

cgaggcagcg cggcuaucgu ggcuggccac gacgggcguu ccuugcgcag cugugcucga     660

cguugucacu gaagcgggaa gggacuggcu gcuauugggc gaagugccgg ggcaggaucu     720

ccugucaucu caccuugcuc cugccgagaa aguauccauc auggcugaug caaugcggcg     780

gcugcauacg cuugauccgg cuaccugccc auucgaccac caagcgaaac aucgcaucga     840

gcgagcacgu acucggaugg aagccggucu ugucgaucag gaugaucugg acgaagagca     900

ucaggggcuc gcgccagccg aacuguucgc caggcucaag gcgcgcaugc ccgacggcga     960

ggaucucguc gugacccaug gcgaugccug cuugccgaau aucauggugg aaaauggccg    1020

cuuuucugga uucaucgacu guggccggcu ggguguggcg gaccgcuauc aggacauagc    1080

guuggcuacc cgugauauug cugaagagcu uggcggcgaa ugggcugacc gcuuccucgu    1140

gcuuuacggu aucgccgcuc ccgauucgca gcgcaucgcc uucuaucgcc uucuugacga    1200

guucuucuga guuuaaacag accacaacgg uuucccucua gcgggaucaa uuccgccccu    1260

cucccucccc ccccccuaac guuacuggcc gaagccgcuu ggaauaaggc cggugugcgu    1320

uugucuauau guuauuuucc accauauugc cgucuuuugg caaugugagg gcccggaaac    1380

cuuggcccug ucuucuugac gagcauuccu aggggucuuu ccccucucgc caaaggaaug    1440

caaggucugu uggaugucgu gaaggaagca guuccucugg gaagcuucuu gaagacaaac    1500

aacgucugua gcgacccuuu gcaggcagcg gaacccccca ccuggcgaca ggugccucug    1560

cggccaaaag ccacguguau aagauacacc ugcaaaggcg guacaacccc agugccacgu    1620

ugugaguugg auaguugugg aaagagucaa auggcucucc ucaagcguau ucaacaaggg    1680

gcugaaggau gcccagaagg uaccccauug uaugggaucu gaucuggggc cucggugcac    1740

augcucuaca uguguuuagu cgagguuaaa aaaacgucua ggccccccga accacgggga    1800

cgugguuuuc cuuugaaaaa cacgauaaua ccauggcgcc uaucacggcu uacgcucaac    1860

agacgcgggg ccuacuuggc uguaucauca ccagccucac aggccgggac aagaaccagg    1920

ucgaggggga gguucaggug guuuccaccg caacgcaauc uuuccuggcg accugcguca    1980

acggcgugug uuggacuguc uaccauggug ccggcucgaa gacccuggcc ggcccgaagg 2040
gcccaaucac ccaaauguac accaaugugg accaagaccu cgucggcugg ccggcgcccc 2100
ccggggcgcg cucccugaca ccgugcaccu gcggcagcuc ggaccucuac cuggucacga 2160
ggcaugcuga ugucauuccg gugcgccggc ggggcgacag cagggggagu cuacucucuc 2220
ccaggcccau cuccuacuua aagggcuccu cagguggucc acugcuuugc ccccuggggc 2280
acgcuguggg caucuuccgg gccgcugugu gcacccgggg gguugcaaag gcgguggauu 2340
uuguaccugu ugagucuaug gaaaccacca ugcggucucc ggucuuuacg gauaauucau 2400
cucccccggc cguaccgcag acauuccaag uggcccaucu acacgcuccc acuggcagug 2460
gcaagagcac uaaggugccg gcugcguacg cagcccaagg guacaaggua cucgucuuga 2520
acccauccgu ugccgcuacc uuaggguuug gggcguacau gucuaaagca caugguguug 2580
agccuaacau cagaacuggg guaaggacca ucaccacggg cgcuuccauc acguauucca 2640
ccuacgguaa guuccuugcc gacggugguu gcucuggggg cgccuaugac aucauaauau 2700
gugaugagug ccacucaacu gacucgacuu ccaucuuggg cauuggcaca guccuggacc 2760
aagcggagac ggcuggagcg cggcucgucg ugcucgccac cgcuacgccu ccgggaucgg 2820
ucaccgugcc acaucccaau aucgaggagg uggccuugcc cagcaccgga gaaauucccu 2880
ucuacggcaa agccaucccc auugagacca ucaagggggg gaggcaccuc aucuucugcc 2940
acuccaagaa gaaaugugac gagcucgcug caaagcuggu gggccucgga guuaacgcug 3000
uugcguacua ccggggucuu gauguguccg ucauaccaac aagcggagau gucguugucg 3060
uggcaacaga cgcucuaaug acgggcuuca ccggcgacuu ugacucagug aucgacugua 3120
auacuugugu cacccagaca guugauuuca gcuuggaccc uaccuucacc auugagacga 3180
caaccgugcc ccaagacgcg gugucgcguu cgcagcgacg aggcaggacu ggcaggggca 3240
ggaugggcau auacagguuu guggcuccag gggaacggcc cucgggcaug uucgauucuu 3300
cgguccugug ugagugcuau gacgcgggcu gugcuuggua ugagcucacg cccgccgaga 3360
ccucagucag guugcgggcu uaccuaaaua caccagggcu gcccgucugc caggaccacc 3420
uggaguuuug ggaggggguc uucacaggcc ucacccacau agaugcccau uucuuguccc 3480
agacuaagca ggcaggagau aacuuccccu accugguagc auaccaggcu acggugugcg 3540
ccagggccca ggcucccccu ccaucguggg aucaaaugug gaagugucuc auacggcuga 3600
agccuacacu acacgggcca acgccccugu uguauaggcu aggagccguc cagaaugagg 3660
ucauccucac acaucccaua accaaauaca ucauggcaug caugucggcu gaccuagagg 3720
ucgucacuag caccugggug cuggucggcg ggguccuugc agcucuggcc gcguacugcc 3780
ugacgacggg cagcgugguc auugugggca ggaucaucuu guccgggaag ccggcuauca 3840
uuccugacag ggaaguccuc uaccgggagu ucgaugaaau ggaagagugu gccucacacc 3900
uccccuacau cgaacaggga augcagcucg ccgaacaauu caagcagaag gcgcucgggu 3960
ugcugcagac agccaccaag caagcggaag ccgcugcucc ugugguggag uccaaguggc 4020

gagcccuuga ggccuucugg gcgaagcaca uguggaauuu caucagcggg auacaguacu 4080
uagcaggcuu guccacucug ccugggaacc ccgcgauagc aucacugaug gcauucacag 4140
ccucuaucac cagcccgcuu accacccaac acacccuccu guuuaacauc uugggaggau 4200
ggguggccgc ccaacuugcc ccccccggug cugccucggc uuucgugggc gccggcauug 4260
cuggcgcagc uguuggcagc auaggccuug ggaaggugcu uguggacauc cuggcggguu 4320
auggagcagg gguggcaggc gcgcucgugg ccuucaaggu caugagcggc gagaugcccu 4380
ccaccgagga ccuggucaac uuacucccug ccauccucuc uccuggugcc cuugucgucg 4440
gggucgugug cgcagcaaua cugcgucggc augugggccc gggggagggg gcugugcaau 4500
ggaugaaccg gcugauagcg uucgccucgc gggguaacca cgucuccccc acgcacuaug 4560
ugccugagag cgacgcugca gcgcguguca cacagauccu cucuagccuc accaucacuc 4620
agcuacugaa gaggcuccac caguggauua augaggacug cuccacacca ugcuccggcu 4680
cguggcuuag ggacguuugg gacuggauau gcacgguuuu gagugacuuc aagaccuggc 4740
uccaguccaa gcuccugcca cgguuaccgg gaguuccauu ccuuucaugc caacgugggu 4800
auaagggggu cuggcgggga gauggcauca ugcagaccuc cugcccaugu ggagcacaaa 4860
ucgccggaca ugucaagaac gguuccauga ggaucguugg gccuaaaacc uguagcaaca 4920
cguggcacgg aacauucccc auuaacgcgc acaccacggg ccccugcaca cccuccccag 4980
cgccgaacua cucuaaggcg uuguggcggg uggcugcuga ggaguacgug gaagucacgc 5040
ggguggggga uuuccauuac gugacgggca ugaccacuga caacguaaaa ugcccaugcc 5100
agguuccggc ccccgaguuc uucacagagg uggauggggu acggcugcac agguacgcuc 5160
cggcgugcaa accucuccua cgggaugagg ucacauucca ggucgggcuc aaccaguucc 5220
cgguuggguc acagcuccca ugcgagcccg aaccggaugu aucagugcuc acuuccaugc 5280
uuaccgaccc uucccacauc acagcagaga cggcuaagcg uaggcuggcc agagggucuu 5340
cccccucuuu ggccagcucu ucagcuaguc aguugucugc gcccucauug aaggcgacau 5400
gcaccaccca ucaugacucc ccagacgcug accucauuga ggccaaccuc cuguggcggc 5460
aggagauggg agggaacauc acccgugugg agucagagaa caagguggua auccuggacu 5520
cuuuugaccc gcuucgagcg gaggaggacg agagggaggu gucuguugcg gcggagaucc 5580
ugcggaaaac caggaaguuc cccccagcga ugcccauaug ggcacgcccg gacuacaacc 5640
caccgcugcu agagacuugg aaggacccgg acuacguccc uccaguggug cacgggugcc 5700
cauugccacc uaccaagacc ccuccaauac caccuccgcg gaggaaaaag acaguugucc 5760
ugacagaguc caccgugucu ucugcccugg cggagcuugc cacaaagacc uuuggcagcu 5820
ccggaucguc ggccgucgac agcggcacag cgaccgcccc cccuaaccag cucuccgacg 5880
aaguggauac aggauccgac guugagucgu acuccuccau gcccccccuu gagggagagc 5940
cgggggaccc cgaucucagc gacgggucuu ggucuacugu aagugaggag gcuggugagg 6000
acgucgucug cugcucgaug uccuacacau ggacaggcgc cuugaucacg ccgugcgccg 6060
cggaggagag caagcugccc aucaaugcgc ugagcaacuc uuugcugcgc caccacaaca 6120

uggucuaugc cacaacaucc cgcagcgcaa gccaacggca gaaaaagguc accuuugaca 6180
gacugcaagu ccuggacgac cauuaccggg acgugcucaa ggagaugaag gcgaaggcgu 6240
ccacaguuaa ggcuaaacuu cuauccguag aagaggccug caagcugacg cccccacacu 6300
cagccagguc caaauuuggc uauggggcga aggacguccg gaaccuaucc agcaaggccg 6360
uuaaccacau caacuccgug uggaaggacu ugcuggaaga cacugagaca ccaauugaca 6420
ccaccaucau ggcaaaaaau gaggucuucu guguucaacc agagaaggga ggccgcaagc 6480
cagcucgccu uaucguauac ccagacuugg gggugcgugu gugcgagaaa auggcccuuu 6540
acgacguggu cuccacucuu ccucaggccg ugaugggcuc cucauacgga uuccaguacu 6600
cuccugggca gcgggucgag uuccugguga augccuggaa aucaaagaag aacccuaugg 6660
gcuucgcaua ugacacccgc uguuuugacu caacggucac cgagaacgac auccguguug 6720
aggagucaau uuaccaaugu ugugacuugg cccccgaggc cagacaggug auaaggucgc 6780
ucacagagcg gcuuuauguc gggggccccc ugacuaauuc aaaagggcag aacugcgguu 6840
aucgccggug ccgcgccagc ggcgugcuga cgacuagcug cgguaauacc cucacauguu 6900
acuugaaggc cucugcagcc ugucgagcug caaagcucca ggacugcacg augcucgugu 6960
gcggggacga ccuugucguu aucugugaaa gcgcggggac ccaggaggac gcggcgagcc 7020
uacgagucuu cacggaggcu augacuaggu acuccgcccc ccccggggac ccgccccgac 7080
cggaauacga cuuggaguug auaacaucau gcuccuccaa cgugucgguc gcgcacgaug 7140
caucuggcaa acggguguau uaccucaccc gugaccccac caccccccuu gcgcgggcug 7200
cgugggagac agcuaaacac acuccaguca acuccuggcu aggcaacauc aucauguaug 7260
cgcccacccu cugggcaagg augauucuga ugacucacuu cuucuccauc cuucuagcuc 7320
aggagcagcu ugaaaaagcc cuggauuguc agaucuacgg ggccacuuac uccauugaac 7380
cacuugaccu accucagauc auucaacgac uccauggucu uagcgcauuc ucacuccaua 7440
guuacucucc aggugaaauc aauagggugg cuucaugccu caggaaacuu gggguaccgc 7500
ccuugcgagu cuggagacau cgggccagaa guguccgcgc uaagcuacug ucccaggggg 7560
ggagggcugc cacuuguggc aaguaccucu ucaacugggc aguaaggacc aagcucaaac 7620
ucacuccaau cccggcugcg ucccaguugg acuuguccgg cugguucauu gcugguuaca 7680
gcgggggaga cauauaucac agccugucuc gugcccgacc ccgcugguuu auguugugcc 7740
uacuccuacu uucugugggg guaggcaucu accugcuccc caaucgauga acggggggcu 7800
aaacacucca ggccaauagg ccauucuguu uuuuuuuuuu uuuuuuuuuu uuuuuuuuuu 7860
uuuuuuuuuu uuuuuuuuuu uuccuuuuuu uuuuuuuuuu ucccuuucuu uugguggcuc 7920
caucuuagcc cuagucacgg cuagcuguga aagguccgug agccgcauga cugcagagag 7980
ugcugauacu ggccucucug cagaucaugu 8010

<210> 10
<211> 9594
<212> RNA

<213>  Hepatitis C virus

<400>  10
   gccagccccc ugaugggggc gacacuccac cauagaucac uccccuguga ggaacuacug      60
   ucuucacgca gaaagcgucu agccauggcg uuaguaugag ugucgugcag ccuccaggac     120
   cccccucccc gggagagcca uaguggucug cggaaccggu gaguacaccg gaauugccag     180
   gacgaccggg uccuuucuug gaucaacccg cucaaugccu ggagauuugg gcgugccccc     240
   gcgagacugc uagccgagua guguuggguc gcgaaaggcc uugugguacu gccugauagg     300
   gugcuugcga gugccccggg aggucucgua gaccgugcau caugagcaca aauccuaaac     360
   cucaaagaaa aaccaaacgu aacaccaacc gccgcccaca ggacgucaag uucccgggcg     420
   guggucagau cguuggugga guuuaccugu ugccgcgcag gggccccagg uugggugugc     480
   gcgcgacuag gaagacuucc gagcggucgc aaccucguga aaggcggcaa ccuaucccca     540
   aggcucgccg ccccgagggc agggccuggg cucagcccgg guacccuugg ccccucuaug     600
   gcaaugaggg ucuggggugg gcaggguggc uccugucacc ccgcggcucc cggccuaauu     660
   ggggccccac ggaccccggg cguaggucgc guaauuuggg uaaggucauc gauacccuca     720
   caugcggcuu cgccgaccuc augggguaca uuccgcucgu cggcgccccc cuaggaggcg     780
   uugccagggc ccuggcgcau ggcguccggg uucuggagga cggcgugaac uaugcaacag     840
   ggaauuugcc cgguugcucu uucucuaucu uccucuuggc uuugcugucc uguuugacug     900
   ucccagcuuc cgcuuaugaa gugcgcaacg uguccgggac auaccauguc acgaacgacu     960
   gcgccaacuc aagcauugug uaugaggcag cggacauaau caugcacacc ccugggugcg    1020
   ugcccugcgu ccgggagggc aauuccuccc gcugcugggu agcgcucacu cccacgcucg    1080
   cggccaggaa caggagcauc cccacuacga cgauacgacg ccacgucgau uugcucguug    1140
   gggcggcugc cuucugcucc gccauguacg ugggggaucu cugcgggucu guuuuccuag    1200
   ucucccaacu guucaccuuc ucaccucgcc gguaugagac gguacaagaa ugcaauugcu    1260
   caaucuaucc cggccacgua ucaggucacc gcauggcuug ggauaugaug augaauuggu    1320
   cgcccacagc agcucuagug gugucacagu uacuccgaau cccacaagcc gucguggaua    1380
   ugguggcagg ggcccacugg gggguucugg cgggucuugc cuacuauucc auggugggga    1440
   acugggcuaa agucuugauu gugaugcuac ucuuugccgg cguugacggg accaccaccg    1500
   ugacaggggg agcggcggcc uuuggcacca ggagccuugc guccuucuuu acauuagggc    1560
   cgucucagaa aauccaacuu guaaacacca acggcaguug gcacaucaau aggacugccu    1620
   ugaauuguaa ugacucccuc cagacugggu uccuugcugc gcuguucuau gcacacaaga    1680
   ucaacacguc uggaugccca gagcgcaugg ccagcugccg cccuauugac aaguucgcuc    1740
   aggggugggg ucccaucacc cauggugcgc cugacaccuc ggaccagagg cccuacugcu    1800
   ggcacuacgc accucgaccg ugugguaucg uacccgcguc ggaggugugu ggcccagugu    1860
   auuguuucac cccaagcccu guaguggugg ggacgaccga ucgcuucggc guccccacgu    1920
   auagcugggg ggagaauaag acggacgugc ugcuccucaa caacacgcgg ccgccgcagg    1980
   gcaauugguu cggcugcaca uggaugaaug gcaccggguu caccaagacg ugugggggcc 2040
   ccccguguga uaucgggggg gucggcaaca acaccuugac cugccccacg gauugcuucc 2100
   ggaagcaccc cgaggccacu uauaccaaau gcggcucggg gccuugguug acgccuaggu 2160
   gucuaguuga cuacccauac aggcuuuggc acuaccccug cacugucaac uuuaccaucu 2220
   ucaaggucag gauguaugug gggggcgugg agcacaggcu caacgcugcg ugcaacugga 2280
   cucgaggaga gcgcugugac cuagaggaca gggacagauc agagcuuagc ccgcugcuac 2340
   ugucuacaac agaguggcag auacugcccu guuccuucac cacccuaccg gcucugucca 2400
   cuggcuugau ccaccuccau cagaacaucg uggacgugca auaccuguac gguguagggu 2460
   cagcaguugu cuccguugug aucagauggg aguacguccu gcugcucuuc cuccuccugg 2520
   cggacgcgcg cguuugugcc ugcuuaugga ugaugcuguu gauagcccag gcugaagccg 2580
   ccuuggagaa ccugguaguc cucaacgcgg cguccguggc cggagcgcau ggcauccucu 2640
   cuuuccuugu guucuucugc gcugccuggu acaucaaagg caagcuagug ccuggggugg 2700
   cguaugcucu cuauggugua uggccgcugc uccugcuccu gcuggcguug ccacaacggg 2760
   cguacgccau ggaccgggag auggcugcau cgugcggagg cgcgguuuuc gugggucugg 2820
   uacucuugac cuugucacca cacuauaaag cauuccuugc caaguccaua uggugguuac 2880
   aauauuuaau caccagggcc gaggcgcauu ugcaagugug gguccccccc cucaacguuc 2940
   gggggggccg cgaugccauc auccuccuca cgugcgcggu ccacccagag cuaauuuuug 3000
   acaucaccaa aauuuugcuc gccauauuug gcccgcucau ggugcuccag gcuggucuaa 3060
   cuagagugcc guacuuugug cgcgcucaag ggcucauccg ugugugcaug uuggugcgga 3120
   aagucgcugg gggucauuau auccaaaugg cucucaugag guuggccgca cugacgggca 3180
   cguacguuua ugaucaucuu acuccgcugc gggacugggc ccaugcgggc uugcgagacc 3240
   uugcgguggc aguugagccc gucguuuucu ccaacacaga gaccaagauu aucaccuggg 3300
   gggcggacac cgcggcgugu ggggacauca ucuugggucu accugucucc gcccgaaggg 3360
   ggagagaggu acuccuagga ccggccgaua acuuuggaga gcaagggugg cgacuccuug 3420
   cgcccaucac ggccuacucc caacagacgc ggggcuuacu uggcuguauc aucaccggcc 3480
   ucacaggucg agacaagaac caggucgaag gggagguuca ggugguuucc accgcaacac 3540
   agucuuucuu ggcaaccugc gucaacggug uguguuggac ugucuaccau ggugccggcu 3600
   caaagacccu agccggcccg aaggggccaa ucacccagau guacaccaac guagaccaag 3660
   accucguugg cuggcaggcg ccccccgggg cgcguuccau gacgccgugc accugcggca 3720
   gcucggaccu uuacuugguc acgaggcaug cugaugucau uccggugcgc cggcggggug 3780
   acagcagagg gagccuacuu ucccccaggc ccgucuccua cuugaagggc ucuucaggug 3840
   guccacugcu cugccccuug gggcacgucg ugggcaucuu ucgggcugcc gugugcaccc 3900
   gggggguugc gaaggcggug gacuucauac ccaucgaguc uauggaaacu accaugcggu 3960
   cuccggucuu uacggauaau ucaucucccc cggccguacc gcagacuuuu caaguggccc 4020
   aucugcacgc ccccacuggc agcggcaaga gcaccaaggu gccagcugcg uaugcagccc 4080
   agggguacaa ggugcuuguc uugaauccau ccguugccgc caccuugggu uuuggggcgu 4140
   auauguccaa ggcauacggu gucgacccua acauuagaac ugggguaagg accaucacca 4200
   caggcgcucc caucacguac uccaccuacg gcaaguuccu ugccgacggu gguugcuccg 4260
   ggggcgcuua cgacaucaua auaugcgaug agugccacuc aaccgacucg acuuccauuu 4320
   ugggcauugg cacgguccug gaucaagcgg agacggcugg agcgcgacuc gucgugcucg 4380
   ccaccgcuac gccuccggga ucggucacug ugccacaccc caacaucgag gagguggccu 4440
   uguccaacac cggagagauu cccuucuaug gcaaagccau cccccucgag gucaucaagg 4500
   gggggaggca ucucauuuuc ugucauucua agaagaagug ugaugagcuc gcugcaaagc 4560
   ugucgggccu uggggucaac gcuguagcgu acuaccgggg ucuugaugug uccgucauac 4620
   caacaagcgg ggacgucguu gucguggcaa cagacgcucu aaugacgggc uacaccggug 4680
   acuuugacuc ugugaucgac uguaauacau gugucaccca gacagucgac uucagccugg 4740
   accccaccuu caccauugag acgacgaccg ugccccaaga cgcagugucg cgcucgcagc 4800
   ggcgagggag gacugguagg gguagagggg gcauauacag guuugugacu ccaggagagc 4860
   ggcccucggg cauguucgau uccucggucc ugugugaaug cuaugacgcg ggcugugcuu 4920
   gguacgagcu cacgcccgcc gagaccucgg uuaggcugcg ggcuuaccua aauacaccag 4980
   gguugcccgu cugccaggac cauuuggagu ucugggaagg cgucuucaca ggccucacuc 5040
   auauagaugc ccacuucuug ucucagacua agcaggcagg agacaacuuc cccuaccugg 5100
   uggcauacca ggccacagug ugcgccaggg cccaggcacc accuccauca ugggaucaaa 5160
   uguggaagug ucucauacgg cuaaaaccua cacuacacgg gccaacaccc cuguuguaca 5220
   ggcuaggagc cguccaaaac gaggucaccc ucacacaccc caugaccaaa uacaucauga 5280
   caugcauguc ggcugaccua gaggucguca ccagcacuug ggugcuggug ggcggguucc 5340
   ucgcagcuuu ggccgcguac ugcuugacaa cgggcagcgu gguuaucaua ggcaggauca 5400
   ucuuguccgg aaggccggcu aucauucccg auagggaagu ucucuaccag gaguucgaug 5460
   aaauggaaga gugcgccuca caccuucccu acaucgaaca aggaaugcag cucgccgagc 5520
   aauucaagca gaaggcgcuc ggguuguugc aaacggccac caagcaagcg gaggccgcug 5580
   cucccguggu ggaguccaaa uggcaagccc uugaggccuu cugggcgaag cacaugugga 5640
   acuucaucag cgggauacag uaucuagcag gcuuguccac ucugccugga aaccccgcga 5700
   uagcaucgcu gauggcauuu acagccucua ucacuagccc gcucaccacc caacauaccc 5760
   uucuauuuaa caucuugggg ggaugggugg ccgcccaacu cgcccccccc agcgcugcuu 5820
   cagccuucgu aggcgccggc aucgccggcg cggcuguugg cagcauaggu cuugggaagg 5880
   ugcucgugga cauccuagcg gguuauggag cagggguggc aggcgcacuc guggccuuca 5940
   aggucaugag cggcgaagug cccuccacug aggaccuggu caacuuacuc ccugccaucc 6000
   ucuccccugg ugcccugguc gucggggucg ugugcgcagc gauacugcgu cggcaugugg 6060
   gcccagggga gggggccgug caguggauga accggcugau agcguucgcu ucgcggggua 6120
   accacgucuc ccccacgcac uaugugccug agagcgacgc cgcagcgcgu gucacccaga 6180
   uccucuccag ccuuaccauc acucagcugc uaaagaggcu ccaccagugg auuaaugagg 6240
   acuguuccac gccaugcucc gguucguggc ucagggaugu uugggacugg auaugcacgg 6300
   uuuugaccga cuucaaaacc uggcuccagu ccaagcuccu gccacgguug ccgggacucc 6360
   cuuucuuuuc augucaacgu ggauauaaag gagucuggcu gggagauggc guuaugcaaa 6420
   cuaccugucc auguggugca caaaucagcg gacaugucaa aaacggcucc augaagaucg 6480
   uggggccuaa aaccugcagc aacacguggc acgggacguu ccccaucaac gcauacacca 6540
   caggccccug cacacccucc ccggcgccga acuauuccaa ggcguugugg cgaguggcug 6600
   cugaggagua uguggagguc acgcgggugg gggauuucca cuacgugacg ggcaugacca 6660
   cugacaacgu aaaaugccca ugccaggucc cggcccccga auucuucacg gaguuggaug 6720
   gggugcggcu gcacagguac gcuccggcgu gcaagccucu cuuacgggau gaggucacau 6780
   uccaggucgg gcucaaccag uauccgguug gaucacagcu uccaugugag cccgagccgg 6840
   auguaacagu gcucacuucc augcucaccg accccuccca uauuacagca gagacggcua 6900
   agcguaggcu ggccagaggg uccccccccu cuuuggccag cuccucagcu agccaguugu 6960
   cugcgccuuc cuugaaggcg acaugcacua cccaccauga cuccccagau gcugaccuca 7020
   ucgaggccaa ccuccugugg cggcaggaga ugggcgggaa caucacccgc guggagucag 7080
   agaacaaggu aguaauucug gacucuuuug aaccgcuucg agcggaggag gaugauaggg 7140
   aaguguccgu agcggcggag auccugcgga gaaccaggaa auuccccgca gcgaugccca 7200
   uaugggcacg gccggacuac aacccaccac uccuagaguc uuggaagaac ccagacuacg 7260
   ucccuccagu gguacacggg ugcccauuac caccuauuaa ggccccuccg auaccaccuc 7320
   cacggagaaa gaggacgguc auccugacag aauccaccgu gucuucugcc uuggcggagc 7380
   uugcuacaaa gaccuuuggc agcuccggau cgucggccgu ugacagcggc acagcgacug 7440
   gcccuccuga ucaguccucc ggugacggag auacaggauc cgacgcugag ucgugcuccu 7500
   ccaugccccc ccuugagggg gagccggggg accccgaucu cagcgacggg ucuuggucua 7560
   ccgugagcga ggaggccggu gaggacgucg ucugcugcuc gauguccuac acauggacag 7620
   gcgccuuaau cacaccgugc gccgcagagg agagcaagcu gccuaucaac gcguugagca 7680
   acucuuugcu gcgccaccac aacauggucu augccacaac aucccgcagc gcgggccaac 7740
   ggcagaagaa ggucacuuuu gacagagugc agguccugga cgaccauuac cgggacgugc 7800
   ucaaggagau gaaggcgaag gcguccacag uuaaggcuaa acuucuaccu guagaagaag 7860
   ccugcaggcu gacgccccca cacucggcca gauccaaguu uggcuauggg gcgaaggacg 7920
   uccggaaccu auccagcaag gccgucaacc acauccacuc cguguggaag gacuugcugg 7980
   aagacucuga gacgccgauu gacaccacca ucauggcaaa aaaugagguc uuuuguguuc 8040
   aaccagagaa aggaggccgc aagucagcuc gucuuaucgu auucccagac uuggggguuc 8100
   gugugugcga gaaaauggcc cuuuacgacg ugguuuccac ucuuccucag gccgugaugg 8160
   gcuccucaua cggguuccag uacucuccug gacagcgggu cgaguuccug gugaaugccu 8220
   ggaaaucaaa gaagaacccu augggcuucg cauaugacac ccgcuguuuu gacucaacgg 8280
   ucacugagag ugacauccgu guugaggagu caauuuacca auguugugac uuggcccccg 8340
   aggccagaca ggucauaagg ucgcucacgg agcggcuuua uaucgggggc ccccugacua 8400
   auucaaaagg gcagagcugc ggcuaucgcc ggugccgcgc cagcggugug cugacgacca 8460
   gcugcgguaa uacccucaca uguuacuuga aggcuucugc agccugucga gcugcaaagc 8520
   uccaggacug cacaauguua gugugcggag acgaccuugu cguuaucugu gaaagugcgg 8580
   gaacccagga ggacgcggcg agccuacgag ucuucacgga ggcuaugacu agguacucug 8640
   ccccccccgg ggacccgccc cagccagaau acgacuugga gcugauaaca ucaugcuccu 8700
   ccaacgucuc ggucgcgcac gaugcacuug gcaagcgggu guauuaucug acccgcgacc 8760
   ccaccacccc ccuugcgcgg gcugcguggg agacagcaag acacacucca guuaacuccu 8820
   ggcuaggcaa caucaucaug uaugcgccca cccuaugggc aaggaugauu cugaugaccc 8880
   acuucuuuuc cauccuucua gcucaggaac aacuugaaaa agcccuagau ugucagaucu 8940
   acggggccac uuacuccauu gagccacuug accuaccuca gaucauucag cgacuccacg 9000
   gccuuagcgc auuuucacuc cauagcuacu cuccagguga gaucaauagg guggcuucau 9060
   gccucaggaa acuuggggua ccacccuugc gagucuggag acaucgggcc agaagugucc 9120
   gcgcuaagcu acugucccag ggggggaggg ccgccacuug uggcaaauac cucuucaacu 9180
   gggcaguaag gaccaagcuc aaacucacuc caauuccggc ugcgucccag uuggacuugu 9240
   ccggcugguu cauugcuggu uacagcgggg gagacauaua ucacagccug ucucgcgccc 9300
   gaccccgcug guucaugugg ugccuacucc uacuuuccgu agggguaggc aucuaucugc 9360
   uccccaaucg augaacgggg ggcuaaacac uccaggccaa uaggccauuc uguuuuuuuu 9420
   uuuuuuuuuu uuuuuuuuuu uuuuuuuuuu uuuuuuuuuu uuuuuuccuu uuuuuuuuuu 9480
   uuuuucccuu ucuuuuggug gcuccaucuu agcccuaguc acggcuagcu gugaaagguc 9540
   cgugagccgc augacugcag agagugcuga uacuggccuc ucugcagauc augu 9594

<210> 11
<211> 3010
<212> PRT
<213> Hepatitis C virus

<400> 11

Met Ser Thr Asn Pro Lys Pro Gln Arg Lys Thr Lys Arg Asn Thr Asn
1               5                   10                  15

Arg Arg Pro Gln Asp Val Lys Phe Pro Gly Gly Gly Gln Ile Val Gly
            20                  25                  30

Gly Val Tyr Leu Leu Pro Arg Arg Gly Pro Arg Leu Gly Val Arg Ala
        35                  40                  45

Thr Arg Lys Thr Ser Glu Arg Ser Gln Pro Arg Glu Arg Arg Gln Pro
    50                  55                  60

Ile Pro Lys Ala Arg Arg Pro Glu Gly Arg Ala Trp Ala Gln Pro Gly
65                  70                  75                  80

Tyr Pro Trp Pro Leu Tyr Gly Asn Glu Gly Leu Gly Trp Ala Gly Trp
                85                  90                  95

Leu Leu Ser Pro Arg Gly Ser Arg Pro Asn Trp Gly Pro Thr Asp Pro
            100                 105                 110

Arg Arg Arg Ser Arg Asn Leu Gly Lys Val Ile Asp Thr Leu Thr Cys
        115                 120                 125

Gly Phe Ala Asp Leu Met Gly Tyr Ile Pro Leu Val Gly Ala Pro Leu
    130                 135                 140

Gly Gly Val Ala Arg Ala Leu Ala His Gly Val Arg Val Leu Glu Asp
145                 150                 155                 160

Gly Val Asn Tyr Ala Thr Gly Asn Leu Pro Gly Cys Ser Phe Ser Ile
                165                 170                 175

Phe Leu Leu Ala Leu Leu Ser Cys Leu Thr Val Pro Ala Ser Ala Tyr
            180                 185                 190

Glu Val Arg Asn Val Ser Gly Thr Tyr His Val Thr Asn Asp Cys Ala
        195                 200                 205

Asn Ser Ser Ile Val Tyr Glu Ala Ala Asp Ile Ile Met His Thr Pro
    210                 215                 220

Gly Cys Val Pro Cys Val Arg Glu Gly Asn Ser Ser Arg Cys Trp Val
225                 230                 235                 240

Ala Leu Thr Pro Thr Leu Ala Ala Arg Asn Arg Ser Ile Pro Thr Thr
                245                 250                 255

Thr Ile Arg Arg His Val Asp Leu Leu Val Gly Ala Ala Ala Phe Cys
            260                 265                 270

Ser Ala Met Tyr Val Gly Asp Leu Cys Gly Ser Val Phe Leu Val Ser
        275                 280                 285

Gln Leu Phe Thr Phe Ser Pro Arg Arg Tyr Glu Thr Val Gln Glu Cys
    290                 295                 300

Asn Cys Ser Ile Tyr Pro Gly His Val Ser Gly His Arg Met Ala Trp
305                 310                 315                 320

Asp Met Met Met Asn Trp Ser Pro Thr Ala Ala Leu Val Val Ser Gln
                325                 330                 335

Leu Leu Arg Ile Pro Gln Ala Val Val Asp Met Val Ala Gly Ala His
            340                 345                 350

Trp Gly Val Leu Ala Gly Leu Ala Tyr Tyr Ser Met Val Gly Asn Trp
        355                 360                 365

Ala Lys Val Leu Ile Val Met Leu Leu Phe Ala Gly Val Asp Gly Thr
    370                 375                 380

Thr Thr Val Thr Gly Gly Ala Ala Ala Phe Gly Thr Arg Ser Leu Ala
385                 390                 395                 400

Ser Phe Phe Thr Leu Gly Pro Ser Gln Lys Ile Gln Leu Val Asn Thr
                405                 410                 415

Asn Gly Ser Trp His Ile Asn Arg Thr Ala Leu Asn Cys Asn Asp Ser
            420                 425                 430

Leu Gln Thr Gly Phe Leu Ala Ala Leu Phe Tyr Ala His Lys Ile Asn
        435                 440                 445

Thr Ser Gly Cys Pro Glu Arg Met Ala Ser Cys Arg Pro Ile Asp Lys
    450                 455                 460

Phe Ala Gln Gly Trp Gly Pro Ile Thr His Gly Ala Pro Asp Thr Ser
465                 470                 475                 480

Asp Gln Arg Pro Tyr Cys Trp His Tyr Ala Pro Arg Pro Cys Gly Ile
                485                 490                 495

Val Pro Ala Ser Glu Val Cys Gly Pro Val Tyr Cys Phe Thr Pro Ser
            500                 505                 510

Pro Val Val Val Gly Thr Thr Asp Arg Phe Gly Val Pro Thr Tyr Ser
        515                 520                 525

Trp Gly Glu Asn Lys Thr Asp Val Leu Leu Leu Asn Asn Thr Arg Pro
    530                 535                 540

Pro Gln Gly Asn Trp Phe Gly Cys Thr Trp Met Asn Gly Thr Gly Phe
545                 550                 555                 560

Thr Lys Thr Cys Gly Gly Pro Pro Cys Asp Ile Gly Gly Val Gly Asn
                565                 570                 575

Asn Thr Leu Thr Cys Pro Thr Asp Cys Phe Arg Lys His Pro Glu Ala
            580                 585                 590

Thr Tyr Thr Lys Cys Gly Ser Gly Pro Trp Leu Thr Pro Arg Cys Leu
        595                 600                 605

Val Asp Tyr Pro Tyr Arg Leu Trp His Tyr Pro Cys Thr Val Asn Phe
    610                 615                 620

Thr Ile Phe Lys Val Arg Met Tyr Val Gly Gly Val Glu His Arg Leu
625                 630                 635                 640

Asn Ala Ala Cys Asn Trp Thr Arg Gly Glu Arg Cys Asp Leu Glu Asp
                645                 650                 655

Arg Asp Arg Ser Glu Leu Ser Pro Leu Leu Leu Ser Thr Thr Glu Trp
            660                 665                 670

Gln Ile Leu Pro Cys Ser Phe Thr Thr Leu Pro Ala Leu Ser Thr Gly
        675                 680                 685

Leu Ile His Leu His Gln Asn Ile Val Asp Val Gln Tyr Leu Tyr Gly
    690                 695                 700

Val Gly Ser Ala Val Val Ser Val Val Ile Arg Trp Glu Tyr Val Leu
705                 710                 715                 720

Leu Leu Phe Leu Leu Leu Ala Asp Ala Arg Val Cys Ala Cys Leu Trp
                725                 730                 735

Met Met Leu Leu Ile Ala Gln Ala Glu Ala Ala Leu Glu Asn Leu Val
            740                 745                 750

Val Leu Asn Ala Ala Ser Val Ala Gly Ala His Gly Ile Leu Ser Phe
        755                 760                 765

Leu Val Phe Phe Cys Ala Ala Trp Tyr Ile Lys Gly Lys Leu Val Pro
    770                 775                 780

Gly Val Ala Tyr Ala Leu Tyr Gly Val Trp Pro Leu Leu Leu Leu Leu
785                 790                 795                 800

Leu Ala Leu Pro Gln Arg Ala Tyr Ala Met Asp Arg Glu Met Ala Ala
                805                 810                 815

Ser Cys Gly Gly Ala Val Phe Val Gly Leu Val Leu Leu Thr Leu Ser
            820                 825                 830

Pro His Tyr Lys Ala Phe Leu Ala Lys Ser Ile Trp Trp Leu Gln Tyr
        835                 840                 845

Leu Ile Thr Arg Ala Glu Ala His Leu Gln Val Trp Val Pro Pro Leu
    850                 855                 860

Asn Val Arg Gly Gly Arg Asp Ala Ile Ile Leu Leu Thr Cys Ala Val
865                 870                 875                 880

His Pro Glu Leu Ile Phe Asp Ile Thr Lys Ile Leu Leu Ala Ile Phe

                885                 890                 895

Gly Pro Leu Met Val Leu Gln Ala Gly Leu Thr Arg Val Pro Tyr Phe
            900                 905                 910

Val Arg Ala Gln Gly Leu Ile Arg Val Cys Met Leu Val Arg Lys Val
        915                 920                 925

Ala Gly Gly His Tyr Ile Gln Met Ala Leu Met Arg Leu Ala Ala Leu
    930                 935                 940

Thr Gly Thr Tyr Val Tyr Asp His Leu Thr Pro Leu Arg Asp Trp Ala
945                 950                 955                 960

His Ala Gly Leu Arg Asp Leu Ala Val Ala Val Glu Pro Val Val Phe
                965                 970                 975

Ser Asn Thr Glu Thr Lys Ile Ile Thr Trp Gly Ala Asp Thr Ala Ala
            980                 985                 990

Cys Gly Asp Ile Ile Leu Gly Leu Pro Val Ser Ala Arg Arg Gly Arg
        995                 1000                1005

Glu Val Leu Leu Gly Pro Ala Asp Asn Phe Gly Glu Gln Gly Trp
    1010                1015                1020

Arg Leu Leu Ala Pro Ile Thr Ala Tyr Ser Gln Gln Thr Arg Gly
    1025                1030                1035

Leu Leu Gly Cys Ile Ile Thr Gly Leu Thr Gly Arg Asp Lys Asn
    1040                1045                1050

Gln Val Glu Gly Glu Val Gln Val Val Ser Thr Ala Thr Gln Ser
    1055                1060                1065

Phe Leu Ala Thr Cys Val Asn Gly Val Cys Trp Thr Val Tyr His
    1070                1075                1080

Gly Ala Gly Ser Lys Thr Leu Ala Gly Pro Lys Gly Pro Ile Thr
    1085                1090                1095

Gln Met Tyr Thr Asn Val Asp Gln Asp Leu Val Gly Trp Gln Ala
    1100                1105                1110

Pro Pro Gly Ala Arg Ser Met Thr Pro Cys Thr Cys Gly Ser Ser
    1115                1120                1125

Asp Leu Tyr Leu Val Thr Arg His Ala Asp Val Ile Pro Val Arg
    1130                1135                1140

Arg Arg Gly Asp Ser Arg Gly Ser Leu Leu Ser Pro Arg Pro Val
    1145                1150                1155

Ser Tyr Leu Lys Gly Ser Ser Gly Gly Pro Leu Leu Cys Pro Leu
    1160                1165                1170

Gly His Val Val Gly Ile Phe Arg Ala Ala Val Cys Thr Arg Gly
    1175                1180                1185

Val Ala Lys Ala Val Asp Phe Ile Pro Ile Glu Ser Met Glu Thr
    1190                1195                1200

Thr Met Arg Ser Pro Val Phe Thr Asp Asn Ser Ser Pro Pro Ala
    1205                1210                1215

Val Pro Gln Thr Phe Gln Val Ala His Leu His Ala Pro Thr Gly
    1220                1225                1230

Ser Gly Lys Ser Thr Lys Val Pro Ala Ala Tyr Ala Ala Gln Gly
    1235                1240                1245

Tyr Lys Val Leu Val Leu Asn Pro Ser Val Ala Ala Thr Leu Gly
    1250                1255                1260

Phe Gly Ala Tyr Met Ser Lys Ala Tyr Gly Val Asp Pro Asn Ile
    1265                1270                1275

Arg Thr Gly Val Arg Thr Ile Thr Thr Gly Ala Pro Ile Thr Tyr
    1280                1285                1290

Ser Thr Tyr Gly Lys Phe Leu Ala Asp Gly Gly Cys Ser Gly Gly
    1295                1300                1305

Ala Tyr Asp Ile Ile Ile Cys Asp Glu Cys His Ser Thr Asp Ser
    1310                1315                1320

Thr Ser Ile Leu Gly Ile Gly Thr Val Leu Asp Gln Ala Glu Thr
    1325                1330                1335

Ala Gly Ala Arg Leu Val Val Leu Ala Thr Ala Thr Pro Pro Gly
    1340                1345                1350

Ser Val Thr Val Pro His Pro Asn Ile Glu Glu Val Ala Leu Ser
    1355                1360                1365

Asn Thr Gly Glu Ile Pro Phe Tyr Gly Lys Ala Ile Pro Leu Glu
    1370                1375                1380

Val Ile Lys Gly Gly Arg His Leu Ile Phe Cys His Ser Lys Lys
    1385                1390                1395

Lys Cys Asp Glu Leu Ala Ala Lys Leu Ser Gly Leu Gly Val Asn
    1400                1405                1410

Ala Val Ala Tyr Tyr Arg Gly Leu Asp Val Ser Val Ile Pro Thr
    1415                1420                1425

Ser Gly Asp Val Val Val Ala Thr Asp Ala Leu Met Thr Gly
    1430                1435                1440

Tyr Thr Gly Asp Phe Asp Ser Val Ile Asp Cys Asn Thr Cys Val
        1445                1450                1455

Thr Gln Thr Val Asp Phe Ser Leu Asp Pro Thr Phe Thr Ile Glu
        1460                1465                1470

Thr Thr Thr Val Pro Gln Asp Ala Val Ser Arg Ser Gln Arg Arg
        1475                1480                1485

Gly Arg Thr Gly Arg Gly Arg Gly Gly Ile Tyr Arg Phe Val Thr
        1490                1495                1500

Pro Gly Glu Arg Pro Ser Gly Met Phe Asp Ser Ser Val Leu Cys
        1505                1510                1515

Glu Cys Tyr Asp Ala Gly Cys Ala Trp Tyr Glu Leu Thr Pro Ala
        1520                1525                1530

Glu Thr Ser Val Arg Leu Arg Ala Tyr Leu Asn Thr Pro Gly Leu
        1535                1540                1545

Pro Val Cys Gln Asp His Leu Glu Phe Trp Glu Gly Val Phe Thr
        1550                1555                1560

Gly Leu Thr His Ile Asp Ala His Phe Leu Ser Gln Thr Lys Gln
        1565                1570                1575

Ala Gly Asp Asn Phe Pro Tyr Leu Val Ala Tyr Gln Ala Thr Val
        1580                1585                1590

Cys Ala Arg Ala Gln Ala Pro Pro Pro Ser Trp Asp Gln Met Trp
        1595                1600                1605

Lys Cys Leu Ile Arg Leu Lys Pro Thr Leu His Gly Pro Thr Pro
        1610                1615                1620

Leu Leu Tyr Arg Leu Gly Ala Val Gln Asn Glu Val Thr Leu Thr
        1625                1630                1635

His Pro Met Thr Lys Tyr Ile Met Thr Cys Met Ser Ala Asp Leu
        1640                1645                1650

Glu Val Val Thr Ser Thr Trp Val Leu Val Gly Gly Val Leu Ala
        1655                1660                1665

Ala Leu Ala Ala Tyr Cys Leu Thr Thr Gly Ser Val Val Ile Ile
        1670                1675                1680

Gly Arg Ile Ile Leu Ser Gly Arg Pro Ala Ile Ile Pro Asp Arg
        1685                1690                1695

Glu Val Leu Tyr Gln Glu Phe Asp Glu Met Glu Glu Cys Ala Ser
        1700                1705                1710

His Leu Pro Tyr Ile Glu Gln Gly Met Gln Leu Ala Glu Gln Phe
        1715                1720                1725

Lys Gln Lys Ala Leu Gly Leu Leu Gln Thr Ala Thr Lys Gln Ala
        1730                1735                1740

Glu Ala Ala Ala Pro Val Val Glu Ser Lys Trp Gln Ala Leu Glu
        1745                1750                1755

Ala Phe Trp Ala Lys His Met Trp Asn Phe Ile Ser Gly Ile Gln
        1760                1765                1770

Tyr Leu Ala Gly Leu Ser Thr Leu Pro Gly Asn Pro Ala Ile Ala
        1775                1780                1785

Ser Leu Met Ala Phe Thr Ala Ser Ile Thr Ser Pro Leu Thr Thr
        1790                1795                1800

Gln His Thr Leu Leu Phe Asn Ile Leu Gly Gly Trp Val Ala Ala
        1805                1810                1815

Gln Leu Ala Pro Pro Ser Ala Ala Ser Ala Phe Val Gly Ala Gly
        1820                1825                1830

Ile Ala Gly Ala Ala Val Gly Ser Ile Gly Leu Gly Lys Val Leu
        1835                1840                1845

Val Asp Ile Leu Ala Gly Tyr Gly Ala Gly Val Ala Gly Ala Leu
        1850                1855                1860

Val Ala Phe Lys Val Met Ser Gly Glu Val Pro Ser Thr Glu Asp
        1865                1870                1875

Leu Val Asn Leu Leu Pro Ala Ile Leu Ser Pro Gly Ala Leu Val
        1880                1885                1890

Val Gly Val Val Cys Ala Ala Ile Leu Arg Arg His Val Gly Pro
        1895                1900                1905

Gly Glu Gly Ala Val Gln Trp Met Asn Arg Leu Ile Ala Phe Ala
        1910                1915                1920

Ser Arg Gly Asn His Val Ser Pro Thr His Tyr Val Pro Glu Ser

1925                    1930                    1935

Asp Ala Ala Ala Arg Val Thr Gln Ile Leu Ser Ser Leu Thr Ile
    1940                    1945                    1950

Thr Gln Leu Leu Lys Arg Leu His Gln Trp Ile Asn Glu Asp Cys
    1955                    1960                    1965

Ser Thr Pro Cys Ser Gly Ser Trp Leu Arg Asp Val Trp Asp Trp
    1970                    1975                    1980

Ile Cys Thr Val Leu Thr Asp Phe Lys Thr Trp Leu Gln Ser Lys
    1985                    1990                    1995

Leu Leu Pro Arg Leu Pro Gly Leu Pro Phe Phe Ser Cys Gln Arg
    2000                    2005                    2010

Gly Tyr Lys Gly Val Trp Leu Gly Asp Gly Val Met Gln Thr Thr
    2015                    2020                    2025

Cys Pro Cys Gly Ala Gln Ile Ser Gly His Val Lys Asn Gly Ser
    2030                    2035                    2040

Met Lys Ile Val Gly Pro Lys Thr Cys Ser Asn Thr Trp His Gly
    2045                    2050                    2055

Thr Phe Pro Ile Asn Ala Tyr Thr Thr Gly Pro Cys Thr Pro Ser
    2060                    2065                    2070

Pro Ala Pro Asn Tyr Ser Lys Ala Leu Trp Arg Val Ala Ala Glu
    2075                    2080                    2085

Glu Tyr Val Glu Val Thr Arg Val Gly Asp Phe His Tyr Val Thr
    2090                    2095                    2100

Gly Met Thr Thr Asp Asn Val Lys Cys Pro Cys Gln Val Pro Ala
    2105                    2110                    2115

Pro Glu Phe Phe Thr Glu Leu Asp Gly Val Arg Leu His Arg Tyr
    2120                    2125                    2130

Ala Pro Ala Cys Lys Pro Leu Leu Arg Asp Glu Val Thr Phe Gln
    2135                    2140                    2145

Val Gly Leu Asn Gln Tyr Pro Val Gly Ser Gln Leu Pro Cys Glu
    2150                    2155                    2160

Pro Glu Pro Asp Val Thr Val Leu Thr Ser Met Leu Thr Asp Pro
    2165                    2170                    2175

Ser His Ile Thr Ala Glu Thr Ala Lys Arg Arg Leu Ala Arg Gly
    2180                    2185                    2190

Ser Pro Pro Ser Leu Ala Ser Ser Ser Ala Ser Gln Leu Ser Ala
        2195                2200                2205

Pro Ser Leu Lys Ala Thr Cys Thr Thr His His Asp Ser Pro Asp
        2210                2215                2220

Ala Asp Leu Ile Glu Ala Asn Leu Leu Trp Arg Gln Glu Met Gly
        2225                2230                2235

Gly Asn Ile Thr Arg Val Glu Ser Glu Asn Lys Val Val Ile Leu
        2240                2245                2250

Asp Ser Phe Glu Pro Leu Arg Ala Glu Glu Asp Asp Arg Glu Val
        2255                2260                2265

Ser Val Ala Ala Glu Ile Leu Arg Arg Thr Arg Lys Phe Pro Ala
        2270                2275                2280

Ala Met Pro Ile Trp Ala Arg Pro Asp Tyr Asn Pro Pro Leu Leu
        2285                2290                2295

Glu Ser Trp Lys Asn Pro Asp Tyr Val Pro Pro Val Val His Gly
        2300                2305                2310

Cys Pro Leu Pro Pro Ile Lys Ala Pro Pro Ile Pro Pro Pro Arg
        2315                2320                2325

Arg Lys Arg Thr Val Ile Leu Thr Glu Ser Thr Val Ser Ser Ala
        2330                2335                2340

Leu Ala Glu Leu Ala Thr Lys Thr Phe Gly Ser Ser Gly Ser Ser
        2345                2350                2355

Ala Val Asp Ser Gly Thr Ala Thr Gly Pro Pro Asp Gln Ser Ser
        2360                2365                2370

Gly Asp Gly Asp Thr Gly Ser Asp Ala Glu Ser Cys Ser Ser Met
        2375                2380                2385

Pro Pro Leu Glu Gly Glu Pro Gly Asp Pro Asp Leu Ser Asp Gly
        2390                2395                2400

Ser Trp Ser Thr Val Ser Glu Glu Ala Gly Glu Asp Val Val Cys
        2405                2410                2415

Cys Ser Met Ser Tyr Thr Trp Thr Gly Ala Leu Ile Thr Pro Cys
        2420                2425                2430

Ala Ala Glu Glu Ser Lys Leu Pro Ile Asn Ala Leu Ser Asn Ser
        2435                2440                2445

Leu Leu Arg His His Asn Met Val Tyr Ala Thr Thr Ser Arg Ser
        2450                2455                2460

Ala Gly Gln Arg Gln Lys Lys Val Thr Phe Asp Arg Val Gln Val
        2465                2470                2475

Leu Asp Asp His Tyr Arg Asp Val Leu Lys Glu Met Lys Ala Lys
        2480                2485                2490

Ala Ser Thr Val Lys Ala Lys Leu Leu Pro Val Glu Glu Ala Cys
        2495                2500                2505

Arg Leu Thr Pro Pro His Ser Ala Arg Ser Lys Phe Gly Tyr Gly
        2510                2515                2520

Ala Lys Asp Val Arg Asn Leu Ser Ser Lys Ala Val Asn His Ile
        2525                2530                2535

His Ser Val Trp Lys Asp Leu Leu Glu Asp Ser Glu Thr Pro Ile
        2540                2545                2550

Asp Thr Thr Ile Met Ala Lys Asn Glu Val Phe Cys Val Gln Pro
        2555                2560                2565

Glu Lys Gly Gly Arg Lys Ser Ala Arg Leu Ile Val Phe Pro Asp
        2570                2575                2580

Leu Gly Val Arg Val Cys Glu Lys Met Ala Leu Tyr Asp Val Val
        2585                2590                2595

Ser Thr Leu Pro Gln Ala Val Met Gly Ser Ser Tyr Gly Phe Gln
        2600                2605                2610

Tyr Ser Pro Gly Gln Arg Val Glu Phe Leu Val Asn Ala Trp Lys
        2615                2620                2625

Ser Lys Lys Asn Pro Met Gly Phe Ala Tyr Asp Thr Arg Cys Phe
        2630                2635                2640

Asp Ser Thr Val Thr Glu Ser Asp Ile Arg Val Glu Glu Ser Ile
        2645                2650                2655

Tyr Gln Cys Cys Asp Leu Ala Pro Glu Ala Arg Gln Val Ile Arg
        2660                2665                2670

Ser Leu Thr Glu Arg Leu Tyr Ile Gly Gly Pro Leu Thr Asn Ser
        2675                2680                2685

Lys Gly Gln Ser Cys Gly Tyr Arg Arg Cys Arg Ala Ser Gly Val
        2690                2695                2700

Leu Thr Thr Ser Cys Gly Asn Thr Leu Thr Cys Tyr Leu Lys Ala
        2705                2710                2715

Ser Ala Ala Cys Arg Ala Ala Lys Leu Gln Asp Cys Thr Met Leu
        2720                2725                2730

Val Cys Gly Asp Asp Leu Val Val Ile Cys Glu Ser Ala Gly Thr
        2735                2740                2745

Gln Glu Asp Ala Ala Ser Leu Arg Val Phe Thr Glu Ala Met Thr
        2750                2755                2760

Arg Tyr Ser Ala Pro Pro Gly Asp Pro Pro Gln Pro Glu Tyr Asp
        2765                2770                2775

Leu Glu Leu Ile Thr Ser Cys Ser Ser Asn Val Ser Val Ala His
        2780                2785                2790

Asp Ala Leu Gly Lys Arg Val Tyr Tyr Leu Thr Arg Asp Pro Thr
        2795                2800                2805

Thr Pro Leu Ala Arg Ala Ala Trp Glu Thr Ala Arg His Thr Pro
        2810                2815                2820

Val Asn Ser Trp Leu Gly Asn Ile Ile Met Tyr Ala Pro Thr Leu
        2825                2830                2835

Trp Ala Arg Met Ile Leu Met Thr His Phe Phe Ser Ile Leu Leu
        2840                2845                2850

Ala Gln Glu Gln Leu Glu Lys Ala Leu Asp Cys Gln Ile Tyr Gly
        2855                2860                2865

Ala Thr Tyr Ser Ile Glu Pro Leu Asp Leu Pro Gln Ile Ile Gln
        2870                2875                2880

Arg Leu His Gly Leu Ser Ala Phe Ser Leu His Ser Tyr Ser Pro
        2885                2890                2895

Gly Glu Ile Asn Arg Val Ala Ser Cys Leu Arg Lys Leu Gly Val
        2900                2905                2910

Pro Pro Leu Arg Val Trp Arg His Arg Ala Arg Ser Val Arg Ala
        2915                2920                2925

Lys Leu Leu Ser Gln Gly Gly Arg Ala Ala Thr Cys Gly Lys Tyr
        2930                2935                2940

Leu Phe Asn Trp Ala Val Arg Thr Lys Leu Lys Leu Thr Pro Ile
        2945                2950                2955

Pro Ala Ala Ser Gln Leu Asp Leu Ser Gly Trp Phe Ile Ala Gly

2960                    2965                    2970

Tyr Ser Gly Gly Asp Ile Tyr His Ser Leu Ser Arg Ala Arg Pro
    2975                    2980                    2985

Arg Trp Phe Met Trp Cys Leu Leu Leu Leu Ser Val Gly Val Gly
    2990                    2995                    3000

Ile Tyr Leu Leu Pro Asn Arg
    3005                    3010

<210>  12
<211>  30
<212>  DNA
<213>  Artificial

<220>
<223>  primer for HCV

<400>  12
tcatgcggct cacggacctt tcacagctag                                        30

<210>  13
<211>  30
<212>  DNA
<213>  Artificial

<220>
<223>  primer for HCV

<400>  13
atcgtcttca cgcagaaagc gtctagccat                                        30

<210>  14
<211>  26
<212>  DNA
<213>  Artificial

<220>
<223>  primer for HCV

<400>  14
gcctattggc ctggagtgtt tagctc                                            26

<210>  15
<211>  29
<212>  DNA
<213>  Artificial

<220>
<223>  primer for HCV

<400>  15
atggcgttag tatgagtgtc gtgcagcct                                         29

<210>  16
<211>  31
<212>  DNA
<213>  Artificial

<220>

<223>  primer for HCV

<400>  16
tcgggcacga gacaggctgt gatatatgtc t                                    31


<210>  17
<211>  19
<212>  DNA
<213>  Artificial

<220>
<223>  primer for HCV

<400>  17
tgcacggtct acgagacct                                                  19


<210>  18
<211>  24
<212>  DNA
<213>  Artificial

<220>
<223>  primer for HCV

<400>  18
ctcgcaagca ccctatcagc cagt                                            24


<210>  19
<211>  19
<212>  DNA
<213>  Artificial

<220>
<223>  primer for HCV

<400>  19
aggcattgag cgggtttat                                                  19


<210>  20
<211>  38
<212>  DNA
<213>  Artificial

<220>
<223>  primer for RACE

<400>  20
ctagactcga gtcgacatcg tttttttttt tttttttt                             38


<210>  21
<211>  20
<212>  DNA
<213>  Artificial

<220>
<223>  primer for HCV

<400>  21
atcttagccc tagtcacggc                                                 20


<210>  22
<211>  20
<212>  DNA

<213>  Artificial

<220>
<223>  primer for RACE

<400>  22
ctagactcga gtcgacatcg                                                    20


<210>  23
<211>  29
<212>  DNA
<213>  Artificial

<220>
<223>  primer for HCV

<400>  23
ctagctgtaa aggtccgtga gccgcatga                                          29


<210>  24
<211>  17
<212>  DNA
<213>  Artificial

<220>
<223>  primer

<400>  24
gtaaaacgac ggccagt                                                       17


<210>  25
<211>  17
<212>  DNA
<213>  Artificial

<220>
<223>  primer

<400>  25
caggaaacag ctatgac                                                       17


<210>  26
<211>  20
<212>  DNA
<213>  Artificial

<220>
<223>  primer for HCV

<400>  26
aggaagactt ccgagcggtc                                                    20


<210>  27
<211>  32
<212>  DNA
<213>  Artificial

<220>
<223>  primer for HCV

<400>  27
ggaacttgcc cggttgctct ttctctatct tc                                      32

<210>  28
<211>  25
<212>  DNA
<213>  Artificial

<220>
<223>  primer for HCV

<400>  28
attccatggt ggggaactgg gctaa                                          25


<210>  29
<211>  30
<212>  DNA
<213>  Artificial

<220>
<223>  primer for HCV

<400>  29
taacaatacc ttgacctgcc ccacggactg                                     30


<210>  30
<211>  29
<212>  DNA
<213>  Artificial

<220>
<223>  primer for HCV

<400>  30
aacatcgtgg acgtgcaata cctgtacgg                                      29


<210>  31
<211>  20
<212>  DNA
<213>  Artificial

<220>
<223>  primer for HCV

<400>  31
gaccctacac cgtacaggta                                                20


<210>  32
<211>  26
<212>  DNA
<213>  Artificial

<220>
<223>  primer for HCV

<400>  32
ttggaccggg agatggctgc atcgtg                                         26


<210>  33
<211>  21
<212>  DNA
<213>  Artificial

<220>
<223>  primer for HCV

<400>  33

cacccaaatg tacaccaatg t                                                21

<210>  34
<211>  22
<212>  DNA
<213>  Artificial

<220>
<223>  primer for HCV

<400>  34
tacccgttga gtctatggaa ac                                               22

<210>  35
<211>  20
<212>  DNA
<213>  Artificial

<220>
<223>  primer for HCV

<400>  35
cacttggaat gtctgcggta                                                  20

<210>  36
<211>  25
<212>  DNA
<213>  Artificial

<220>
<223>  primer for HCV

<400>  36
agggggggag gcatctcatt ttctg                                            25

<210>  37
<211>  26
<212>  DNA
<213>  Artificial

<220>
<223>  primer for HCV

<400>  37
tgctatgacg cgggctgtgc ttggta                                           26

<210>  38
<211>  21
<212>  DNA
<213>  Artificial

<220>
<223>  primer for HCV

<400>  38
ggtcattgtg ggcaggatca t                                                21

<210>  39
<211>  20
<212>  DNA
<213>  Artificial

<220>

<223>  primer for HCV

<400>  39
ctgcctggaa accccgcgat                                                    20


<210>  40
<211>  24
<212>  DNA
<213>  Artificial

<220>
<223>  primer for HCV

<400>  40
tggcagcata ggccttggga aggt                                               24


<210>  41
<211>  21
<212>  DNA
<213>  Artificial

<220>
<223>  primer for HCV

<400>  41
aagacctggc tccagtccaa g                                                  21


<210>  42
<211>  21
<212>  DNA
<213>  Artificial

<220>
<223>  primer for HCV

<400>  42
ttccatgctc accgacccct c                                                  21


<210>  43
<211>  20
<212>  DNA
<213>  Artificial

<220>
<223>  primer for HCV

<400>  43
accttattct ctgactccac                                                    20


<210>  44
<211>  21
<212>  DNA
<213>  Artificial

<220>
<223>  primer for HCV

<400>  44
cagaagaagg tcacctttga c                                                  21


<210>  45
<211>  25
<212>  DNA

<213>  Artificial

<220>
<223>  primer for HCV

<400>  45
gcagcgggtc gagttcctgg tgaat                                          25


<210>  46
<211>  26
<212>  DNA
<213>  Artificial

<220>
<223>  primer for HCV

<400>  46
ctacggggcc tgttactcca ttgaac                                         26


<210>  47
<211>  26
<212>  DNA
<213>  Artificial

<220>
<223>  primer for HCV

<400>  47
cttgaaaaag ccctggattg tcagat                                         26


<210>  48
<211>  35
<212>  DNA
<213>  Artificial

<220>
<223>  primer for HCV

<400>  48
acatgatctg cagagaggcc agtatcagca ctctc                               35


<210>  49
<211>  31
<212>  DNA
<213>  Artificial

<220>
<223>  primer for HCV

<400>  49
gccagccccc tgatgggggc gacactccac c                                   31


<210>  50
<211>  25
<212>  DNA
<213>  Artificial

<220>
<223>  primer for HCV

<400>  50
agccgcatgt aagggtatcg atgac                                          25

<210> 51
<211> 20
<212> DNA
<213> Artificial

<220>
<223> primer for HCV

<400> 51
acatgatctg cagagaggcc 20

<210> 52
<211> 30
<212> DNA
<213> Artificial

<220>
<223> primer for HCV

<400> 52
gagtacaccg gaattgccag gacgaccggg 30

<210> 53
<211> 25
<212> DNA
<213> Artificial

<220>
<223> primer for HCV

<400> 53
attccatggt ggggaactgg gccaa 25

<210> 54
<211> 24
<212> DNA
<213> Artificial

<220>
<223> primer for HCV

<400> 54
catccatgtg cagccgaacc aatt 24

<210> 55
<211> 30
<212> DNA
<213> Artificial

<220>
<223> primer for HCV

<400> 55
aggggtagtg ccaaagcctg tatgggtagt 30

<210> 56
<211> 31
<212> DNA
<213> Artificial

<220>
<223> primer for HCV

<400> 56

at aat gaccc ccggcgact t t ccgcact aa c 31

<210> 57
<211> 20
<212> DNA
<213> Art i f i ci al

<220>
<223> pri mer f or HCV

<400> 57
cat ggt agac agt ccagcac 20

<210> 58
<211> 24
<212> DNA
<213> Art i f i ci al

<220>
<223> pri mer f or HCV

<400> 58
gacat gcat g t cat gat gt a t t t g 24

<210> 59
<211> 20
<212> DNA
<213> Art i f i ci al

<220>
<223> pri mer f or HCV

<400> 59
ct cat gacct t aaaggccac 20

<210> 60
<211> 30
<212> DNA
<213> Art i f i ci al

<220>
<223> pri mer f or ECMV I RES

<400> 60
t gcacat gct ct acat gt gt t t agt cgagg 30

<210> 61
<211> 9594
<212> RNA
<213> Hepat i t i s C vi r us

<400> 61
gccagccccc ugaugggggc gacacuccac cauagaucac uccccuguga ggaacuacug 60

ucuucacgca gaaagcgucu agccauggcg uuaguaugag ugucgugcag ccuccaggac 120

cccccucccc gggagagcca uaguggucug cggaaccggu gaguacaccg gaauugccag 180

gacgaccggg uccuuucuug gaucaacccg cucaaugccu ggagauuugg gcgugccccc 240

gcgagacugc uagccgagua guguuggguc gcgaaaggcc uugugguacu gccugauagg 300

gugcuugcga gugccccggg aggucucgua gaccgugcau caugagcaca aauccuaaac 360

cucaaagaaa aaccaaacgu aacaccaacc gccgcccaca ggacgucaag uucccgggcg 420
guggucagau cguuggugga guuuaccugu ugccgcgcag gggccccagg uugggugugc 480
gcgcgacuag gaagacuucc gagcggucgc aaccucguga aaggcggcaa ccuaucccca 540
aggcucgccg ccccgagggc agggccuggg cucagcccgg guacccuugg ccccucuaug 600
gcaaugaggg ucuggggugg gcaggguggc uccugucacc ccgcggcucc cggccuaauu 660
ggggccccac ggacccccgg cguaggucgc guaauuuggg uaaggucauc gauacccuca 720
caugcggcuu cgccgaccuc augggguaca uuccgcucgu cggcgccccc cuaggaggcg 780
uugccagggc ccuggcgcau ggcguccggg uucuggagga cggcgugaac uaugcaacag 840
ggaauuugcc cgguugcucu uucucuaucu uccucuuggc uuugcugucc uguuugacug 900
ucccagcuuc cgcuuaugaa gugcgcaacg uguccgggac auaccauguc acgaacgacu 960
gcgccaacuc aagcauugug uaugaggcag cggacauaau caugcacacc ccugggugcg 1020
ugcccugcgu ccgggagggc aauuccuccc gcugcugggu agcgcucacu cccacgcucg 1080
cggccaggaa caggagcauc cccacuacga cgauacgacg ccacgucgau uugcucguug 1140
gggcggcugc cuucugcucc gccauguacg ugggggaucu cugcgggucu guuuuccuag 1200
ucucccaacu guucaccuuc ucaccucgcc gguaugagac gguacaagaa ugcaauugcu 1260
caaucuaucc cggccacgua ucaggucacc gcauggcuug ggauaugaug augaauuggu 1320
cgcccacagc agcucuagug gugucacagu uacuccgaau cccacaagcc gucguggaua 1380
ugguggcagg ggcccacugg gggguucugg cgggucuugc cuacuauucc auggugggga 1440
acugggcuaa agucuugauu gugaugcuac ucuuugccgg cguugacggg accaccaccg 1500
ugacaggggg agcggcggcc uuuggcacca ggagccuugc guccuucuuu acauuagggc 1560
cgucucagaa aauccaacuu guaaacacca acggcaguug gcacaucaau aggacugccu 1620
ugaauuguaa ugacucccuc cagacugggu uccuugcugc gcuguucuau gcacacaaga 1680
ucaacacguc uggaugccca gagcgcaugg ccagcugccg cccuauugac aaguucgcuc 1740
aggggugggg ucccaucacc cauggugcgc cugacaccuc ggaccagagg cccuacugcu 1800
ggcacuacgc accucgaccg ugugguaucg uacccgcguc ggaggugugu ggcccagugu 1860
auuguuucac cccaagcccu guaguggugg ggacgaccga ucgcuucggc guccccacgu 1920
auagcugggg ggagaauaag acggacgugc ugcuccucaa caacacgcgg ccgccgcagg 1980
gcaauugguu cggcugcaca uggaugaaug gcaccggguu caccaagacg ugugggggcc 2040
ccccguguga uaucgggggg gucggcaaca acaccuugac cugccccacg gauugcuucc 2100
ggaagcaccc cgaggccacu uauaccaaau gcggcucggg gccuugguug acgccuaggu 2160
gucuaguuga cuacccauac aggcuuuggc acuaccccug cacugucaac uuuaccaucu 2220
ucaaggucag gauguaugug gggggcgugg agcacaggcu caacgcugcg ugcaacugga 2280
cucgaggaga gcgcugugac cuagaggaca gggacagauc agagcuuagc ccgcugcuac 2340
ugucuacaac agaguggcag auacugcccu guuccuucac cacccuaccg gcucugucca 2400
cuggcuugau ccaccuccau cagaacaucg uggacgugca auaccuguac gguguagggu 2460

cagcaguugu cuccguugug aucagauggg aguacguccu gcugcucuuc cuccuccugg 2520
cggacgcgcg cguuugugcc ugcuuaugga ugaugcuguu gauagcccag gcugaagccg 2580
ccuuggagaa ccugguaguc cucaacgcgg cguccguggc cggagcgcau ggcauccucu 2640
cuuuccuugu guucuucugc gcugccuggu acaucaaagg caagcuagug ccuggggugg 2700
cguaugcucu cuauggugua uggccgcugc uccugcuccu gcuggcguug ccacaacggg 2760
cguacgccau ggaccgggag auggcugcau cgugcggagg cgcgguuuuc gugggucugg 2820
uacucuugac cuugucacca cacuauaaag cauuccuugc caaguccaua uggugguuac 2880
aauauuuaau caccagggcc gaggcgcauu ugcaagugug gguccccccc cucaacguuc 2940
gggggggccg cgaugccauc auccuccuca cgugcgcggu ccacccagag cuaauuuuug 3000
acaucaccaa aauuuugcuc gccauauuug gcccgcucau ggugcuccag gcuggucuaa 3060
cuagagugcc guacuuugug cgcgcucaag ggcucauccg ugugugcaug uuggugcgga 3120
aagucgcugg gggucauuau auccaaaugg cucucaugag guuggccgca cugacgggca 3180
cguacguuua ugaucaucuu acuccgcugc gggacugggc ccaugcgggc uugcgagacc 3240
uugcgguggc aguugagccc gucguuuucu ccaacacaga gaccaagauu aucaccuggg 3300
gggcggacac cgcggcgugu ggggacauca ucuugggucu accugucucc gcccgaaggg 3360
ggagagaggu acuccuagga ccggccgaua acuuuggaga gcaagggugg cgacuccuug 3420
cgcccaucac ggccuacucc caacagacgc ggggcuuacu uggcuguauc aucaccggcc 3480
ucacaggucg agacaagaac caggucgaag gggagguuca ggugguuucc accgcaacac 3540
agucuuucuu ggcaaccugc gucaacggug uguguuggac ugucuaccau ggugccggcu 3600
caaagacccu agccggcccg aaggggccaa ucacccagau guacaccaac guagaccaag 3660
accucguugg cuggcaggcg cccccggggg cgcguuccau gacgccgugc accugcggca 3720
gcucggaccu uuacuugguc acgaggcaug cugaugucau uccggugcgc cggcggggug 3780
acagcagagg gagccuacuu ucccccaggc ccgucuccua cuugaagggc ucuucaggug 3840
guccacugcu cugccccuug gggcacgucg ugggcaucuu ucgggcugcc gugugcaccc 3900
gggggguugc gaaggcggug gacuucauac ccaucgaguc uauggaaacu accaugcggu 3960
cuccggucuu uacggauaau ucaucucccc cggccguacc gcagacuuuu caaguggccc 4020
aucugcacgc ccccacuggc agcggcaaga gcaccaaggu gccagcugcg uaugcagccc 4080
agggguacaa ggugcuuguc uugaauccau ccguugccgc caccuugggu uuuggggcgu 4140
auauguccaa ggcauacggu gucgacccua acauuagaac ugggguaagg accaucacca 4200
caggcgcucc caucacguac uccaccuacg gcaaguuccu ugccgacggu gguugcuccg 4260
ggggcgcuua cgacaucaua auaugcgaug agugccacuc aaccgacucg acuuccauuu 4320
ugggcauugg cacgguccug gaucaagcgg agacggcugg agcgcgacuc gucgugcucg 4380
ccaccgcuac gccuccggga ucggucacug ugccacaccc caacaucgag gagguggccu 4440
uguccaacac cggagagauu cccuucuaug gcaaagccau cccccucgag gucaucaagg 4500

gggggaggca ucucauuuuc ugucauucua agaagaagug ugaugagcuc gcugcaaagc 4560
ugucgggccu uggggucaac gcuguagcgu acuaccgggg ucuugaugug uccgucauac 4620
caacaagcgg ggacgucguu gucguggcaa cagacgcucu aaugacgggc uacaccggug 4680
acuuugacuc ugugaucgac uguaauacau gugucaccca gacagucgac uucagccugg 4740
accccaccuu caccauugag acgacgaccg ugccccaaga cgcagugucg cgcucgcagc 4800
ggcgagggag gacugguagg gguagagggg gcauauacag guuugugacu ccaggagagc 4860
ggcccucggg cauguucgau uccucggucc ugugugaaug cuaugacgcg ggcugugcuu 4920
gguacgagcu cacgcccgcc gagaccucgg uuaggcugcg ggcuuaccua aauacaccag 4980
gguugcccgu cugccaggac cauuuggagu ucugggaagg cgucuucaca ggccucacuc 5040
auauagaugc ccacuucuug ucucagacua agcaggcagg agacaacuuc cccuaccugg 5100
uggcauacca ggccacagug ugcgccaggg cccaggcacc accuccauca ugggaucaaa 5160
uguggaagug ucucauacgg cuaaaaccua cacuacacgg gccaacaccc cuguuguaca 5220
ggcuaggagc cguccaaaac gaggucaccc ucacacaccc caugaccaaa uacaucauga 5280
caugcauguc ggcugaccua gaggucguca ccagcacuug ggugcuggug ggcggggucc 5340
ucgcagcuuu ggccgcguac ugcuugacaa cgggcagcgu gguuaucaua ggcaggauca 5400
ucuuguccgg aaggccggcu aucauucccg auagggaagu ucucuaccag gaguucgaug 5460
aaauggaaga gugcgccuca caccuucccu acaucgaaca aggaaugcag cucgccgagc 5520
aauucaagca gaaggcgcuc ggguuguugc aaacggccac caagcaagcg gaggccgcug 5580
cucccguggu ggaguccaaa uggcaagccc uugaggccuu cugggcgaag cacaugugga 5640
acuucaucag cgggauacag uaucuagcag gcuuguccac ucugccugga aaccccgcga 5700
uagcaucgcu gauggcauuu acagccucua ucacuagccc gcucaccacc cuacauaccc 5760
uucuauuuaa caucuugggg ggaugggugg ccgcccaacu cgcccccccc agcgcugcuu 5820
cagccuucgu aggcgccggc aucgccggcg cggcuguugg cagcauaggu cuugggaagg 5880
ugcucgugga cauccuagcg gguuauggag caggggugge aggcgcacuc guggccuuca 5940
aggucaugag cggcgaagug cccuccacug aggaccuggu caacuuacuc ccugccaucc 6000
ucuccccugg ugcccugguc gucggggucg ugugcgcagc gauacugcgu cggcaugugg 6060
gcccagggga gggggccgug caguggauga accggcugau agcguucgcu ucgcggggua 6120
accacgucuc ccccacgcac uaugugccug agagcgacgc cgcagcgcgu gucacccaga 6180
uccucuccag ccuuaccauc acucagcugc uaaagaggcu ccaccagugg auuaauaagg 6240
acuguuccac gccaugcucc gguucguggc ucagggaugu uugggacugg auaugcacgg 6300
uuuugaccga cuucaaaacc uggcuccagu ccaagcuccu gccacgguug ccgggacucc 6360
cuuucuuuuc augucaacgu ggauauaaag gagucuggcu gggagauggc guuaugcaaa 6420
cuaccugucc auguggugca caaaucagcg gacaugucaa aaacggcucc augaagaucg 6480
uggggccuaa aaccugcagc aacacguggc acgggacguu ccccaucaac gcauacacca 6540
caggccccug cacacccucc ccggcgccga acuauuccaa ggcguugugg cgaguggcug 6600

cugaggagua uguggagguc acgcgggugg gggauuucca cuacgugacg ggcaugacca 6660
cugacaacgu aaaaugccca ugccaggucc cggcccccga auucuucacg gaguuggaug 6720
gggugcggcu gcacagguac gcuccggcgu gcaagccucu cuuacgggau gaggucacau 6780
uccaggucgg gcucaaccag uauccgguug gaucacagcu uccaugugag cccgagccgg 6840
auguaacagu gcucacuucc augcucaccg accccuccca uauuacagca gagacggcua 6900
agcguaggcu ggccagaggg uccccccccu cuuuggccag cuccucagcu agccaguugu 6960
cugcgccuuc cuugaaggcg acaugcacua cccaccauga cuccccagau gcugaccuca 7020
ucgaggccaa ccuccugugg cggcaggaga ugggcgggaa caucacccgc guggagucag 7080
agaacaaggu aguaauucug gacucuuuug aaccgcuucg agcggaggag gaugauaggg 7140
aaguguccgu agcggcggag auccugcgga gaaccaggaa auuccccgca gcgaugccca 7200
uaugggcacg gccggacuac aacccaccac uccuagaguc uuggaagaac ccagacuacg 7260
ucccuccagu gguacacggg ugcccauuac caccuauuaa ggccccuccg auaccaccuc 7320
cacggagaaa gaggacgguc auccugacag aauccaccgu gucuucugcc uuggcggagc 7380
uugcuacaaa gaccuuuggc agcuccggau cgucggccgu ugacagcggc acagcgacug 7440
gcccuccuga ucaguccucc ggugacggag auacaggauc cgacgcugag ucgugcuccu 7500
ccaugccccc ccuugagggg gagccggggg accccgaucu cagcgacggg ucuuggucua 7560
ccgugagcga ggaggccggu gaggacgucg ucugcugcuc gauguccuac acauggacag 7620
gcgccuuaau cacaccgugc gccgcagagg agagcaagcu gccuaucaac gcguugagca 7680
acucuuugcu gcgccaccac aacauggucu augccacaac aucccgcagc gcgggccaac 7740
ggcagaagaa ggucacuuuu gacagagugc agguccugga cgaccauuac cgggacgugc 7800
ucaaggagau gaaggcgaag gcguccacag uuaaggcuaa acuucuaccu guagaagaag 7860
ccugcaggcu gacgccccca cacucggcca gauccaaguu uggcuauggg gcgaaggacg 7920
uccggaaccu auccagcaag gccgucaacc acauccacuc cguguggaag gacuugcugg 7980
aagacucuga gacgccgauu gacaccacca ucauggcaaa aaaugagguc uuuuguguuc 8040
aaccagagaa aggaggccgc aagucagcuc gucuuaucgu auucccagac uuggggguuc 8100
gugugugcga gaaaauggcc cuuuacgacg ugguuuccac ucuuccucag gccgugaugg 8160
gcuccucaua cggguuccag uacucuccug gacagcgggu cgaguuccug gugaaugccu 8220
ggaaaucaaa gaagaacccu augggcuucg cauaugacac ccgcuguuuu gacucaacgg 8280
ucacugagag ugacauccgu guugaggagu caauuuacca auguugugac uuggcccccg 8340
aggccagaca ggucauaagg ucgcucacgg agcggcuuua uaucgggggc ccccugacua 8400
auucaaaagg gcagagcugc ggcuaucgcc ggugccgcgc cagcggugug cugacgacca 8460
gcugcgguaa uacccucaca uguuacuuga aggcuucugc agccugucga gcugcaaagc 8520
uccaggacug cacaauguua gugugcggag acgaccuugu cguuaucugu gaaagugcgg 8580
gaacccagga ggacgcggcg agccuacgag ucuucacgga ggcuaugacu agguacucug 8640

ccccccccgg ggacccgccc cagccagaau acgacuugga gcugauaaca ucaugcuccu 8700
ccaacgucuc ggucgcgcac gaugcacuug gcaagcgggu guauuaucug acccgcgacc 8760
ccaccacccc ccuugcgcgg gcugcguggg agacagcaag acacacucca guuaacuccu 8820
ggcuaggcaa caucaucaug uaugcgccca cccuaugggc aaggaugauu cugaugaccc 8880
acuucuuuuc cauccuucua gcucaggaac aacuugaaaa agcccuagau ugucagaucu 8940
acggggccac uuacuccauu gagccacuug accuaccuca gaucauucag cgacuccacg 9000
gccuuagcgc auuuucacuc cauagcuacu cuccagguga gaucaauagg guggcuucau 9060
gccucaggaa acuuggggua ccacccuugc gagucuggag acaucgggcc agaaguccc 9120
gcgcuaagcu acugucccag ggggggaggg ccgccacuug uggcaaauac cucuucaacu 9180
gggcaguaag gaccaagcuc aaacucacuc caauuccggc ugcgucccag uuggacuugu 9240
ccggcugguu cauugcuggu uacagcgggg gagacauaua ucacagccug ucucgcgccc 9300
gaccccgcug guucaugugg ugccuacucc uacuuuccgu agggguaggc aucuaucugc 9360
uccccaaucg augaacgggg ggcuaaacac uccaggccaa uaggccauuc uguuuuuuuu 9420
uuuuuuuuuu uuuuuuuuuu uuuuuuuuuu uuuuuuuuuu uuuuuuccuu uuuuuuuuuu 9480
uuuuucccuu ucuuuuggug gcuccaucuu agcccuaguc acggcuagcu gugaaagguc 9540
cgugagccgc augacugcag agagugcuga uacuggccuc ucugcagauc augu 9594

<210> 62
<211> 3010
<212> PRT
<213> Hepatitis C virus

<400> 62

Met Ser Thr Asn Pro Lys Pro Gln Arg Lys Thr Lys Arg Asn Thr Asn
1               5                   10                  15

Arg Arg Pro Gln Asp Val Lys Phe Pro Gly Gly Gly Gln Ile Val Gly
            20                  25                  30

Gly Val Tyr Leu Leu Pro Arg Arg Gly Pro Arg Leu Gly Val Arg Ala
        35                  40                  45

Thr Arg Lys Thr Ser Glu Arg Ser Gln Pro Arg Glu Arg Arg Gln Pro
    50                  55                  60

Ile Pro Lys Ala Arg Arg Pro Glu Gly Arg Ala Trp Ala Gln Pro Gly
65                  70                  75                  80

Tyr Pro Trp Pro Leu Tyr Gly Asn Glu Gly Leu Gly Trp Ala Gly Trp
                85                  90                  95

Leu Leu Ser Pro Arg Gly Ser Arg Pro Asn Trp Gly Pro Thr Asp Pro
            100                 105                 110

Arg Arg Arg Ser Arg Asn Leu Gly Lys Val Ile Asp Thr Leu Thr Cys

        115                 120                 125

Gly Phe Ala Asp Leu Met Gly Tyr Ile Pro Leu Val Gly Ala Pro Leu
    130                 135                 140

Gly Gly Val Ala Arg Ala Leu Ala His Gly Val Arg Val Leu Glu Asp
145                 150                 155                 160

Gly Val Asn Tyr Ala Thr Gly Asn Leu Pro Gly Cys Ser Phe Ser Ile
                165                 170                 175

Phe Leu Leu Ala Leu Leu Ser Cys Leu Thr Val Pro Ala Ser Ala Tyr
            180                 185                 190

Glu Val Arg Asn Val Ser Gly Thr Tyr His Val Thr Asn Asp Cys Ala
        195                 200                 205

Asn Ser Ser Ile Val Tyr Glu Ala Ala Asp Ile Ile Met His Thr Pro
    210                 215                 220

Gly Cys Val Pro Cys Val Arg Glu Gly Asn Ser Ser Arg Cys Trp Val
225                 230                 235                 240

Ala Leu Thr Pro Thr Leu Ala Ala Arg Asn Arg Ser Ile Pro Thr Thr
                245                 250                 255

Thr Ile Arg Arg His Val Asp Leu Leu Val Gly Ala Ala Ala Phe Cys
            260                 265                 270

Ser Ala Met Tyr Val Gly Asp Leu Cys Gly Ser Val Phe Leu Val Ser
        275                 280                 285

Gln Leu Phe Thr Phe Ser Pro Arg Arg Tyr Glu Thr Val Gln Glu Cys
    290                 295                 300

Asn Cys Ser Ile Tyr Pro Gly His Val Ser Gly His Arg Met Ala Trp
305                 310                 315                 320

Asp Met Met Met Asn Trp Ser Pro Thr Ala Ala Leu Val Val Ser Gln
                325                 330                 335

Leu Leu Arg Ile Pro Gln Ala Val Val Asp Met Val Ala Gly Ala His
            340                 345                 350

Trp Gly Val Leu Ala Gly Leu Ala Tyr Tyr Ser Met Val Gly Asn Trp
        355                 360                 365

Ala Lys Val Leu Ile Val Met Leu Leu Phe Ala Gly Val Asp Gly Thr
    370                 375                 380

Thr Thr Val Thr Gly Gly Ala Ala Ala Phe Gly Thr Arg Ser Leu Ala
385                 390                 395                 400

Ser Phe Phe Thr Leu Gly Pro Ser Gln Lys Ile Gln Leu Val Asn Thr
                405                 410                 415

Asn Gly Ser Trp His Ile Asn Arg Thr Ala Leu Asn Cys Asn Asp Ser
            420                 425                 430

Leu Gln Thr Gly Phe Leu Ala Ala Leu Phe Tyr Ala His Lys Ile Asn
        435                 440                 445

Thr Ser Gly Cys Pro Glu Arg Met Ala Ser Cys Arg Pro Ile Asp Lys
    450                 455                 460

Phe Ala Gln Gly Trp Gly Pro Ile Thr His Gly Ala Pro Asp Thr Ser
465                 470                 475                 480

Asp Gln Arg Pro Tyr Cys Trp His Tyr Ala Pro Arg Pro Cys Gly Ile
                485                 490                 495

Val Pro Ala Ser Glu Val Cys Gly Pro Val Tyr Cys Phe Thr Pro Ser
            500                 505                 510

Pro Val Val Val Gly Thr Thr Asp Arg Phe Gly Val Pro Thr Tyr Ser
        515                 520                 525

Trp Gly Glu Asn Lys Thr Asp Val Leu Leu Leu Asn Asn Thr Arg Pro
    530                 535                 540

Pro Gln Gly Asn Trp Phe Gly Cys Thr Trp Met Asn Gly Thr Gly Phe
545                 550                 555                 560

Thr Lys Thr Cys Gly Gly Pro Pro Cys Asp Ile Gly Gly Val Gly Asn
                565                 570                 575

Asn Thr Leu Thr Cys Pro Thr Asp Cys Phe Arg Lys His Pro Glu Ala
            580                 585                 590

Thr Tyr Thr Lys Cys Gly Ser Gly Pro Trp Leu Thr Pro Arg Cys Leu
        595                 600                 605

Val Asp Tyr Pro Tyr Arg Leu Trp His Tyr Pro Cys Thr Val Asn Phe
    610                 615                 620

Thr Ile Phe Lys Val Arg Met Tyr Val Gly Gly Val Glu His Arg Leu
625                 630                 635                 640

Asn Ala Ala Cys Asn Trp Thr Arg Gly Glu Arg Cys Asp Leu Glu Asp
                645                 650                 655

Arg Asp Arg Ser Glu Leu Ser Pro Leu Leu Leu Ser Thr Thr Glu Trp
            660                 665                 670

Gln Ile Leu Pro Cys Ser Phe Thr Thr Leu Pro Ala Leu Ser Thr Gly
        675                 680                 685

Leu Ile His Leu His Gln Asn Ile Val Asp Val Gln Tyr Leu Tyr Gly
    690                 695                 700

Val Gly Ser Ala Val Val Ser Val Val Ile Arg Trp Glu Tyr Val Leu
705                 710                 715                 720

Leu Leu Phe Leu Leu Leu Ala Asp Ala Arg Val Cys Ala Cys Leu Trp
                725                 730                 735

Met Met Leu Leu Ile Ala Gln Ala Glu Ala Ala Leu Glu Asn Leu Val
            740                 745                 750

Val Leu Asn Ala Ala Ser Val Ala Gly Ala His Gly Ile Leu Ser Phe
        755                 760                 765

Leu Val Phe Phe Cys Ala Ala Trp Tyr Ile Lys Gly Lys Leu Val Pro
    770                 775                 780

Gly Val Ala Tyr Ala Leu Tyr Gly Val Trp Pro Leu Leu Leu Leu Leu
785                 790                 795                 800

Leu Ala Leu Pro Gln Arg Ala Tyr Ala Met Asp Arg Glu Met Ala Ala
                805                 810                 815

Ser Cys Gly Gly Ala Val Phe Val Gly Leu Val Leu Leu Thr Leu Ser
            820                 825                 830

Pro His Tyr Lys Ala Phe Leu Ala Lys Ser Ile Trp Trp Leu Gln Tyr
        835                 840                 845

Leu Ile Thr Arg Ala Glu Ala His Leu Gln Val Trp Val Pro Pro Leu
    850                 855                 860

Asn Val Arg Gly Gly Arg Asp Ala Ile Ile Leu Leu Thr Cys Ala Val
865                 870                 875                 880

His Pro Glu Leu Ile Phe Asp Ile Thr Lys Ile Leu Leu Ala Ile Phe
                885                 890                 895

Gly Pro Leu Met Val Leu Gln Ala Gly Leu Thr Arg Val Pro Tyr Phe
            900                 905                 910

Val Arg Ala Gln Gly Leu Ile Arg Val Cys Met Leu Val Arg Lys Val
        915                 920                 925

Ala Gly Gly His Tyr Ile Gln Met Ala Leu Met Arg Leu Ala Ala Leu
    930                 935                 940

Thr Gly Thr Tyr Val Tyr Asp His Leu Thr Pro Leu Arg Asp Trp Ala
945                 950                 955                 960

His Ala Gly Leu Arg Asp Leu Ala Val Ala Val Glu Pro Val Val Phe
                965                 970                 975

Ser Asn Thr Glu Thr Lys Ile Ile Thr Trp Gly Ala Asp Thr Ala Ala
            980                 985                 990

Cys Gly Asp Ile Ile Leu Gly Leu Pro Val Ser Ala Arg Arg Gly Arg
        995                 1000                1005

Glu Val Leu Leu Gly Pro Ala Asp Asn Phe Gly Glu Gln Gly Trp
    1010                1015                1020

Arg Leu Leu Ala Pro Ile Thr Ala Tyr Ser Gln Gln Thr Arg Gly
    1025                1030                1035

Leu Leu Gly Cys Ile Ile Thr Gly Leu Thr Gly Arg Asp Lys Asn
    1040                1045                1050

Gln Val Glu Gly Glu Val Gln Val Val Ser Thr Ala Thr Gln Ser
    1055                1060                1065

Phe Leu Ala Thr Cys Val Asn Gly Val Cys Trp Thr Val Tyr His
    1070                1075                1080

Gly Ala Gly Ser Lys Thr Leu Ala Gly Pro Lys Gly Pro Ile Thr
    1085                1090                1095

Gln Met Tyr Thr Asn Val Asp Gln Asp Leu Val Gly Trp Gln Ala
    1100                1105                1110

Pro Pro Gly Ala Arg Ser Met Thr Pro Cys Thr Cys Gly Ser Ser
    1115                1120                1125

Asp Leu Tyr Leu Val Thr Arg His Ala Asp Val Ile Pro Val Arg
    1130                1135                1140

Arg Arg Gly Asp Ser Arg Gly Ser Leu Leu Ser Pro Arg Pro Val
    1145                1150                1155

Ser Tyr Leu Lys Gly Ser Ser Gly Gly Pro Leu Leu Cys Pro Leu
    1160                1165                1170

Gly His Val Val Gly Ile Phe Arg Ala Ala Val Cys Thr Arg Gly
    1175                1180                1185

Val Ala Lys Ala Val Asp Phe Ile Pro Ile Glu Ser Met Glu Thr
    1190                1195                1200

Thr Met Arg Ser Pro Val Phe Thr Asp Asn Ser Ser Pro Pro Ala

1205                 1210                 1215

Val Pro Gln Thr Phe Gln Val Ala His Leu His Ala Pro Thr Gly
    1220                 1225                 1230

Ser Gly Lys Ser Thr Lys Val Pro Ala Ala Tyr Ala Ala Gln Gly
    1235                 1240                 1245

Tyr Lys Val Leu Val Leu Asn Pro Ser Val Ala Ala Thr Leu Gly
    1250                 1255                 1260

Phe Gly Ala Tyr Met Ser Lys Ala Tyr Gly Val Asp Pro Asn Ile
    1265                 1270                 1275

Arg Thr Gly Val Arg Thr Ile Thr Thr Gly Ala Pro Ile Thr Tyr
    1280                 1285                 1290

Ser Thr Tyr Gly Lys Phe Leu Ala Asp Gly Gly Cys Ser Gly Gly
    1295                 1300                 1305

Ala Tyr Asp Ile Ile Ile Cys Asp Glu Cys His Ser Thr Asp Ser
    1310                 1315                 1320

Thr Ser Ile Leu Gly Ile Gly Thr Val Leu Asp Gln Ala Glu Thr
    1325                 1330                 1335

Ala Gly Ala Arg Leu Val Val Leu Ala Thr Ala Thr Pro Pro Gly
    1340                 1345                 1350

Ser Val Thr Val Pro His Pro Asn Ile Glu Glu Val Ala Leu Ser
    1355                 1360                 1365

Asn Thr Gly Glu Ile Pro Phe Tyr Gly Lys Ala Ile Pro Leu Glu
    1370                 1375                 1380

Val Ile Lys Gly Gly Arg His Leu Ile Phe Cys His Ser Lys Lys
    1385                 1390                 1395

Lys Cys Asp Glu Leu Ala Ala Lys Leu Ser Gly Leu Gly Val Asn
    1400                 1405                 1410

Ala Val Ala Tyr Tyr Arg Gly Leu Asp Val Ser Val Ile Pro Thr
    1415                 1420                 1425

Ser Gly Asp Val Val Val Val Ala Thr Asp Ala Leu Met Thr Gly
    1430                 1435                 1440

Tyr Thr Gly Asp Phe Asp Ser Val Ile Asp Cys Asn Thr Cys Val
    1445                 1450                 1455

Thr Gln Thr Val Asp Phe Ser Leu Asp Pro Thr Phe Thr Ile Glu
    1460                 1465                 1470

Thr Thr Thr Val Pro Gln Asp Ala Val Ser Arg Ser Gln Arg Arg
    1475                1480                1485

Gly Arg Thr Gly Arg Gly Arg Gly Gly Ile Tyr Arg Phe Val Thr
    1490                1495                1500

Pro Gly Glu Arg Pro Ser Gly Met Phe Asp Ser Ser Val Leu Cys
    1505                1510                1515

Glu Cys Tyr Asp Ala Gly Cys Ala Trp Tyr Glu Leu Thr Pro Ala
    1520                1525                1530

Glu Thr Ser Val Arg Leu Arg Ala Tyr Leu Asn Thr Pro Gly Leu
    1535                1540                1545

Pro Val Cys Gln Asp His Leu Glu Phe Trp Glu Gly Val Phe Thr
    1550                1555                1560

Gly Leu Thr His Ile Asp Ala His Phe Leu Ser Gln Thr Lys Gln
    1565                1570                1575

Ala Gly Asp Asn Phe Pro Tyr Leu Val Ala Tyr Gln Ala Thr Val
    1580                1585                1590

Cys Ala Arg Ala Gln Ala Pro Pro Pro Ser Trp Asp Gln Met Trp
    1595                1600                1605

Lys Cys Leu Ile Arg Leu Lys Pro Thr Leu His Gly Pro Thr Pro
    1610                1615                1620

Leu Leu Tyr Arg Leu Gly Ala Val Gln Asn Glu Val Thr Leu Thr
    1625                1630                1635

His Pro Met Thr Lys Tyr Ile Met Thr Cys Met Ser Ala Asp Leu
    1640                1645                1650

Glu Val Val Thr Ser Thr Trp Val Leu Val Gly Gly Val Leu Ala
    1655                1660                1665

Ala Leu Ala Ala Tyr Cys Leu Thr Thr Gly Ser Val Val Ile Ile
    1670                1675                1680

Gly Arg Ile Ile Leu Ser Gly Arg Pro Ala Ile Ile Pro Asp Arg
    1685                1690                1695

Glu Val Leu Tyr Gln Glu Phe Asp Glu Met Glu Glu Cys Ala Ser
    1700                1705                1710

His Leu Pro Tyr Ile Glu Gln Gly Met Gln Leu Ala Glu Gln Phe
    1715                1720                1725

Lys Gln Lys Ala Leu Gly Leu Leu Gln Thr Ala Thr Lys Gln Ala
    1730                1735                1740

Glu Ala Ala Ala Pro Val Val Glu Ser Lys Trp Gln Ala Leu Glu
    1745                1750                1755

Ala Phe Trp Ala Lys His Met Trp Asn Phe Ile Ser Gly Ile Gln
    1760                1765                1770

Tyr Leu Ala Gly Leu Ser Thr Leu Pro Gly Asn Pro Ala Ile Ala
    1775                1780                1785

Ser Leu Met Ala Phe Thr Ala Ser Ile Thr Ser Pro Leu Thr Thr
    1790                1795                1800

Leu His Thr Leu Leu Phe Asn Ile Leu Gly Gly Trp Val Ala Ala
    1805                1810                1815

Gln Leu Ala Pro Pro Ser Ala Ala Ser Ala Phe Val Gly Ala Gly
    1820                1825                1830

Ile Ala Gly Ala Ala Val Gly Ser Ile Gly Leu Gly Lys Val Leu
    1835                1840                1845

Val Asp Ile Leu Ala Gly Tyr Gly Ala Gly Val Ala Gly Ala Leu
    1850                1855                1860

Val Ala Phe Lys Val Met Ser Gly Glu Val Pro Ser Thr Glu Asp
    1865                1870                1875

Leu Val Asn Leu Leu Pro Ala Ile Leu Ser Pro Gly Ala Leu Val
    1880                1885                1890

Val Gly Val Val Cys Ala Ala Ile Leu Arg Arg His Val Gly Pro
    1895                1900                1905

Gly Glu Gly Ala Val Gln Trp Met Asn Arg Leu Ile Ala Phe Ala
    1910                1915                1920

Ser Arg Gly Asn His Val Ser Pro Thr His Tyr Val Pro Glu Ser
    1925                1930                1935

Asp Ala Ala Ala Arg Val Thr Gln Ile Leu Ser Ser Leu Thr Ile
    1940                1945                1950

Thr Gln Leu Leu Lys Arg Leu His Gln Trp Ile Asn Lys Asp Cys
    1955                1960                1965

Ser Thr Pro Cys Ser Gly Ser Trp Leu Arg Asp Val Trp Asp Trp
    1970                1975                1980

Ile Cys Thr Val Leu Thr Asp Phe Lys Thr Trp Leu Gln Ser Lys
    1985                1990                1995

Leu Leu Pro Arg Leu Pro Gly Leu Pro Phe Phe Ser Cys Gln Arg
    2000                2005                2010

Gly Tyr Lys Gly Val Trp Leu Gly Asp Gly Val Met Gln Thr Thr
    2015                2020                2025

Cys Pro Cys Gly Ala Gln Ile Ser Gly His Val Lys Asn Gly Ser
    2030                2035                2040

Met Lys Ile Val Gly Pro Lys Thr Cys Ser Asn Thr Trp His Gly
    2045                2050                2055

Thr Phe Pro Ile Asn Ala Tyr Thr Thr Gly Pro Cys Thr Pro Ser
    2060                2065                2070

Pro Ala Pro Asn Tyr Ser Lys Ala Leu Trp Arg Val Ala Ala Glu
    2075                2080                2085

Glu Tyr Val Glu Val Thr Arg Val Gly Asp Phe His Tyr Val Thr
    2090                2095                2100

Gly Met Thr Thr Asp Asn Val Lys Cys Pro Cys Gln Val Pro Ala
    2105                2110                2115

Pro Glu Phe Phe Thr Glu Leu Asp Gly Val Arg Leu His Arg Tyr
    2120                2125                2130

Ala Pro Ala Cys Lys Pro Leu Leu Arg Asp Glu Val Thr Phe Gln
    2135                2140                2145

Val Gly Leu Asn Gln Tyr Pro Val Gly Ser Gln Leu Pro Cys Glu
    2150                2155                2160

Pro Glu Pro Asp Val Thr Val Leu Thr Ser Met Leu Thr Asp Pro
    2165                2170                2175

Ser His Ile Thr Ala Glu Thr Ala Lys Arg Arg Leu Ala Arg Gly
    2180                2185                2190

Ser Pro Pro Ser Leu Ala Ser Ser Ser Ala Ser Gln Leu Ser Ala
    2195                2200                2205

Pro Ser Leu Lys Ala Thr Cys Thr Thr His His Asp Ser Pro Asp
    2210                2215                2220

Ala Asp Leu Ile Glu Ala Asn Leu Leu Trp Arg Gln Glu Met Gly
    2225                2230                2235

Gly Asn Ile Thr Arg Val Glu Ser Glu Asn Lys Val Val Ile Leu

2240                2245                2250

Asp Ser Phe Glu Pro Leu Arg Ala Glu Glu Asp Asp Arg Glu Val
    2255                2260                2265

Ser Val Ala Ala Glu Ile Leu Arg Arg Thr Arg Lys Phe Pro Ala
    2270                2275                2280

Ala Met Pro Ile Trp Ala Arg Pro Asp Tyr Asn Pro Pro Leu Leu
    2285                2290                2295

Glu Ser Trp Lys Asn Pro Asp Tyr Val Pro Pro Val Val His Gly
    2300                2305                2310

Cys Pro Leu Pro Pro Ile Lys Ala Pro Pro Ile Pro Pro Pro Arg
    2315                2320                2325

Arg Lys Arg Thr Val Ile Leu Thr Glu Ser Thr Val Ser Ser Ala
    2330                2335                2340

Leu Ala Glu Leu Ala Thr Lys Thr Phe Gly Ser Ser Gly Ser Ser
    2345                2350                2355

Ala Val Asp Ser Gly Thr Ala Thr Gly Pro Pro Asp Gln Ser Ser
    2360                2365                2370

Gly Asp Gly Asp Thr Gly Ser Asp Ala Glu Ser Cys Ser Ser Met
    2375                2380                2385

Pro Pro Leu Glu Gly Glu Pro Gly Asp Pro Asp Leu Ser Asp Gly
    2390                2395                2400

Ser Trp Ser Thr Val Ser Glu Glu Ala Gly Glu Asp Val Val Cys
    2405                2410                2415

Cys Ser Met Ser Tyr Thr Trp Thr Gly Ala Leu Ile Thr Pro Cys
    2420                2425                2430

Ala Ala Glu Glu Ser Lys Leu Pro Ile Asn Ala Leu Ser Asn Ser
    2435                2440                2445

Leu Leu Arg His His Asn Met Val Tyr Ala Thr Thr Ser Arg Ser
    2450                2455                2460

Ala Gly Gln Arg Gln Lys Lys Val Thr Phe Asp Arg Val Gln Val
    2465                2470                2475

Leu Asp Asp His Tyr Arg Asp Val Leu Lys Glu Met Lys Ala Lys
    2480                2485                2490

Ala Ser Thr Val Lys Ala Lys Leu Leu Pro Val Glu Glu Ala Cys
    2495                2500                2505

Arg Leu Thr Pro Pro His Ser Ala Arg Ser Lys Phe Gly Tyr Gly
    2510                2515                2520

Ala Lys Asp Val Arg Asn Leu Ser Ser Lys Ala Val Asn His Ile
    2525                2530                2535

His Ser Val Trp Lys Asp Leu Leu Glu Asp Ser Glu Thr Pro Ile
    2540                2545                2550

Asp Thr Thr Ile Met Ala Lys Asn Glu Val Phe Cys Val Gln Pro
    2555                2560                2565

Glu Lys Gly Gly Arg Lys Ser Ala Arg Leu Ile Val Phe Pro Asp
    2570                2575                2580

Leu Gly Val Arg Val Cys Glu Lys Met Ala Leu Tyr Asp Val Val
    2585                2590                2595

Ser Thr Leu Pro Gln Ala Val Met Gly Ser Ser Tyr Gly Phe Gln
    2600                2605                2610

Tyr Ser Pro Gly Gln Arg Val Glu Phe Leu Val Asn Ala Trp Lys
    2615                2620                2625

Ser Lys Lys Asn Pro Met Gly Phe Ala Tyr Asp Thr Arg Cys Phe
    2630                2635                2640

Asp Ser Thr Val Thr Glu Ser Asp Ile Arg Val Glu Glu Ser Ile
    2645                2650                2655

Tyr Gln Cys Cys Asp Leu Ala Pro Glu Ala Arg Gln Val Ile Arg
    2660                2665                2670

Ser Leu Thr Glu Arg Leu Tyr Ile Gly Gly Pro Leu Thr Asn Ser
    2675                2680                2685

Lys Gly Gln Ser Cys Gly Tyr Arg Arg Cys Arg Ala Ser Gly Val
    2690                2695                2700

Leu Thr Thr Ser Cys Gly Asn Thr Leu Thr Cys Tyr Leu Lys Ala
    2705                2710                2715

Ser Ala Ala Cys Arg Ala Ala Lys Leu Gln Asp Cys Thr Met Leu
    2720                2725                2730

Val Cys Gly Asp Asp Leu Val Val Ile Cys Glu Ser Ala Gly Thr
    2735                2740                2745

Gln Glu Asp Ala Ala Ser Leu Arg Val Phe Thr Glu Ala Met Thr
    2750                2755                2760

Arg Tyr Ser Ala Pro Pro Gly Asp Pro Pro Gln Pro Glu Tyr Asp
    2765                2770                2775

Leu Glu Leu Ile Thr Ser Cys Ser Ser Asn Val Ser Val Ala His
    2780                2785                2790

Asp Ala Leu Gly Lys Arg Val Tyr Tyr Leu Thr Arg Asp Pro Thr
    2795                2800                2805

Thr Pro Leu Ala Arg Ala Ala Trp Glu Thr Ala Arg His Thr Pro
    2810                2815                2820

Val Asn Ser Trp Leu Gly Asn Ile Ile Met Tyr Ala Pro Thr Leu
    2825                2830                2835

Trp Ala Arg Met Ile Leu Met Thr His Phe Phe Ser Ile Leu Leu
    2840                2845                2850

Ala Gln Glu Gln Leu Glu Lys Ala Leu Asp Cys Gln Ile Tyr Gly
    2855                2860                2865

Ala Thr Tyr Ser Ile Glu Pro Leu Asp Leu Pro Gln Ile Ile Gln
    2870                2875                2880

Arg Leu His Gly Leu Ser Ala Phe Ser Leu His Ser Tyr Ser Pro
    2885                2890                2895

Gly Glu Ile Asn Arg Val Ala Ser Cys Leu Arg Lys Leu Gly Val
    2900                2905                2910

Pro Pro Leu Arg Val Trp Arg His Arg Ala Arg Ser Val Arg Ala
    2915                2920                2925

Lys Leu Leu Ser Gln Gly Gly Arg Ala Ala Thr Cys Gly Lys Tyr
    2930                2935                2940

Leu Phe Asn Trp Ala Val Arg Thr Lys Leu Lys Leu Thr Pro Ile
    2945                2950                2955

Pro Ala Ala Ser Gln Leu Asp Leu Ser Gly Trp Phe Ile Ala Gly
    2960                2965                2970

Tyr Ser Gly Gly Asp Ile Tyr His Ser Leu Ser Arg Ala Arg Pro
    2975                2980                2985

Arg Trp Phe Met Trp Cys Leu Leu Leu Leu Ser Val Gly Val Gly
    2990                2995                3000

Ile Tyr Leu Leu Pro Asn Arg
    3005                3010

<210>  63
<211>  9594
<212>  RNA
<213>  Hepatitis C virus

<400>  63
gccagccccc ugaugggggc gacacuccac cauagaucac uccccuguga ggaacuacug      60

ucuucacgca gaaagcgucu agccauggcg uuaguaugag ugucgugcag ccuccaggac     120

ccccccuccc gggagagcca uaguggucug cggaaccggu gaguacaccg gaauugccag     180

gacgaccggg uccuuucuug gaucaacccg cucaaugccu ggagauuugg gcgugccccc     240

gcgagacugc uagccgagua guguuggguc gcgaaaggcc uugugguacu gccugauagg     300

gugcuugcga gugccccggg aggucucgua gaccgugcau caugagcaca aauccuaaac     360

cucaaagaaa aaccaaacgu aacaccaacc gccgcccaca ggacgucaag uucccgggcg     420

guggucagau cguuggugga guuuaccugu ugccgcgcag gggccccagg uugggugugc     480

gcgcgacuag gaagacuucc gagcggucgc aaccucguga aaggcggcaa ccuaucccca     540

aggcucgccg ccccgagggc agggccuggg cucagcccgg guacccuugg ccccucuaug     600

gcaaugaggg ucuggggugg gcaggguggc uccugucacc ccgcggcucc cggccuaauu     660

ggggccccac ggacccccgg cguaggucgc guaauuuggg uaaggucauc gauacccuca     720

caugcggcuu cgccgaccuc augggguaca uuccgcucgu cggcgccccc cuaggaggcg     780

uugccagggc ccuggcgcau ggcguccggg uucuggagga cggcgugaac uaugcaacag     840

ggaauuugcc cgguugcucu uucucuaucu uccucuuggc uuugcugucc uguuugacug     900

ucccagcuuc cgcuuaugaa gugcgcaacg uguccgggac auaccauguc acgaacgacu     960

gcgccaacuc aagcauugug uaugaggcag cggacauaau caugcacacc ccugggugcg    1020

ugcccugcgu ccgggagggc aauuccuccc gcugcugggu agcgcucacu cccacgcucg    1080

cggccaggaa caggagcauc cccacuacga cgauacgacg ccacgucgau uugcucguug    1140

gggcggcugc cuucugcucc gccauguacg ugggggaucu cugcgggucu guuuuccuag    1200

ucucccaacu guucaccuuc ucaccucgcc gguaugagac gguacaagaa ugcaauugcu    1260

caaucuaucc cggccacgua ucaggucacc gcauggcuug ggauaugaug augaauuggu    1320

cgcccacagc agcucuagug gugucacagu uacuccgaau cccacaagcc gucguggaua    1380

ugguggcagg ggcccacugg gggguucugg cgggucuugc cuacuauucc auggugggga    1440

acugggcuaa agucuugauu gugaugcuac ucuuugccgg cguugacggg accaccaccg    1500

ugacaggggg agcggcggcc uuuggcacca ggagccuugc guccuucuuu acauuagggc    1560

cgucucagaa aauccaacuu guaaacacca acggcaguug gcacaucaau aggacugccu    1620

ugaauuguaa ugacucccuc cagacugggu uccuugcugc gcuguucuau gcacacaaga    1680

ucaacacguc uggaugccca gagcgcaugg ccagcugccg cccuauugac aaguucgcuc    1740

aggggugggg ucccaucacc cauggugcgc cugacaccuc ggaccagagg cccuacugcu    1800

ggcacuacgc accucgaccg ugugguaucg uacccgcguc ggaggugugu ggcccagugu    1860

auuguuucac cccaagcccu guaguggugg ggacgaccga ucgcuucggc guccccacgu    1920

auagcugggg ggagaauaag acggacgugc ugcuccucaa caacacgcgg ccgccgcagg 1980
gcaauugguu cggcugcaca uggaugaaug gcaccggguu caccaagacg ugugggggcc 2040
ccccguguga uaucgggggg gucggcaaca acaccuugac cugccccacg gauugcuucc 2100
ggaagcaccc cgaggccacu uauaccaaau gcggcucggg gccuugguug acgccuaggu 2160
gucuaguuga cuacccauac aggcuuuggc acuaccccug cacugucaac uuuaccaucu 2220
ucaaggucag gauguaugug gggggcgugg agcacaggcu caacgcugcg ugcaacugga 2280
cucgaggaga gcgcugugac cuagaggaca gggacagauc agagcuuagc ccgcugcuac 2340
ugucuacaac agaguggcag auacugcccu guuccuucac cacccuaccg gcucugucca 2400
cuggcuugau ccaccuccau cagaacaucg uggacgugca auaccuguac gguguagggu 2460
cagcaguugu cuccguugug aucagauggg aguacguccu gcugcucuuc cuccuccugg 2520
cggacgcgcg cguuugugcc ugcuuaugga ugaugcuguu gauagcccag gcugaagccg 2580
ccuuggagaa ccugguaguc cucaacgcgg cguccguggc cggagcgcau ggcauccucu 2640
cuuuccuugu guucuucugc gcugccuggu acaucaaagg caagcuagug ccuggggugg 2700
cguaugcucu cuauggugua uggccgcugc uccugcuccu gcuggcguug ccacaacggg 2760
cguacgccau ggaccgggag auggcugcau cgugcggagg cgcgguuuuc gugggucugg 2820
uacucuugac cuugucacca cacuauaaag cauuccuugc caaguccaua uggugguuac 2880
aauauuuaau caccagggcc gaggcgcauu ugcaagugug gguccccccc cucaacguuc 2940
gggggggccg cgaugccauc auccuccuca cgugcgcggu ccacccagag cuaauuuuug 3000
acaucaccaa aauuuugcuc gccauauuug gcccgcucau ggugcuccag gcuggucuaa 3060
cuagagugcc guacuuugug cgcgcucaag ggcucauccg ugugugcaug uuggugcgga 3120
aagucgcugg gggucauuau auccaaaugg cucucaugag guuggccgca cugacgggca 3180
cguacguuua ugaucaucuu acuccgcugc gggacugggc ccaugcgggc uugcgagacc 3240
uugcgguggc aguugagccc gucguuuucu ccaacacaga gaccaagauu aucaccuggg 3300
gggcggacac cgcggcgugu ggggacauca ucuugggucu accugucucc gcccgaaggg 3360
ggagagaggu acuccuagga ccggccgaua acuuuggaga gcaagggugg cgacuccuug 3420
cgcccaucac ggccuacucc caacagacgc ggggcuuacu uggcuguauc aucaccggcc 3480
ucacaggucg agacaagaac caggucgaag gggagguuca ggugguuucc accgcaacac 3540
agucuuucuu ggcaaccugc gucaacggug uguguuggac ugucuaccau ggugccggcu 3600
caaagacccu agccggcccg aaggggccaa ucacccagau guacaccaac guagaccaag 3660
accucguugg cuggcaggcg ccccucgggg cgcguuccau gacgccgugc accugcggca 3720
gcucggaccu uuacuugguc acgaggcaug cugaugucau uccggugcgc cggcggggug 3780
acagcagagg gagccuacuu ucccccaggc ccgucuccua cuugaagggc ucuucaggug 3840
guccacugcu cugccccuug gggcacgucg ugggcaucuu ucgggcugcc gugugcaccc 3900
gggggguugc gaaggcggug gacuucauac ccaucgaguc uauggaaacu accaugcggu 3960

cuccggucuu uacggauaau ucaucucccc cggccguacc gcagacuuuu caaguggccc 4020
aucugcacgc ccccacuggc agcggcaaga gcaccaaggu gccagcugcg uaugcagccc 4080
agggguacaa ggugcuuguc uugaauccau ccguugccgc caccuugggu uuuggggcgu 4140
auauguccaa ggcauacggu gucgacccua acauuagaac ugggguaagg accaucacca 4200
caggcgcucc caucacguac uccaccuacg gcaaguuccu ugccgacggu gguugcuccg 4260
ggggcgcuua cgacaucaua auaugcgaug agugccacuc aaccgacucg acuuccauuu 4320
ugggcauugg cacgguccug gaucaagcgg agacggcugg agcgcgacuc gucgugcucg 4380
ccaccgcuac gccuccggga ucggucacug ugccacaccc caacaucgag gagguggccu 4440
uguccaacac cggagagauu cccuucuaug gcaaagccau cccccucgag gucaucaagg 4500
gggggaggca ucucauuuuc ugucauucua agaagaagug ugaugagcuc gcugcaaagc 4560
ugucgggccu uggggucaac gcuguagcgu acuaccgggg ucuugaugug uccgucauac 4620
caacaagcgg ggacgucguu gucguggcaa cagacgcucu aaugacgggc uacaccggug 4680
acuuugacuc ugugaucgac uguaauacau gugucaccca gacagucgac uucagccugg 4740
accccaccuu caccauugag acgacgaccg ugccccaaga cgcagugucg cgcucgcagc 4800
ggcgagggag gacugguagg gguagagggg gcauauacag guuugugacu ccaggagagc 4860
ggcccucggg cauguucgau uccucggucc ugugugaaug cuaugacgcg ggcugugcuu 4920
gguacgagcu cacgcccgcc gagaccucgg uuaggcugcg ggcuuaccua aauacaccag 4980
gguugcccgu cugccaggac cauuuggagu ucugggaagg cgucuucaca ggccucacuc 5040
auauagaugc ccacuucuug ucucagacua agcaggcagg agacaacuuc cccuaccugg 5100
uggcauacca ggccacagug ugcgccaggg cccaggcacc accuccauca ugggaucaaa 5160
uguggaagug ucucauacgg cuaaaaccua cacuacacgg gccaacaccc cuguuguaca 5220
ggcuaggagc cguccaaaac gaggucaccc ucacacaccc caugaccaaa uacaucauga 5280
caugcauguc ggcugaccua gaggucguca ccagcacuug ggugcuggug ggcggggucc 5340
ucgcagcuuu ggccgcguac ugcuugacaa cgggcagcgu gguuaucaua ggcaggauca 5400
ucuuguccgg aaggccggcu aucauucccg auagggaagu ucucuaccag gaguucgaug 5460
aaauggaaga gugcgccuca caccuucccu acaucgaaca aggaaugcag cucgccgagc 5520
aauucaagca gaaggcgcuc ggguuguugc aaacggccac caagcaagcg gaggccgcug 5580
cucccguggu ggaguccaaa uggcaagccc uugaggccuu cugggcgaag cacaugugga 5640
acuucaucag cgggauacag uaucuagcag gcuuguccac ucugccugga aaccccgcga 5700
uagcaucgcu gauggcauuu acagccucua ucacuagccc gcucaccacc cuacauaccc 5760
uucuauuuaa caucuugggg ggaugggugg ccgcccaacu cgcccccccc agcgcugcuu 5820
cagccuucgu aggcgccggc aucgccggcg cggcuguugg cagcauaggu cuugggaagg 5880
ugcucgugga cauccuagcg gguuauggag cagggguggc aggcgcacuc guggccuuca 5940
aggucaugag cggcgaagug cccuccacug aggaccuggu caacuuacuc ccugccaucc 6000
ucuccccugg ugcccugguc gucggggucg ugugcgcagc gauacugcgu cggcaugugg 6060

gcccagggga gggggccgug caguggauga accggcugau agcguucgcu ucgcggggua 6120
accacgucuc ccccacgcac uaugugccug agagcgacgc cgcagcgcgu gucacccaga 6180
uccucuccag ccuuaccauc acucagcugc uaaagaggcu ccaccagugg auuaauaagg 6240
acuguuccac gccaugcucc gguucguggc ucagggaugu uugggacugg auaugcacgg 6300
uuuugaccga cuucaaaacc uggcuccagu ccaagcuccu gccacgguug ccgggacucc 6360
cuuucuuuuc augucaacgu ggauauaaag gagucuggcu gggagauggc guuaugcaaa 6420
cuaccugucc augugguca caaaucagcg gacaugucaa aaacggcucc augaagaucg 6480
uggggccuaa aaccugcagc aacacguggc acgggacguu ccccaucaac gcauacacca 6540
caggccccug cacacccucc ccggcgccga acuauuccaa ggcguugugg cgaguggcug 6600
cugaggagua uguggagguc acgcgggugg gggauuucca cuacgugacg ggcaugacca 6660
cugacaacgu aaaaugccca ugccaggucc cggcccccga auucuucacg gaguuggaug 6720
gggugcggcu gcacagguac gcuccggcgu gcaagccucu cuuacgggau gaggucacau 6780
uccaggucgg gcucaaccag uauccgguug gaucacagcu uccaugugag cccgagccgg 6840
auguaacagu gcucacuucc augcucaccg accccuccca uauuacagca gagacggcua 6900
agcguaggcu ggccagaggg uccccccccu cuuuggccag cuccucagcu agccaguugu 6960
cugcgccuuc cuugaaggcg acaugcacua cccaccauga cuccccagau gcugaccuca 7020
ucgaggccaa ccuccugugg cggcaggaga ugggcgggaa caucacccgc guggagucag 7080
agaacaaggu aguaauucug gacucuuuug aaccgcuucg agcggaggag gaugauaggg 7140
aaguguccgu agcggcggag auccugcgga gaaccaggaa auuccccgca gcgaugccca 7200
uaugggcacg gccggacuac aacccaccac uccuagaguc uuggaagaac ccagacuacg 7260
ucccuccagu gguacacggg ugcccauuac caccuauuaa ggccccuccg auaccaccuc 7320
cacggagaaa gaggacgguc auccugacag aauccaccgu gucuucugcc uuggcggagc 7380
uugcuacaaa gaccuuuggc agcuccggau cgucggccgu ugacagcggc acagcgacug 7440
gcccuccuga ucaguccucc ggugacggag auacaggauc cgacgcugag ucgugcuccu 7500
ccaugccccc ccuugagggg gagccggggg accccgaucu cagcgacggg ucuuggucua 7560
ccgugagcga ggaggccggu gaggacgucg ucugcugcuc gauguccuac acauggacag 7620
gcgccuuaau cacaccgugc gccgcagagg agagcaagcu gccuaucaac gcguugagca 7680
acucuuugcu gcgccaccac aacauggucu augccacaac aucccgcagc gcgggccaac 7740
ggcagaagaa ggucacuuuu gacagagugc agguccugga cgaccauuac cgggacgugc 7800
ucaaggagau gaaggcgaag gcguccacag uuaaggcuaa acuucuaccu guagaagaag 7860
ccugcaggcu gacgccccca cacucggcca gauccaaguu uggcuauggg gcgaaggacg 7920
uccggaaccu auccagcaag gccgucaacc acauccacuc cguguggaag gacuugcugg 7980
aagacucuga gacgccgauu gacaccacca ucauggcaaa aaaugagguc uuuuguguuc 8040
aaccagagaa aggaggccgc aagucagcuc gucuuaucgu auucccagac uuggggguuc 8100

gugugugcga gaaaauggcc cuuuacgacg ugguuuccac ucuuccucag gccgugaugg 8160

gcuccucaua cggguuccag uacucuccug gacagcgggu cgaguuccug gugaaugccu 8220

ggaaaucaaa gaagaacccu augggcuucg cauaugacac ccgcuguuuu gacucaacgg 8280

ucacugagag ugacauccgu guugaggagu caauuuacca auguugugac uuggcccccg 8340

aggccagaca ggucauaagg ucgcucacgg agcggcuuua uaucgggggc ccccugacua 8400

auucaaaagg gcagagcugc ggcuaucgcc ggugccgcgc cagcggugug cugacgacca 8460

gcugcgguaa uacccucaca uguuacuuga aggcuucugc agccugucga gcugcaaagc 8520

uccaggacug cacaauguua gugugcggag acgaccuugu cguuaucugu gaaagugcgg 8580

gaacccagga ggacgcggcg agccuacgag ucuucacgga ggcuaugacu agguacucug 8640

ccccccccgg ggacccgccc cagccagaau acgacuugga gcugauaaca ucaugcuccu 8700

ccaacgucuc ggucgcgcac gaugcacuug gcaagcgggu guauuaucug acccgcgacc 8760

ccaccacccc ccuugcgcgg gcugcguggg agacagcaag acacacucca guuaacuccu 8820

ggcuaggcaa caucaucaug uaugcgccca cccuaugggc aaggaugauu cugaugaccc 8880

acuucuuuuc cauccuucua gcucaggaac aacuugaaaa agcccuagau ugucagaucu 8940

acggggccac uuacuccauu gagccacuug accuaccuca gaucauucag cgacuccacg 9000

gccuuagcgc auuuucacuc cauagcuacu cuccagguga gaucaauagg guggcuucau 9060

gccucaggaa acuuggggua ccacccuugc gagucuggag acaucgggcc agaaguccc 9120

gcgcuaagcu acugucccag ggggggaggg ccgccacuug uggcaaauac cucuucaacu 9180

gggcaguaag gaccaagcuc aaacucacuc caauuccggc ugcgucccag uuggacuugu 9240

ccggcugguu cauugcuggu uacagcgggg gagacauaua ucacagccug ucucgcgccc 9300

gaccccgcug guucaugugg ugccuacucc uacuuuccgu agggguaggc aucuaucugc 9360

uccccaaucg augaacgggg ggcuaaacac uccaggccaa uaggccauuc uguuuuuuuu 9420

uuuuuuuuuu uuuuuuuuuu uuuuuuuuuu uuuuuuuuuu uuuuuuccuu uuuuuuuuuu 9480

uuuuucccuu ucuuuuggug gcuccaucuu agcccuaguc acggcuagcu gugaaagguc 9540

cgugagccgc augacugcag agagugcuga uacuggccuc ucugcagauc augu 9594

<210> 64
<211> 3010
<212> PRT
<213> Hepatitis C virus

<400> 64

Met Ser Thr Asn Pro Lys Pro Gln Arg Lys Thr Lys Arg Asn Thr Asn
1               5                   10                  15

Arg Arg Pro Gln Asp Val Lys Phe Pro Gly Gly Gly Gln Ile Val Gly
            20                  25                  30

Gly Val Tyr Leu Leu Pro Arg Arg Gly Pro Arg Leu Gly Val Arg Ala
        35                  40                  45

Thr Arg Lys Thr Ser Glu Arg Ser Gln Pro Arg Glu Arg Arg Gln Pro
    50                  55                  60

Ile Pro Lys Ala Arg Arg Pro Glu Gly Arg Ala Trp Ala Gln Pro Gly
65                  70                  75                  80

Tyr Pro Trp Pro Leu Tyr Gly Asn Glu Gly Leu Gly Trp Ala Gly Trp
                85                  90                  95

Leu Leu Ser Pro Arg Gly Ser Arg Pro Asn Trp Gly Pro Thr Asp Pro
            100                 105                 110

Arg Arg Arg Ser Arg Asn Leu Gly Lys Val Ile Asp Thr Leu Thr Cys
        115                 120                 125

Gly Phe Ala Asp Leu Met Gly Tyr Ile Pro Leu Val Gly Ala Pro Leu
    130                 135                 140

Gly Gly Val Ala Arg Ala Leu Ala His Gly Val Arg Val Leu Glu Asp
145                 150                 155                 160

Gly Val Asn Tyr Ala Thr Gly Asn Leu Pro Gly Cys Ser Phe Ser Ile
                165                 170                 175

Phe Leu Leu Ala Leu Leu Ser Cys Leu Thr Val Pro Ala Ser Ala Tyr
            180                 185                 190

Glu Val Arg Asn Val Ser Gly Thr Tyr His Val Thr Asn Asp Cys Ala
        195                 200                 205

Asn Ser Ser Ile Val Tyr Glu Ala Ala Asp Ile Ile Met His Thr Pro
    210                 215                 220

Gly Cys Val Pro Cys Val Arg Glu Gly Asn Ser Ser Arg Cys Trp Val
225                 230                 235                 240

Ala Leu Thr Pro Thr Leu Ala Ala Arg Asn Arg Ser Ile Pro Thr Thr
                245                 250                 255

Thr Ile Arg Arg His Val Asp Leu Leu Val Gly Ala Ala Ala Phe Cys
            260                 265                 270

Ser Ala Met Tyr Val Gly Asp Leu Cys Gly Ser Val Phe Leu Val Ser
        275                 280                 285

Gln Leu Phe Thr Phe Ser Pro Arg Arg Tyr Glu Thr Val Gln Glu Cys
    290                 295                 300

Asn Cys Ser Ile Tyr Pro Gly His Val Ser Gly His Arg Met Ala Trp
305                 310                 315                 320

Asp Met Met Met Asn Trp Ser Pro Thr Ala Ala Leu Val Val Ser Gln
                325                 330                 335

Leu Leu Arg Ile Pro Gln Ala Val Val Asp Met Val Ala Gly Ala His
            340                 345                 350

Trp Gly Val Leu Ala Gly Leu Ala Tyr Tyr Ser Met Val Gly Asn Trp
        355                 360                 365

Ala Lys Val Leu Ile Val Met Leu Leu Phe Ala Gly Val Asp Gly Thr
    370                 375                 380

Thr Thr Val Thr Gly Gly Ala Ala Ala Phe Gly Thr Arg Ser Leu Ala
385                 390                 395                 400

Ser Phe Phe Thr Leu Gly Pro Ser Gln Lys Ile Gln Leu Val Asn Thr
                405                 410                 415

Asn Gly Ser Trp His Ile Asn Arg Thr Ala Leu Asn Cys Asn Asp Ser
            420                 425                 430

Leu Gln Thr Gly Phe Leu Ala Ala Leu Phe Tyr Ala His Lys Ile Asn
        435                 440                 445

Thr Ser Gly Cys Pro Glu Arg Met Ala Ser Cys Arg Pro Ile Asp Lys
    450                 455                 460

Phe Ala Gln Gly Trp Gly Pro Ile Thr His Gly Ala Pro Asp Thr Ser
465                 470                 475                 480

Asp Gln Arg Pro Tyr Cys Trp His Tyr Ala Pro Arg Pro Cys Gly Ile
                485                 490                 495

Val Pro Ala Ser Glu Val Cys Gly Pro Val Tyr Cys Phe Thr Pro Ser
            500                 505                 510

Pro Val Val Val Gly Thr Thr Asp Arg Phe Gly Val Pro Thr Tyr Ser
        515                 520                 525

Trp Gly Glu Asn Lys Thr Asp Val Leu Leu Leu Asn Asn Thr Arg Pro
    530                 535                 540

Pro Gln Gly Asn Trp Phe Gly Cys Thr Trp Met Asn Gly Thr Gly Phe
545                 550                 555                 560

Thr Lys Thr Cys Gly Gly Pro Pro Cys Asp Ile Gly Gly Val Gly Asn
                565                 570                 575

Asn Thr Leu Thr Cys Pro Thr Asp Cys Phe Arg Lys His Pro Glu Ala
            580                 585                 590

Thr Tyr Thr Lys Cys Gly Ser Gly Pro Trp Leu Thr Pro Arg Cys Leu

        595                 600                 605

Val Asp Tyr Pro Tyr Arg Leu Trp His Tyr Pro Cys Thr Val Asn Phe
    610                 615                 620

Thr Ile Phe Lys Val Arg Met Tyr Val Gly Gly Val Glu His Arg Leu
625                 630                 635                 640

Asn Ala Ala Cys Asn Trp Thr Arg Gly Glu Arg Cys Asp Leu Glu Asp
                645                 650                 655

Arg Asp Arg Ser Glu Leu Ser Pro Leu Leu Leu Ser Thr Thr Glu Trp
            660                 665                 670

Gln Ile Leu Pro Cys Ser Phe Thr Thr Leu Pro Ala Leu Ser Thr Gly
        675                 680                 685

Leu Ile His Leu His Gln Asn Ile Val Asp Val Gln Tyr Leu Tyr Gly
    690                 695                 700

Val Gly Ser Ala Val Val Ser Val Val Ile Arg Trp Glu Tyr Val Leu
705                 710                 715                 720

Leu Leu Phe Leu Leu Leu Ala Asp Ala Arg Val Cys Ala Cys Leu Trp
                725                 730                 735

Met Met Leu Leu Ile Ala Gln Ala Glu Ala Ala Leu Glu Asn Leu Val
            740                 745                 750

Val Leu Asn Ala Ala Ser Val Ala Gly Ala His Gly Ile Leu Ser Phe
        755                 760                 765

Leu Val Phe Phe Cys Ala Ala Trp Tyr Ile Lys Gly Lys Leu Val Pro
    770                 775                 780

Gly Val Ala Tyr Ala Leu Tyr Gly Val Trp Pro Leu Leu Leu Leu Leu
785                 790                 795                 800

Leu Ala Leu Pro Gln Arg Ala Tyr Ala Met Asp Arg Glu Met Ala Ala
                805                 810                 815

Ser Cys Gly Gly Ala Val Phe Val Gly Leu Val Leu Leu Thr Leu Ser
            820                 825                 830

Pro His Tyr Lys Ala Phe Leu Ala Lys Ser Ile Trp Trp Leu Gln Tyr
        835                 840                 845

Leu Ile Thr Arg Ala Glu Ala His Leu Gln Val Trp Val Pro Pro Leu
    850                 855                 860

Asn Val Arg Gly Gly Arg Asp Ala Ile Ile Leu Leu Thr Cys Ala Val
865                 870                 875                 880

His Pro Glu Leu Ile Phe Asp Ile Thr Lys Ile Leu Leu Ala Ile Phe
                885                 890                 895

Gly Pro Leu Met Val Leu Gln Ala Gly Leu Thr Arg Val Pro Tyr Phe
            900                 905                 910

Val Arg Ala Gln Gly Leu Ile Arg Val Cys Met Leu Val Arg Lys Val
        915                 920                 925

Ala Gly Gly His Tyr Ile Gln Met Ala Leu Met Arg Leu Ala Ala Leu
    930                 935                 940

Thr Gly Thr Tyr Val Tyr Asp His Leu Thr Pro Leu Arg Asp Trp Ala
945                 950                 955                 960

His Ala Gly Leu Arg Asp Leu Ala Val Ala Val Glu Pro Val Val Phe
                965                 970                 975

Ser Asn Thr Glu Thr Lys Ile Ile Thr Trp Gly Ala Asp Thr Ala Ala
            980                 985                 990

Cys Gly Asp Ile Ile Leu Gly Leu Pro Val Ser Ala Arg Arg Gly Arg
        995                 1000                1005

Glu Val Leu Leu Gly Pro Ala Asp Asn Phe Gly Glu Gln Gly Trp
    1010                1015                1020

Arg Leu Leu Ala Pro Ile Thr Ala Tyr Ser Gln Gln Thr Arg Gly
    1025                1030                1035

Leu Leu Gly Cys Ile Ile Thr Gly Leu Thr Gly Arg Asp Lys Asn
    1040                1045                1050

Gln Val Glu Gly Glu Val Gln Val Val Ser Thr Ala Thr Gln Ser
    1055                1060                1065

Phe Leu Ala Thr Cys Val Asn Gly Val Cys Trp Thr Val Tyr His
    1070                1075                1080

Gly Ala Gly Ser Lys Thr Leu Ala Gly Pro Lys Gly Pro Ile Thr
    1085                1090                1095

Gln Met Tyr Thr Asn Val Asp Gln Asp Leu Val Gly Trp Gln Ala
    1100                1105                1110

Pro Leu Gly Ala Arg Ser Met Thr Pro Cys Thr Cys Gly Ser Ser
    1115                1120                1125

Asp Leu Tyr Leu Val Thr Arg His Ala Asp Val Ile Pro Val Arg
    1130                1135                1140

Arg Arg Gly Asp Ser Arg Gly Ser Leu Leu Ser Pro Arg Pro Val
    1145                1150                1155

Ser Tyr Leu Lys Gly Ser Ser Gly Gly Pro Leu Leu Cys Pro Leu
    1160                1165                1170

Gly His Val Val Gly Ile Phe Arg Ala Ala Val Cys Thr Arg Gly
    1175                1180                1185

Val Ala Lys Ala Val Asp Phe Ile Pro Ile Glu Ser Met Glu Thr
    1190                1195                1200

Thr Met Arg Ser Pro Val Phe Thr Asp Asn Ser Ser Pro Pro Ala
    1205                1210                1215

Val Pro Gln Thr Phe Gln Val Ala His Leu His Ala Pro Thr Gly
    1220                1225                1230

Ser Gly Lys Ser Thr Lys Val Pro Ala Ala Tyr Ala Ala Gln Gly
    1235                1240                1245

Tyr Lys Val Leu Val Leu Asn Pro Ser Val Ala Ala Thr Leu Gly
    1250                1255                1260

Phe Gly Ala Tyr Met Ser Lys Ala Tyr Gly Val Asp Pro Asn Ile
    1265                1270                1275

Arg Thr Gly Val Arg Thr Ile Thr Thr Gly Ala Pro Ile Thr Tyr
    1280                1285                1290

Ser Thr Tyr Gly Lys Phe Leu Ala Asp Gly Gly Cys Ser Gly Gly
    1295                1300                1305

Ala Tyr Asp Ile Ile Ile Cys Asp Glu Cys His Ser Thr Asp Ser
    1310                1315                1320

Thr Ser Ile Leu Gly Ile Gly Thr Val Leu Asp Gln Ala Glu Thr
    1325                1330                1335

Ala Gly Ala Arg Leu Val Val Leu Ala Thr Ala Thr Pro Pro Gly
    1340                1345                1350

Ser Val Thr Val Pro His Pro Asn Ile Glu Glu Val Ala Leu Ser
    1355                1360                1365

Asn Thr Gly Glu Ile Pro Phe Tyr Gly Lys Ala Ile Pro Leu Glu
    1370                1375                1380

Val Ile Lys Gly Gly Arg His Leu Ile Phe Cys His Ser Lys Lys
    1385                1390                1395

Lys Cys Asp Glu Leu Ala Ala Lys Leu Ser Gly Leu Gly Val Asn
    1400                1405                1410

Ala Val Ala Tyr Tyr Arg Gly Leu Asp Val Ser Val Ile Pro Thr
    1415                1420                1425

Ser Gly Asp Val Val Val Val Ala Thr Asp Ala Leu Met Thr Gly
    1430                1435                1440

Tyr Thr Gly Asp Phe Asp Ser Val Ile Asp Cys Asn Thr Cys Val
    1445                1450                1455

Thr Gln Thr Val Asp Phe Ser Leu Asp Pro Thr Phe Thr Ile Glu
    1460                1465                1470

Thr Thr Thr Val Pro Gln Asp Ala Val Ser Arg Ser Gln Arg Arg
    1475                1480                1485

Gly Arg Thr Gly Arg Gly Arg Gly Gly Ile Tyr Arg Phe Val Thr
    1490                1495                1500

Pro Gly Glu Arg Pro Ser Gly Met Phe Asp Ser Ser Val Leu Cys
    1505                1510                1515

Glu Cys Tyr Asp Ala Gly Cys Ala Trp Tyr Glu Leu Thr Pro Ala
    1520                1525                1530

Glu Thr Ser Val Arg Leu Arg Ala Tyr Leu Asn Thr Pro Gly Leu
    1535                1540                1545

Pro Val Cys Gln Asp His Leu Glu Phe Trp Glu Gly Val Phe Thr
    1550                1555                1560

Gly Leu Thr His Ile Asp Ala His Phe Leu Ser Gln Thr Lys Gln
    1565                1570                1575

Ala Gly Asp Asn Phe Pro Tyr Leu Val Ala Tyr Gln Ala Thr Val
    1580                1585                1590

Cys Ala Arg Ala Gln Ala Pro Pro Pro Ser Trp Asp Gln Met Trp
    1595                1600                1605

Lys Cys Leu Ile Arg Leu Lys Pro Thr Leu His Gly Pro Thr Pro
    1610                1615                1620

Leu Leu Tyr Arg Leu Gly Ala Val Gln Asn Glu Val Thr Leu Thr
    1625                1630                1635

His Pro Met Thr Lys Tyr Ile Met Thr Cys Met Ser Ala Asp Leu
    1640                1645                1650

Glu Val Val Thr Ser Thr Trp Val Leu Val Gly Gly Val Leu Ala

1655                 1660                 1665

Ala Leu Ala Ala Tyr Cys Leu Thr Thr Gly Ser Val Val Ile Ile
    1670                 1675                 1680

Gly Arg Ile Ile Leu Ser Gly Arg Pro Ala Ile Ile Pro Asp Arg
    1685                 1690                 1695

Glu Val Leu Tyr Gln Glu Phe Asp Glu Met Glu Glu Cys Ala Ser
    1700                 1705                 1710

His Leu Pro Tyr Ile Glu Gln Gly Met Gln Leu Ala Glu Gln Phe
    1715                 1720                 1725

Lys Gln Lys Ala Leu Gly Leu Leu Gln Thr Ala Thr Lys Gln Ala
    1730                 1735                 1740

Glu Ala Ala Ala Pro Val Val Glu Ser Lys Trp Gln Ala Leu Glu
    1745                 1750                 1755

Ala Phe Trp Ala Lys His Met Trp Asn Phe Ile Ser Gly Ile Gln
    1760                 1765                 1770

Tyr Leu Ala Gly Leu Ser Thr Leu Pro Gly Asn Pro Ala Ile Ala
    1775                 1780                 1785

Ser Leu Met Ala Phe Thr Ala Ser Ile Thr Ser Pro Leu Thr Thr
    1790                 1795                 1800

Leu His Thr Leu Leu Phe Asn Ile Leu Gly Gly Trp Val Ala Ala
    1805                 1810                 1815

Gln Leu Ala Pro Pro Ser Ala Ala Ser Ala Phe Val Gly Ala Gly
    1820                 1825                 1830

Ile Ala Gly Ala Ala Val Gly Ser Ile Gly Leu Gly Lys Val Leu
    1835                 1840                 1845

Val Asp Ile Leu Ala Gly Tyr Gly Ala Gly Val Ala Gly Ala Leu
    1850                 1855                 1860

Val Ala Phe Lys Val Met Ser Gly Glu Val Pro Ser Thr Glu Asp
    1865                 1870                 1875

Leu Val Asn Leu Leu Pro Ala Ile Leu Ser Pro Gly Ala Leu Val
    1880                 1885                 1890

Val Gly Val Val Cys Ala Ala Ile Leu Arg Arg His Val Gly Pro
    1895                 1900                 1905

Gly Glu Gly Ala Val Gln Trp Met Asn Arg Leu Ile Ala Phe Ala
    1910                 1915                 1920

Ser Arg Gly Asn His Val Ser Pro Thr His Tyr Val Pro Glu Ser
    1925                1930                1935

Asp Ala Ala Ala Arg Val Thr Gln Ile Leu Ser Ser Leu Thr Ile
    1940                1945                1950

Thr Gln Leu Leu Lys Arg Leu His Gln Trp Ile Asn Lys Asp Cys
    1955                1960                1965

Ser Thr Pro Cys Ser Gly Ser Trp Leu Arg Asp Val Trp Asp Trp
    1970                1975                1980

Ile Cys Thr Val Leu Thr Asp Phe Lys Thr Trp Leu Gln Ser Lys
    1985                1990                1995

Leu Leu Pro Arg Leu Pro Gly Leu Pro Phe Phe Ser Cys Gln Arg
    2000                2005                2010

Gly Tyr Lys Gly Val Trp Leu Gly Asp Gly Val Met Gln Thr Thr
    2015                2020                2025

Cys Pro Cys Gly Ala Gln Ile Ser Gly His Val Lys Asn Gly Ser
    2030                2035                2040

Met Lys Ile Val Gly Pro Lys Thr Cys Ser Asn Thr Trp His Gly
    2045                2050                2055

Thr Phe Pro Ile Asn Ala Tyr Thr Thr Gly Pro Cys Thr Pro Ser
    2060                2065                2070

Pro Ala Pro Asn Tyr Ser Lys Ala Leu Trp Arg Val Ala Ala Glu
    2075                2080                2085

Glu Tyr Val Glu Val Thr Arg Val Gly Asp Phe His Tyr Val Thr
    2090                2095                2100

Gly Met Thr Thr Asp Asn Val Lys Cys Pro Cys Gln Val Pro Ala
    2105                2110                2115

Pro Glu Phe Phe Thr Glu Leu Asp Gly Val Arg Leu His Arg Tyr
    2120                2125                2130

Ala Pro Ala Cys Lys Pro Leu Leu Arg Asp Glu Val Thr Phe Gln
    2135                2140                2145

Val Gly Leu Asn Gln Tyr Pro Val Gly Ser Gln Leu Pro Cys Glu
    2150                2155                2160

Pro Glu Pro Asp Val Thr Val Leu Thr Ser Met Leu Thr Asp Pro
    2165                2170                2175

Ser His Ile Thr Ala Glu Thr Ala Lys Arg Arg Leu Ala Arg Gly
    2180                2185                2190

Ser Pro Pro Ser Leu Ala Ser Ser Ser Ala Ser Gln Leu Ser Ala
    2195                2200                2205

Pro Ser Leu Lys Ala Thr Cys Thr Thr His His Asp Ser Pro Asp
    2210                2215                2220

Ala Asp Leu Ile Glu Ala Asn Leu Leu Trp Arg Gln Glu Met Gly
    2225                2230                2235

Gly Asn Ile Thr Arg Val Glu Ser Glu Asn Lys Val Val Ile Leu
    2240                2245                2250

Asp Ser Phe Glu Pro Leu Arg Ala Glu Glu Asp Asp Arg Glu Val
    2255                2260                2265

Ser Val Ala Ala Glu Ile Leu Arg Arg Thr Arg Lys Phe Pro Ala
    2270                2275                2280

Ala Met Pro Ile Trp Ala Arg Pro Asp Tyr Asn Pro Pro Leu Leu
    2285                2290                2295

Glu Ser Trp Lys Asn Pro Asp Tyr Val Pro Pro Val Val His Gly
    2300                2305                2310

Cys Pro Leu Pro Pro Ile Lys Ala Pro Pro Ile Pro Pro Pro Arg
    2315                2320                2325

Arg Lys Arg Thr Val Ile Leu Thr Glu Ser Thr Val Ser Ser Ala
    2330                2335                2340

Leu Ala Glu Leu Ala Thr Lys Thr Phe Gly Ser Ser Gly Ser Ser
    2345                2350                2355

Ala Val Asp Ser Gly Thr Ala Thr Gly Pro Pro Asp Gln Ser Ser
    2360                2365                2370

Gly Asp Gly Asp Thr Gly Ser Asp Ala Glu Ser Cys Ser Ser Met
    2375                2380                2385

Pro Pro Leu Glu Gly Glu Pro Gly Asp Pro Asp Leu Ser Asp Gly
    2390                2395                2400

Ser Trp Ser Thr Val Ser Glu Glu Ala Gly Glu Asp Val Val Cys
    2405                2410                2415

Cys Ser Met Ser Tyr Thr Trp Thr Gly Ala Leu Ile Thr Pro Cys
    2420                2425                2430

Ala Ala Glu Glu Ser Lys Leu Pro Ile Asn Ala Leu Ser Asn Ser
    2435                2440                2445

Leu Leu Arg His His Asn Met Val Tyr Ala Thr Thr Ser Arg Ser
    2450                2455                2460

Ala Gly Gln Arg Gln Lys Lys Val Thr Phe Asp Arg Val Gln Val
    2465                2470                2475

Leu Asp Asp His Tyr Arg Asp Val Leu Lys Glu Met Lys Ala Lys
    2480                2485                2490

Ala Ser Thr Val Lys Ala Lys Leu Leu Pro Val Glu Glu Ala Cys
    2495                2500                2505

Arg Leu Thr Pro Pro His Ser Ala Arg Ser Lys Phe Gly Tyr Gly
    2510                2515                2520

Ala Lys Asp Val Arg Asn Leu Ser Ser Lys Ala Val Asn His Ile
    2525                2530                2535

His Ser Val Trp Lys Asp Leu Leu Glu Asp Ser Glu Thr Pro Ile
    2540                2545                2550

Asp Thr Thr Ile Met Ala Lys Asn Glu Val Phe Cys Val Gln Pro
    2555                2560                2565

Glu Lys Gly Gly Arg Lys Ser Ala Arg Leu Ile Val Phe Pro Asp
    2570                2575                2580

Leu Gly Val Arg Val Cys Glu Lys Met Ala Leu Tyr Asp Val Val
    2585                2590                2595

Ser Thr Leu Pro Gln Ala Val Met Gly Ser Ser Tyr Gly Phe Gln
    2600                2605                2610

Tyr Ser Pro Gly Gln Arg Val Glu Phe Leu Val Asn Ala Trp Lys
    2615                2620                2625

Ser Lys Lys Asn Pro Met Gly Phe Ala Tyr Asp Thr Arg Cys Phe
    2630                2635                2640

Asp Ser Thr Val Thr Glu Ser Asp Ile Arg Val Glu Glu Ser Ile
    2645                2650                2655

Tyr Gln Cys Cys Asp Leu Ala Pro Glu Ala Arg Gln Val Ile Arg
    2660                2665                2670

Ser Leu Thr Glu Arg Leu Tyr Ile Gly Gly Pro Leu Thr Asn Ser
    2675                2680                2685

Lys Gly Gln Ser Cys Gly Tyr Arg Arg Cys Arg Ala Ser Gly Val

2690                2695                2700

Leu Thr Thr Ser Cys Gly Asn Thr Leu Thr Cys Tyr Leu Lys Ala
2705                2710                2715

Ser Ala Ala Cys Arg Ala Ala Lys Leu Gln Asp Cys Thr Met Leu
2720                2725                2730

Val Cys Gly Asp Asp Leu Val Val Ile Cys Glu Ser Ala Gly Thr
2735                2740                2745

Gln Glu Asp Ala Ala Ser Leu Arg Val Phe Thr Glu Ala Met Thr
2750                2755                2760

Arg Tyr Ser Ala Pro Pro Gly Asp Pro Pro Gln Pro Glu Tyr Asp
2765                2770                2775

Leu Glu Leu Ile Thr Ser Cys Ser Ser Asn Val Ser Val Ala His
2780                2785                2790

Asp Ala Leu Gly Lys Arg Val Tyr Tyr Leu Thr Arg Asp Pro Thr
2795                2800                2805

Thr Pro Leu Ala Arg Ala Ala Trp Glu Thr Ala Arg His Thr Pro
2810                2815                2820

Val Asn Ser Trp Leu Gly Asn Ile Ile Met Tyr Ala Pro Thr Leu
2825                2830                2835

Trp Ala Arg Met Ile Leu Met Thr His Phe Phe Ser Ile Leu Leu
2840                2845                2850

Ala Gln Glu Gln Leu Glu Lys Ala Leu Asp Cys Gln Ile Tyr Gly
2855                2860                2865

Ala Thr Tyr Ser Ile Glu Pro Leu Asp Leu Pro Gln Ile Ile Gln
2870                2875                2880

Arg Leu His Gly Leu Ser Ala Phe Ser Leu His Ser Tyr Ser Pro
2885                2890                2895

Gly Glu Ile Asn Arg Val Ala Ser Cys Leu Arg Lys Leu Gly Val
2900                2905                2910

Pro Pro Leu Arg Val Trp Arg His Arg Ala Arg Ser Val Arg Ala
2915                2920                2925

Lys Leu Leu Ser Gln Gly Gly Arg Ala Ala Thr Cys Gly Lys Tyr
2930                2935                2940

Leu Phe Asn Trp Ala Val Arg Thr Lys Leu Lys Leu Thr Pro Ile
2945                2950                2955

Pro Ala Ala Ser Gln Leu Asp Leu Ser Gly Trp Phe Ile Ala Gly
    2960                2965                2970

Tyr Ser Gly Gly Asp Ile Tyr His Ser Leu Ser Arg Ala Arg Pro
    2975                2980                2985

Arg Trp Phe Met Trp Cys Leu Leu Leu Leu Ser Val Gly Val Gly
    2990                2995                3000

Ile Tyr Leu Leu Pro Asn Arg
    3005                3010

<210> 65
<211> 783
<212> RNA
<213> Hepatitis C virus

<400> 65
gccucacacc uucccuacau cgaacaagga augcagcucg ccgagcaauu caagcagaag 60

gcgcucgggu uguugcaaac ggccaccaag caagcggagg ccgcugcucc cgugguggag 120

uccaaauggc aagcccuuga ggccuucugg gcgaagcaca uguggaacuu caucagcggg 180

auacaguauc uagcaggcuu guccacucug ccuggaaacc ccgcgauagc aucgcugaug 240

gcauuuacag ccucuaucac uagcccgcuc accacccuac auacccuucu auuuaacauc 300

uuggggggau ggguggccgc ccaacucgcc ccccccagcg cugcuucagc cuucguaggc 360

gccggcaucg ccggcgcggc uguuggcagc auaggucuug ggaaggugcu cguggacauc 420

cuagcggguu auggagcagg gguggcaggc gcacucgugg ccuucaaggu caugagcggc 480

gaagugcccu ccacugagga ccuggucaac uuacucccug ccauccucuc cccuggugcc 540

cuggucgucg gggucgugug cgcagcgaua cugcgucggc augugggccc aggggagggg 600

gccgugcagu ggaugaaccg gcugauagcg uucgcuucgc gggguaacca cgucuccccc 660

acgcacuaug ugccugagag cgacgccgca gcgcguguca cccagauccu cuccagccuu 720

accaucacuc agcugcuaaa gaggcuccac caguggauua auaaggacug uuccacgcca 780

ugc 783

<210> 66
<211> 261
<212> PRT
<213> Hepatitis C virus

<400> 66

Ala Ser His Leu Pro Tyr Ile Glu Gln Gly Met Gln Leu Ala Glu Gln
1               5                   10                  15

Phe Lys Gln Lys Ala Leu Gly Leu Leu Gln Thr Ala Thr Lys Gln Ala
            20                  25                  30

Glu Ala Ala Ala Pro Val Val Glu Ser Lys Trp Gln Ala Leu Glu Ala

35                  40                  45

Phe Trp Ala Lys His Met Trp Asn Phe Ile Ser Gly Ile Gln Tyr Leu
    50                  55                  60

Ala Gly Leu Ser Thr Leu Pro Gly Asn Pro Ala Ile Ala Ser Leu Met
65                  70                  75                  80

Ala Phe Thr Ala Ser Ile Thr Ser Pro Leu Thr Thr Leu His Thr Leu
                85                  90                  95

Leu Phe Asn Ile Leu Gly Gly Trp Val Ala Ala Gln Leu Ala Pro Pro
            100                 105                 110

Ser Ala Ala Ser Ala Phe Val Gly Ala Gly Ile Ala Gly Ala Ala Val
        115                 120                 125

Gly Ser Ile Gly Leu Gly Lys Val Leu Val Asp Ile Leu Ala Gly Tyr
    130                 135                 140

Gly Ala Gly Val Ala Gly Ala Leu Val Ala Phe Lys Val Met Ser Gly
145                 150                 155                 160

Glu Val Pro Ser Thr Glu Asp Leu Val Asn Leu Leu Pro Ala Ile Leu
                165                 170                 175

Ser Pro Gly Ala Leu Val Val Gly Val Val Cys Ala Ala Ile Leu Arg
            180                 185                 190

Arg His Val Gly Pro Gly Glu Gly Ala Val Gln Trp Met Asn Arg Leu
        195                 200                 205

Ile Ala Phe Ala Ser Arg Gly Asn His Val Ser Pro Thr His Tyr Val
    210                 215                 220

Pro Glu Ser Asp Ala Ala Ala Arg Val Thr Gln Ile Leu Ser Ser Leu
225                 230                 235                 240

Thr Ile Thr Gln Leu Leu Lys Arg Leu His Gln Trp Ile Asn Lys Asp
                245                 250                 255

Cys Ser Thr Pro Cys
            260

<210>  67
<211>  8010
<212>  RNA
<213>  Artificial

<220>
<223>  subgenomic HCV RNA replicon

<400>  67
   uaauacgacu cacuauagcc agcccccgau ugggggcgac acuccaccau agaucacucc      60
   ccugugagga acuacugucu ucacgcagaa agcgucuagc cauggcguua guaugagugu 120
   cgugcagccu ccaggacccc cccucccggg agagccauag uggucugcgg aaccggugag 180
   uacaccggaa uugccaggac gaccgggucc uuucuuggau caacccgcuc aaugccugga 240
   gauuugggcg ugcccccgcg agacugcuag ccgaguagug uugggucgcg aaaggccuug 300
   ugguacugcc ugauagggug cuugcgagug ccccgggagg ucucguagac cgugcaccau 360
   gagcacgaau ccuaaaccuc aaagaaaaac caaacguaac accaacgggc gcgccaugau 420
   ugaacaagau ggauugcacg cagguucucc ggccgcuugg guggagaggc uauucggcua 480
   ugacugggca caacagacaa ucggcugcuc ugaugccgcc guguuccggc ugucagcgca 540
   ggggcgcccg guucuuuuug ucaagaccga ccuguccggu gcccugaaug aacugcagga 600
   cgaggcagcg cggcuaucgu ggcuggccac gacgggcguu ccuugcgcag cugugcucga 660
   cguugucacu gaagcgggaa gggacuggcu gcuauugggc gaagugccgg ggcaggaucu 720
   ccugucaucu caccuugcuc cugccgagaa aguauccauc auggcugaug caaugcggcg 780
   gcugcauacg cuugauccgg cuaccugccc auucgaccac caagcgaaac aucgcaucga 840
   gcgagcacgu acucggaugg aagccggucu ugucgaucag gaugaucugg acgaagagca 900
   ucaggggcuc gcgccagccg aacuguucgc caggcucaag gcgcgcaugc ccgacggcga 960
   ggaucucguc gugacccaug gcgaugccug cuugccgaau aucauggugg aaaauggccg 1020
   cuuuucugga uucaucgacu guggccggcu ggguguggcg gaccgcuauc aggacauagc 1080
   guuggcuacc cgugauauug cugaagagcu uggcggcgaa ugggcugacc gcuuccucgu 1140
   gcuuuacggu aucgccgcuc ccgauucgca gcgcaucgcc uucuaucgcc uucuugacga 1200
   guucuucuga guuuaaacag accacaacgg uuucccucua gcgggaucaa uuccgccccu 1260
   cucccucccc ccccccuaac guuacuggcc gaagccgcuu ggaauaaggc cggugugcgu 1320
   uugucuauau guuauuuucc accauauugc cgucuuuugg caaugugagg gcccggaaac 1380
   cuuggcccug ucuucuugac gagcauuccu aggggucuuu ccccucucgc caaaggaaug 1440
   caaggucugu uggaugucgu gaaggaagca guuccucugg gaagcuucuu gaagacaaac 1500
   aacgucugua gcgacccuuu gcaggcagcg gaacccccca ccuggcgaca ggugccucug 1560
   cggccaaaag ccacguguau aagauacacc ugcaaaggcg guacaacccc agugccacgu 1620
   ugugaguugg auaguugugg aaagagucaa auggcucucc ucaagcguau ucaacaaggg 1680
   gcugaaggau gcccagaagg uaccccauug uaugggaucu gaucuggggc cucggugcac 1740
   augcucuaca uguguuuagu cgagguuaaa aaaacgucua ggccccccga accacgggga 1800
   cgugguuuuc cuuugaaaaa cacgauaaua ccauggcgcc caucacggcc uacucccaac 1860
   agacgcgggg cuuacuuggc uguaucauca ccggccucac aggucgagac aagaaccagg 1920
   ucgaagggga gguucaggug guuuccaccg caacacaguc uuucuuggca accugcguca 1980
   acggugugug uuggacuguc uaccauggug ccggcucaaa gacccuagcc ggcccgaagg 2040
   ggccaaucac ccagauguac accaacguag accaagaccu cguuggcugg caggcgcccc 2100
   ccggggcgcg uuccaugacg ccgugcaccu gcggcagcuc ggaccuuuac uuggucacga 2160
   ggcaugcuga ugucauuccg gugcgccggc ggggugacag cagagggagc cuacuuuccc 2220
   ccaggcccgu cuccuacuug aagggcucuu cagguggucc acugcucugc cccuuggggc 2280
   acgucguggg caucuuucgg gcugccgugu gcacccgggg gguugcgaag gcgguggacu 2340
   ucauacccau cgagucuaug gaaacuacca ugcggucucc ggucuuuacg gauaauucau 2400
   cucccccggc cguaccgcag acuuuucaag uggcccaucu gcacgccccc acuggcagcg 2460
   gcaagagcac caaggugcca gcugcguaug cagcccaggg guacaaggug cuugucuuga 2520
   auccauccgu ugccgccacc uuggguuuug gggcguauau guccaaggca uacggugucg 2580
   acccuaacau uagaacuggg guaaggacca ucaccacagg cgcucccauc acguacucca 2640
   ccuacggcaa guuccuugcc gacggugguu gcuccggggg cgcuuacgac aucauaauau 2700
   gcgaugagug ccacucaacc gacucgacuu ccauuuuggg cauuggcacg guccuggauc 2760
   aagcggagac ggcuggagcg cgacucgucg ugcucgccac cgcuacgccu ccgggaucgg 2820
   ucacugugcc acaccccaac aucgaggagg uggccuuguc caacaccgga gagauucccu 2880
   ucuauggcaa agccaucccc cucgagguca ucaagggggg gaggcaucuc auuuucuguc 2940
   auucuaagaa gaagugugau gagcucgcug caaagcuguc gggccuuggg gucaacgcug 3000
   uagcguacua ccggggucuu gauguguccg ucauaccaac aagcggggac gucguugucg 3060
   uggcaacaga cgcucuaaug acgggcuaca ccggugacuu ugacucugug aucgacugua 3120
   auacaugugu cacccagaca gucgacuuca gccuggaccc caccuucacc auugagacga 3180
   cgaccgugcc ccaagacgca gugucgcgcu cgcagcggcg agggaggacu gguaggggua 3240
   gagggggcau auacagguuu gugacuccag gagagcggcc cucgggcaug uucgauuccu 3300
   cgguccugug ugaaugcuau gacgcgggcu gugcuuggua cgagcucacg cccgccgaga 3360
   ccucgguuag gcugcgggcu uaccuaaaua caccaggguu gcccgucugc caggaccauu 3420
   uggaguucug ggaaggcguc uucacaggcc ucacucauau agaugcccac uucuugucuc 3480
   agacuaagca ggcaggagac aacuuccccu accugguggc auaccaggcc acagugugcg 3540
   ccagggccca ggcaccaccu ccaucauggg aucaaaugug gaagugucuc auacggcuaa 3600
   aaccuacacu acacgggcca acaccccugu uguacaggcu aggagccguc caaaacgagg 3660
   ucacccucac acaccccaug accaaauaca ucaugacaug caugucggcu gaccuagagg 3720
   ucgucaccag cacuugggug cuggugggcg ggguccucgc agcuuuggcc gcguacugcu 3780
   ugacaacggg cagcgugguu aucauaggca ggaucaucuu guccggaagg ccggcuauca 3840
   uucccgauag ggaaguucuc uaccaggagu ucgaugaaau ggaagagugc gccucacacc 3900
   uucccuacau cgaacaagga augcagcucg ccgagcaauu caagcagaag gcgcucgggu 3960
   uguugcaaac ggccaccaag caagcggagg ccgcugcucc cgugguggag uccaaauggc 4020
   aagcccuuga ggccuucugg gcgaagcaca uguggaacuu caucagcggg auacaguauc 4080
   uagcaggcuu guccacucug ccuggaaacc ccgcgauagc aucgcugaug gcauuuacag 4140
   ccucuaucac uagcccgcuc accacccuac auacccuucu auuuaacauc uuggggggau 4200
   ggguggccgc ccaacucgcc ccccccagcg cugcuucagc cuucguaggc gccggcaucg 4260
   ccggcgcggc uguuggcagc auaggucuug ggaaggugcu cguggacauc cuagcggguu 4320
   auggagcagg gguggcaggc gcacucgugg ccuucaaggu caugagcggc gaagugcccu 4380
   ccacugagga ccuggucaac uuacucccug ccauccucuc cccuggugcc cuggucgucg 4440
   gggucgugug cgcagcgaua cugcgucggc augugggccc aggggagggg gccgugcagu 4500
   ggaugaaccg gcugauagcg uucgcuucgc gggguaacca cgucuccccc acgcacuaug 4560
   ugccugagag cgacgccgca gcgcguguca cccagauccu cuccagccuu accaucacuc 4620
   agcugcuaaa gaggcuccac caguggauua auaaggacug uuccacgcca ugcuccgguu 4680
   cguggcucag ggauguuugg gacuggauau gcacgguuuu gaccgacuuc aaaaccuggc 4740
   uccaguccaa gcuccugcca cgguugccgg gacucccuuu cuuuucaugu caacguggau 4800
   auaaaggagu cuggcuggga gauggcguua ugcaaacuac cuguccaugu ggugcacaaa 4860
   ucagcggaca ugucaaaaac ggcuccauga agaucguggg gccuaaaacc ugcagcaaca 4920
   cguggcacgg gacguucccc aucaacgcau acaccacagg ccccugcaca cccuccccgg 4980
   cgccgaacua uuccaaggcg uuguggcgag uggcugcuga ggaguaugug gaggucacgc 5040
   ggguggggga uuuccacuac gugacgggca ugaccacuga caacguaaaa ugcccaugcc 5100
   aggucccggc ccccgaauuc uucacggagu uggauggggu gcggcugcac agguacgcuc 5160
   cggcgugcaa gccucucuua cgggaugagg ucacauucca ggucgggcuc aaccaguauc 5220
   cgguuggauc acagcuucca ugugagcccg agccggaugu aacagugcuc acuuccaugc 5280
   ucaccgaccc cucccauauu acagcagaga cggcuaagcg uaggcuggcc agaggguccc 5340
   cccccucuuu ggccagcucc ucagcuagcc aguugucugc gccuuccuug aaggcgacau 5400
   gcacuaccca ccaugacucc ccagaugcug accucaucga ggccaaccuc cuguggcggc 5460
   aggagauggg cgggaacauc acccgcgugg agucagagaa caagguagua auucuggacu 5520
   cuuuugaacc gcuucgagcg gaggaggaug auagggaagu guccguagcg gcggagaucc 5580
   ugcggagaac caggaaauuc cccgcagcga ugcccauaug ggcacggccg gacuacaacc 5640
   caccacuccu agagucuugg aagaacccag acuacguccc uccaguggua cacgggugcc 5700
   cauuaccacc uauuaaggcc ccuccgauac caccuccacg gagaaagagg acggucaucc 5760
   ugacagaauc caccgugucu ucugccuugg cggagcuugc uacaaagacc uuuggcagcu 5820
   ccggaucguc ggccguugac agcggcacag cgacuggccc uccugaucag uccuccggug 5880
   acggagauac aggauccgac gcugagucgu gcuccuccau gcccccccuu gagggggagc 5940
   cgggggaccc cgaucucagc gacgggucuu ggucuaccgu gagcgaggag gccggugagg 6000
   acgucgucug cugcucgaug uccuacacau ggacaggcgc cuuaaucaca ccgugcgccg 6060
   cagaggagag caagcugccu aucaacgcgu ugagcaacuc uuugcugcgc caccacaaca 6120
   uggucuaugc cacaacaucc cgcagcgcgg gccaacggca gaagaagguc acuuuugaca 6180
   gagugcaggu ccuggacgac cauuaccggg acgugcucaa ggagaugaag gcgaaggcgu 6240
   ccacaguuaa ggcuaaacuu cuaccuguag aagaagccug caggcugacg cccccacacu 6300
   cggccagauc caaguuuggc uauggggcga aggacguccg gaaccuaucc agcaaggccg 6360
   ucaaccacau ccacuccgug uggaaggacu ugcuggaaga cucugagacg ccgauugaca 6420
   ccaccaucau ggcaaaaaau gaggucuuuu guguucaacc agagaaagga ggccgcaagu 6480
   cagcucgucu uaucguauuc ccagacuugg ggguucgugu gugcgagaaa auggcccuuu 6540
   acgacguggu uuccacucuu ccucaggccg ugaugggcuc cucauacggg uuccaguacu 6600
   cuccuggaca gcgggucgag uuccugguga augccuggaa aucaaagaag aacccuaugg 6660
   gcuucgcaua ugacacccgc uguuuugacu caacggucac ugagagugac auccguguug 6720
   aggagucaau uuaccaaugu ugugacuugg cccccgaggc cagacagguc auaaggucgc 6780
   ucacggagcg gcuuuauauc gggggccccc ugacuaauuc aaaagggcag agcugcggcu 6840
   aucgccggug ccgcgccagc ggugugcuga cgaccagcug cgguaauacc cucacauguu 6900
   acuugaaggc uucugcagcc ugucgagcug caaagcucca ggacugcaca auguuagugu 6960
   gcggagacga ccuugucguu aucugugaaa gugcgggaac ccaggaggac gcggcgagcc 7020
   uacgagucuu cacggaggcu augacuaggu acucugcccc ccccggggac ccgccccagc 7080
   cagaauacga cuuggagcug auaacaucau gcuccuccaa cgucucgguc gcgcacgaug 7140
   cacuuggcaa gcggguguau uaucugaccc gcgaccccac caccccccuu gcgcgggcug 7200
   cgugggagac agcaagacac acuccaguua acuccuggcu aggcaacauc aucauguaug 7260
   cgcccacccu augggcaagg augauucuga ugacccacuu cuuuuccauc cuucuagcuc 7320
   aggaacaacu ugaaaaagcc cuagauuguc agaucuacgg ggccacuuac uccauugagc 7380
   cacuugaccu accucagauc auucagcgac uccacggccu uagcgcauuu ucacuccaua 7440
   gcuacucucc aggugagauc aauagggugg cuucaugccu caggaaacuu gggguaccac 7500
   ccuugcgagu cuggagacau cgggccagaa guguccgcgc uaagcuacug ucccaggggg 7560
   ggagggccgc cacuuguggc aaauaccucu ucaacugggc aguaaggacc aagcucaaac 7620
   ucacuccaau uccggcugcg ucccaguugg acuuguccgg cugguucauu gcugguuaca 7680
   gcgggggaga cauauaucac agccugucuc gcgcccgacc ccgcugguuc augguggucc 7740
   uacuccuacu uuccguaggg guaggcaucu aucugcuccc caaucgauga acggggggcu 7800
   aaacacucca ggccaauagg ccauucuguu uuuuuuuuuu uuuuuuuuuu uuuuuuuuuu 7860
   uuuuuuuuuu uuuuuuuuuu uuccuuuuuu uuuuuuuuuu ucccuuucuu uugguggcuc 7920
   caucuuagcc cuagucacgg cuagcuguga aagguccgug agccgcauga cugcagagag 7980
   ugcugauacu ggccucucug cagaucaugu 8010

<210> 68
<211> 8010
<212> RNA
<213> Artificial

<220>
<223> subgenomic HCV RNA replicon

<400>  68
uaauacgacu cacuauagcc agcccccgau ugggggcgac acuccaccau agaucacucc      60
ccugugagga acuacugucu ucacgcagaa agcgucuagc cauggcguua guaugagugu     120
cgugcagccu ccaggacccc cccucccggg agagccauag uggucugcgg aaccggugag     180
uacaccggaa uugccaggac gaccgggucc uuucuuggau caacccgcuc aaugccugga     240
gauuugggcg ugcccccgcg agacugcuag ccgaguagug uugggucgcg aaaggccuug     300
ugguacugcc ugauagggug cuugcgagug ccccgggagg ucucguagac cgugcaccau     360
gagcacgaau ccuaaaccuc aaagaaaaac caaacguaac accaacgggc gcgccaugau     420
ugaacaagau ggauugcacg cagguucucc ggccgcuugg guggagaggc uauucggcua     480
ugacugggca caacagacaa ucggcugcuc ugaugccgcc guguuccggc ugucagcgca     540
ggggcgcccg guucuuuuug ucaagaccga ccuguccggu gcccugaaug aacugcagga     600
cgaggcagcg cggcuaucgu ggcuggccac gacgggcguu ccuugcgcag cugugcucga     660
cguugucacu gaagcgggaa gggacuggcu gcuauugggc gaagugccgg ggcaggaucu     720
ccugucaucu caccuugcuc cugccgagaa aguauccauc auggcugaug caaugcggcg     780
gcugcauacg cuugauccgg cuaccugccc auucgaccac caagcgaaac aucgcaucga     840
gcgagcacgu acucggaugg aagccggucu ugucgaucag gaugaucugg acgaagagca     900
ucaggggcuc gcgccagccg aacuguucgc caggcucaag gcgcgcaugc ccgacggcga     960
ggaucucguc gugacccaug gcgaugccug cuugccgaau aucauggugg aaaauggccg    1020
cuuuucugga uucaucgacu guggccggcu ggguguggcg gaccgcuauc aggacauagc    1080
guuggcuacc cgugauauug cugaagagcu uggcggcgaa ugggcugacc gcuuccucgu    1140
gcuuuacggu aucgccgcuc ccgauucgca gcgcaucgcc uucuaucgcc uucuugacga    1200
guucuucuga guuuaaacag accacaacgg uuucccucua gcgggaucaa uuccgccccu    1260
cucccucccc ccccccuaac guuacuggcc gaagccgcuu ggaauaaggc cggugugcgu    1320
uugucuauau guuauuuucc accauauugc cgucuuuugg caaugugagg gcccggaaac    1380
cuuggcccug ucuucuugac gagcauuccu aggggucuuu ccccucucgc caaaggaaug    1440
caaggucugu uggaugucgu gaaggaagca guuccucugg gaagcuucuu gaagacaaac    1500
aacgucugua gcgacccuuu gcaggcagcg gaacccccca ccuggcgaca ggugccucug    1560
cggccaaaag ccacguguau aagauacacc ugcaaaggcg guacaacccc agugccacgu    1620
ugugaguugg auaguugugg aaagagucaa auggcucucc ucaagcguau ucaacaaggg    1680
gcugaaggau gcccagaagg uaccccauug uaugggaucu gaucuggggc cucggugcac    1740
augcucuaca uguguuuagu cgagguuaaa aaaacgucua ggccccccga accacgggga    1800
cgugguuuuc cuuugaaaaa cacgauaaua ccauggcgcc caucacggcc uacucccaac    1860
agacgcgggg cuuacuuggc uguaucauca ccggccucac aggucgagac aagaaccagg    1920
ucgaagggga gguucaggug guuuccaccg caacacaguc uuucuuggca accugcguca    1980
acggugugug uuggacuguc uaccauggug ccggcucaaa gacccuagcc ggcccgaagg    2040

ggccaaucac ccagauguac accaacguag accaagaccu cguuggcugg caggcgcccc 2100
ucggggcgcg uuccaugacg ccgugcaccu gcggcagcuc ggaccuuuac uuggucacga 2160
ggcaugcuga ugucauuccg gugcgccggc ggggugacag cagagggagc cuacuuuccc 2220
ccaggcccgu cuccuacuug aagggcucuu cagguggucc acugcucugc cccuuggggc 2280
acgucguggg caucuuucgg gcugccgugu gcacccgggg gguugcgaag gcgguggacu 2340
ucauacccau cgagucuaug gaaacuacca ugcggucucc ggucuuuacg gauaauucau 2400
cucccccggc cguaccgcag acuuuucaag uggcccaucu gcacgccccc acuggcagcg 2460
gcaagagcac caaggugcca gcugcguaug cagcccaggg guacaaggug cuugucuuga 2520
auccauccgu ugccgccacc uuggguuuug gggcguauau guccaaggca uacggugucg 2580
acccuaacau uagaacuggg guaaggacca ucaccacagg cgcucccauc acguacucca 2640
ccuacggcaa guuccuugcc gacggugguu gcuccggggg cgcuuacgac aucauaauau 2700
gcgaugagug ccacucaacc gacucgacuu ccauuuuggg cauuggcacg guccuggauc 2760
aagcggagac ggcuggagcg cgacucgucg ugcucgccac cgcuacgccu ccgggaucgg 2820
ucacugugcc acaccccaac aucgaggagg uggccuuguc caacaccgga gagauucccu 2880
ucuauggcaa agccaucccc cucgagguca ucaagggggg gaggcaucuc auuuucuguc 2940
auucuaagaa gaagugugau gagcucgcug caaagcuguc gggccuuggg gucaacgcug 3000
uagcguacua ccggggucuu gauguguccg ucauaccaac aagcggggac gucguugucg 3060
uggcaacaga cgcucuaaug acgggcuaca ccggugacuu ugacucugug aucgacugua 3120
auacaugugu cacccagaca gucgacuuca gccuggaccc caccuucacc auugagacga 3180
cgaccgugcc ccaagacgca gugucgcgcu cgcagcggcg agggaggacu gguaggggua 3240
gagggggcau auacagguuu gugacuccag gagagcggcc cucgggcaug uucgauuccu 3300
cgguccugug ugaaugcuau gacgcgggcu gugcuuggua cgagcucacg cccgccgaga 3360
ccucgguuag gcugcgggcu uaccuaaaua caccaggguu gcccgucugc caggaccauu 3420
uggaguucug ggaaggcguc uucacaggcc ucacucauau agaugcccac uucuugucuc 3480
agacuaagca ggcaggagac aacuuccccu accugguggc auaccaggcc acagugugcg 3540
ccagggccca ggcaccaccu ccaucauggg aucaaaugug gaagugucuc auacggcuaa 3600
aaccuacacu acacgggcca acaccccugu uguacaggcu aggagccguc caaaacgagg 3660
ucacccucac acaccccaug accaaauaca ucaugacaug caugucggcu gaccuagagg 3720
ucgucaccag cacuugggug cuggugggcg ggguccucgc agcuuuggcc gcguacugcu 3780
ugacaacggg cagcgugguu aucauaggca ggaucaucuu guccggaagg ccggcuauca 3840
uucccgauag ggaaguucuc uaccaggagu ucgaugaaau ggaagagugc gccucacacc 3900
uucccuacau cgaacaagga augcagcucg ccgagcaauu caagcagaag gcgcucgggu 3960
uguugcaaac ggccaccaag caagcggagg ccgcugcucc cgugguggag uccaaauggc 4020
aagcccuuga ggccuucugg gcgaagcaca uguggaacuu caucagcggg auacaguauc 4080
uagcaggcuu guccacucug ccuggaaacc ccgcgauagc aucgcugaug gcauuuacag 4140

ccucuaucac uagcccgcuc accacccuac auacccuucu auuuaacauc uuggggggau 4200
ggguggccgc ccaacucgcc ccccccagcg cugcuucagc cuucguaggc gccggcaucg 4260
ccggcgcggc uguuggcagc auaggucuug ggaaggugcu cguggacauc cuagcggguu 4320
auggagcagg gguggcaggc gcacucgugg ccuucaaggu caugagcggc gaagugcccu 4380
ccacugagga ccuggucaac uuacucccug ccauccucuc cccuggugcc cuggucgucg 4440
gggucgugug cgcagcgaua cugcgucggc augugggccc aggggagggg gccgugcagu 4500
ggaugaaccg gcugauagcg uucgcuucgc gggguaacca cgucuccccc acgcacuaug 4560
ugccugagag cgacgccgca gcgcguguca cccagauccu cuccagccuu accaucacuc 4620
agcugcuaaa gaggcuccac caguggauua auaaggacug uuccacgcca ugcuccgguu 4680
cguggcucag ggauguuugg gacuggauau gcacgguuuu gaccgacuuc aaaaccuggc 4740
uccaguccaa gcuccugcca cgguugccgg gacucccuuu cuuuucaugu caacguggau 4800
auaaaggagu cuggcuggga gauggcguua ugcaaacuac cuguccaugu ggugcacaaa 4860
ucagcggaca ugucaaaaac ggcuccauga agaucguggg gccuaaaacc ugcagcaaca 4920
cguggcacgg gacguucccc aucaacgcau acaccacagg ccccugcaca cccuccccgg 4980
cgccgaacua uuccaaggcg uuguggcgag uggcugcuga ggaguaugug gaggucacgc 5040
ggguggggga uuuccacuac gugacgggca ugaccacuga caacguaaaa ugcccaugcc 5100
aggucccggc ccccgaauuc uucacggagu uggauggggu gcggcugcac agguacgcuc 5160
cggcgugcaa gccucucuua cgggaugagg ucacauucca ggucgggcuc aaccaguauc 5220
cgguuggauc acagcuucca ugugagcccg agccggaugu aacagugcuc acuuccaugc 5280
ucaccgaccc cucccauauu acagcagaga cggcuaagcg uaggcuggcc agaggguccc 5340
cccccucuuu ggccagcucc ucagcuagcc aguugucugc gccuuccuug aaggcgacau 5400
gcacuaccca ccaugacucc ccagaugcug accucaucga ggccaaccuc cuguggcggc 5460
aggagauggg cgggaacauc acccgcgugg agucagagaa caagguagua auucuggacu 5520
cuuuugaacc gcuucgagcg gaggaggaug auagggaagu guccguagcg gcggagaucc 5580
ugcggagaac caggaaauuc cccgcagcga ugcccauaug ggcacggccg gacuacaacc 5640
caccacuccu agagucuugg aagaacccag acuacguccc uccaguggua cacgggugcc 5700
cauuaccacc uauuaaggcc ccuccgauac caccuccacg gagaaagagg acggucaucc 5760
ugacagaauc caccgugucu ucugccuugg cggagcuugc uacaaagacc uuuggcagcu 5820
ccggaucguc ggccguugac agcggcacag cgacuggccc uccugaucag uccuccggug 5880
acggagauac aggauccgac gcugagucgu gcuccuccau gcccccccuu gagggggagc 5940
cgggggaccc cgaucucagc gacgggucuu ggucuaccgu gagcgaggag gccggugagg 6000
acgucgucug cugcucgaug uccuacacau ggacaggcgc cuuaaucaca ccgugcgccg 6060
cagaggagag caagcugccu aucaacgcgu ugagcaacuc uuugcugcgc caccacaaca 6120
uggucuaugc cacaacaucc cgcagcgcgg gccaacggca gaagaagguc acuuuugaca 6180

gagugcaggu ccuggacgac cauuaccggg acgugcucaa ggagaugaag gcgaaggcgu 6240
ccacaguuaa ggcuaaacuu cuaccuguag aagaagccug caggcugacg cccccacacu 6300
cggccagauc caaguuuggc uauggggcga aggacguccg gaaccuaucc agcaaggccg 6360
ucaaccacau ccacuccgug uggaaggacu ugcuggaaga cucugagacg ccgauugaca 6420
ccaccaucau ggcaaaaaau gaggucuuuu guguucaacc agagaaagga ggccgcaagu 6480
cagcucgucu uaucguauuc ccagacuugg ggguucgugu gugcgagaaa auggcccuuu 6540
acgacguggu uuccacucuu ccucaggccg ugaugggcuc cucauacggg uuccaguacu 6600
cuccuggaca gcgggucgag uuccugguga augccuggaa aucaaagaag aacccuaugg 6660
gcuucgcaua ugacacccgc uguuuugacu caacggucac ugagagugac auccguguug 6720
aggagucaau uuaccaaugu ugugacuugg cccccgaggc cagacagguc auaaggucgc 6780
ucacggagcg gcuuuauauc gggggccccc ugacuaauuc aaaagggcag agcugcggcu 6840
aucgccggug ccgcgccagc ggugugcuga cgaccagcug cgguaauacc cucacauguu 6900
acuugaaggc uucugcagcc ugucgagcug caaagcucca ggacugcaca auguuagugu 6960
gcggagacga ccuugucguu aucugugaaa gugcgggaac ccaggaggac gcggcgagcc 7020
uacgagucuu cacggaggcu augacuaggu acucugcccc ccccggggac ccgccccagc 7080
cagaauacga cuuggagcug auaacaucau gcuccuccaa cgucucgguc gcgcacgaug 7140
cacuuggcaa gcggguguau uaucugaccc gcgaccccac caccccccuu gcgcgggcug 7200
cgugggagac agcaagacac acuccaguua acuccuggcu aggcaacauc aucauguaug 7260
cgcccacccu augggcaagg augauucuga ugacccacuu cuuuuccauc cuucuagcuc 7320
aggaacaacu ugaaaaagcc cuagauuguc agaucuacgg ggccacuuac uccauugagc 7380
cacuugaccu accucagauc auucagcgac uccacggccu uagcgcauuu ucacuccaua 7440
gcuacucucc aggugagauc aauagggugg cuucaugccu caggaaacuu gggguaccac 7500
ccuugcgagu cuggagacau cgggccagaa guguccgcgc uaagcuacug ucccaggggg 7560
ggagggccgc cacuuguggc aaauaccucu ucaacugggc aguaaggacc aagcucaaac 7620
ucacuccaau uccggcugcg ucccaguugg acuuguccgg cugguucauu gcugguuaca 7680
gcgggggaga cauauaucac agccugucuc gcgcccgacc ccgcugguuc auguggugcc 7740
uacuccuacu uuccguaggg guaggcaucu aucugcuccc caaucgauga acggggggcu 7800
aaacacucca ggccaauagg ccauucuguu uuuuuuuuuu uuuuuuuuuu uuuuuuuuuu 7860
uuuuuuuuuu uuuuuuuuuu uuccuuuuuu uuuuuuuuuu ucccuuucuu uugguggcuc 7920
caucuuagcc cuagucacgg cuagcuguga aagguccgug agccgcauga cugcagagag 7980
ugcugauacu ggccucucug cagaucaugu 8010

## Description

### FIELD OF THE INVENTION

The present invention relates to a hepatitis C virus (hereinafter also referred to "HCV") gene, a replicon RNA derived from the gene, a replicon-replicating cell into which the replicon RNA is introduced, and a method for screening a drug using the replicon-replicating cell.

### BACKGROUND ART

HCV is a causal factor for chronic hepatitis C. According to statistics from WHO, it is estimated that 170 million people are infected in the world. HCV is a virus classified in the genus Flavivirus in the family Flaviviridae. It is thought to infect via blood or blood components, and to proliferate in the liver. Although those who are infected with HCV cause relatively benign symptoms in the early stages of infection, it develops into a chronic disease at a high frequency. After an asymptomatic period for a certain period of time, it develops into chronic hepatitis. Furthermore, as the period of infection prolongs, conditions of disease deteriorates to liver cirrhosis and eventually to liver cancer at a high frequency. It is thought that the hepatitis virus relates to 95% of the liver cancer and 80% of them are caused by HCV infection.

For a treatment of chronic hepatitis C, interferon is widely used. Recently, by improvement of pharmaceutical formulations of interferon, as well as improvement of administration methods such as a combination therapy of interferon and ribavirin or the like, HCV is eliminated out of the body and a rate of sustained virological response (SVR) is gradually increasing. Yet the rate of SVR by the interferon administration is still about 50% and there are thought to be many HCVs exhibiting resistance against the interferon treatment. Accordingly, development of a drug having therapeutic effects on the interferon-resistant viruses is desired.

In the development of such a drug, a system for screening the drug is required. It has been reported that HCV was infected to cells derived from human or monkey cells and proliferated *in vitro.* But such a proliferation system showed a low efficiency of infection and low efficiency of proliferation, and thus cannot be used as the system screening the drug.

Recently, Wakita *et al.* isolated HCV genotype 2a gene from a patient with fulminant hepatitis C (Patent Literature 1). From this isolated JFH1 strain, a full-length RNA was synthesized *in vitro.* When the full-length RNA was introduced into cells derived from human hepatocarcinoma (Huh7 cells), a replicon RNA which autonomously replicates in the cells was able to be obtained. In addition, it was confirmed that infectious particles were released in the culture supernatant of the cells into which the replicon RNA was introduced (Non-patent Literature 1). Hence, a system for reinfection and proliferation can be constructed by introducing the replicon RNA of the JFH1 strain into the cells derived from human hepatocarcinoma (Huh7 cells) and culturing the obtained infectious particles again with the cells derived from human hepatocarcinomana. Using this system for reinfection and proliferation, screening for a drug against HCV has been started.

However, the JFH1 strain is an HCV of the genotype 2a and an interferon-sensitive HCV. For that, the JFH1 strain does not have an HCV gene region showing the interferon resistance. Also, a factor in a host, which factor acts on a region defining the interferon resistance, cannot be specified. Thus, there is a possibility that a drug effective against interferon-resistant HCV cannot be screened.

Recently, Lemon *et al.* reported a system for infection and proliferation in which a replicon RNA of H77 strain belonging to genotype 1a was introduced into the cells derived from human hepatocarcinoma (Huh7 cells) (Non-patent Literature 2). However, when virus particles obtained from the culture supernatant of the cells in which this replicon RNA was introduced were again infected to cells derived from human hepatocarcinoma, the infectivity titer was about 400 times lower, compared with that of the above-described JFH1 strain. Therefore, the replicon RNA of the H77 strain is thought to release virus particles which have lost infectivity. Consequently, it is thought that the RNA replicon of the H77 strain which is replicable *in vitro* has lost a mechanism to produce the infectious particles and does not retain a proliferation mechanism intrinsic to HCV.
Accordingly, there is a possibility that a system for screening using the system for infection and proliferation of this replicon RNA of H77 cannot screen an effective drug against HCV having a proliferation mechanism *in vivo.*

As described above, since there are no effective systems for culturing HCV *in vitro,* it has been difficult to carry out screening for a drug useful for the HCV treatment. For instance, as for interferon which is currently widely used for the HCV treatment, direct therapeutic methods have been developed and improved using patients as test subjects, which has been imposed a very heavy burden on the patients. Although the above-described replicon RNAs reported by Wakita and Lemon enabled some drugs to be screened, these replicon RNAs have problems described above. Hence, it is thought that a drug which can be widely used for the HCV treatment cannot be screened.

Patent Literature 1: Japanese Laid-open Patent Application (Kokai) No. 2002-171978
Non-patent Literature 1: "Nature Medicine" (U.S.A.) 2005, volume 11, p791-796
Non-patent Literature 2: "Proceeding of the National Academy of Science of the United State of America" 2006, volume 103, p2310-2315

### DISCLOSURE OF THE INVENTION

### PROBLEMS WHICH THE INVENTION TRIES TO SOLVE

The present inventors intensively studied, in order to obtain a drug which can be widely used for the HCV treatment, an effective system for proliferating HCV, in particular, an *in vitro* system for proliferating HCV having the genotype 1b gene, resistant to interferon and capable of producing infectious particles. First, a full-length of HCV genome was isolated from the serum of a patient with fulminant hepatitis and the nucleic acid sequence with 9594 bases shown in SEQ ID NO: 1 was determined. When a replicon RNA was prepared from this HCV gene and transfected into cells derived from human hepatocarcinoma, it was confirmed that the replicon RNA autonomously replicated in the cells and replication of RNA occurred. Further, by introducing two mutations of amino acid sequence in an NS4B protein region of this replicon RNA, replication efficiency of RNA markedly increased and lots of virus particles were released into the culture supernatant. And then, by culturing this HCV particles again with the cells derived from human hepatocarcinoma, construction of a system for reinfection and proliferation was possible. And, the present inventors found that this system for reinfection and proliferation using the replicon-replicating cells and infectious particles was useful as a system for screening a drug for the HCV treatment, in particular, a system for screening a drug for an interferon-resistant virus. The present invention is based on such discoveries.

### MEANS FOR SOLVING THE PROBLEMS

Accordingly, the present invention relates to a HCV gene comprising a polynucleotide selected from the group consisting of:
(A) a polynucleotide having the nucleic acid sequence shown in SEQ ID NO: 5;
(B) a polynucleotide having the nucleic acid sequence shown in SEQ ID NO: 7;
(C) a polynucleotide having the nucleic acid sequence shown in SEQ ID NO: 65;
(D) a polynucleotide coding for a polypeptide having the amino acid sequence shown in SEQ ID NO: 6;
(E) a polynucleotide coding for a polypeptide having the amino acid sequence shown in SEQ ID NO: 8; and
(F) a polynucleotide coding for a polypeptide having the amino acid sequence shown in SEQ ID NO: 66.
In a preferred mode of the HCV gene according to the present invention, the HCV gene is a polynucleotide having the nucleic acid sequence shown in SEQ ID NO: 1, SEQ ID NO: 3, SEQ ID NO: 10, SEQ ID NO: 61 or SEQ ID NO: 63.
Also, the present invention relates to a HCV genotype 1b gene comprising nucleotides coding for the 1804th amino acid leucine and the 1966th amino acid lysine in the amino acid sequence of a HCV polyprotein and a polynucleotide coding for an NS4B protein.
Additionally, the present invention also relates to a DNA comprising a single stranded DNA having the nucleic acid sequence with uridine being substituted by thymine in the nucleic acid sequence of the above-described HCV gene.

The present invention also relates to a polypeptide having the amino acid sequence shown in SEQ ID NO: 6, SEQ ID NO: 8 or SEQ ID NO: 66.
Further, the present invention also relates to a HCV polyprotein, wherein a peptide in an NS4B region is a polypeptide having the amino acid sequence shown in SEQ ID NO: 6, SEQ ID NO: 8 or SEQ ID NO: 66.
Also, the present invention also relates to at least one HCV protein selected from the group consisting of a core protein having the amino acid sequence from the first to the 191 st amino acid in the amino acid sequence shown in SEQ ID NO: 2, SEQ ID NO: 4, SEQ ID NO: 11, SEQ ID NO: 62 or SEQ ID NO: 64, an E1 protein having the amino acid sequence from the 192nd to the 383rd amino acid, an E2 protein having the amino acid sequence from the 384th to the 746th amino acid, a P7 protein having the amino acid sequence from the 747th to the 809th amino acid, an NS2 protein having the amino acid sequence from the 810th to the1026th amino acid, an NS3 protein having the amino acid sequence from the 1027th to 1657th amino acid, an NS4A protein having the amino acid sequence from the 1658th to 1711th amino acid, an NS4B protein having the amino acid sequence from the 1712th to the 1972nd amino acid, an NS5A protein having the amino acid sequence from the 1973rd to the 2419th amino acid, and an NS5B protein having the amino acid sequence from the 2420th to the 3010th amino acid.

The present invention also relates to a replicon RNA comprising a polynucleotide selected from the group consisting of:
(A) a polynucleotide having the nucleic acid sequence shown in SEQ ID NO: 5;
(B) a polynucleotide having the nucleic acid sequence shown in SEQ ID NO: 7;
(C) a polynucleotide having the nucleic acid sequence shown in SEQ ID NO: 65;
(D) a polynucleotide coding for a polypeptide having the amino acid sequence shown in SEQ ID NO: 6;
(E) a polynucleotide coding for a polypeptide having the amino acid sequence shown in SEQ ID NO: 8;
(F) a polynucleotide coding for a polypeptide having the amino acid sequence shown in SEQ ID NO: 66; and
(G) a polynucleotide having a nucleic acid sequence having a homology of not less than 90% with the nucleic acid sequence shown in SEQ ID NO: 5, SEQ ID NO: 7 or SEQ ID NO: 65.
Also, the present invention relates to a replicon RNA of the genotype 1b, the replicon RNA comprising nucleotides coding for the 1804th amino acid leucine and the 1966th amino acid lysine in the amino acid sequence of an HCV polyprotein and a polynucleotide coding for an NS4B protein.
In a preferred mode of the replicon RNA according to the present invention, the replicon RNA comprises:
(A) a polynucleotide from the first to the 341st nucleotide in the 5' untranslated region of the HCV, a polynucleotide coding for a polypeptide from the 1027th to the 3010th amino acid in the HCV polyprotein and a polynucleotide of the 3' untranslated region; or
(B) a polynucleotide from the first to the 341 st nucleotide in the 5' untranslated region, a polynucleotide coding for the HCV polyprotein composed of 3010 amino acids and a polynucleotide of the 3' untranslated region.
In a preferred mode of the replicon RNA according to the present invention, the replicon RNA is resistant to interferon.
In another preferred mode of the replicon RNA according to the present invention, the replicon RNA comprises:
(A) a polynucleotide having the first to the 341st nucleotide in the nucleic acid sequence shown in SEQ ID NO: 1 and a polynucleotide having the 3420th to the 9594th nucleotide in the nucleic acid sequence shown in SEQ ID NO: 1;
(B) a polynucleotide having the first to the 341 st nucleotide in the nucleic acid sequence shown in SEQ ID NO: 3 and a polynucleotide having the 3420th to the 9594th nucleotide in the nucleic acid sequence shown in SEQ ID NO: 3;
(C) a polynucleotide having the first to the 341 st nucleotide in the nucleic acid sequence shown in SEQ ID NO: 10 and a polynucleotide having the 3420th to the 9594th nucleotide in the nucleic acid sequence shown in SEQ ID NO: 10;
(D) a polynucleotide having the first to the 341st nucleotide in the nucleic acid sequence shown in SEQ ID NO: 61 and a polynucleotide having the 3420th to the 9594th nucleotide in the nucleic acid sequence shown in SEQ ID NO: 61;
(E) a polynucleotide having the first to the 341 st nucleotide in the nucleic acid sequence shown in SEQ ID NO: 63 and a polynucleotide having the 3420th to the 9594th nucleotide in the nucleic acid sequence shown in SEQ ID NO: 63;
(F) a polynucleotide having a nucleic acid sequence having a homology of not less than 90% with the nucleic acid sequence from the first to the 341 st nucleotide in the nucleic acid sequence shown in SEQ ID NO: 1 and a polynucleotide having a nucleic acid sequence having a homology of not less than 90% with the nucleic acid sequence from the 3420th to the 9594th nucleotide in the nucleic acid sequence shown in SEQ ID NO: 1;
(G) a polynucleotide having a nucleic acid sequence having a homology of not less than 90% with the nucleic acid sequence from the first to the 341 st nucleotide in the nucleic acid sequence shown in SEQ ID NO: 3 and the polynucleotide having a nucleic acid sequence having a homology of not less than 90% with the nucleic acid sequence from the 3420th to the 9594th nucleotide in the nucleic acid sequence shown in SEQ ID NO: 3;
(H) a polynucleotide having a nucleic acid sequence having a homology of not less than 90% with the nucleic acid sequence from the first to the 341 st nucleotide in the nucleic acid sequence shown in SEQ ID NO: 10 and a polynucleotide having a nucleic acid sequence having a homology of not less than 90% with the nucleic acid sequence from the 3420th to the 9594th nucleotide in the nucleic acid sequence shown in SEQ ID NO: 10;
(I) a polynucleotide having a nucleic acid sequence having a homology of not less than 90% with the nucleic acid sequence from the first to the 341 st nucleotide in the nucleic acid sequence shown in SEQ ID NO: 61 and a polynucleotide having a nucleic acid sequence having a homology of not less than 90% with the nucleic acid sequence from the 3420th to the 9594th nucleotide in the nucleic acid sequence shown in SEQ ID NO: 61; or
(J) a polynucleotide having a nucleic acid sequence having a homology of not less than 90% with the nucleic acid sequence from the first to the 341 st nucleotide in the nucleic acid sequence shown in SEQ ID NO: 63 and a polynucleotide having a nucleic acid sequence having a homology of not less than 90% with the nucleic acid sequence from the 3420th to the 9594th nucleotide in the nucleic acid sequence shown in SEQ ID NO: 63.
Also, in another preferred mode of the replicon RNA according to the present invention, the above-described replicon RNA is a polynucleotide having the nucleic acid sequence shown in SEQ ID NO: 1, SEQ ID NO: 3, SEQ ID NO: 10, SEQ ID NO: 61 or SEQ ID NO: 63; or a polynucleotide having a nucleic acid sequence having a homology of not less than 90% with the nucleic acid sequence shown in SEQ ID NO: 1, SEQ ID NO: 3, SEQ ID NO: 10, SEQ ID NO: 61 or SEQ ID NO: 63.
Further, in another preferred mode of the replicon RNA according to the present invention, the replicon RNA comprises at least one selection marker gene or reporter gene and at least one IRES sequence.

Further, the present invention relates to a DNA coding for the above-described replicon RNA.
Further, the present invention relates to a vector comprising the above-described DNA.
Also, the present invention relates to a replicon-replicating cell made by introducing at least one selected from the group consisting of the above-described replicon RNA, the above-described DNA and the above-described vector into a cell.
In a preferred mode of the replicon-replicating cell according to the present invention, the above-described cell is a cell derived from a hepatocyte. Preferably the above-described cell derived from the hepatocyte is a Huh-7 cell.

The present invention relates to a replicon RNA produced by the above-described replicon-replicating cell.
Further, the present invention relates to at least one HCV protein selected from the group consisting of CORE, E1, E2, P7, NS2, NS3, NS4A, NS4B, NS5A and NS5B produced by the above-described replicon-replicating cell.
Further, the present invention relates to a HCV particle produced by the above-described replicon-replicating cell.
Also, the present invention relates to a method for screening a substance controlling HCV infection, which method comprises the steps of contacting the above-described replicon-replicating cell with the above-described substance and analyzing a degree of increase in the replicon RNA.
In a preferred mode of the method for screening according to the present invention, the above-described analysis of the degree of increase in the replicon RNA is detection of the replicon RNA or HCV protein.
In the present specification, "replicon RNA" means RNA which is produced based on viral RNA and has an ability to autonomously replicate in cells. As long as it can cause RNA replication, the replicon RNA includes ones capable and incapable of generating a virus particle.
Also, in the present specification, "interferon resistance" means that replication or proliferation of HCV is not significantly suppressed by administration of interferon *in vitro* and *in vivo.*

### EFFECTS OF THE INVENTION

The HCV gene according to the present invention allows an HCV gene capable of replicating *in vivo* to be analyzed *in vitro.* By using this HCV gene, the RNA replicon according to the present invention can be produced. Further, by the replicon-replicating cells in which the above-described RNA replicon is introduced, screening of a drug against HCV is possible. The replicon RNA produced from the HCV gene according to the present invention is an interferon-resistant RNA replicon of the genotype 1b. In addition, it is possible that the replicon-replicating cells into which this replicon RNA is introduced produce the infectious virus particles. Therefore, it is possible to provide an *in vitro* model with the same proliferation mechanisms of HCV, which is the genotype 1b and resistant to interferon, as *in vivo* proliferation mechanisms. And this proliferation model of HCV enables a drug for suppressing aggravation of hepatitis to be screened and a pharmaceutical to be developed.

### BRIEF DESCRIPTION OF THE DRAWINGS

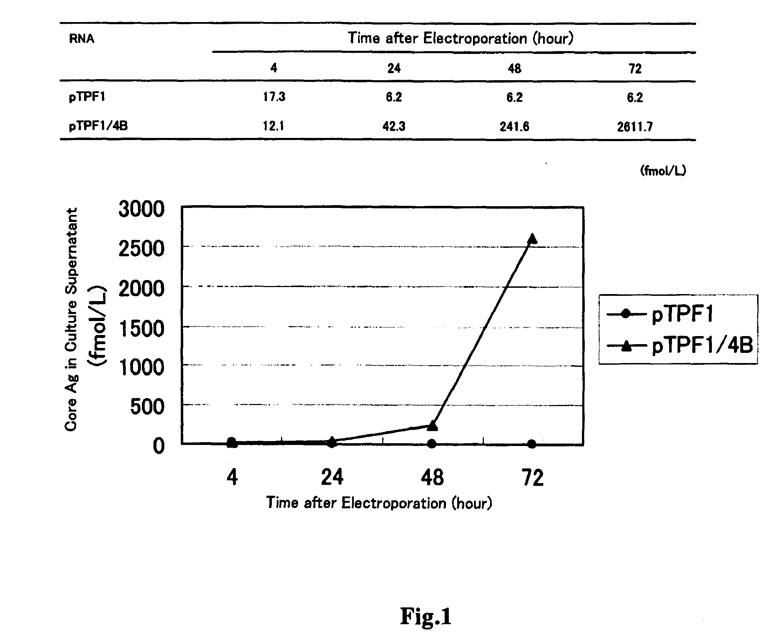

[Figure 1] Figure 1 shows the production of the core protein by introducing the replicon RNA according to the present invention into cells. pTPF1 and pTPF1/4B were introduced into Huh-7 cells by electroporation and 4, 24, 48 and 72 hours later, the concentration of the core protein in the culture supernatant was measured.

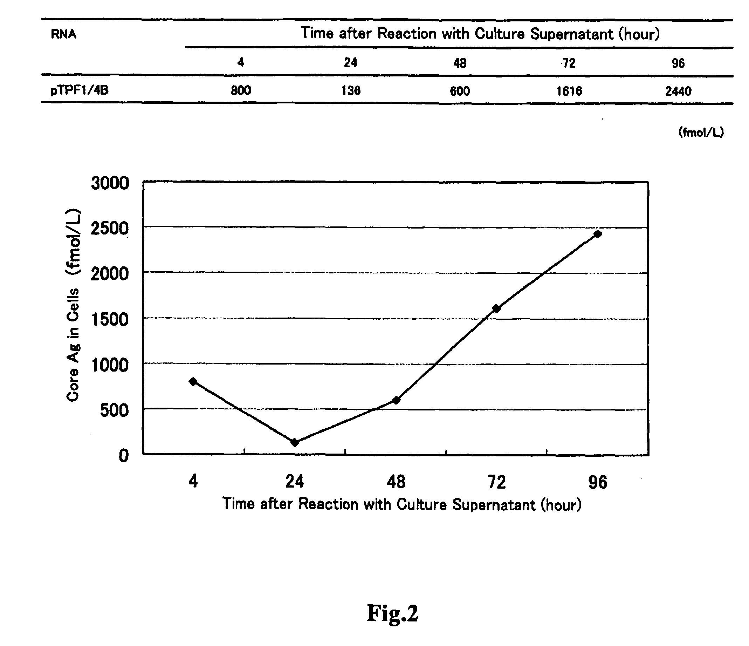

[Figure 2] Figure 2 shows reinfection of the infective particles produced from the replicon-replicating cells according to the present invention to cells. At 4, 24, 48, 72 and 96 hours after the infection, the concentration of the core protein in the culture supernatant was measured.

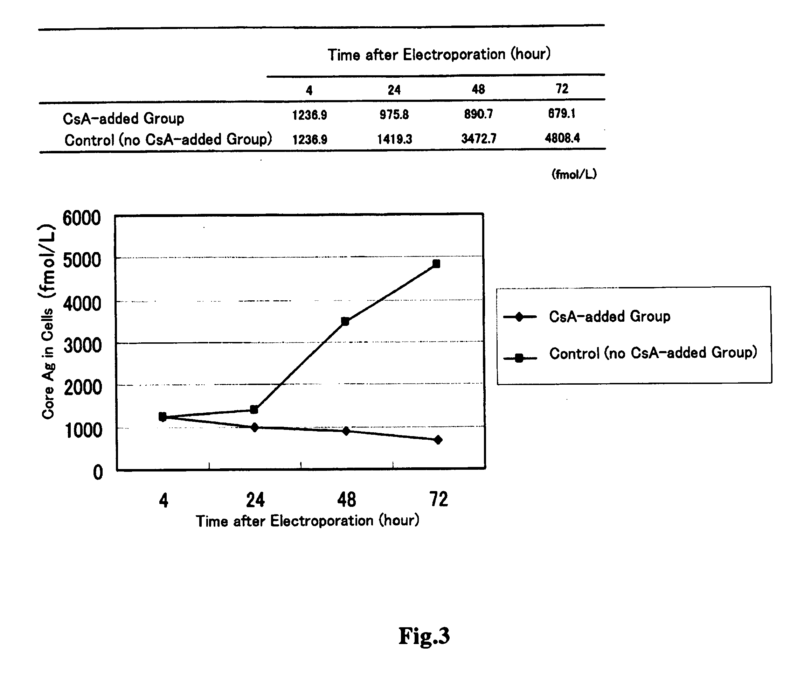

[Figure 3] Figure 3 shows inhibitory effects of cyclosporin A on the production of the core protein using the method for screening according to the present invention. The replicon RNA was introduced into cells with or without adding cyclosporin A, and 4, 24, 48, 72 and 96 hours later, the concentration of the core protein in the culture supernatant was measured.

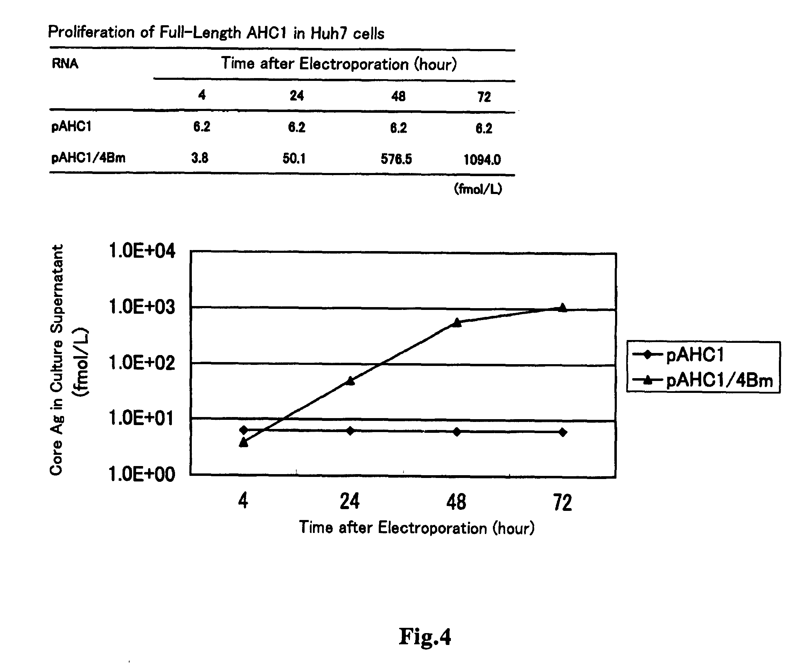

[Figure 4] Figure 4 shows the production of the core protein by introducing the replicon RNA according to the present invention into cells. pAHC1 and pAHC/4Bm were introduced into Huh-7 cells by electroporation, and 4, 24, 48 and 72 hours later, the concentration of the core protein in the culture supernatant was measured.

### BEST MODE FOR CARRYING OUT THE INVENTION

The best mode of the present invention will be described below but the present invention is by no means limited to this mode.
As long as the HCV gene according to the present invention is an HCV gene belonging to the genotype 1b it is not restricted. Preferably, the gene contains a polynucleotide coding for an NS4B protein according to the present invention. Also, the gene is preferred to be an HCV gene exhibiting interferon resistance.
The HCV gene can be classified into at least six types of the genotypes based on its nucleic acid sequence. Among them, HCV belonging to the genotype 1 is further classified into genotype 1a and genotype 1b. Specifically, the genotype of genotype 1b includes an HCV having a polynucleotide having a nucleic acid sequence having a homology of not less than 90% with the nucleic acid sequence shown in SEQ ID NO: 5 or 7.

Examples of the NS4B protein according to the present invention include a polypeptide having the amino acid sequence shown in SEQ ID NO: 6, SEQ ID NO: 8 or SEQ ID NO: 66. The HCV gene is composed of a region coding for a core protein, E1 protein and E2 protein, which are viral structural proteins; and a P7 protein, NS2 protein, NS3 protein, NS4A protein, NS4B protein, NS5A protein and NS5B protein, which are non-structural proteins, between the 5' untranslated region (5' UTR) and 3' untranslated region (3' UTR). The HCV gene, after infecting, functions as mRNA in host cells and a polyprotein with a length of about 3000 consecutive amino acids is synthesized. Thereafter, it is cleaved by a signal peptidase and signal peptidyl peptidase of the host cells as well as a protease encoded by the HCV genome, to yield the above-described three types of the structural proteins and seven types of the non-structural proteins.

Among these ten types of the HCV proteins, the NS4B protein forms a complex with other non-structural proteins from NS3 to NS5B, which complex forms an RNA replicating complex with proteins of the host cells. The RNA replicating complex is thought to replicate the genome RNA and plays an important role in viral replication. A polypeptide having the amino acid sequence shown in SEQ ID NO: 6 according to the present invention (hereinafter referred to as "TPF1-NS4B polypeptide") which is encoded by the NS4B region of the HCV gene obtained from patients with fulminant hepatitis, a polypeptide having the amino acid sequence shown in SEQ ID NO: 8 according to the present invention (hereinafter referred to as "TPF1-mutated NS4B polypeptide"), and a polypeptide having the amino acid sequence shown in SEQ ID NO: 66 according to the present invention (hereinafter also referred to as AHC1-mutated NS4B polypeptide), in particular the TPF1-mutated NS4B polypeptide and AHC1-mutated NS4B polypeptide exhibit prominent effects on the replication of the HCV gene.
Therefore, the HCV gene according to the present invention is an HCV gene preferably comprising a polynucleotide coding for TPF1-NS4B polypeptide or TPF1-mutated NS4B polypeptide, more preferably a polynucleotide having the nucleic acid sequence shown in SEQ ID NO:5 (herein after referred to as TPF1-NS4B polynucleotide), most preferably a polynucleotide having the nucleic acid sequence shown in SEQ ID NO:7 (TPF1-mutated NS4B polynucleotide) or a polynucleotide having the nucleic acid sequence shown in SEQ ID NO:65 (AHC1-mutated NS4B polynucleotide). However, the HCV gene according to the present invention is not restricted as long as it exhibits the prominent effects on the replication of the HCV gene. For instance, the HCV gene according to the present invention can be a HCV gene having a homology of preferably not less than 90%, more preferably not less than 95%, still more preferably not less than 97%, still more preferably not less than 99%, with the nucleic acid sequence shown in SEQ ID NO: 5, SEQ ID NO: 7 or SEQ ID NO: 65.

As long as the HCV gene according to the present invention contains a polynucleotide in an NS4B region, it is not restricted, and includes a partial HCV gene containing a partial polynucleotide of HCV and HCV gene having the full-length HCV polynucleotide.
Examples of the partial polynucleotide include nucleotides having an arbitrary nucleic acid sequence region in SEQ ID NO: 1, 3,10,61 or 63, and, in particular, partial nucleotides of 5'UTR (nucleic acid sequence from the first to the 341 st nucleotide), core (nucleic acid sequence from the 342nd to the 914th nucleotide), E1 region (nucleic acid sequence from the 915th to the 1490th nucleotide), E2 region (nucleic acid sequence from the 1491 st to the 2579th nucleotide), P7 region (nucleic acid sequence from the 2580th to the 2768th nucleotide), NS2 region (nucleic acid sequence from the 2769th to the 3419th nucleotide), NS3 region (nucleic acid sequence from the 3420th to the 5312th nucleotide), NS4A region (nucleic acid sequence from the 5313th to the 5474th nucleotide), NS4B region (nucleic acid sequence from the 5475th to the 6257th nucleotide), NS5A region (nucleic acid sequence from the 6258th to the 7598th nucleotide), NS5B region (nucleic acid sequence from the 7599th to the 9371st nucleotide) and 3' untranslated region (nucleic acid sequence from the 9372nd to the 9594th nucleotide). Also, examples of the HCV gene having the full-length HCV polynucleotide include HCV genes having the nucleic acid sequence shown in SEQ ID NO: 1, SEQ ID NO: 3, SEQ ID NO: 10, SEQ ID NO: 61, or SEQ ID NO: 63.

The HCV gene according to the present invention includes a gene isolated from a patient with fulminant hepatitis. Among hepatitis, fulminant hepatitis means one which develops the second-degree encephalopathy or more severe encephalopathy and a prothrombin time of not more than 40% within eight weeks after the onset of the disease. It divided into acute hepatitis C type which develops encephalopathy within ten days and subacute hepatitis C type in which encephalopathy appears after ten or more days.

Cloning of the HCV gene according to the present invention can, for example, be carried out as follows. Total RNA is prepared from the serum of a patient with fulminant hepatitis C using acidity guanidine isothiocyanate·phenol·chloroform method (for example, ISOGEN-LS, manufactured by Nippon Gene) or the like. cDNA is synthesized from the total RNA by a reverse transcription reaction using a 3' UTR specific primer and mouse leukemia virus reverse transcriptase (Superscript II, manufactured by Life technologies).

The synthesized HCV cDNA is amplified by PCR using specific primers from 5' UTR to 3' UTR (PCR Protocols, Academic Press (1990)). The amplified HCV cDNA is cloned into a pGEM-T EASY vector (manufactured by Promega) to determine the nucleic acid sequence.

Both termini of the HCV gene can be obtained by 5'-RACE using a 5' UTR specific primer and 3'-RACE using a 3' UTR specific primer (Proc. Natl. Acad. Sci. USA, 85, 8998 (1988)). The obtained cDNA fragments can be ligated together to obtain the full-length HCV genome.

The DNA according to the present invention is not restricted as long as it is a DNA corresponding to the above-described HCV gene which is RNA. An example includes double stranded DNA composed of a single strand cDNA synthesized with a reverse transcriptase from the HCV gene and a complementary strand of the single strand cDNA.

As long as the polypeptide according to the present invention is a polypeptide encoded by the polynucleotide having the nucleic acid sequence shown in SEQ ID NO: 1, 3, 10, 61, or 63, or a polypeptide containing the1804th leucine and the1966th lysine in the amino acid sequence of the HCV polyprotein, its region and length are not restricted. Preferably it is a polypeptide having the amino acid sequence shown in SEQ ID NO:6, SEQ ID NO:8, or SEQ ID NO:66.

The HCV protein according to the present invention includes a core protein having the amino acid sequence from the first to the 191 st amino acid in the amino acid sequence shown in SEQ ID NO: 2, 4, 11, 62 or 64, an E1 protein having the amino acid sequence from the 192nd to the 383rd amino acid, an E2 protein having the amino acid sequence from the 384th to the 746th amino acid, a P7 protein having the amino acid sequence from the 747th to the 809th amino acid, an NS2 protein having the amino acid sequence from the 810th to the1026th amino acid, an NS3 protein having the amino acid sequence from the 1027th to 1657th amino acid, an NS4A protein having the amino acid sequence from the 1658th to 1711th amino acid, an NS4B protein having the amino acid sequence from the 1712th to the 1972nd amino acid, an NS5A protein having the amino acid sequence from the 1973rd to the 2419th amino acid, or an NS5B protein having the amino acid sequence from the 2420th to the 3010th amino acid.

As long as the replicon RNA according to the present invention is RNA containing a polynucleotide having the nucleic acid sequence of the genotype 1b and having an ability to autonomously replicate in cells, it is not restricted. Examples of the nucleotide region involving in the replication of the HCV replicon RNA especially include the nucleotide regions coding for the 5' UTR, 3' UTR, and nonstructural proteins such as an NS3 protein, NS4A protein, NS4B protein, NS5A protein, and NS5B protein. In the replicon RNA according to the present invention, all of these regions are important but the region coding for the NS4B protein is important in terms of increasing replication efficiency. In particular, the NS4B protein is preferably the TPF1-NS4B polypeptide which is a polypeptide having the amino acid sequence shown in SEQ ID NO:6, more preferably the TPF1-mutated NS4B polypeptide which is a polypeptide having the amino acid sequence shown in SEQ ID NO:8, or the AHC1-mutated NS4B polypeptide which is a polypeptide having the amino acid sequence shown in SEQ ID NO:66.

Therefore, the replicon RNA according to the present invention is preferably a replicon RNA containing a polynucleotide coding for the TPF1-NS4B polypeptide, in particular a polynucleotide having the nucleic acid sequence shown in SEQ ID NO: 5 (TPF1-NS4B polynucleotide), more preferably a replicon RNA containing a polynucleotide coding for the TPF1-mutated NS4B polypeptide, in particular a polynucleotide having the nucleic acid sequence shown in SEQ ID NO: 7 (TPF1-NS4B mutated polynucleotide) or a polynucleotide coding for the AHC1-mutated NS4B polypeptide, in particular a polynucleotide having the nucleic acid sequence shown in SEQ ID NO: 65 (AHC1-mutated NS4B polynucleotide). However, the replicon RNA according to the present invention is not restricted to the above-described replicon RNA containing the polynucleotide of the NS4B region and includes a replicon RNA containing a polynucleotide having a nucleic acid sequence having a homology of preferably not less than 90%, more preferably not less than 95%, still more preferably not less than 97%, most preferably not less than 99%, with the nucleic acid sequence shown in SEQ ID NO:5, SEQ ID NO:7 or SEQ ID NO:65.

The TPF1-mutated NS4B polynucleotide (SEQ ID NO: 7) is a polynucleotide wherein the 278th nucleotide A is substituted with U and the 763rd nucleotide G is substituted with A in the TPF1-NS4B polynucleotide (SEQ ID NO: 5). The TPF1-mutated NS4B polypeptide (SEQ ID NO: 8) is a polypeptide wherein the 93rd amino acid glutamine (Q) is substituted with leucine (L) and the 255th amino acid glutamic acid (E) is substituted with lysine (K) in the TPF1-NS4B polypeptide (SEQ ID NO: 6).

In a mode of the HCV gene and replicon RNA according to the present invention, nucleotides coding for the1804th amino acid leucine and the 1966th amino acid lysine in the amino acid sequence of the HCV polyprotein can be included.
The positions of the 1804th amino acid leucine and the1966th amino acid lysine are positions in HCV genotype 1b gene composed of 3010 amino acids.

The1804th amino acid leucine and the1966th amino acid lysine are amino acids contained in the NS4B protein. Thus far, an NS4B protein containing these amino acids has not been reported. Hence, an HCV polyprotein containing these amino acids and RNA replicon containing a polynucleotide coding for these amino acids have not been reported.

Although a nucleic acid sequence of the HCV genotype 1b gene is not particularly restricted, it includes, for example, a nucleic acid sequence having a homology of not less than 90% with the nucleic acid sequence shown in SEQ ID NO:1, SEQ ID N0:3, SEQ ID NO:10, SEQ ID NO:61, or SEQ ID NO:63.

As long as the replicon RNA according to the present invention can replicate in cells, its structure is not restricted. Examples include those containing the full-length HCV RNA and a subgenomic replicon RNA containing a part of the RNA. For instance, the subgenomic replicon RNA can contain a nucleotide region coding for the 5' untranslated region (hereinafter also referred to as 5' UTR), the 3' untranslated region (hereinafter also referred to as 3' UTR), and the NS3 protein, NS4A protein, NS4B protein, NS5A protein and NS5B protein, which are nonstructural proteins, preferably a polynucleotide having the nucleic acid sequence from the first to the 341 st nucleotide in the nucleic acid sequence shown in SEQ ID NO:1, SEQ ID NO:3, SEQ ID NO:61 or SEQ ID NO:63; and a polynucleotide having the nucleic acid sequence from 3420th to the 9564th nucleotide in the nucleic acid sequence shown in SEQ ID NO:1, SEQ ID NO:3, SEQ ID NO:61 or SEQ ID NO:63.

5' UTR is usually composed of 341 nucleotides in the HCV genotype 1b gene. Although the nucleic acid sequence of 5' UTR contained in the replicon RNA is not restricted, it preferably contains its entire length of the sequence. The length of the 3' UTR varies depending on the viral strain. 3' UTR is usually composed of variable region with 41 nucleotides, a poly U region whose length varies depending on the strain and 3' X region with 98 nucleotides. Although the nucleic acid sequence and length of 3'UTR contained in the replicon RNA is not restricted, it preferably contains the entire length of 3'UTR in the strain.

Also, in addition to the full-length RNA and subgenomic RNA, a selection marker gene, reporter gene, or IRES sequence may be contained. Examples of such a replicon RNA include a replicon RNA having the polynucleotide shown in SEQ ID NO:9, SEQ ID NO:67, or SEQ ID NO:68.

Examples of the selection marker include antibiotic resistance genes. Examples of the preferred selection marker genes in the present invention include neomycin resistance genes, thymidine kinase genes, kanamycin resistance genes, pyrithiamin resistance genes, adenylyl transferase genes, zeocin resistance genes and puromycin resistance genes. The neomycin resistance genes and thymidine kinase genes are preferred and the neomycin resistance genes are most preferred. However, the selection marker gene in the present invention is not limited thereto.

An example of the reporter gene includes structural genes of an enzyme which catalyzes a luminescent reaction and coloring reaction. Examples of the preferred reporter gene in the present invention include chloramphenicol acetyltransferase genes derived from transposon Tn9, β-glucuronidase or β-galactosidase genes derived from *E. coli,* luciferase genes, green fluorescent protein genes, aequorin genes derived from jellyfish and secreted placental alkaline phosphatase (SEAP) genes. However, the reporter gene according to the present invention is not limited thereto.

Examples of the IRES sequence include, but are not limited to, EMCV IRES (internal ribosome entry site of an encephalomyocarditis virus), FMDV IRES, HCV IRES and the like. EMCV IRES and HCV IRES are preferred and EMCV IRES is most preferred.

The replicon RNA according to the present invention is preferably resistant to interferon. When a patient with HCV is treated with interferon, whether interferon is effective or not is thought to depend on, for example, a factor attributed to the virus and a factor attributed to the host. The factor attributed to the virus includes an HCV gene region exhibiting the interferon resistance. The replicon RNA according to the present invention preferably contains the HCV gene area exhibiting the interferon resistance. The HCV gene area exhibiting the interferon resistance is not particularly restricted and can be, for example, an ISDR region which is thought be an index for IFN sensitivity in an NS5A region.

The replicon RNA according to the present invention is preferably an RNA replicon containing a polynucleotide having the nucleic acid sequence of the genotype 1b but includes, for example, a RNA replicon containing a polynucleotide having a nucleic acid sequence having a homology of not less than 90% with the nucleic acid sequence shown in SEQ ID NO:5, SEQ ID NO:7, or SEQ ID NO:65.

As long as the DNA according to the present invention is a form of linear DNA and DNA coding for the above-described replicon RNA, it is not restricted. It can, for example, contain an RNA promoter to generate a replicon RNA.

The replicon RNA according to the present invention can be produced using an arbitrary gene engineering technique. Although it is not restricted, the replicon RNA can, for example, be produced by the method below. A DNA coding for the above-described replicon RNA is inserted into a cloning vector by a conventional method to produce a DNA clone. This DNA is inserted downstream of an RNA promoter to produce a DNA clone capable of generating a replicon RNA. The above-described RNA promoter is preferably one contained in a plasmid clone. Examples of the RNA promoter include, but are not limited to, T7 RNA promoters, SP6 RNA promoters and SP3 RNA promoters with particularly the T7 RNA promoters being preferred.

A vector into which the DNA is to be inserted is not particularly restricted and examples thereof include plasmid vectors, linear double-stranded DNA vectors and virus vectors such as adenovirus vectors, adeno-associated virus vectors, retroviral vectors and lentivirus vectors. The plasmid vectors are preferred.

The replicon RNA according to the present invention can be produced from the above-described vector into which the DNA is inserted. The RNA is synthesized with the DNA clone as a template using an RNA polymerase. The RNA synthesis can be started from the 5' untranslated region by a conventional method. In cases where a template DNA is a plasmid clone, the above-mentioned DNA region linked to the downstream of the RNA promoter can be excised from the plasmid clone using a restriction enzyme and the RNA is synthesized with the resulting DNA fragment as a template. The 3' terminus of the synthesized RNA is preferably identical to the 3' untranslated region of virus genome RNA. It is preferred that other sequence be not added or deleted. For instance, in the case of a preferred mode of the full-length replicon RNA according to the present invention, DNA is inserted into a vector having a T7 RNA promoter upstream of 5'UTR and a restriction enzyme *Xba* I site in the 3'UTR terminus. After digestion with *Xba* I, an HCV genome RNA can be synthesized using a T7 RNA polymerase.

The replicon-replicating cells according to the present invention can be prepared by introducing the above-described RNA replicon into arbitrary cells. Cells into which the replicon RNA is introduced are not particularly restricted and preferably are cells derived from human liver, cells derived from mouse liver or cells derived from monkey liver. Examples of the cells particularly include Huh7 cells, HepG2 cells, or Hep3B cells which are cells derived from human hepatocarcinoma, or IMY-N9 cells, HeLa cells, CHO cells, COS cells, Vero cells and 293 cells. The introduction of the replicon RNA into the cells can be carried out by an arbitrary transfection method. Examples of such an introduction method include electroporation, particle gun methods and lipofection methods. A method by the electroporation is especially preferred.

When a replicon RNA containing a selection marker gene or reporter gene for the introduction into cells is used, cells in which the replicon RNA is introduced and replicating continuously, can be selected using expression of the selection marker gene or reporter gene. For instance, in cases where a neomycin resistance gene is contained in the replicon RNA as the selection marker gene, cells into which the replicon RNA is transfected are plated in a culture dish, and G418 (neomycin) is added at a concentration of 0.05 mg/ml to 3.0 mg/ml. Thereafter, the medium is changed twice a week to continue the culture. At two to three weeks after plating, the resulting cells can be visualized as a colony.

The replicon-replicating cells according to the present invention produce a replicon RNA, HCV protein and HCV particle. Therefore, by using the replicon-replicating cells, the replicon RNA, HCV protein and HCV particle can be produced.

The replicon RNA which replicates in the replicon-replicating cells can be extracted from the cells using an arbitrary RNA extraction method. The RNA extracted from the cells can be made to function as a replicon RNA by being again introduced into the other cells. The HCV protein according to the present invention can be used one secreted in the cells or into a culture supernatant. The produced HCV protein can be extracted and purified using a known method. Also, as for the HCV particles produced by replicon-replicating cells, those secreted in the cells or into the culture supernatant can be used. The HCV protein and HCV particle according to the present invention can be used as a vaccine by adding modifications to the replicon RNA to modify the RNA, virus protein or virus particle and to weaken pathogenicity.

By using the above-described replicon-replicating cells, a substance to control the infection of HCV can be screened. "To control the infection of HCV" means for example to control (e.g. to promote or to suppress) the replication of HCV RNA and to control (e.g. to promote or to suppress) the translation from RNA to a protein. Concretely, by contacting the replicon-replicating cells with a test substance and analyzing the degree of increase of the replicon RNA, the screening of the test substance can be carried out. The degree of increase of the replicon RNA means a change of the replication rate or amount of the replicon RNA. Concretely, by detecting or measuring the amount of the replicon RNA in replicon-replicating cells, and comparing with the amount of the replicon RNA in the replicon replicating cells without contacting with the test substance, which cells are a control, the test substance can be screened. Also, by detecting or measuring the amount of the HCV protein in cells or the supernatant, and comparing with that in control replicon replicating cells which do not contact with the test substance, the test substance can be screened. The HCV protein which can be detected or measured in the screening is not particularly restricted and is preferably a core protein. The core protein can be measured using a commercially available kit. In addition, by automating the screening method, adaptation to a high-throughput screening method is possible.

Further, the screening method according to the present invention is useful as a method for evaluating effects of the screened drug. In cases where the evaluation of the drug needs to be carried out by this screening method, it can be used as a method for producing a drug.

### «Action»

The HCV gene and replicon RNA containing the nucleotides coding for the1804th amino acid leucine and the1966th amino acid lysine in the amino acid sequence of the HCV polyprotein are not completely clarified but can be speculated as follows. Yet, the present invention is by no means limited to the description below.
The present inventors repeated the operation in the Example 9 using the genotype 1b strain coding for other 3010 amino acids, instead of the AHC1 strain used in the Example 9 described later, and obtained the same results as in Example 9. As a subgenomic replicon having an adaptive mutation other than the NS4B protein, a replicon with the 5308th base T being mutated to C and the 1656th amino acid V (valine) in 3010 amino acids of the HCV polyprotein being mutated to A (alanine); and a replicon with, in addition to the above mutation, the 6846th base A being mutated to G and the 2169th amino acid T (tyrosine) in 3010 amino acids of the HCV polyprotein being mutated to A (alanine) were obtained.

From the experimental results obtained from the TPF1 strain, AHC1 strain and the above-described genotype 1b strain, the following can be considered. The replicon RNA of the genotype 1bcontaining the nucleotides coding for the 1804th leucine and the 1966th lysine increases in RNA replication efficiency, compared with the replicon RNA without these nucleotides. Thus, since the replicon RNA of HCV genotype 1b has these two adaptive mutations, the RNA replication efficiency increases.
By transfecting cells using the replicon RNA having the above-described two adaptive mutations in the above-described NS4B protein, the replicon-replicating cells can be obtained with certainty. The replicon RNA obtained from these replicon-replicating cells may be introduced with one or more other adaptive mutations in addition to the above-mentioned two adaptive mutations in the NS4B protein. Therefore, by introducing one or more other adaptive mutations in addition to the above-mentioned two adaptive mutations in the NS4B protein, the replication efficiency of the replicon RNA may increase.

The adaptive mutations other than two of the above-described adaptive mutations in the above-described NS4B protein include known adaptive mutations and unknown adaptive mutations. As the known adaptive mutations, mutations shown in Table 1 have been reported.

**[Table1]**

| Region | Position of aa | aa |
|---|---|---|
| NS3 | 1115 | P→L |
| | 1133 | V→I |
| | 1202 | E→G |
| | 1261 | T→S |
| | 1268 | M→V |
| | 1280 | T→I |
| | 1283 | R→G |
| | 1287 | T→A |
| | 1304 | G→S |
| | 1383 | E→A |
| | 1452 | I→L |
| | 1577 | K→R |
| | 1609 | K→E |
| NS4A | 1691 | K→R |
| NS4B | 1846 | K→T |
| | 1897 | V→L/M/A |
| | 1963 | P→S |
| NS5A | Ins2041 | K |
| | 2043 | S→Y |
| | 2163 | E→G |
| | 2177 | D→N/G |
| | 2189 | R→G |
| | 2196 | P→S |
| | 2197 | S→P/C |
| | 2199 | A→T/D/S |
| | 2202 | del S |
| | 2204 | S→I/R |
| | 2330 | K→E |
| | 2371 | Q-R |
| | 2379 | A→V |
| | 2411 | E→K |
| NS5B | 2442 | I→V |
| | 2884 | R→G |
| | 2933 | Q→R |
| | 3004 | I→T |

### EXAMPLES

### «Example 1: Isolation and Analysis of Full-Length Fulminant Hepatitis C Virus Gene»

### (A) Extraction of RNA from serum

RNA was purified from the serum drawn from a patient at the acute stage of fulminant hepatitis (250 µl) using High Pure Viral Nucleic Acid Kit (Roche diagnostics corporation) in accordance with the method recommended by the manufacturer.

### (B) Synthesis of cDNA and Amplification of cDNA by PCR

To the purified RNA, was added XR58R primer to carry out a reverse transcription reaction at 42°C for an hour using SuperSucript II reverse transcriptase (Invitrogen) in accordance with the method recommended by the manufacturer, thereby obtaining cDNA. To the obtained reaction solution, was added RNaseH (Invitrogen) and the mixture was allowed to react at 37°C for 30 minutes, to digest RNA. This reaction solution was subjected to polymerase chain reaction (PCR) involving 30 rounds of a thermal cycle reaction using HC-LongAl primer and 1b9405R primer with Takara LA Taq DNA polymerase (Takara Shuzo), the thermal cycle reaction being composed of 94°C for 20 seconds and 68°C for nine minutes, thereby amplifying cDNA. Further, an aliquot of the obtained reaction solution was subjected to PCR using HC85F and HC9302R primers to amplify HCV cDNA.

### (C) Cloning of cDNA

The amplified DNA fragment was separated by electrophoresis using 0.7% agarose gel and the DNA fragment was collected using QIAquick gel purification kit (QIAGEN) in accordance with the method recommended by the manufacturer. The collected DNA fragment was subjected to a ligation reaction with pGEM-T easy vector (Promega) and DH5a strain was transformed by the resulting plasmid. Ampicillin-resistant transformants were selected and cultured using 2YT culture medium. The plasmid was purified from the cultured bacteria using Wizard Plus SV Miniprep DNA Purification System.

### (D) Determination of the nucleic acid sequence.

The nucleic acid sequence of HCV cDNA was determined using a primer designed based on the sequence of the genotype 1b of HCV. Using CEQ DTCS Quick Start Kit (Beckman Coulter), a reaction was carried out in accordance with the method by the manufacturer and an analysis was carried out using CEQ2000 XL DNA analysis system (Software version 4.0.0, Beckman Coulter). The obtained data was analyzed using Sequencher (Version 4.1.2, Gene Codes Corporation). The obtained HCV clone was named pTPF1-0193.

### (E) Cloning of cDNA of 5' Untranslated Region and Determination of nucleic acid Sequence Thereof

Further, from the RNA obtained in the step according to the above-described (A), cDNA of the terminus of 5' untranslated region was obtained by 5'RACE method, which was carried out using a kit of 5'RACE System for Rapid Amplification of cDNA Ends, Version2.0 (Invitrogen) in accordance with the attached instructions. Chiba-as was used as the antisense primer for the cDNA synthesis. cDNA was synthesized using SuperScript II Reverse Transcriptase (Invitrogen). After purified with S.N.A.P column, cDNA was subjected to a TdT-tailing reaction and dCTP was then added to the resultant. With 5'RACE Abridged Anchor primer which came in the kit and KY78 primer, the first PCR was carried out using Takara LA Taq DNA polymerase (Takara Shuzo). Using an aliquot of this PCR product as a template, and with UTP primer which came in the kit and KM2 primer, the second PCR was carried out using Takara LA Taq DNA polymerase (Takara Shuzo), thereby obtaining a PCR product. This PCR product was cloned into the pGEM-T easy vector. The nucleic acid sequence was determined in accordance with the step according to the above-described (D). An HCV cDNA clone containing the nucleotide from the first to the 709th in the obtained SEQ ID NO: 1 was named pTPF1-0007.

### (F) Cloning of cDNA of 3' Untranslated Region and Determination of nucleic acid Sequence Thereof

From the RNA obtained in the step according to the above-described (A), cDNA of the terminus of 3' untranslated region was obtained by 3'RACE method. First, to the RNA from the patient, was added Poly(A) using Poly(A) Tailing Kit (Ambion) in accordance with the attached instructions. The above-described steps (B) to (D) were repeated except that dT-Adp primer was used instead of the XR58R primer, 3UTR-1F and Adp primers were used as the primers for the first PCR, and XR58F and Adp primers were used as the primers for the second PCR. The obtained HCV cDNA clone was named pTPF1-8994.

The obtained HCV strain was named TPF1 strain. The TPF1 strain is an HCV with the full-length of 9594 bases and the nucleic acid sequence is shown in SEQ ID NO: 1. The polynucleotide of the obtained TPF1 strain had a translation region coding for consecutive 3010 amino acids between the 342th and the 9374th base. The amino acid sequence of the polyprotein of the TPF1 strain is shown in SEQ ID NO: 2.

The primers used for cloning and determining the nucleic acid sequence are shown below.
XR58R (SEQ ID NO: 12): 5'-tcatgcggct cacggacctt tcacagctag-3'
HClongA1 (SEQ ID NO: 13): 5'-atcgtcttca cgcagaaagc gtctagccat-3'
1b9405R (SEQ ID NO: 14): 5'-gcctattggc ctggagtgtt tagctc-3'
HC85F (SEQ ID NO: 15): 5'-atggcgttag tatgagtgtc gtgcagcct-3'
HC9302R (SEQ ID NO: 16): 5'-tcgggcacga gacaggctgt gatatatgtc t-3'
chiba-as (SEQ ID NO: 17): 5'-tgcacggtct acgagacct-3'
KY78 (SEQ ID NO: 18): 5'-ctcgcaagca ccctatcagc cagt-3'
KM2 (SEQ ID NO: 19): 5'-aggcattgag cgggtttat-3'
dT-Adp (SEQ ID NO: 20): 5'-ctagactcga gtcgacatcg tttttttttt tttttttt-3'
3UTR-1F (SEQ ID NO: 21): 5'-atcttagccc tagtcacggc-3'
Adp (SEQ ID NO: 22): 5'-ctagactcga gtcgacatcg-3'
XR58F (SEQ ID NO: 23): 5'-ctagctgtaa aggtccgtga gccgcatga-3'
M13 Primer M3 (SEQ ID NO: 24): 5'-gtaaaacgac ggccagt-3'
M13 Primer RV (SEQ ID NO: 25): 5'-caggaaacag ctatgac-3'
104 (SEQ ID NO:26): 5'-aggaagactt ccgagcggtc-3'
HC841 S (SEQ ID NO:27): 5'-ggaacttgcc cggttgctct ttctctatct tc-3'
E1 (SEQ ID NO:28): 5'-attccatggt ggggaactgg gctaa-3'
HC2069S (SEQ ID NO:29): 5'-taacaatacc ttgacctgcc ccacggactg-3'
HC2430S (SEQ ID NO:30): 5'-aacatcgtgg acgtgcaata cctgtacgg-3'
HC2461 AS (SEQ ID NO:31): 5'-gaccctacac cgtacaggta-3'
HC2769S (SEQ ID NO:32): 5'-ttggaccggg agatggctgc atcgtg-3'
HC3632F (SEQ ID NO:33): 5'-cacccaaatg tacaccaatg t-3'
HC3928S (SEQ ID NO:34): 5'-tacccgttga gtctatggaa ac-3'
HC4016AS (SEQ ID NO:35): 5'-cacttggaat gtctgcggta-3'
HC4498S (SEQ ID NO:36): 5'-agggggggag gcatctcatt ttctg-3'
HC4888F (SEQ ID NO:37): 5'-tgctatgacg cgggctgtgc ttggta-3'
HC5381F (SEQ ID NO:38): 5'-ggtcattgtg ggcaggatca t-3'
HC5692S (SEQ ID NO: 39): 5'-ctgcctggaa accccgcgat-3'
HC5858F (SEQ ID NO: 40): 5'-tggcagcata ggccttggga aggt-3'
HC6315F (SEQ ID NO: 41): 5'-aagacctggc tccagtccaa g-3'
5A- (SEQ ID NO: 42): 5'-ttccatgctc accgacccct c-3'
HC7090S (SEQ ID NO: 43): 5'-gtggagtcag agaataaggt-3'
HC7743F (SEQ ID NO: 44): 5'-cagaagaagg tcacctttgac-3'
HC8192S (SEQ ID NO: 45): 5'-gcagcgggtc gagttcctgg tgaat-3'
HC8939F (SEQ ID NO: 46): 5'-ctacggggcc tgttactcca ttgaac-3'

### «Example 2: Preparation of subgenomic RNA replicon»

The full-length of the polynucleotide of the hepatitis C virus TPF1 strain was inserted into downstream of a T7 RNA promoter sequence in pBluescriptIISK(+) (hereinafter referred to as pTPF1).

Next, a region coding for a structural protein of pTPF1 and a part of a region coding for a nonstructural protein were replaced with a neomycin resistance gene (neomycin phosphotransferase, NPT-II) and EMCV-IRES (internal ribosome entry site of an encephalomyocarditis virus), thereby constructing plasmid DNA pRepTPF 1. This construction procedure was carried out in accordance with a reported method (Lohmann et al., Science, (1999) 285, p.110-113).

Specifically, pTPF1 was first digested with restriction enzymes, *Age* I and *Bsr* GI. To the resulting cleavage site, a PCR-amplified fragment from 5'UTR to the core region derived from pTPF1 and the neomycin resistance gene derived from pcDNA3.1(+), which fragment was digested with restriction enzymes, *Age* I and *Pme* I, and a PCR-amplified fragment from EMCV-IRES to NS3 region, which fragment was digested with restriction enzymes, *Pme* I and *Bsr* GI, were inserted and ligated. RNA was synthesized with this plasmid DNA pRepTPF1 digested with *Xba* I as a template using Megascript T7 kit (Ambion). The RNA was purified in accordance with the method recommended by the manufacturer.

Human hepatocarcinoma cells (Huh7, JCRB0403) were cultured in Dulbecco's modified Eagle medium (D-MEM, IWAKI) containing 10% fetal bovine serum (FBS) with penicillin and streptomycin (50 U/mL and 50 µg/mL, respectively), under 5% CO2 conditions at 37°C. The cells before confluency were detached from the culture dish using a trypsin-EDTA treatment and resuspended in medium containing serum to inactivate trypsin. After washing twice with PBS, the cells were resuspended in Cytomix (120 mM Potassium chloride, 10 mM Potassium phosphate, 5 mM Magnesium chloride, 25 mM HEPES, 0.15 mM Calcium chloride, 2 mM EGTA, pH 7.6) and 1.25% DMSO was added, followed by transferring the mixture into an electroporation cuvette with a gap of 0.4 cm.

An appropriate amount of RNA is added to the cells and the mixture is then sufficiently cooled on ice for five minutes. A pulse is applied at 960 uF and 250 V using an electroporator (Bio-Rad). Immediately, the cells were resuspended in 8 ml of medium and a part of them is spread on a plate. After incubation for a certain period of time, G418 (neomycin) was added to the culture plate at a concentration of 1 mg/ml. Thereafter, the culture was continued with the culture medium being changed every four days. A colony of surviving cells was cloned from the culture plate about 20 days after plating, and the culture was continued. Such cloning of the colony made it possible to establish cells in which pRepTPF1 replicon RNA autonomously replicates. Whether or not the replicon RNA replicates was analyzed by a quantitative RT-PCR method measuring the copy number of replicating replicon RNAs contained in cellular RNAs.

### Method for Quantifying Minus Strand

Whether or not the autonomous replication of the replicon RNA took place was checked if the minus strand of the 5'UTR region of HCV RNA in the cells can be detected or not. The method for specifically quantifying the minus strand was carried out in the same manner as the method for specifically detecting the minus strand RNA, which method is described in Japanese Patent Application No. 08-187097.
The statistically significant amount of the minus strand was detected from the cells into which RNA was introduced by electroporation, the RNA being synthesized *in vitro* using pRepTPF-1 as a template. Thus it was confirmed that the replicon RNA autonomously replicated in the cells.

### «Example 3: Analysis of Adaptive Mutations»

Intracellular RNAs were extracted from the replicon RNA-replicating cell line using ISOGEN (Nippon Gene) in accordance with the condition recommended by the manufacturer, which cell line was established by transfecting RNA synthesized *in vitro* with pRepTPF1 as a template into Huh7 cells in accordance with Example 2.

DNA for the almost entire region of the replicon RNA was amplified from this intracellular RNA in the same manner of obtaining the gene from TPF1 as described in Example 1. Specifically, cDNA corresponding to the replicon RNA was synthesized with the extracted intracellular RNA as a template using SuperSucript II reverse transcriptase (Invitrogen) and XR58R primer.

Using an aliquot of this cDNA, a polymerase chain reaction (PCR) was carried out in the presence of EMCV-S1 primer: 5'-tgcacatgct ctacatgtgt ttagtcgagg-3' (SEQ ID NO: 60) and HC9405R primer, using Takara LA Taq DNA polymerase (Takara Shuzo), which PCR involved 30 rounds of a thermal cycle reaction composed of 94°C for 20 seconds and 68°C for 6 minutes, thereby amplifying cDNA. When the nucleic acid sequence of the clone cloned into the pGEM-T easy vector was determined, it was found that the 5752nd base A was substituted with T and the 6237th base G was substituted with A. Consequently, Q (glutamine) at the amino acid corresponding to the amino acid number 1804 in SEQ ID NO: 2 was mutated to L (leucine) and E (glutamic acid) at the amino acid corresponding to the amino acid number 1966 was mutated to K (lysine).

Next, effects of the above-described amino acid substitution on the replication of the replicon RNA were examined. First, the adaptive mutations at the amino acid number 1804 (from Q to L) and at the amino acid number 1906 (from E to K) were introduced into the HCV RNA replicon pRepTPF1 prepared in Example 2 using , Quick Mutagenesis Kit (Stratagene) in accordance with the method recommended by the manufacturer. A replicon RNA in which this amino acid substitution was introduced was named pRep4B.

RNA was synthesized using pRepTPF1 which did not have a nucleic acid sequence causing the mutations and pRep4B having the amino acid mutations, both of which plasmid DNAs were cleaved with *Xba* I, as templates using Megascript T7 kit (Ambion). RNA was purified in accordance with the method recommended by the manufacturer. Each of the purified RNAs was transfected into Huh7cells. The cells were cultured for about 20 days in the presence of G418 and surviving cells were stained with crystal violet. The number of the stained cells was measured to calculate the number of colony per 1 µg of the amount of the replicon RNA transfected.

When 1 µg of RepTPF1 RNA was transfected, one G418 resistant colony was selected whereas 10⁴ colonies were selected when 1µg of Rep4B RNA was transfected. Hence, the nucleic acid mutations which caused the amino acid mutations in the replicon were thought to be adaptive mutations which increased the replication efficiency of the replicon RNA in Huh7 cells.

### «Example 4: Effects of Adaptive Mutations on HCV RNA Replication »

The full-length HCV DNA pTPF1 prepared in Example 2 was digested with a restriction enzyme, *Sfi* I. A fragment obtained by digesting pRep4B with the restriction enzyme, *Sfi* I was inserted and ligated to the region of the cleavage, thereby preparing a full-length HCV DNA pTPF1/4B in which the adaptive mutations were inserted.

The replication efficiency of a full-length HCV RNA synthesized from pTPF1/4B in which the adaptive mutations were inserted was compared with the case of pTPF1. Specifically, the same method as described in Example 2 was carried out. The full-length HCV RNA was synthesized *in vitro* and transfected into Huh7 cells. The transfected cells were immediately resuspended in 10 ml of the culture medium and plated 1 ml each in a 12-well plate (diameter 22.1 mm) to start a culture. Four hours, 24 hours, 48 hours and 72 hours later, culture supernatant was collected. The collected culture supernatant was centrifuged at 2k rpm for 10 minutes and supernatant was collected. The supernatant (100 µl) was measured using a kit for HCV core antigen (FUJIREBIO, Lumipulse).

As shown in Figure 1, the measured value of the core antigen in the supernatant of the pTPF1/4B in which the adaptive mutations were introduced was higher at any point, compared with the case of the pTPF1 which did not have the adaptive mutations and served as a control. This indicates that, by introducing the adaptive mutations according to the present invention into the full-length HCV RNA replicon, replication occurs at a high efficiency in cells and the core protein is secreted into the supernatant. It indicates that a full-length genome similar to the structure replicating in the liver is replicable *in vitro.*
In particular, it is thought that, in the TPF1-NS4B polypeptide according to the present invention, by using the polynucleotide coding for the TPF1-mutated NS4B polypeptide having the above-described adaptive mutations as the replicon RNA, the replication efficiency of the RNA increases.

### «Example 5: Construction of HCV Particle Capable of Reinfection»

Whether or not the core antigen secreted into the culture medium in Example 4 was capable of forming the virus particle and of *in vitro* reinfection was examined. Specifically, the full-length HCV RNA synthesized from pTPF1/4B was transfected into Huh7 cells. After culturing the cells for 72 hours, culture supernatant was collected. The collected culture supernatant was centrifuged at 2k rpm for 10 minutes and then filtered (0.45 µm, Millipore) to remove broken cells and the like.

The filtered supernatant was allowed to react with Huh7 cells cultured in a 12-well plate (diameter 22.1 µm) for three hours at 4°C. After the reaction, the plate was transferred to an incubator with 5% CO2 conditions at 37°C to culture the cells. Four hours, 24 hours, 48 hours, 72 hours and 96 hours later, the cells were detached by 1 mM EDTA-PBS and collected by centrifugation. Cell pellet was dissolved in 50µl of RIPA buffer (20 mM Tris-HCl (pH 7.5), 150 mM NaCl, 1 mM EDTA, 1% NP40, 0.1 % Deoxycholate, 0.1 % SDS, Complete protease inhibitor cocktail (Roche diagnostics corporation) and supernatant was collected by centrifugation at 10k rpm for 5 minutes. The supernatant (5 µl) was measured using a kit for the HCV core antigen (FUJIREBIO, Lumipulse).

As shown in Figure 2, the core antigen in the cells treated with the culture supernatant of pTPF1/4B exhibited a decrease once from the 4 hour to the 24 hour after the start of the culturing. Thereafter, it began to increase at the 48 hour and remained increasing at the 96 hour. This indicates that in the culture supernatant into which the full-length HCV RNA according to the present invention replicated in the cells and was secreted, the virus particles capable of reinfecting naive Huh7 cells are contained.

### «Example 6: Cloning of HCV Full-Length Genome from Patients with Interferon-Sensitive Acute Hepatitis»

Cloning of the HCV full-length genome from patients with acute hepatitis for whom an interferon treatment was effective was attempted. RNA was extracted from patient's serum using an RNA extracting reagent, ISOGEN-LS (Nippon Gene), in accordance with the appended instructions.

The HCV gene from the 85th to the 9302nd nucleotide was obtained from this RNA in the same manner as described in Example 1 in which the full-length genome was obtained from TPF1. The obtained RNA was cloned into the pGEM-T easy vector. When the nucleic acid sequence was determined, it was found to be a typical full-length genome belonging to the genotype 1b.

Subsequently, the nucleic acid sequence of the 3' untranslated region was determined. To the extracted RNA (2.5 µl), was added 5 pmole (0.5 µl) of primer 8913F, and the mixture was kept at 70°C for 3 minutes and quickly cooled on ice. To the resultant, was added 5x First-Strand Buffer 2 µl, 0.1 M DTT 1 µl, 20 mM dNTP 0.5 µl, RNase Inhibitor (Takara Shuzo) 20 units and SuperSucript II reverse transcriptase (Invitrogen) 0.5µl, and then sterilized water treated with diethylpyrocarbonate was added to the mixture to attain a final volume of 10µl. This mixture was allowed to react at 42°C for 60 minutes. To digest RNA, 12 U of RNaseH (Takara Shuzo, 60 U/µl) was added to the mixture. The mixture was kept at 37°C for 30 minutes, followed by incubation at 72°C for three minutes to inactivate the RNaseH. The resultant was used as cDNA.

This cDNA (2µL) was subjected to PCR using primers 8913F and RP2 in the same manner as described above. An aliquot of this PCR product was subjected to the second PCR using 8939F and R1 primers, thereby obtaining a PCR product with about 600 bases. This PCR product was cloned into the pGEM-T Easy vector to determine the sequence.

On the other hand, the 5' terminus of the HCV cDNA was isolated and the nucleic acid sequence was determined as follows: An aliquot of the cDNA reaction mixture treated with the above-described RNaseH was subjected to PCR using HCLongH1 and HC705R with Takara EX Taq DNA polymerase (Takara Shuzo), which PCR involved 35 repeated rounds of a thermal cycle composed of 94°C for 20 seconds, 55°C for 30 seconds and 72°C for 1 minute, to amplify a fragment corresponding to the nucleotides from the first to the 709th nucleotide of the HCV cDNA previously reported. This PCR product was cloned into the pGEM-T Easy vector to determine the sequence.

By the operations described above, the entire virus genome was obtained. This was named AHC1 strain. The AHC1 strain had a full-length of 9594 nucleotide and the determined nucleic acid sequence of the entire virus genome had a translated region coding for 3010 consecutive amino acids between the 342nd and the 9374th nucleotide. The nucleic acid sequence is shown in SEQ ID NO: 10, and the amino acid sequence is shown in SEQ ID NO: 11.

The primers used for the cloning and gene analysis are shown below.
XR58R (SEQ ID NO: 12): 5'-tcatgcggct cacggacctt tcacagctag-3'
HClongA1 (SEQ ID NO: 13): 5'-atcgtcttca cgcagaaagc gtctagccat-3'
1b9405R (SEQ ID NO: 14): 5'-gcctattggc ctggagtgtt tagctc-3'
HC85F (SEQ ID NO: 15): 5'-atggcgttag tatgagtgtc gtgcagcct-3'
HC9302R (SEQ ID NO: 16): 5'-tcgggcacga gacaggctgt gatatatgtc t-3'
HC8913F (SEQ ID NO: 47): 5'-cttgaaaaag ccctggattg tcagat-3'
HC8939F (SEQ ID NO: 46): 5'-ctacggggcc tgttactcca ttgaac-3'
R1 (SEQ ID NO: 48): 5'-acatgatctg cagagaggcc agtatcagca ctctc-3
HClongH1 (SEQ ID NO: 49): 5'-gccagccccc tgatgggggc gacactccac c-3'
HC705R (SEQ ID NO: 50): 5'-agccgcatgt aagggtatcg atgac-3"
RP2 (SEQ ID NO: 51): 5'-acatgatctg cagagaggcc-3'
M13PrimerM3 (SEQ ID NO: 24): 5'-gtaaaacgac ggccagt-3'
M13PrimerRV (SEQ ID NO: 25): 5'-caggaaacag ctatgac-3'
HC161S (SEQ ID NO: 52): 5'-gagtacaccggaattgccaggacgaccggg-3'
104 (SEQ ID NO: 26): 5'-aggaagactt ccgagcggtc-3'
HC841 S (SEQ ID NO: 27): 5'-ggaacttgcc cggttgctct ttctctatct tc-3'
HC1405S (SEQ ID NO: 53): 5'-attccatggt ggggaactgg gccaa-3'
E1 (SEQ ID NO: 28): 5'-attccatggt ggggaactgg gctaa-3'
HC2006AS (SEQ ID NO: 54): 5'-catccatgtg cagccgaacc aatt-3'
HC2199AS (SEQ ID NO: 55): 5'-aggggtagtg ccaaagcctg tatgggtagt-3'
HC2430S (SEQ ID NO: 30): 5'-aacatcgtgg acgtgcaata cctgtacgg-3'
HC2769S (SEQ ID NO: 32): 5'-ttggaccggg agatggctgc atcgtg-3'
HC3111AS (SEQ ID NO: 56): 5'-ataatgaccc ccggcgactt tccgcactaa c-3'
HC3591AS (SEQ ID NO: 57): 5'-catggtagac agtccagcac-3'
HC4016AS (SEQ ID NO: 35): 5'-cacttggaat gtctgcggta-3'
HC4498S (SEQ ID NO: 36): 5'-agggggggag gcatctcatt ttctg-3'
HC4888F (SEQ ID NO: 37): 5'-tgctatgacg cgggctgtgc ttggta-3'
1b5290AS (SEQ ID NO: 58): 5'-gacatgcatg tcatgatgta tttg-3'
HC5950AS (SEQ ID NO: 59): 5'-ctcatgacct taaaggccac-3' HC5858F (SEQ ID NO: 40): 5'-tggcagcata ggccttggga aggt-3'
HC6315F (SEQ ID NO: 41): 5'-aagacctggc tccagtccaa g-3'
spa-1 (SEQ ID NO: 42): 5'-ttccatgctc accgacccct c-3'
HC7090AS (SEQ ID NO: 43): 5'-accttattct ctgactccac-3' HC7743F (SEQ ID NO: 44): 5'-cagaagaagg tcacctttgac-3'
HC8192S (SEQ ID NO: 45): 5'-gcagcgggtc gagttcctgg tgaat-3'

### «Example 7: Inhibition of Replication of HCV RNA Replicon by Interferon»

Evaluation of an inhibitory action of interferon on the replication of the HCV RNA replicon was attempted using the full-length HCV RNA replicon. The full-length HCV RNA used for the evaluation of the inhibitory action of interferon was pTPF1/4B which was replicable with high efficiency in Huh7 cells in Example 4 and a chimera between AHC1 obtained in Example 6 and pTPF1/4B was also used. As for a chimera vector, the full-length HCV DNA pTPF1 was digested with restriction enzymes, *Age* I and *Bsr* GI and a fragment obtained by digesting AHC 1 with restriction enzymes, Age I and *Bsr* GI were ligated to and inseted into the region of the cleavage of pTPF1, thereby preparing HCV DNA pTPF1/AHC1 AgeBsr in which the structural protein region of AHC1 was inserted.

The full-length HCV RNAs of pTPF1/4B and pTPF1/AHC1AgeBsr were synthesized using the same method as in Example 2 and transfected into Huh7 cells. The transfected cells were immediately resuspended in 15 ml of the culture medium and plated 1 ml each in a 12-well plate (diameter 22.1 mm) to start a culture.

At twenty four hours after the culture was started, the medium was replaced with culture medium dissolving various concentrations (from 0.1 IU/ml to 300 IU/ml) of interferon. After culturing for another 24 hours, the cells were detached by 1 mM EDTA-PBS and collected by centrifugation. Cell pellet was dissolved in 50 µl of RIPA buffer (20 mM Tris-HCl (pH 7.5), 150 mM NaCl, 1 mM EDTA, 1% NP40, 0.1% Deoxycholate, 0.1% SDS, Complete protease inhibitor cocktail (Roche diagnostics corporation) and supernatant was collected by centrifugation at 10k rpm for 5 minutes. The supernatant in an amount of 5 µl was measured using a kit for HCV core antigen (FUJIREBIO, Lumipulse).

The concentration of interferon at which the amount of the core antigen in the cells exhibits 50% of the amount of the core antigen in a control (a group with no interferon being added) was calculated based on a plot, which concentration was set to as IC50. The results are shown in Table 2.

**[Table 2]**

| RNA | Inhibitory activity for replication of HCV RNA replicon : IC50 (IU/m) |
|---|---|
| pTPF1/4B | >300 |
| pTPF1/AHC1_AgeBsr | 33 |

The results of the above-described experiment revealed that pTPF1/4B was resistant to interferon whereas pTPF1/AHC1_AgeBsr was sensitive to interferon. From this, it is thought that the full-length HCV RNA replicon according to the present invention is an interferon-resistant RNA replicon and useful in development of a therapeutic agent against a HCV showing the interferon resistance.

### «Example 8: Inhibition of Replication of HCV RNA Replicon by Cyclosporin A»

Evaluation of inhibitory action by cyclosporin A on the replication of the HCV RNA replicon was attempted. pTPF1/4B was used for the evaluation. Huh7 cells were transfected using the same method as described in Example 2 and plated in a 12-well plate. After culturing for 4 hours, the medium was replaced with a medium dissolving 1 µg of cyclosporin A.

For the cells at culturing for 24 hours, 48 hours, and 72 hours after the replacement of the culture medium containing cyclosporin A, the amount of the core antigen in cells was measured using the same method as described in Example 7, using a kit for HCV core antigen (FUJIREBIO, Lumipulse).

As shown in Figure 3, it was confirmed that the group in which cyclosporin A (CsA) was added reached a maximum at 4 hours after transfection and thereafter decreased. On the other hand, it was confirmed that a control group (a group in which CsA was not added) once decreased from 4 hours to 24 hours and started increasing from 48 hours and remained increasing 72 hours later. Therefore, it was confirmed that CsA had anti-HCV activity. This indicated that the full-length HCV RNA according to the present invention can be a test system (screening method) for a therapeutic agent for HCV, which agent is thus far reported. In addition, it can be used as a test system for screening various agents affecting on the replication of HCV and/or the translation of a HCV protein.

### «Example 9: Effects of Adaptive Mutations on NS4B Protein of TPF1 strain»

Effects of the adaptive mutations of two amino acids in the NS4B protein which was obtained by the RNA replicon using TPF1 strain on replication of the replicon RNA in the other HCV genes of genotype 1b were evaluated. The mutations were introduced in the nucleotides such that the corresponding mutations were introduced in a protein of the NS4B region with 3010 amino acids of the AHC1 strain obtained in Example 6. Specifically, using Quick Mutagenesis Kit (Stratagene) in accordance with the method recommended by the manufacturer, nucleotide mutations were introduced so as to mutate the 1804th amino acid from Q (glutamine) to L (leucine) and the 1966th amino acid from E (glutamic acid) to K (lysine). The nucleic acid sequence of RNA of the obtained clone is shown in SEQ ID NO: 61 and its amino acid sequence is shown in SEQ ID NO: 62.

The same procedures as described in Example 2 were repeated except that a full-length polynucleotide of the AHC1 strain with the obtained adaptive mutations was used, thereby obtaining a plasmid DNA pRepAHC1/4B having the adaptive mutations. RepAHC1/4B replicon RNA was obtained using a fragment obtaining by digesting this pRepAHC1/4B with a restriction enzyme *Xba* I as a template, and transfected into human hepatocarcinoma cells (Huh7, JCRB0403), thereby establishing a cell line in which the RepAHC1/4B replicon RNA autonomously replicates.
When the same procedures were repeated using, as a control, the full-length polynucleotide of the AHC1 strain in which the adaptive mutations were not introduced, although a cell line in which the replicon RNA autonomously replicates was established, the efficiency was about one thousandth, compared with the RepAHC1/4B replicon. Hence, it was proved that the replicon RNA efficiently replicates autonomously by introducing the nucleotide mutations coding for the two adaptive mutations of the NS4B protein into the HCV genotype 1b gene.

Subsequently, the procedures described in Example 3 were repeated except that the obtained cell line was used and the nucleotide sequence of the replicated replicon RNA was determined. As a result, it was found that the 3685th nucleotide C was mutated to T and thus the amino acid corresponding to the 1115th amino acid number in SEQ ID NO: 11 of P (proline) was mutated to L (leucine). This mutation was introduced into the above-described pRepAHC1/4B to obtain a plasmid pRepAHC1/4Bm. RepAHC1/4B which was a replicon RNA obtained from pRepAHC1/4B, and RepAHC1/4Bm which was a replicon RNA obtained from pRepAHC1/4Bm were transfected into Huh7 and the number of colonies was calculated. The nucleic acid sequence of RepAHC1/4B is shown in SEQ ID NO: 67 and the nucleic acid sequence of RepAHC1/4Bm is shown in SEQ ID NO: 68.
When 1µg of RepAHC1/4B RNA was transfected, about 10³ G418-resistant colonies were selected whereas about 10⁶ colonies were selected when 1µg of RepAHC1/4Bm RNA was transfected. Therefore, it was proved that the replication efficiency of the replicon RNA increased by introducing one or more other adaptive mutations in addition to two of the above-described adaptive mutations in the NS4B protein.

Next, the same procedures as described in Example 4 were repeated except that, as the full-length HCV DNA, pAHC1 was used instead of pTPF1 and pRepAHC1/4Bm was used instead of pRep4B, thereby preparing pAHC1 and pAHC1/4Bm which were the full-length HCV DNA. AHC1 which was a replicon RNA prepared from pAHC1 and AHC1/4Bm which was a replicon RNA prepared pAHC1/4Bm were transfected into Huh7 cells and then the amount of the core antigen in the culture supernatant was measured. The results are shown in Figure 4.
As shown in Figure 4, the measured value of the core antigen in the culture supernatant 24 hours, 48 hours, and 72 hours after transfection with the replicon RNA of AHC1/4Bm having the adaptive mutations was higher than the measured value of the core antigen in the culture supernatant from cells transfected with replicon RNA of AHC1 as a control. The nucleic acid sequence of AHC1/4Bm which is a replicon RNA is shown in SEQ ID NO: 63, and the encoded amino acid sequence is shown in SEQ ID NO: 64.

### INDUSTRIAL APPLICATIBILITY

The replicon RNA according to the present invention can be introduced into cells, can autonomously replicate and generate a HCV gene, HCV protein, and infectious particle. The replicon-replicating cells into which this replicon RNA is introduced reflect *in vivo* proliferation mechanisms of HCV as an *in vitro* model of the HCV infection. These replicon-replicating cells can be used in a method for screening a therapeutic agent of HCV. Further, the above-described method for screening can be used, besides the screening of the therapeutic agent for HCV, for quality control in the process of the production of the therapeutic agent and thus used as a method for producing a pharmaceutical.
As described above, the present invention has been illustrated along the certain modes, yet variations and modifications obvious to those skilled in the art are within the scope of the present invention.

## Claims

1. A hepatitis C virus (HCV) gene comprising a polynucleotide selected from the group consisting of:
(A) a polynucleotide having the nucleic acid sequence shown in SEQ ID NO: 5;
(B) a polynucleotide having the nucleic acid sequence shown in SEQ ID NO: 7;
(C) a polynucleotide having the nucleic acid sequence shown in SEQ ID NO: 65;
(D) a polynucleotide coding for a polypeptide having the amino acid sequence shown in SEQ ID NO: 6;
(E) a polynucleotide coding for a polypeptide having the amino acid sequence shown in SEQ ID NO: 8; and
(F) a polynucleotide coding for a polypeptide having the amino acid sequence shown in SEQ ID NO: 66.

2. A HCV gene according to claim 1, said gene being a polynucleotide having the nucleic acid sequence shown in SEQ ID NO: 1, SEQ ID NO: 3, SEQ ID NO: 10, SEQ ID NO: 61 or SEQ ID NO: 63.

3. A HCV genotype 1b gene, said gene comprising nucleotides coding for the 1804th amino acid leucine and the 1966th amino acid lysine in the amino acid sequence of HCV polyprotein and a polynucleotide coding for an NS4B protein.

4. A DNA comprising a single stranded DNA having the nucleic acid sequence with uridine being substituted by thymine in the nucleic acid sequence of said HCV gene according to any one of claims 1 to 3.

5. A polypeptide having the amino acid sequence shown in SEQ ID NO: 6, SEQ ID NO: 8 or SEQ ID NO: 66.

6. A HCV polyprotein, wherein a peptide in an NS4B region is a polypeptide having the amino acid sequence shown in SEQ ID NO: 6, SEQ ID NO: 8 or SEQ ID NO: 66.

7. At least one HCV protein selected from the group consisting of a core protein having the amino acid sequence from the first to the 191 st amino acid in the amino acid sequence shown in SEQ ID NO: 2, SEQ ID NO: 4, SEQ ID NO: 11, SEQ ID NO: 62 or SEQ ID NO: 64, an E1 protein having the amino acid sequence from the 192nd to the 383rd amino acid, an E2 protein having the amino acid sequence from the 384th to the 746th amino acid, a P7 protein having the amino acid sequence from the 747th to the 809th amino acid, an NS2 protein having the amino acid sequence from the 810th to the1026th amino acid, an NS3 protein having the amino acid sequence from the 1027th to 1657th amino acid, an NS4A protein having the amino acid sequence from the 1658th to 1711th amino acid, an NS4B protein having the amino acid sequence from the 1712th to the 1972nd amino acid, an NS5A protein having the amino acid sequence from the 1973rd to the 2419th amino acid, and an NS5B protein having the amino acid sequence from the 2420th to the 3010th amino acid.

8. A replicon RNA comprising a polynucleotide selected from the group consisting of:
(A) a polynucleotide having the nucleic acid sequence shown in SEQ ID NO: 5;
(B) a polynucleotide having the nucleic acid sequence shown in SEQ ID NO: 7;
(C) a polynucleotide having the nucleic acid sequence shown in SEQ ID NO: 65;
(D) a polynucleotide coding for a polypeptide having the amino acid sequence shown in SEQ ID NO: 6;
(E) a polynucleotide coding for a polypeptide having the amino acid sequence shown in SEQ ID NO: 8;
(F) a polynucleotide coding for a polypeptide having the amino acid sequence shown in SEQ ID NO: 66; and
(G) a polynucleotide having a nucleic acid sequence having a homology of not less than 90% with the nucleic acid sequence shown in SEQ ID NO: 5, SEQ ID NO: 7 or SEQ ID NO: 65.

9. A replicon RNA of the genotype 1b, said replicon RNA comprising nucleotides coding for the 1804th amino acid leucine and the 1966th amino acid lysine in the amino acid sequence of a HCV polyprotein and a polynucleotide coding for an NS4B protein.

10. The replicon RNA according to claims 8 or 9, said replicon RNA comprising:
(A) a polynucleotide from the first to the 341 st nucleotide in the 5' untranslated region of said HCV, a polynucleotide coding for a polypeptide from the 1027th to the 3010th amino acid in said HCV polyprotein and a polynucleotide of the 3' untranslated region; or
(B) a polynucleotide from the first to the 341 st nucleotide in the 5' untranslated region, a polynucleotide coding for the HCV polyprotein composed of 3010 amino acids and a polynucleotide of the 3' untranslated region.

11. The replicon RNA according to any one of claims 8 to 10, said replicon RNA being resistant to interferon.

12. The replicon RNA according to any one of claims 8 to 11, said replicon RNA comprising:
(A) a polynucleotide having the first to the 341 st nucleotide in the nucleic acid sequence shown in SEQ ID NO: 1 and a polynucleotide having the 3420th to the 9594th nucleotide in the nucleic acid sequence shown in SEQ ID NO: 1;
(B) a polynucleotide having the first to the 341 st nucleotide in the nucleic acid sequence shown in SEQ ID NO: 3 and a polynucleotide having the 3420th to the 9594th nucleotide in the nucleic acid sequence shown in SEQ ID NO: 3;
(C) a polynucleotide having the first to the 341 st nucleotide in the nucleic acid sequence shown in SEQ ID NO: 10 and a polynucleotide having the 3420th to the 9594th nucleotide in the nucleic acid sequence shown in SEQ ID NO: 10;
(D) a polynucleotide having the first to the 341 st nucleotide in the nucleic acid sequence shown in SEQ ID NO: 61 and a polynucleotide having the 3420th to the 9594th nucleotide in the nucleic acid sequence shown in SEQ ID NO: 61;
(E) a polynucleotide having the first to the 341 st nucleotide in the nucleic acid sequence shown in SEQ ID NO: 63 and a polynucleotide having the 3420th to the 9594th nucleotide in the nucleic acid sequence shown in SEQ ID NO: 63;
(F) a polynucleotide having a nucleic acid sequence having a homology of not less than 90% with the nucleic acid sequence from the first to the 341 st nucleotide in the nucleic acid sequence shown in SEQ ID NO: 1 and a polynucleotide having a nucleic acid having a homology of not less than 90% with the nucleic acid sequence from the 3420th to the 9594th nucleotide in the nucleic acid sequence shown in SEQ ID NO: 1;
(G) a polynucleotide having a nucleic acid sequence having a homology of not less than 90% with the nucleic acid sequence from the first to the 341 st nucleotide in the nucleic acid sequence shown in SEQ ID NO: 3 and the polynucleotide having a nucleic acid sequence having a homology of not less than 90% with the nucleic acid sequence from the 3420th to the 9594th nucleotide in the nucleic acid sequence shown in SEQ ID NO: 3;
(H) a polynucleotide having a nucleic acid sequence having a homology of not less than 90% with the nucleic acid sequence from the first to the 341 st nucleotide in the nucleic acid sequence shown in SEQ ID NO: 10 and a polynucleotide having a nucleic acid sequence having a homology of not less than 90% with the nucleic acid sequence from the 3420th to the 9594th nucleotide in the nucleic acid sequence shown in SEQ ID NO: 10;
(I) a polynucleotide having a nucleic acid sequence having a homology of not less than 90% with the nucleic acid sequence from the first to the 341 st nucleotide in the nucleic acid sequence shown in SEQ ID NO: 61 and a polynucleotide having a nucleic acid sequence having a homology of not less than 90% with the nucleic acid sequence from the 3420th to the 9594th nucleotide in the nucleic acid sequence shown in SEQ ID NO: 61; or
(J) a polynucleotide having a nucleic acid sequence having a homology of not less than 90% with the nucleic acid sequence from the first to the 341 st nucleotide in the nucleic acid sequence shown in SEQ ID NO: 63 and a polynucleotide having a nucleic acid sequence having a homology of not less than 90% with the nucleic acid sequence from the 3420th to the 9594th nucleotide in the nucleic acid sequence shown in SEQ ID NO: 63.

13. The replicon RNA according to any one of claims 8 to 12, said replicon RNA being a polynucleotide having the nucleic acid sequence shown in SEQ ID NO: 1, SEQ ID NO: 3, SEQ ID NO: 10, SEQ ID NO: 61 or SEQ ID NO: 63; or a polynucleotide having a nucleic acid sequence having a homology of not less than 90% with the nucleic acid sequence shown in SEQ ID NO: 1, SEQ ID NO: 3, SEQ ID NO: 10, SEQ ID NO: 61 or SEQ ID NO: 63.

14. The replicon RNA according to any one of claims 8 to 13, said replicon RNA comprising at least one selection marker gene or reporter gene and at least one IRES sequence.

15. A DNA coding for said replicon RNA according to any one of claims 8 to 14.

16. A vector comprising said DNA according to claim 15.

17. A replicon-replicating cell prepared by introducing at least one selected from the group consisting of said replicon RNA according to any one of claims 8 to 14, said DNA according to claim 15, and said vector according to claim 16 into a cell.

18. The replicon-replicating cell according to claim 17, wherein said cell is a cell derived from a hepatocyte.

19. The replicon-replicating cell according to claim 18, wherein said cell derived from said hepatocyte is an Huh-7 cell.

20. A replicon RNA produced by said replicon-replicating cell according to any one of claims 17 to 19.

21. At least one HCV protein selected from the group consisting of CORE, E1, E2, P7, NS2, NS3, NS4A, NS4B, NS5A and NS5B produced by said replicon-replicating cell according to any one of claims 17 to 19.

22. A HCV particle produced by said replicon-replicating cell according to any one of claims 17 to 19.

23. A method for screening a substance controlling infection of said HCV, said method comprising the steps of contacting said replicon-replicating cell according to any one of claims 17 to 19 with said substance and analyzing a degree of increase in said replicon RNA.

24. The method for screening according to claim 23, wherein said analysis of said degree of increase in said replicon RNA is detection of said replicon RNA or HCV protein.
